# EUROPEAN PATENT APPLICATION

(11) **EP 2 168 959 A1**
(43) Date of publication of application: **31.03.2010**
(21) Application number: 08765704.5
(22) Date of filing: 18.06.2008
(51) Int. Cl.: C07D 401/14, A61K 31/501, A61P 1/00, A61P 9/04, A61P 9/10, A61P 9/12, A61P 11/00, A61P 11/06, A61P 11/16, A61P 17/00, A61P 17/04, A61P 19/02, A61P 25/00, A61P 25/16, A61P 25/24, A61P 25/28, A61P 27/16, A61P 29/00, A61P 37/08, A61P 43/00, C07D 403/14

(54) **PYRIDAZINONE DERIVATIVE AND PDE INHIBITOR CONTAINING THE SAME AS ACTIVE INGREDIENT**

(30) Priority: 19.06.2007 JP 2007160955
(71) Applicant: Kyorin Pharmaceutical Co., Ltd., Tokyo 101-8311 (JP)
(72) Inventor: KOHNO, Yasushi, Shimotsuga-gun Tochigi 329-0114 (JP); OCHIAI, Koji, Shimotsuga-gun Tochigi 329-0114 (JP); TAKITA, Satoshi, Shimotsuga-gun Tochigi 329-0114 (JP); KOJIMA, Akihiko, Shimotsuga-gun Tochigi 329-0114 (JP); EIRAKU, Tomohiko, Shimotsuga-gun Tochigi 329-0114 (JP); KISHI, Tetsuya, Shimotsuga-gun Tochigi 329-0114 (JP)
(74) Representative: Vossius & Partner
(86) International application number: PCT/JP2008/061087
(87) International publication number: WO 2008/156094

(57) **Abstract**

It is to provide a novel pyridazinone derivative represented by the following general formula (1), which is useful as a pharmaceutical and has a phosphodiesterase inhibitory action: 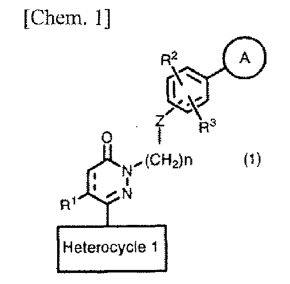 wherein R¹ represents H or C₁₋₆ alkyl, each of R² and R³ represents H, X, C₁₋₆ alkoxy, Z represents O or S, and A represents AA or BB, wherein AA represents: 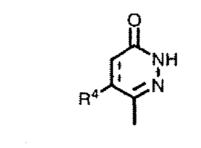 and BB represents: 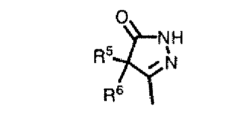 wherein R⁴ represents H or C₁₋₆ alkyl, and each of R⁵ and R⁶ represents C₁₋₆ alkyl.

## Description

### Technical Field

The present invention relates to a pyridazinone derivative, a salt thereof, or a hydrate thereof, which is useful as a phosphodiesterase (PDE) inhibitor.

### Background Art

Phosphodiesterases (PDEs) are enzymes that breakdown cyclic AMP (cAMP) and cyclic GMP (cGMP) which are second messengers in the living body. To date, types 1 to 11 of PDEs have been identified and each type specifically breaks down either cAMP or cGMP, or both. There are differences in the tissue distribution of each type of PDE. It is thought that cellular reactions are controlled by various types of PDEs according to the type of organ.

Up to the present, a large number of PDE inhibitors have been developed. For example, PDE3 inhibitors are anticipated as agents for treating angina pectoris, cardiac failure, hypertension, or the like, or as platelet aggregation inhibitors or antiasthmatic agents; and PDE4 inhibitors are anticipated as agents for treating bronchial asthma, chronic obstructive pulmonary disease (COPD), interstitial pneumonia, allergic rhinitis, atopic dermatitis, rheumatoid arthritis, multiple sclerosis, Crohn's disease, inflammatory colitis, Alzheimer's disease, dementia, Parkinson's disease, depression, or the like. PDE5 inhibitors are already in clinical use as an agent for treating male erectile dysfunction. Moreover, it has been recently reported that the use of minocycline as a PDE10A modulator is effective for patients with Huntington's disease (Patent Document 1), and a patent laid-open publication has been disclosed, which describes PDE10 inhibitors as effective as agents for treating various psychiatric disorders such as Huntington's disease, Alzheimer's disease, dementia, Parkinson's disease, schizophrenia, and the like (Patent Document 2). In addition, recently, the pamphlet of International Publication (Patent Document 3) has also been disclosed which describes that the inhibitors are also effective for obesity and metabolic syndrome has been disclosed.

Some compounds containing pyridazinone rings as the PDE inhibitors have been reported (Patent Documents 4 to 14 and Non-Patent Documents 1 to 9). Also, meribenden, pimobendan, UD-CG-212 (CAS-108381-22-2), and the like have been also reported. In addition, a compound having an alkyl group at a position 2 of a phthalazinone ring and thus having a PDE inhibitory action has been disclosed (Patent Documents 15 and 16). However, there is not known a compound which has the characteristics of the present invention, which is a compound with a pyridazinone ring or pyrazolone ring via an alkyl group at a position 2 of the pyridazinone ring, wherein various hetero ring compounds are linked to the pyridazinone rings.
[Patent Document 1] Pamphlet of WO 01024781
[Patent Document 2] JP-A-2002-363103
[Patent Document 3] Pamphlet of WO 2005120514
[Patent Document 4] JP-A-2-193994
[Patent Document 5] DE 3511110
[Patent Document 6] DE 3006671
[Patent Document 7] Pamphlet of WO 04078751
[Patent Document 8] Pamphlet of WO 04058729
[Patent Document 9] JP-A-58-183687
[Patent Document 10] Pamphlet of WO 03097062
[Patent Document 11] Republished WO98/1444
[Patent Document 12] JP-A-10-109988
[Patent Document 13] JP-A-200b-117647
[Patent Document 14] Pamphlet of WO 2006095666
[Patent Document 15] Pamphlet of WO 2001019818
[Patent Document 16] Pamphlet of WO 9947505
[Non-Patent Document 1] Joshua O Odingo, Expert Opin. Ther. Patents, 15 773 (2005)
[Non-Patent Document 2] Peter Norman, Expert Opin. Ther. Patents, 12 93 (2002)
[Non-Patent Document 3] Miles D Houslay et al., Drug Discovery Today, 10 1503 (2005)
[Non-Patent Document 4] Pasola Fossa et al., Quant. Struct. -Act. Relat., 21 267 (2002)
[Nan-Patent Document 5] Nomoto Yuji et al., Chem, Pharm. bull., 39 352 (1991)
[Non-Patent Document 6] Abou-Zeid K. A. M. et al., Egyptian J. Pharm. Sci., 38 303 (1997)
[Non-Patent Document 7] Hishmat, Orchidee H. et al., Pharmazie 40 460 (1985)
[Non-Patent Document 8] Colletti Steven L. et al., J. Med.chem., 46 349 (2003)
[Non-Patent Document 9] Jonas Ret al., Eur. J. Med. chem., 28 141 (1993)

### Disclosure of the Invention

### Problem that the Invention is to Solve

The present invention aims to provide a pyridazinone derivative having an excellent phosphodiesterase inhibitory action with few side effects.

### Means for Solving the Problem

The present inventors have conducted extensive studies on a highly safe compound having phosphodiesterase inhibitory activity, and as a result, they have found that a novel pyridazinone derivative structurally different from any of the existing PDE inhibitors has a PDE inhibitory action. Thus, the present invention has been completed.

Namely, the present invention relates to
1) a pyridazinone derivative, optically active compound thereof, pharmaceutically acceptable salt thereof, or hydrate thereof, wherein the pyridazinone derivative is represented by the following general formula (1):

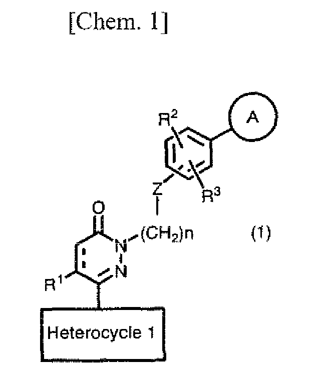

[wherein R¹ represents a hydrogen atom or an alkyl group having 1 to 6 carbon atoms,
R² and R³ are the same as or different from each other and represent a hydrogen atom, a halogen atom, or an alkoxy group having 1 to 6 carbon atoms,
Z represents an oxygen atom or a sulfur atom,
A represents a substituent represented by the general formula:

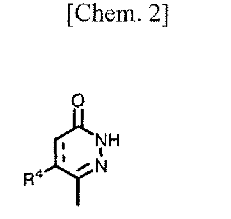

(wherein R⁴ represents a hydrogen atom or an alkyl group having 1 to 6 carbon atoms, and

[Chem. 3] - - -

represents a single bond or a double bond) or a substituent represented by the general formula:

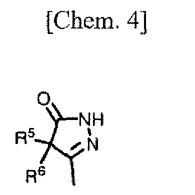

(wherein R⁵ and R⁶ are the same as or different from each other and represent an alkyl group having 1 to 6 carbon atoms),
Heterocycle 1 represents a heterocyclic compound represented by the following general formula (2):

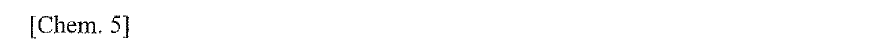

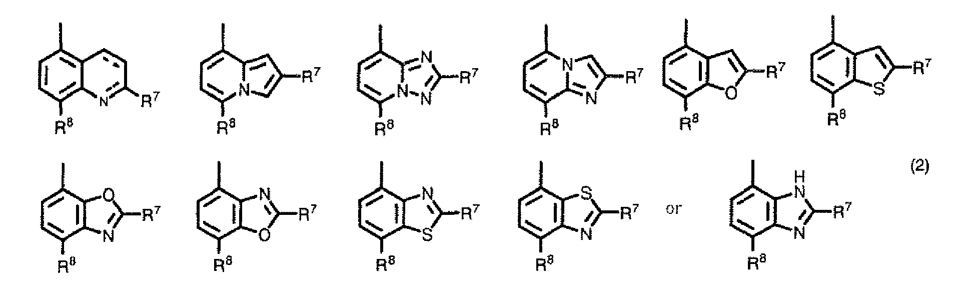

(wherein R⁷ represents a hydrogen atom or an alkyl group having 1 to 6 carbon atoms which may be substituted with halogen atom(s),
R⁸ represents a hydrogen atom or an alkoxy group having 1 to 6 carbon atoms,

[Chem. 6] - - -

represents a single bond or a double bond),
n represents an integer of I to 5, and

[Chem. 7] - - -

represents a single bond or a double bond];

2) the pyridazinone derivative, optically active compound thereof, pharmaceutically acceptable salt thereof, or hydrate thereof according to 1), wherein the compound represented by the general formula (1) is represented by the general formula (1a):

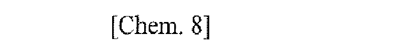

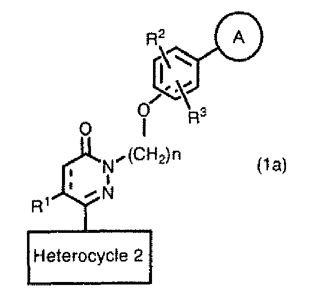

[wherein Heterocycle 2 represents the following general formula (2a):

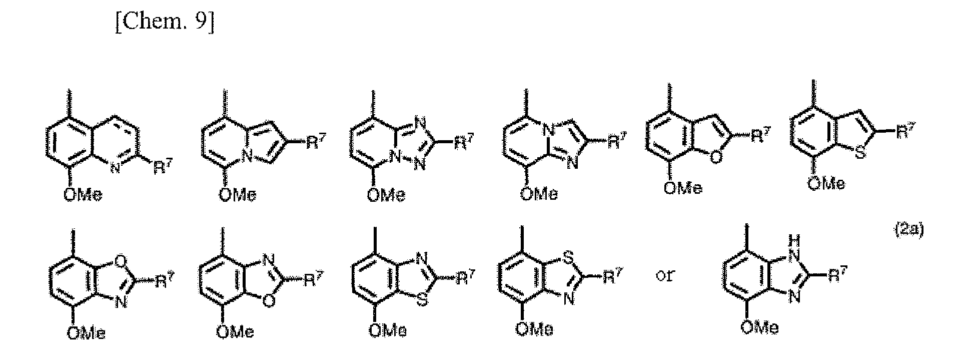

(wherein R⁷ and

[Chem. 10] - - -

are as defined above), and R¹, R², R³, A, n and

[Chem. 11] - - -

are as defined above];

3) the pyridazinone derivative, optically active compound thereof, pharmaceutically acceptable salt thereof, or hydrate thereof according to 1), wherein the compound represented by the general formula (1) is
6-(2-ethyl-8-methoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one,
6-(2-ethyl-8-methoxyquinolin-5-yl)-2-[4-[2,3-difluoro-4-(6-oxo-1,4,5,6-tetrahydropyridazin-3-yl)phenoxy]butyl]-2H-pyridazin-3-one,
6-(2-ethyl-8-methoxyquinolin-5-yl)-2-[4-[4-(6-oxo-1,4,5,6-tetrahydropyridazin-3-yl)phenoxy]butyl]-2H-pyridazin-3-one,
2-[4-[2-fluoro-4-(6-oxo-1,4,5,6-tetrahydropyridazin-3-yl)phenoxy]butyl]-6-(8-methoxy-2-methylquinolin-5-yl)-2H-pyridazin-3-one,
6-(2-ethyl-8-methoxyquinolin-5-yl)-5-methyl-2-[4-[2,3-difluoro-4-(6-oxo-1,4,5,6-tetrahydropyridazin-3-yl)phenoxy]butyl]-4,5-dihydro-2H-pyridazin-3-one,
6-(2-ethyl-8-methoxyquinolin-5-yl)-2-[4-[2,3-difluoro-4-(4-methyl-6-oxo-1,4,5,6-tetrahydropyridazin-3-yl)phenoxy]butyl]-2H-pyridazin-3-one,
6-(4-methoxy-2-trifluoromethyl-1H-benzimidazol-7-yl)-5-methyl-2-[4-[4-(4-methyl-6-oxo-1,4,5,6-tetrahydropyridazin-3-yl)phenoxy]butyl]-4,5-dihydro-2H-pyridazin-3-one,
2-[4-[2,3-difluoro-4-(6-oxo-1,4,5,6-tetrahydropyridazin-3-yl)phenoxy]butyl]-6-(8-methoxy-2-methylquinolin-5-yl)-2H-pyridazin-3-one,
2-[4-[2,3-difluoro-4-(4-methyl-6-oxo-1,4,5,6-tetrahydropyridazin-3-yl)phenoxy]butyl]-6-(8-methoxyquinolin-5-yl)-2H-pyridazin-3-one, or
2-[4-[2,3-difluoro-4-(6-oxo-1,4,5,6-tetrahydropyridazin-3-yl)phenoxy]butyl]-6-(8-methoxyquinolin-5-yl)-2H-pyridazin-3-one;

4) a phosphodiesterase (PDE) inhibitor comprising, as an active ingredient, the pyridazinone derivative, optically active compound thereof, pharmaceutically acceptable salt thereof, or hydrate thereof according to any one of 1) to 3);

5) a pharmaceutical agent comprising, as an active ingredient, the pyridazinone derivative, optically active compound thereof, pharmaceutically acceptable salt thereof, or hydrate thereof according to any one of 1)to 3); and

6) the pharmaceutical agent according to 5), which is an agent for preventing or treating angina pectoris, cardiac failure, hypertension, bronchial asthma, chronic obstructive pulmonary disease (COPD), interstitial pneumonitis, allergic rhinitis, atopic dermatitis, rheumatoid arthritis, multiple sclerosis, Crohn's disease, inflammatory bowel disease, Alzheimer, dementia, Parkinson's disease, or depression.

### Advantage of the Invention

According to the present invention, it has been found that a novel pyridazinone derivative and an addition salt thereof have an excellent PDE inhibitory action. Such a compound having a PDE inhibitory action is useful as an agent for treating angina pectoris, cardiac failure, hypertension, or the like, as a platelet aggregation inhibitor, as an agent for preventing or treating bronchial asthma, chronic obstructive pulmonary disease (COPD), interstitial pneumonitis, allergic rhinitis, atopic dermatitis, rheumatoid arthritis, multiple sclerosis, Crohn's disease, or inflammatory bowel disease, as an agent for preventing or treating various psychiatric disorders such as Huntington's disease, Alzheimer's disease, dementia, Parkinson's disease, depression, schizophrenia, and the like, as an agent for preventing or treating obesity, metabolic syndrome, and the like, and as an agent for treating male erectile dysfunction.

### Best Mode for Carrying out the Inveniton

In the present invention, the alkyl group having 1 to 6 carbon atoms means a linear chained or branched alkyl group having 1 to 6 carbon atoms, and preferably an alkyl group having 1 to 4 carbon atoms. Examples thereof include a methyl group, an ethyl group, a propyl group, an isopropyl group, a butyl group, an isobutyl group, a sec-butyl group, a t-butyl group, and the like.

The alkyl group having 1 to 6 carbon atoms which may be substituted with halogen atom(s) is preferably a linear chained or branched perfluoroalkyl group having 1 to 6 carbon atoms, in which all of the hydrogen atoms are substituted with fluorine atoms, more preferably a linear chained or branched perfluoroalkyl group having 1 to 4 carbon atoms, in which all of the hydrogen atoms are substituted with fluorine atoms, and particularly preferably a trifluoromethyl group.

The alkoxy group having I to 6 carbon atoms means a linear chained or branched alkoxy group having 1 to 6 carbon atoms, and it is preferably an alkoxy group having 1 to 4 carbon atoms. Examples thereof include a methoxy group, an ethoxy group, a propoxy group, an isopropoxy group, a butoxy group, an isobutoxy group, a sec-butoxy group, a t-butoxy group, and the like.

Examples of the halogen atom include a fluorine atom, a chlorine atom, a bromine atom, and an iodine atom.

Examples of the pharmaceutically acceptable salt in the present invention include acid addition salts such as hydrochloride, hydrobromide, acetate, trifluoroacetate, methanesulfonate, citrate, and tartrate.

According to the present invention, the compound represented by the general formula (1) can be prepared via, for example, Synthesis Pathway A as shown below.

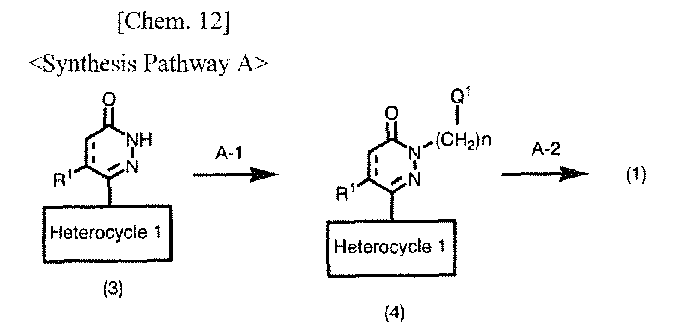

In Synthesis Pathway A, a compound represented by the general formula (4) can be prepared by allowing a compound represented by the general formula (3) to act on a compound represented by the general formula (5) in the presence of a base (Step A-1):

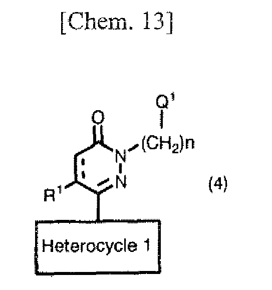

[wherein Q¹ represents a chlorine atom, a bromine atom, an iodine atom, a methanesulfonyloxy group, a trifluoromethanesulfonyloxy group, a benzenesulfonyloxy group, a paratoluenesulfonyloxy group, a t-butyldimethylsilyloxy group, a t-butyldiphenylsilyloxy group, a triisopropylsilyloxy group, a tetrahydropyranyloxy group, a methoxymethyloxy group, or a hydroxyl group, and R¹, Heterocycle 1, n and

[Chem. 14] - - -

are as defined above]

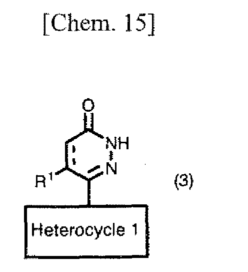

[wherein R¹, Heterocycle 1, and

[Chem. 16] - - -

are as defined above]

[Chem. 17] Q²-(CH₂)n-Q¹ (5)

[wherein Q² represents a chlorine atom, a bromine atom, an iodine atom, a methanesulfonyloxy group, a trifluoromethanesulfonyloxy group, a benzenesulfonyloxy group, or paratoluenesulfonyloxy group, and n and Q¹ are as defined above].

The reaction can be carried out at 0°C to 100°C using n-butyl lithium, sodium hydride, lithium alkoxide, sodium alkoxide, potassium alkoxide, or the like as a base and using tetrahydrofuran (THF), N,N-dimethyl formamide (DMF), or the like as a reaction solvent.

In Synthesis Pathway A, the compound represented by the general formula (1) can be prepared by reacting the compound represented by the general formula (4) with a compound represented by the general formula (6) (Step A-2):

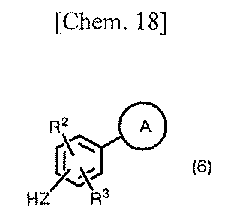

[wherein R², R³, A, and Z are as defined above].

In the case where Q¹ of the compound represented by the general formula (4) is a chlorine atom, a bromine atom, an iodine atom, a methanesulfonyloxy group, a trifluoromethanesulfonyloxy group, a benzenesulfonyloxy group, or a paratoluenesulfonyloxy group, the reaction can be carried out at 0°C to 100°C using n-butyl lithium, sodium hydride, lithium alkoxide, sodium alkoxide, potassium alkoxide, lithium carbonate, sodium carbonate, potassium carbonate, cesium carbonate, or the like as a base and using THF, DMF, or the like as a reaction solvent.

Further, in the case where Q¹ of the compound represented by the general formula (4) is a t-butyldimethylsilyloxy group, a t-butyldiphenylsilyloxy group, or a triisopropylsilyloxy group, it is necessary to allow tetrabutyl ammonium fluoride, hydrofluoric acid, hydrofluoride pyridinium, or the like to act thereon at 0°C to room temperature using THF or the like as a solvent, thereby first transforming a hydroxyl group thereinto. In the case where Q¹ is a tetrahydropyranyloxy group or a methoxymethyloxy group, it is necessary to allow an acid such as, for example, concentrated hydrochloric acid, hydrobromic acid, and the like to act thereon at 0°C to 100°C in a solvent such as acetic acid, thereby transforming a hydroxyl group thereinto. In the case where a chlorine atom, a bromine atom, or an iodine atom is transformed from the obtained hydroxide form, this step can be carried out by allowing a chlorinating agent such as chlorine, carbon tetrachloride, N-chlorosuccinimide (NCS), and the like, a brominating agent such as bromine, carbon tetrabromide, N-bromosuccinimide (NBS), and the like, or an iodinating agent such as iodine, N-iodosuccinimide (NIS), and the like to act thereon at 0°C to room temperature in a solvent such as toluene, methylene chloride, THF, and the like, in the presence of tributylphosphine, triphenylphosphine, triphenoxyphosphine, or the like. Further, in the case where a methanesulfonyloxy group, a trifluoromethanesulfonyloxy group, a benzenesulfonyloxy group, or a paratoluenesulfonyloxy group is transformed from the obtained hydroxide form, this step can be carried out at 0°C to room temperature in a solvent such as methylene chloride, THF, and the like, in the presence of a base such as diisopropyl ethylamine, triethylamine, pyridine, and the like, using the corresponding sulfonyl chloride or sulfonyl anhydride. A compound which transformed by the above can be reacted with the compound represented by the general formula (6) at 0°C to 100°C, using THF, DMF, or the like as a solvent, in the presence of a base such as n-butyl lithium, sodium hydride, lithium alkoxide, sodium alkoxide, potassium alkoxide, lithium carbonate, sodium carbonate, potassium carbonate, cesium carbonate, and the like.

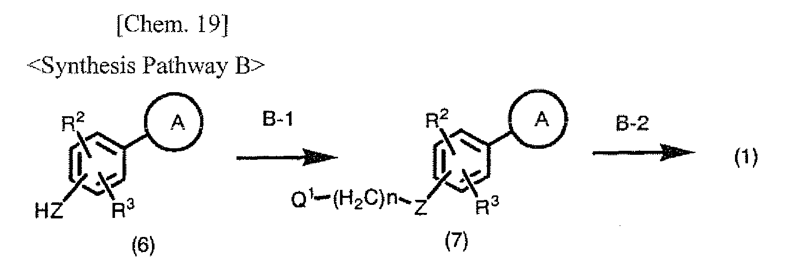

In Synthesis Pathway B, a compound represented by the general formula (7) can be prepared by allowing the compound represented by the general formula (6) to act on the compound represented by the general formula (5) in the presence of a base (Step B-1):

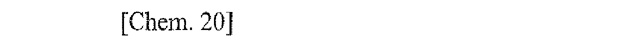

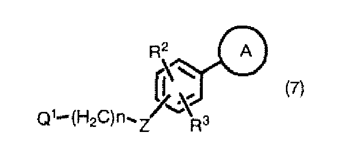

[wherein R², R³, A, Z, Q¹, and n are as defined above].

The reaction can be carried out in the same manner as in Step A-1.

In Synthesis Pathway B, the compound represented by the general formula (1) can be prepared by reacting the compound represented by the general formula (7) with the compound represented by the general formula (3) (Step B-2).

In the case where Q¹ of the compound represented by the general formula (7) is a chlorine atom, a bromine atom, an iodine atom, a methanesulfonyloxy group, a trifluoromethanesulfonyloxy group, a benzenesulfonyloxy group, or a paratoluenesulfonyloxy group, the reaction can be carried out at 0°C to 100°C using n-butyl lithium, sodium hydride, lithium alkoxide, sodium alkoxide, potassium alkoxide, lithium carbonate, sodium carbonate, potassium carbonate, cesium carbonate, or the like as a base and using THF, DMF, or the like as a reaction solvent.

Further, in the case where Q¹ of the compound represented by the general formula (7) is a t-butyldimethylsilyloxy group, a t-butyldiphenylsilyloxy group, or a triisopropylsilyloxy group, it is necessary to allow tetrabutyl ammonium fluoride, hydrofluoric acid, hydrofluoride pyridinium, or the like to act thereon at 0°C to room temperature using THF or the like as a solvent; thereby first transforming a hydroxyl group thereinto. In the case where Q¹ is a tetrahydropyranyloxy group or a methoxymethyloxy group, it is necessary to allow acids such as, for example, concentrated hydrochloric acid, hydrobromic acid, and the like to act thereon at 0°C to 100°C in a solvent such as acetic acid, thereby transforming a hydroxyl group thereinto. In the case where a chlorine atom, a bromine atom, or an iodine atom is transformed from the obtained hydroxide form, this step can be carried out by allowing a chlorinating agent such as chlorine, carbon tetrachloride, NCS, and the like, a brominating agent such as bromine, carbon tetrabromide, NBS, and the like, or an iodinating agent such as iodine, NIS, and the like to act thereon at 0°C to room temperature in a solvent such as toluene, methylene chloride, THF, and the like, in the presence of tributylphosphine, triphenylphosphine, triphenoxyphosphine, or the like. Further, in the case where a methanesulfonyloxy group, a trifluoromethanesulfonyloxy group, a benzenesulfonyloxy group, or a paratoluenesulfonyloxy group is transformed from the obtained hydroxide form, this step can be carried out at 0°C to room temperature in a solvent such as methylene chloride, THF, and the like in the presence of a base such as diisopropyl ethylamine, triethylamine, pyridine, and the like, using the corresponding sulfonyl chloride or sulfonyl anhydride. A compound which transformed by the above can be reacted with the compound represented by the general formula (3) at 0°C to 100°C, using THF, DMF, or the like as a solvent, in the presence of a base such as n-butyl lithium, sodium hydride, lithium alkoxide, sodium alkoxide, potassium alkoxide, lithium carbonate, sodium carbonate, potassium carbonate, cesium carbonate, and the like.

The compound represented by the general formula (1), wherein A is

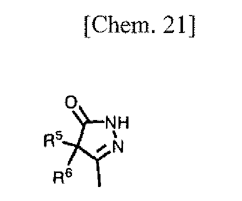

[wherein R⁵ and R⁶ are as defined above], and

[Chem. 22] - - -

is a single bond, that is, a compound represented by the general formula (1b):

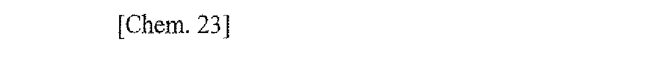

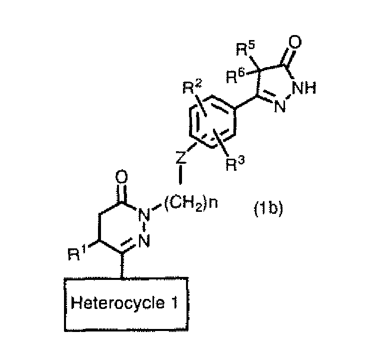

[R¹, R², R³, R⁵, R⁶, Z, Heterocycle 1, and n are as defined above], and the compound in which A is

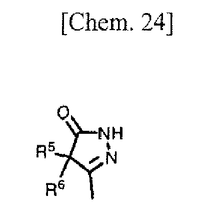

[wherein R⁵ and R⁶ are as defined above], and

[Chem. 25] - - -

is a double bond, that is, a compound represented by the general formula (1c):

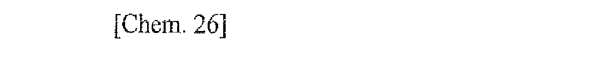

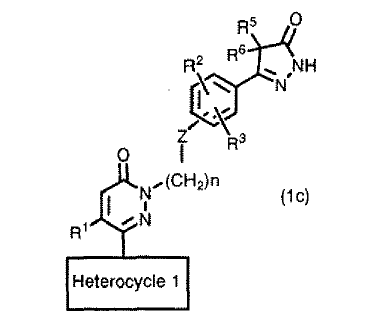

[R¹, R², R³, R⁵, R⁶, Z, Heterocycle 1, and n are as defined above] can be converted to each other as shown in Synthesis Pathways below:

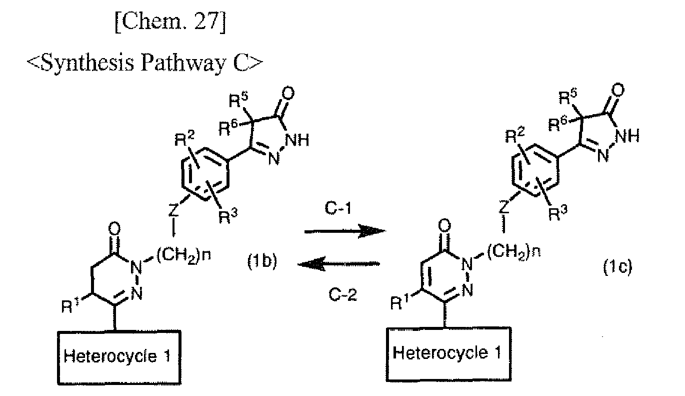

In Synthesis Pathway C, the compound represented by the general formula (1b) can be prepared by reducing the compound represented by the general formula (1c) (Step C-2).

The reaction can be carried out at 80°C to 90°C with the addition of zinc in acetic acid.

In Synthesis Pathway C, the compound represented by the general formula (1c) can be prepared by oxidizing the compound represented by the general formula (1b) (Step C-1).

The reaction can be carried out at 50°C to 60°C with the addition of bromine in acetic acid, or the reaction can be carried out at room temperature to under heating to reflux, using copper (II) chloride in acetonitrile. Further, the reaction can also be carried out by allowing m-nitrobenzene sodium sulfonate to act thereon at room temperature to heating temperature under reflux in an aqueous sodium hydroxide solution.

The compound represented by the general formula (1), wherein A is

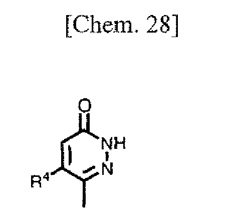

[wherein R⁴ is as defined above], and

[Chem. 29] - - -

is a double bond, that is, a compound represented by the general formula (1d):

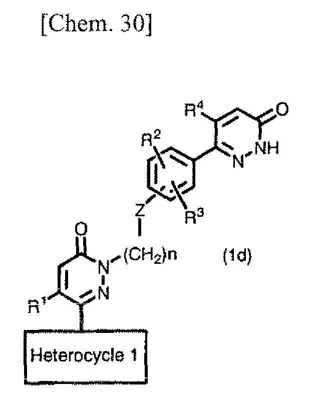

[R¹, R², R³, R⁴, Z, Heterocycle 1, and n are as defined above] can be prepared by oxidizing the compound represented by the general formula (1), wherein A is

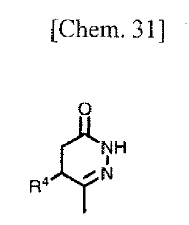

[wherein R⁴ is as defined above], that is, a compound represented by the general formula (1e):

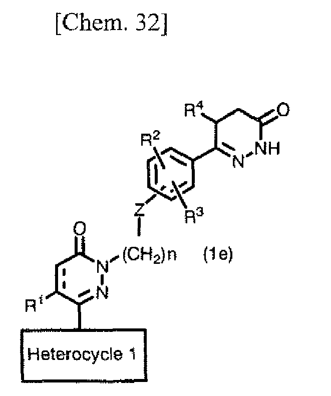

[R¹, R², R³, R⁴, Z, Heterocycle 1, n sand

[Chem. 33] - - -

are as defined above].

The reaction can be carried out in the same manner as in Step C-1.

The compound represented by the general formula (1), wherein A is

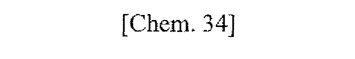

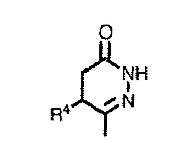

[wherein R⁴ is as defined above], and

[Chem. 35] - - -

is a single bond, that is, a compound represented by the general formula (1f):

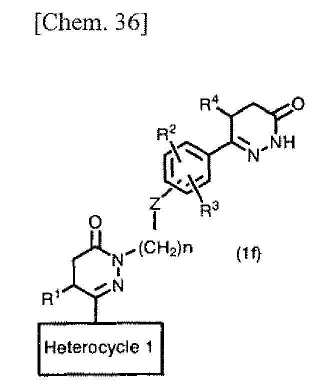

[R¹, R², R³, R⁴, Z, Heterocycle 1, and n are as defined above] can be prepared by reducing the compound represented by the general formula (1), wherein A is

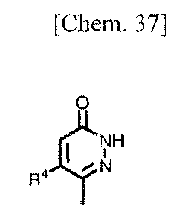

[wherein R⁴ is as defined above], that is, a compound represented by the general formula (1g):

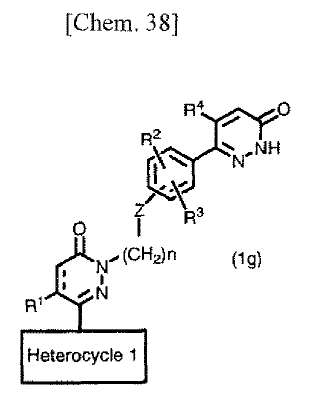

[R¹, R², R³, R⁴, Z, Heterocycle 1, n and

[Chem. 39] - - -

are as defined above].

The reaction can be carried out in the same manner as in Step C-2.

In Synthesis Pathways A and B, the compound represented by the general formula (3), wherein Heterocycle 1 is a quinoline ring and

[Chem. 40] - - -

is a single bond, that is, a compound represented by the general formula (3a-1) can be prepared via, for example, Synthesis Pathway D as shown below:

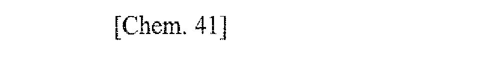

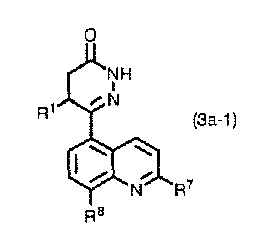

[wherein R¹, R⁷, and R⁸ are as defined above].

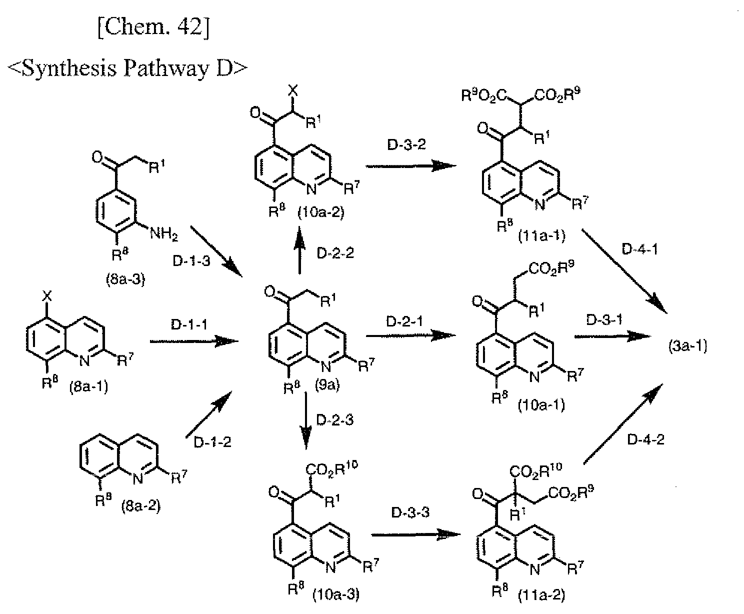

In Synthesis Pathway D, a compound represented by the general formula (9a) can be prepared by treating a compound represented by the general formula (8a-1) with an organometallic reagent, followed by reacting with a compound represented by the general formula (12) or a compound represented by the general formula (13) (Step D-1-1):

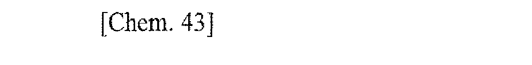

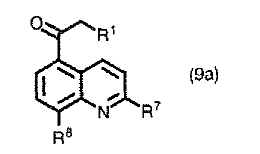

[wherein R¹, R⁷, and R⁸ are as defined above]

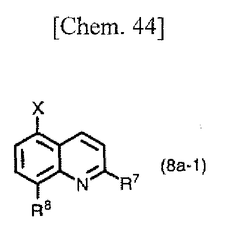

[wherein X represents a halogen atom, and R⁷ and R⁸ are as defined above]

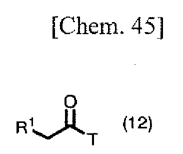

[wherein T represents a halogen atom, an amino group, a dimethyl amino group, or an alkoxy group having 1 to 6 carbon atoms, and R¹ is as defined above]

[Chem. 46] (R¹CH₂CO)₂O (13)

[wherein R¹ is as defined above].

The reaction is preferably carried out by dissolving the compound represented by the general formula (8a-1) in THF, diethyl ether, 1,4-dioxane, or the like, and performing a reaction at -78°C to 0°C, using an organomagnesium reagent such as methyl magnesium chloride, ethyl magnesium chloride, isopropyl magnesium chloride, methyl magnesium bromide, ethyl magnesium bromide, isopropyl magnesium bromide, methyl magnesium iodide, ethyl magnesium iodide, isopropylmagnesium iodide, and the like or an organolithium reagent such as n-butyl lithium, s-butyl lithium, t-butyl lithium, and the like, and preferably n-butyl lithium, and then allowing the compound of the general formula (12) or (13) to react therewith, followed by slowly warming to room temperature.

Further, the compound represented by the general formula (9a) can also be prepared by reacting a compound represented by the general formula (8a-2) with a compound represented by the general formula (14) (Step D-1-2):

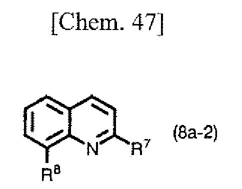

[wherein R⁷ and R⁸ are as defined above]

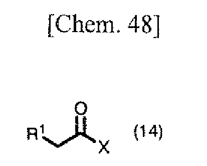

[wherein R¹ and X are as defined above].

The reaction can be carried out by adding a Lewis acid such as aluminum chloride, iron chloride, titanium tetrachloride, tin chloride, and the like, and preferably aluminum chloride, followed by warming to from room temperature to 120°C, using a solvent such as dichlorobenzene, methylene chloride, 1,2-dichloroethane, tetrachloroethane, nitromethane, benzene, chlorobenzene, and the like, and preferably dichlorobenzene.

Further, the compound represented by the general formula (9a) can also be prepared by reacting a compound represented by the general formula (8a-3) with a compound represented by the general formula (15) (Step D-1-3):

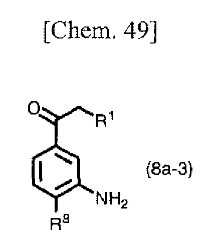

[wherein R¹ and R⁸ are as defined above]

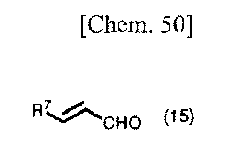

[wherein R⁷ is as defined above].

The reaction can be carried out at 50°C to 100°C, using 70% sulfuric acid or 6 mol/L hydrochloric acid also as a solvent, or using methanol, ethanol, propanol, or butanol, and preferably butanol, as a solvent with the addition of 50% sulfuric acid or concentrated hydrochloric acid. Further, sodium iodide can be added to the reaction system.

In Synthesis Pathway D, a compound represented by the general formula (10a-1) can be prepared by treating the compound represented by the general formula (9a) with a base, followed by reacting with a compound represented by the general formula (16) (Step D-2-1):

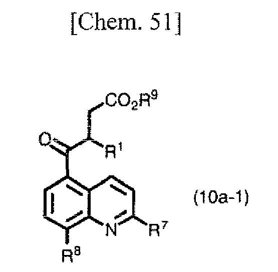

[wherein R⁹ represents an alkyl group having 1 to 6 carbon atoms or a benzyl group, and R¹, R⁷, and R⁸ are as defined above]

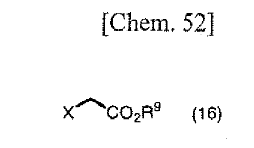

[wherein X and R⁹ are as defined above].

The reaction is preferably carried out by treating the compound represented by the general formula (9a) at -78°C to 0°C using sodium hydride, potassium hydride, sodium alkoxide, potassium alkoxide, lithium diisopropyl amide (LDA), lithium-2,2,6,6-tetramethylpiperizide, lithium bistrimethylsilyl amide, sodium bistrimethylsilyl amide, potassium bistrimethylsilyl amide, or the like as a base and using THF, 1,4-dioxane, 1,2-dimethoxyethane, or the like as a reaction solvent, followed by reacting with the compound of the general formula (16), followed by slowly warming to room temperature.

In Synthesis Pathway D, a compound represented by the general formula (10a-2) can be prepared by halogenating the compound represented by the general formula (9a) (Step D-2-2):

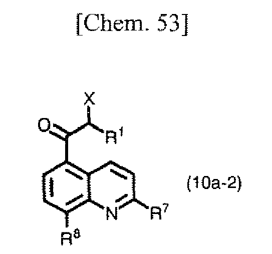

[wherein R¹, R⁷, R⁸, and X are as defined above].

The reaction can be carried out to heating under reflux, using sulfuryl chloride, bromine, iodine, NCS, NBS, NIS, cupric chloride, cupric bromide, or cupric iodide, preferably cupric chloride, cupric bromide, or cupric iodide and using a solvent such as ethyl acetate, THF, 1,4-dioxane, methylene chloride chloroform, and the like, and preferably ethyl acetate.

In Synthesis Pathway D, a compound represented by the general formula (11a-1) can be prepared by allowing a compound represented by the general formula (17) to act on the compound represented by the general formula (10a-2) in the presence of a base (Step D-3-2):

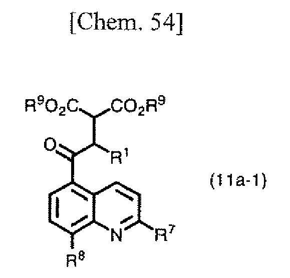

[wherein R¹, R⁷, R⁸, and R⁹ are as defined above] J

[Chem. 55] CH₂(CO₂R⁹)₂ (17)

[wherein R⁹ is as defined above].

The reaction can be carried out by treating the compound of the general formula (17) with an inorganic base such as sodium alkoxide, potassium alkoxide, LDA, lithium-2,2,6,6-tetramethylpiperizide, lithium bistrimethylsilyl amide, sodium bistrimethylsilyl amide, potassium bistrimethylsilyl amide, sodium hydride, potassium hydride, and the like, and preferably sodium hydride at 0°C to room temperature, using THF, DMF, 1,4-dioxane, dimethylsulfoxide (DMSO), or the like as a solvent, and then allowing the compound of the general formula (10a-2) to react therewith at room temperature to heating temperature under reflux.

In Synthesis Pathway D, a compound represented by the general formula (10a-3) can be prepared by allowing the compound represented by the general formula (9a) to act on a compound represented by the general formula (18) in the presence of a base (Step D-2-3):

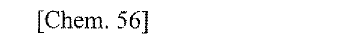

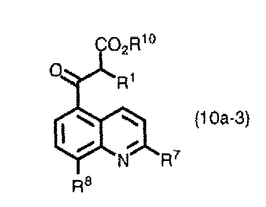

[wherein R¹⁰ represents an alkyl group having 1 to 6 carbon atoms, and R¹, R⁷, and R⁸ are as defined above]

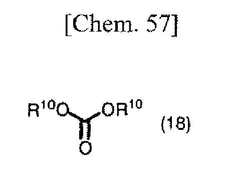

[wherein R¹⁰ is as defined above].

The reaction is preferably carried out to heating under reflux, using a solvent amount of the compound of the general formula (18) in the presence of an inorganic base such as sodium alkoxide, potassium alkoxide, sodium hydride, potassium hydride, and the like, and preferably sodium hydride.

In Synthesis Pathway D, a compound represented by the general formula (11a-2) can be prepared by reacting the compound represented by the general formula (16) with the compound represented by the general formula (10a-3) in the presence of a base (Step D-3-3):

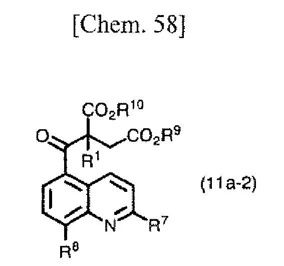

[wherein R¹, R⁷, R⁸, R⁹, and R¹⁰ are as defined above].

The reaction is preferably carried out by treating the compound of the general formula (10a-3) at 0°C to room temperature, using sodium hydride, potassium hydride, sodium alkoxide, potassium alkoxide, LDA, lithium-2,2,6,6-tetramethylpiperizide, lithium bistrimethylsilyl amide, sodium bistrimethylsilyl amide, potassium bistrimethylsilyl amide, or the like as a base and using THF, 1,4-dioxane, 1,2-dimethoxy ethane, or the like as a reaction solvent, followed by reacting with the compound represented by the general formula (16).

In Synthesis Pathway D, the compound represented by the general formula (3a-1) can be prepared by hydrolyzing the compound represented by the general formula (10a-1) and then allowing a hydrazine derivative to react therewith, or by directly allowing a hydrazine derivative to act thereon (Step D-3-1).

If the reaction is performed via the hydrolysis, it is preferably carried out by allowing a base such as an aqueous sodium hydroxide solution, an aqueous potassium hydroxide solution, an aqueous lithium hydroxide solution, and the like to act thereon at 0°C to room temperature, using ethanol, methanol, THF, 1,4-dioxane, or the like as a solvent. Further, in the case where R⁹ is a t-butyl group, it is preferable to perform the hydrolysis using trifluoroacetic acid without a solvent or in methylene chloride as a solvent. The obtained hydrolysate can be reacted with a hydrazine or a salt of the hydrazine such as hydrazine hydrochloride, hydrazine acetate, and the like, or a carbazic ester such as t-butyl carbazate, methyl carbazate, benzyl carbazate, and the like at room temperature or to heating under reflux, using a reaction solvent such as ethanol, benzene, toluene, acetic acid, and the like, and preferably ethanol. Further, in the case of directly reacting the compound represented by the general formula (10a-1) with a hydrazine derivative, the reaction is preferably carried out at room temperature to under heating with the addition of a catalytic amount of acetic acid in a solvent such as methanol, ethanol, and the like, at room temperature to under heating, in acetic acid as a solvent.

Further, the compound represented by the general formula (3a-1) can also be prepared by hydrolyzing the compound represented by the general formula (11a-1) and then allowing a hydrazine derivative to react therewith (Step D-4-1).

The reaction can be carried out by performing the hydrolysis using an aqueous sodium hydroxide solution or an aqueous potassium hydroxide solution and using a reaction solvent such as methanol, ethanol, THF, DMF, DMSO, and the like at room temperature, and then performing a decarboxylation by acidification. Further, if the decarboxylation is insufficient, the decarboxylation can be completed by dissolving the obtained dicarboxylic acid in methanol or ethanol, and then heating to reflux. The subsequent cyclization reaction can be carried out by the reaction with a hydrazine or a hydrazine salt such as hydrazine hydrochloride, hydrazine acetate, and the like, or a carbazic ester such as t-butyl carbazate, methyl carbazate, benzyl carbazate, and the like at room temperature or to heating under reflux in a reaction solvent such as ethanol, benzene, toluene, acetic acid, and the like, and preferably ethanol, as described above.

Further, the compound represented by the general formula (3a-1) can also be prepared by hydrolyzing the compound represented by the general formula (11a-2), and then allowing a hydrazine to react therein (Step D-4-2).

The reaction can be carried out by heating at 80°C to 100°C in hydrochloric acid or hydrobromic acid in the case of using an acidic condition. In the case using an alkaline condition, the hydrolysis can be carried out using an aqueous sodium hydroxide solution or an aqueous potassium hydroxide solution and using a reaction solvent such as methanol, ethanol, THF, DMF, DMSO, and the like at room temperature, and then the decarboxylation can be carried out by acidification. Thereafter, the cyclization reaction can be carried out by the reaction with a hydrazine or a hydrazine salt such as hydrazine hydrochloride, hydrazine acetate, and the like, or a carbazic ester such as t-butyl carbazate, methyl carbazate, benzyl carbazate, and the like at room temperature or heating under reflux in a reaction solvent such as ethanol, benzene, toluene, acetic acid, and the like, and preferably ethanol, as described above.

The compound represented by the general formula (3), wherein Heterocycle 1 is a 1,2,3,4-tetrahydroquinoline ring and

[Chem. 59] - - -

is a single bond, that is, a compound represented by the general formula (3a-2) can be prepared by reducing the compound represented by the general formula (3a-1):

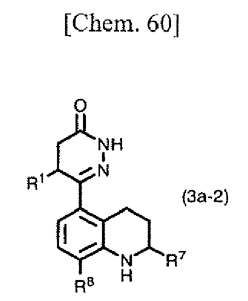

[wherein R¹, R⁷, and R⁸ are as defined above].

The reaction is preferably carried out by adding 5 to 10% palladium/carbon as a catalyst with the use of DMF, methanol, or ethanol as a solvent, and then performing a catalytic reduction under a hydrogen atomosphere at room temperature to under warming, and preferably at 50°C.

The compound represented by the general formula (3), wherein Heterocycle 1 is a triazolopyridine ring and

[Chem. 61] - - -

is a single bond, that is, a compound represented by the general formula (3b) can be prepared via Synthesis Pathway E below:

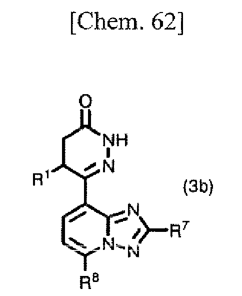

[wherein R¹, R⁷, and R⁸ are as defined above].

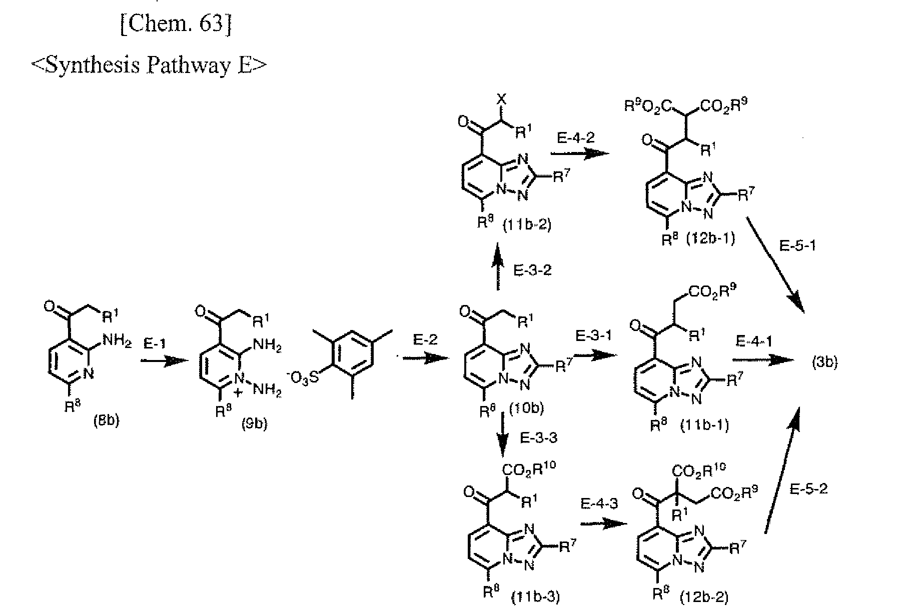

In Synthesis Pathway E, a compound represented by the general formula (9b) can be prepared by allowing the compound represented by the general formula (8b) to act on O-mesitylene sulfonyl hydroxyamine (hereinafter referred to as MSH) (Step E-1):

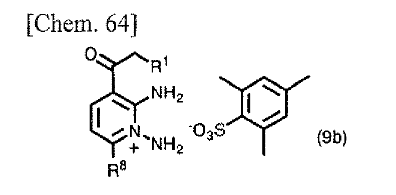

[wherein R¹ and R⁸ are as defined above]

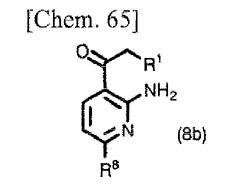

[wherein R¹ and R⁸ are as defined above].

The reaction is preferably carried out by dissolving the compound represented by the general formula (8b) in methylene chloride and allowing a solution of MSH in methylene chloride to act thereon at 0°C to room temperature.

In Synthesis Pathway B, a compound represented by the general formula (10b) can be prepared by allowing the compound represented by the general formula (9b) to act on a compound represented by the general formula (19) in the presence of a base (Step E-2):

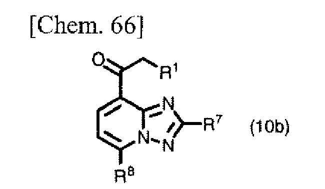

[wherein R¹, R⁷, and R⁸ are as defined above]

[Chem. 67] (R⁷CO)₂O (19)

[wherein R⁷ is as defined above].

The reaction can be carried out at room temperature to heating temperature under reflux, using benzene, toluene, xylene, methanol, ethanol, or the like as a solvent and using a base such as triethylamine, sodium hydroxide, potassium hydroxide, potassium carbonate, and the like, and preferably triethylamine.

In Synthesis Pathway E, a compound represented by the general formula (11b-1) can be prepared by treating the compound represented by the general formula (10b) with a base, followed by reacting with the compound represented by the general formula (16) (Step E-3-1):

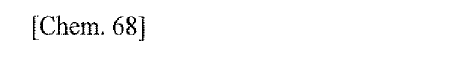

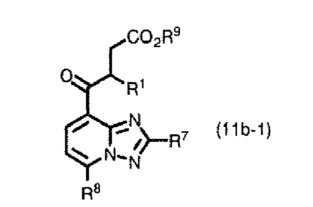

[wherein R¹, R⁷, R⁸, and R⁹ are as defined above]

The reaction can be carried out in the same manner as in Step D-2-1.

In Synthesis Pathway E, a compound represented by the general formula (11b-2) can be prepared by halogenating the compound represented by the general formula (10b) (Step E-3-2):

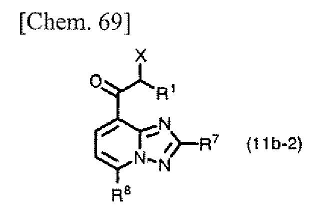

[wherein R¹, R⁷, R⁸, and X are as defined above].

The reaction can be carried out in the same manner as in Step D-2-2.

In Synthesis Pathway E, a compound represented by the general formula (12b-1) can be prepared by allowing a compound represented by the general formula (11b-2) to act on the compound represented by the general formula (17) in the presence of a base (Step E-4-2):

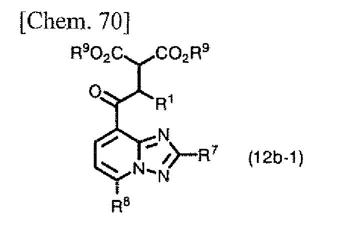

[wherein R¹, R⁷, R⁸, and R⁹ are as defined above].

The reaction can be carried out in the same manner as in Step D-3-2.

In Synthesis Pathway E, a compound represented by the general formula (11b-3) can be prepared by allowing the compound represented by the general formula (10b) to act on the compound represented by the general formula (18) in the presence of a base (Step E-3-3):

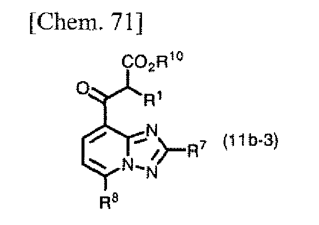

[wherein R¹, R⁷, R⁸, and R¹⁰ are as defined above].

The reaction can be carried out in the same manner as in Step D-2-3.

In Synthesis Pathway E, a compound represented by the general formula (12b-2) can be prepared by reacting the compound represented by the general formula (11b-3) with the compound represented by the general formula (16) in the presence of a base (Step E-4-3):

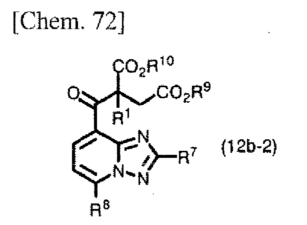

[wherein R¹, R⁷, R⁸, R⁹, and R¹⁰ are as defined above].

The reaction can be carried out in the same manner as in Step D-3-3.

In Synthesis Pathway E, the compound represented by the general formula (3b) can be prepared by hydrolyzing the compound represented by the general formula (11b-1) and then allowing a hydrazine derivative to react therewith or by directly allowing a hydrazine derivative to react therewith (Step E-4-1).

The reaction can be carried out in the same manner as in Step D-3-1.

Further, the compound represented by the general formula (3b) can also be prepared by hydrolyzing the compound represented by the general formula (12b-1) and then allowing a hydrazine derivative to react therewith (Step E-5-1).

The reaction can be carried out in the same manner as in Step D-4-1.

Further, the compound represented by the general formula (3b) can also be prepared by hydrolyzing the compound represented by the general formula (12b-2) and then allowing a hydrazine derivative to react therewith (Step E-5-2).

The reaction can be carried out in the same manner as in Step D-4-2.

In Synthesis Pathway E, the compound represented by the general formula (10b) can also be prepared via Synthesis Pathway E' below.

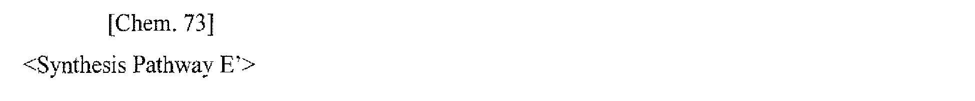

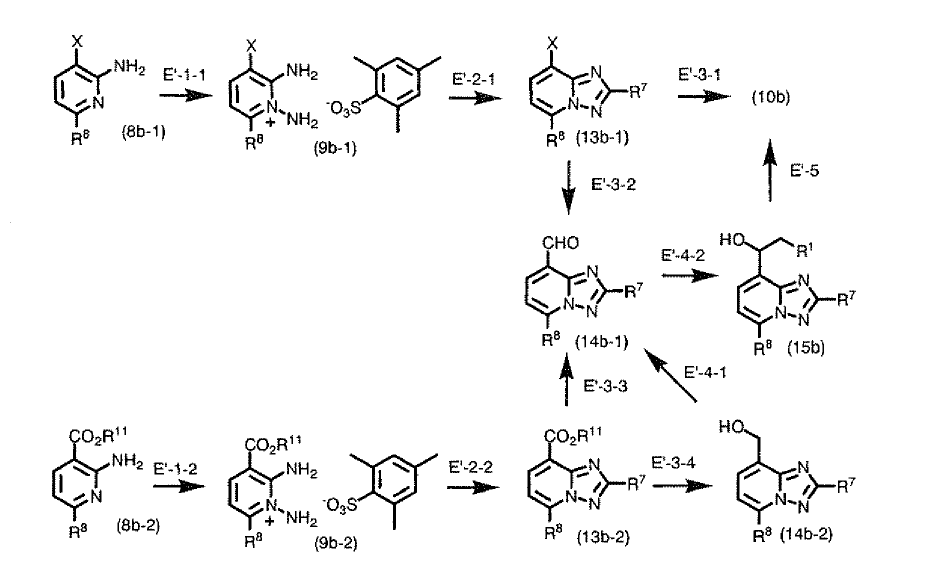

In Synthesis Pathway E', a compound represented by the general formula (9b-1) or a compound represented by the general formula (9b-2) can be prepared by allowing the compound represented by the general formula (8b-1) or the compound represented by the general formula (8b-2) to act on MSH, respectively (Steps E'-1-1 and E'-1-2):

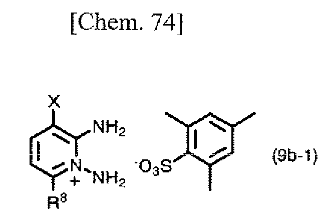

[wherein R⁸ and X are as defined above]

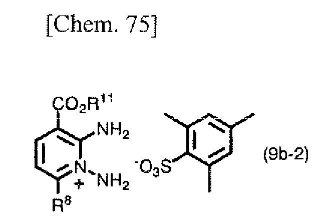

[wherein R¹¹ represents an alkyl group having 1 to 6 carbon atoms or a benzyl group, and R⁸ is as defined above]

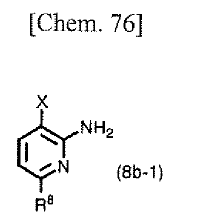

[wherein R⁸ and X are as defined above]

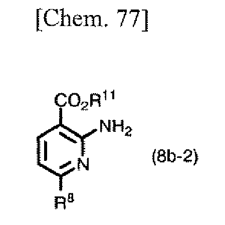

[wherein R⁸ and R¹¹ are as defined above].

The reaction can be carried out in the same manner as in Step E-1.

In Synthesis Pathway E', a compound represented by the general formula (13b-1) or a compound represented by the general formula (13b-2) can be prepared by allowing the compound represented by the general formula (9b-1) or (9b-2) to act on the compound represented by the general formula (19), respectively, in the presence of a base (Steps E'-2-1 and E'-2-2):

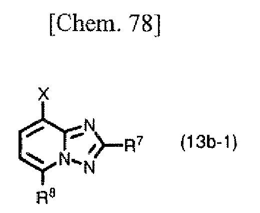

[wherein R⁷, R⁸ and X are as defined above]

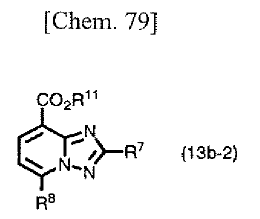

[wherein R⁷, R⁸, and R¹¹ are as defined above]

The reaction can be carried out in the same manner as in Step E-2.

In Synthesis Pathway E', a compound represented by the general formula (14b-2) can be prepared by reducing the compound represented by the general formula (13b-2) (Step E'-3-4):

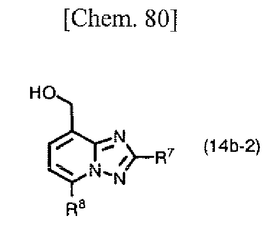

[wherein R⁷ and R⁸ are as defined above].

The reaction can be carried out at a reaction temperature of 0°C to under warming, and preferably at room temperature, using an alkylborane derivative such as borane (BH₃) and 9-borabicyclo[3,3,1]nonane (9-BBN) or a metal hydrogen complex compound such as diisobutylaluminum hydride (DIBAL), lithium borohydride (LiBH₄), sodium borohydride (NaBH₄), lithium aluminum hydride (LiAlH₄), and the like, and preferably lithium borohydride and using THF, ethanol, methanol, or the like as a reaction solvent.

In Synthesis Pathway E', a compound represented by the general formula (14b-1) can be prepared by oxidizing the compound represented by the general formula (14b-2) (Step E'-4-1):

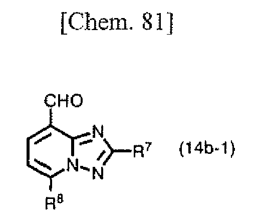

[wherein R⁷ and R⁸ are as defined above].

The reaction can employ a means for oxidizing a generally used alcohol to an aldehyde and a ketone, and examples of the means include a chromium oxide-pyridine complex such as pyridinium chlorochromate, pyridinium dichromate, and the like, a metal oxidant such as chromium oxide, silver carbonate, manganese dioxide, and the like, DMSO oxidation using a DMSO activator such as a sulfur trioxide-pyridine complex, oxalyl chloride, anhydrous trifluoroacetic acid, acetic anhydride, dicyclohexylcarbodiimide (DCC), and the like, and hypervalency iodine oxidation using 4-iodoxybenzoic acid (IBX), dess-Martin periodinane, or the like.

Further, the compound represented by the general formula (14b-1) can also be prepared by treating the compound represented by the general formula (13b-1) with an organometalic reagent, followed by reacting with DMF or formic ester (Step E'-3-2).

The reaction is preferably carried out by dissolving the compound represented by the general formula (13b-1) in THF, ether, 1,4-dioxane, or the like, and performing a reaction at -78°C to 0°C using an organomagnesium reagent such as methyl magnesium chloride, ethyl magnesium chloride, isopropyl magnesium chloride, methyl magnesium bromide, ethyl magnesium bromide, isopropyl magnesium bromide, methyl magnesium iodide, ethyl magnesium iodide, isopropylmagnesium iodide, and the like, or an organolithium reagent such as n-butyl lithium, s-butyl lithium, t-butyl lithium, LDA, and the like, and preferably n-butyl lithium or LDA, as an organic metal reagent, and then allowing DMF or formic ester to react therewith, followed by slowly warming to room temperature.

Further, the compound represented by the general formula (14b-1) can also be prepared by reducing the compound represented by the general formula (13b-2) (Step E'-3-3).

The reaction is preferably carried out at -78°C to 0°C using DIBAL or sodium bis(2-methoxyethoxy) aluminumhydride (trade name: RedAl) as a reducing agent by dissolving the compound in THF, ether, 1,4-dioxane, or the like.

In Synthesis Pathway E', a compound represented by the general formula (15b) can be prepared by reacting the compound represented by the general formula (14b-1) with a compound represented by the general formula (20) (Step E'-4-2):

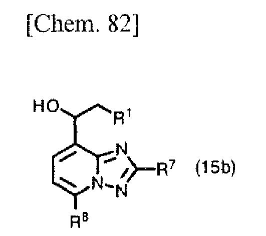

[wherein R¹, R⁷, and R⁸ are as defined above]

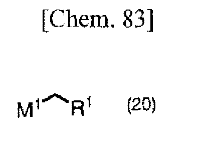

[wherein M¹ represents a lithium atom, MgCl, MgBr, or MgI, and R¹ is as defined above].

The reaction is preferably carried out by mixing both the compounds at -78°C to 0°C, followed by, if necessary, warming to room temperature, using THF, diethyl ether, 1,4-dioxane, or the like as a solvent.

In Synthesis Pathway E', the compound represented by the general formula (10b) can be prepared by oxidizing the compound represented by the general formula (15b) (Step E'-5).

The reaction can be carried out in the same manner as in Step E'-4-1.

Further, the compound represented by the general formula (10b) can also be prepared by treating the compound represented by the general formula (13b-1) with an organometalic reagent, followed by reacting with the compound represented by the general formula (12) or the compound represented by the general formula (13) (Step E'-3-1).

The reaction can be carried out in the same manner as in Step D-1-1.

In Synthesis Pathways E and E', the compound represented by the general formula (10b), wherein R⁸ is an alkoxy group having 1 to 6 carbon atoms, that is, a compound represented by the general formula (10b-1) can also be prepared by two methods as shown in Synthesis Pathway E" below:

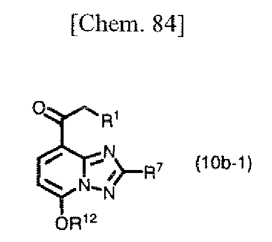

[wherein R¹² represents an alkyl group having 1 to 6 carbon atoms, and R¹ and R⁷ are as defined above].

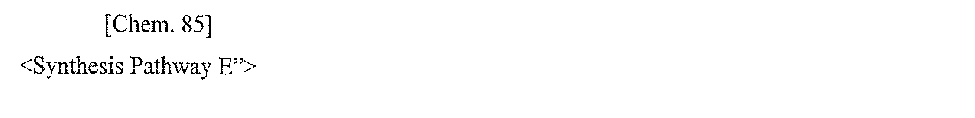

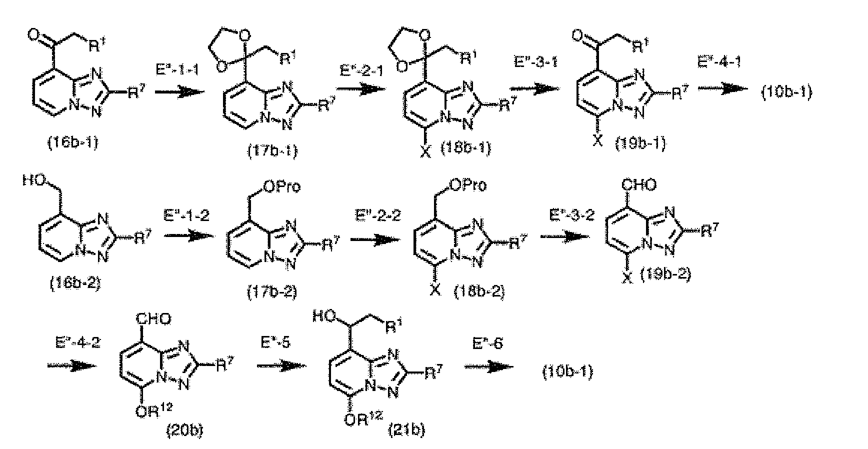

In Synthesis Pathway E", a compound represented by the general formula (17b-1) can be prepared by reacting the compound of the general formula (10b) in which R⁸ is a hydrogen atom, that is, a compound represented by the general formula (16b-1) with ethylene glycol (Step E"-1-1):

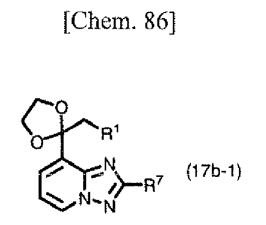

[wherein R¹ and R⁷ are as defined above]

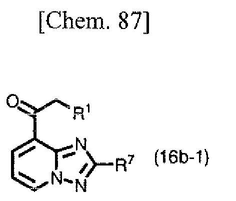

[wherein R¹ and R⁷ are as defined above].

The reaction is preferably carried out to heating under reflux in a solvent such as benzene, toluene, xylene, and the like, preferably under dehydration using a Dea-Stark trap using a catalytic amount of paratoluenesulfonic acid or pyridinium paratoluenesulfonate.

In Synthesis Pathway E", a compound represented by the general formula (18b-1) can be prepared by halogenating the compound represented by the general formula (17b-1) (Step E"-2-1):

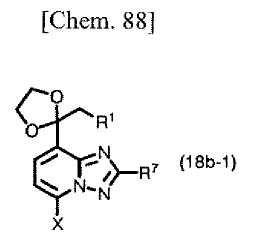

[wherein R¹, R⁷, and X are as defined above].

The reaction is preferably carried out by performing a reaction at -78°C to 0°C in THF as a solvent using a base such as butyl lithium, lithium, hexamethyl disilazide, LDA, and the like, and preferably LDA, and then allowing NCS, NBS, NIS, bromine, iodine, 1,2-dibromoethane, or 1,2-diiodoethane to react therewith.

In Synthesis Pathway E", a compound represented by the general formula (19b-1) can be prepared by deprotecting the compound represented by the general formula (18b-1) (Step E"-3-1):

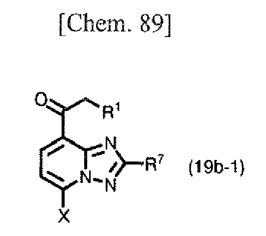

[wherein R¹, R⁷, and X are as defined above].

The reaction is preferably carried out by allowing paratoluenesulfonic acid to act thereon at room temperature to heating temperature under reflux in acetone as a solvent, or by performing a reaction at 0°C to room temperature using hydrogen chloride-containing methanol, ethanol, ethyl acetate, or diethyl ether.

In Synthesis Pathway E", a compound represented by the general formula (17b-2) can be prepared by subjecting the compound of the general formula (14b-2) in which R⁸ is a hydrogen atom, that is, a compound represented by the general formula (16b-2) to various reactions for introducing a protecting group (Step E"-1-2):

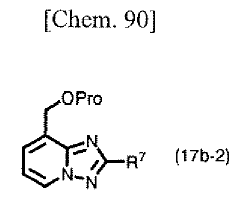

[wherein Pro represents a protecting group used for an alcohol group, such as a methoxymethyl group, a t-butyldimethylsilyl group, a t-butyldiphenylsilyl group, a triisopropylsilyl group, a tetrahydropyranyl group, an acetyl group, and the like, and R⁷ is as defined above]

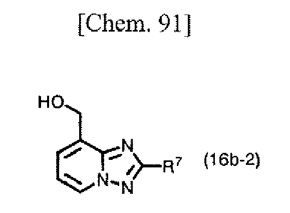

[wherein R⁷ is as defined above].

In the case of the introduction of a methoxymethyl group, it is preferable to allow methoxymethyl chloride or methoxymethylbromide to act thereon at 0°C to room temperature in a solvent such as THF, methylene chloride, acetonitrile, and the like, and preferably methylene chloride in the presence of sodium hydride, triethylamine, ethyl diisopropylamine, or the like. Further, in the case of the introduction of a t-butyldimethylsilyl group, a t-butyldiphenylsilyl group, or a triisopropylsilyl group, it is preferable to allow the corresponding silyl chloride, silyl bromide, or silyl trifluoromethanesulfonate to act thereon at 0°C to room temperature in a solvent such as THF, DMF, acetonitrile, methylene chloride, and the like in the presence of triethylamine, imidazole, or the like. For the introduction of a tetrahydropyranyl group, it is preferable to add an acidic catalyst such as paratoluenesulfonic acid, and the like in the presence of dihydropyrane, and allow it to react in methylene chloride. Further, in the case of the introduction of an acetyl group, acetyl chloride, acetyl bromide, or acetic anhydride can be allowed to react at 0°C to room temperature in a solvent such as THF, 1,4-dioxane, or methylene chloride in the presence of an organic base such as triethylamine, ethyldiisopropyl amine, pyridine, and the like, or the reaction can be carried out using pyridine also as a solvent at 0°C to room temperature.

In Synthesis Pathway E", a compound represented by the general formula (18b-2) can be prepared by halogenating the compound represented by the general formula (17b-2) (Step E"-2-2):

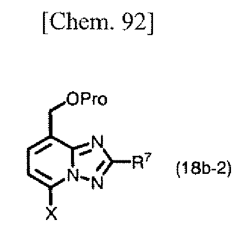

[wherein R⁷, X, and Pro are as defined above].

The reaction can be carried out in the same manner as in Step E"-2-1.

In Synthesis Pathway E", a compound represented by the general formula (19b-2) can be prepared by deprotecting and oxidizing the compound represented by the general formula (18b-2) (Step E"-3-2):

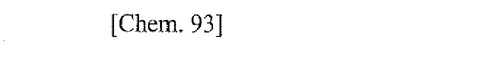

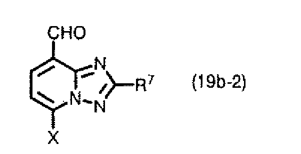

[wherein R⁷ and X are as defined above].

In the case of a methoxymethyl group or a tetrahydropyranyl group, the deprotection reaction is preferably carried out at 0°C to room temperature using hydrogen chloride-containing methanol, ethanol, ethyl acetate, or diethyl ether. In the case of a silyl protecting group, the reaction is preferably carried out at 0°C to room temperature in acetonitrile or THF as a solvent, using potassium fluoride, cesium fluoride, or tetrabutyl ammonium fluoride. Further, in the case of an acetyl group, the reaction is preferably carried out at 0°C to room temperature using an aqueous sodium hydroxide solution, an aqueous potassium hydroxide solution or an aqueous lithium hydroxide solution and using THF, methanol, ethanol, or 1,4-dioxane, or the like as a solvent. Example of the oxidation reaction include a chromium oxide-pyridine complex such as pyridinium chlorochromate, pyridinium dichromate, and the like, a metal oxidant such as chromium oxide, silver carbonate, manganese dioxide, and the like, DMSO oxidation using a DMSO activator including a sulfur trioxide-pyridine complex, oxalyl chloride, anhydrous trifluoroacetic acid, acetic anhydride, DCC, and the like, and hypervalency iodine oxidation such as IBX, dess-Martin periodinane oxidation, and the like.

In Synthesis Pathway E", a compound represented by the general formula (20b) can be prepared by reacting the compound represented by the general formula (19b-2) with a compound represented by the general formula (21) (Step E"-4-2):

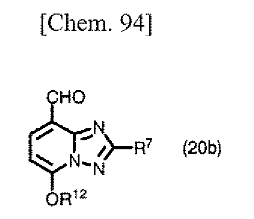

[wherein R⁷ and R¹² are as defined above]

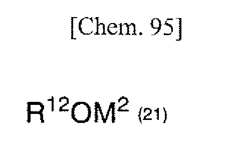

[wherein M² represents a sodium atom, a potassium atom, or a lithium atom, and R¹² is as defined above].

The reaction can be carried out at room temperature to heating temperature under reflux, using methanol, ethanol, propanol, or butanol.

In Synthesis Pathway E", a compound represented by the general formula (21b) can be prepared by reacting the compound represented by the general formula (20b) with the compound represented by the general formula (20) (Step E"-5):

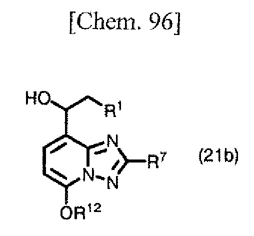

[wherein R¹, R⁷, and R¹² are as defined above].

The reaction can be carried out in the same manner as in Step E'-4-2.

In Synthesis Pathway E", the compound represented by the general formula (10b-1) can be prepared by oxidizing the compound represented by the general formula (21b) (Step E"-6).

The reaction can be carried out in the same manner as in Step E'-4-1.

Further, the compound represented by the general formula (10b-1) can be prepared by reacting the compound represented by the general formula (19b-1) with the compound represented by the general formula (21) (Step E"-4-1).

The reaction can be carried out in the same manner as in Step E"-4-2.

The compound represented by the general formula (3), wherein Heterocycle 1 is an imidazopyridine ring and

[Chem. 97] - - -

is a single bond, that is, a compound represented by the general formula (3c) can be prepared via Synthesis Pathway F below:

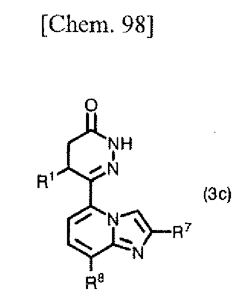

[wherein R¹, R⁷, and R⁸ are as defined above].

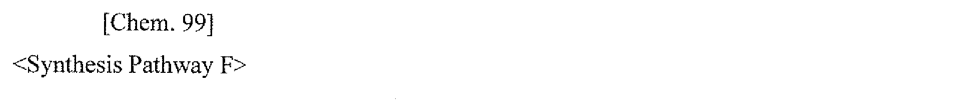

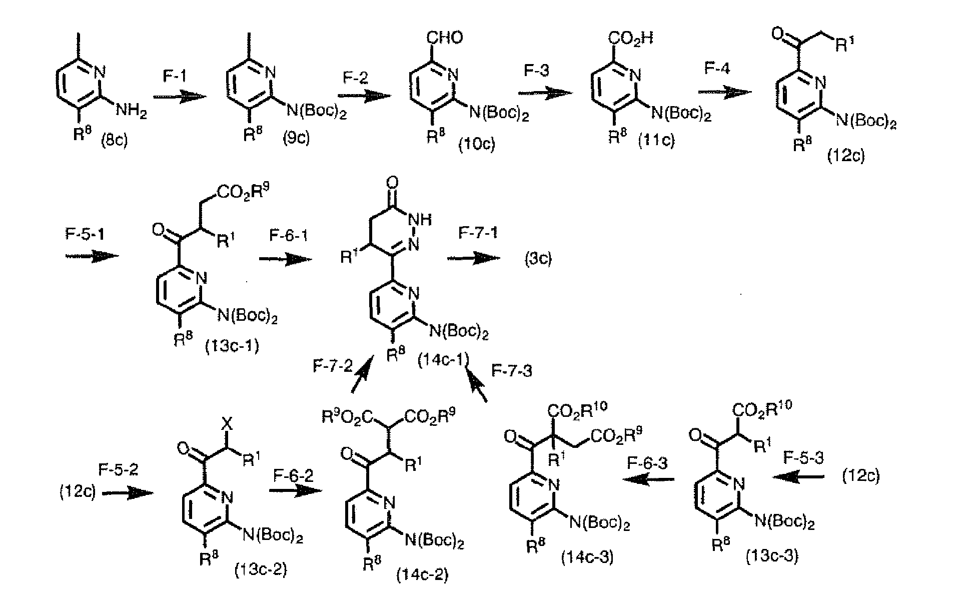

In Synthesis Pathway F, the compound represented by the general formula (9c) can be prepared by the Boc-addition of the compound represented by the general formula (8c) (Step F-1):

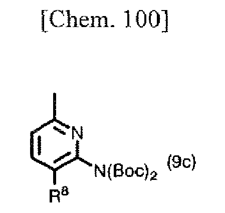

[wherein Boc represents a t-butoxycarbonyl group and R⁸ is as defined above]

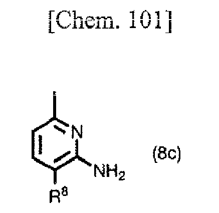

[wherein R⁸ is as defined above].

The reaction can be carried out at room temperature with the addition of di-t-butyl dicarbonate (Boc₂O), triethylamine, and 4-dimethyl aminopyridine (DMAP), using a solvent such as acetonitrile, t-butanol, and the like, and preferably acetonitrile.

In Synthesis Pathway F, a compound represented by the general formula (10c) can be prepared by halogenating the compound represented by the general formula (9c), followed by reacting with N-methylmorpholine-N-oxide (NMO) (Step F-2):

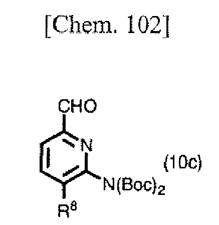

[wherein R⁸ and Boc are as defined above].

The reaction is preferably carried out to heating under reflux in a solvent such as carbon tetrachloride, methylene chloride, chloroform, and the like, and preferably carbon tetrachloride, with the addition of a catalytic amount of a radical initiator such as benzoyl peroxide, 2,2'-azobisisobutyronitrile, and the like, using NCS, NBS, or NIS. Further, the reaction can be efficiently completed by irradiating light instead of heating under reflux.

By adding a Molecular Sieve 4A, NMO to the halogeno form obtained by the reaction as described above, and carrying out the reaction using acetonitrile as a solvent under an inert gas atmosphere at room temperature, its conversion into an aldehyde can be attained.

In Synthesis Pathway F, a compound represented by the general formula (11c) can be prepared by oxidizing the compound represented by the general formula (10c) (Step F-3):

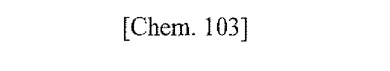

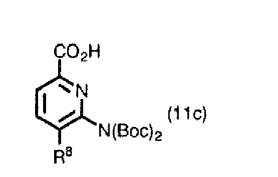

[wherein R⁸ and Boc are as defined above].

The reaction is preferably carried out at room temperature with the addition of sodium chlorite, sodium dihydrogen phosphate, 2-methyl-2-butene, and water, using t-butanol as a solvent.

In Synthesis Pathway F, a compound represented by the general formula (12c) can be prepared by condensing the compound represented by the general formula (11c) with N,O-dimethylhydroxyamine, followed by reacting with the compound represented by the general formula (20) (Step F-4):

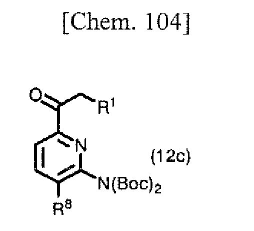

[wherein R¹, R⁸, and Boc are as defined above].

The condensation reaction with N,O-dimethylhydroxyamine is preferably carried out using a condensing agent such as DCC, N,N-diisopropylcarbodiimide (DIPC), diphenylphosphoryl azide (DPPA), diethylphosphoryl cyanide (DEPC), 1-ethyl-3-(3-dimethylaminopropyl)-carbodiimide hydrochloride (WSC), and the like in the presence of an organic base such as triethylamine, pyridine, and the like, or as the occasion demands with the addition of a catalytic amount of DMAP, at 0°C to room temperature, using THF, DMF, DMSO, methylene chloride, and the like as a reaction solvent. It is preferable that the amide form thus obtained is dissolved in a solvent such as THF, diethyl ether, 1,4-dioxane, and the like, and the compound represented by the general formula (20) is added at -78°C to 0°C, followed by, if necessary, warming to room temperature.

In Synthesis Pathway F, a compound represented by the general formula (13c-1) can be prepared by treating the compound represented by the general formula (12c) with a base, followed by reacting with the compound represented by the general formula (16) (Step F-5-1):

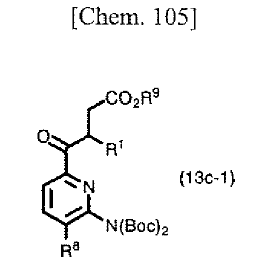

[wherein R¹, R⁸, R⁹, and Boc are as defined above].

The reaction can be carried out in the same manner as in Step D-2-1.

In Synthesis Pathway F, a compound represented by the general formula (13c-2) can be prepared by halogenating the compound represented by the general formula (12c) (Step F-5-2):

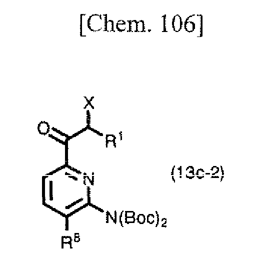

[wherein R¹, R⁸, X, and Boc are as defined above].

The reaction can be carried out in the same manner as in Step D-2-2.

In Synthesis Pathway F, a compound represented by the general formula (14c-2) can be prepared by allowing the compound represented by the general formula (13c-2) to act on the compound represented by the general formula (17) in the presence of a base (Step F-6-2):

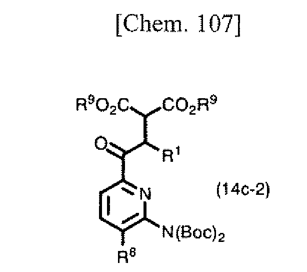

[wherein R¹, R⁸, R⁹, and Boc are as defined above].

The reaction can be carried out in the same manner as in Step D-3-2.

In Synthesis Pathway F, a compound represented by the general formula (13c-3) can be prepared by allowing the compound represented by the general formula (12c) to act on the compound represented by the general formula (18) in the presence of a base (Step F-5-3):

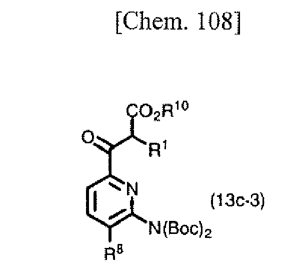

[wherein R¹, R⁸, R¹⁰, and Boc are as defined above].

The reaction can be carried out in the same manner as in Step D-2-3.

In Synthesis Pathway F, a compound represented by the general formula (14c-3) can be prepared by reacting the compound represented by the general formula: (13c-3) with the compound represented by the general formula (16) in the presence of a base (Step F-6-3):

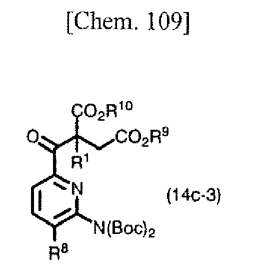

[wherein R¹, R⁸, R⁹, R¹⁰, and Boc are as defined above]_{.}

The reaction can be carried out in the same manner as in Step D-3-3.

In Synthesis Pathway F, a compound represented by the general formula (14c-1) can be prepared by hydrolyzing the compound represented by the general formula (13c-1) and then allowing a hydrazine derivative to react therewith, or by directly allowing a hydrazine derivative to react therewith (Step F-6-1):

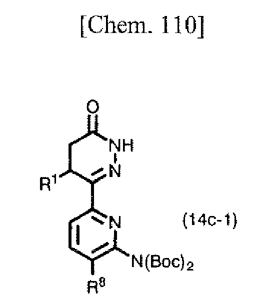

[wherein R¹, R⁸, and Boc are as defined above].

The reaction can be carried out in the same manner as in Step D-3-1.

Further, the compound represented by the general formula (14c-1) can also be prepared by hydrolyzing the compound represented by the general formula (14c-2) and then allowing a hydrazine derivative to react therewith (Step F-7-2).

The reaction can be carried out in the same manner as in Step D-4-1.

Further, the compound represented by the general formula (14c-1) can also be prepared by hydrolyzing the compound represented by the general formula (14c-3) and then allowing a hydrazine derivative to react therewith (Step F-7-3).

The reaction can be carried out in the same manner as in Step D-4-2.

In Synthesis Pathway F, the compound represented by the general formula (3c) can be prepared by deprotecting the compound represented by the general formula (14c-1), followed by reacting with a compound represented by the general formula (22) (Step F-7-1):

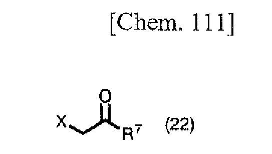

[wherein R⁷ and X are as defined above].

The deprotection reaction can be carried out at room temperature using hydrogen chloride-containing methanol, ethanol, diethyl ether, 1,4-dioxane, ethyl acetate, or trifluoroacetic acid. Subsequently, the reaction with the compound represented by the general formula (22) is preferably carried out at 70°C to heating temperature under reflux, using a solvent such as methanol, ethanol, and the like, and preferably ethanol.

The compound represented by the general formula (3), wherein Heterocycle 1 is an indolidine ring and

[Chem. 112] - - -

is a single bond, that is, a compound represented by the general formula (3d) can be prepared via Synthesis Pathway G below:

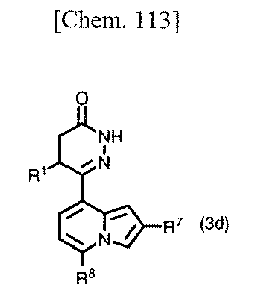

[wherein R¹, R⁷, and R⁸ are as defined above].

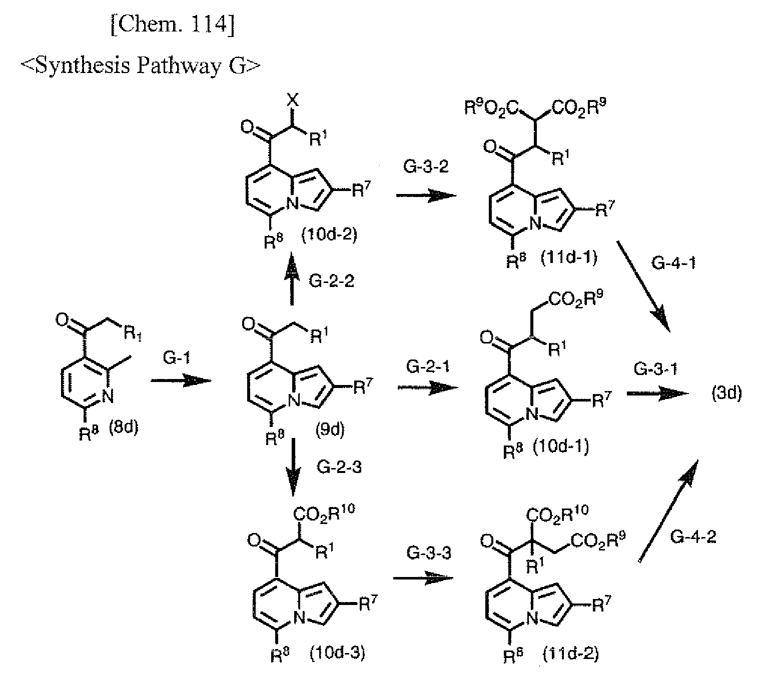

In Synthesis Pathway G, a compound represented by the general formula (9d) can be prepared by reacting a compound represented by the general formula (8d) with the compound represented by the general formula (22) (Step G-1):

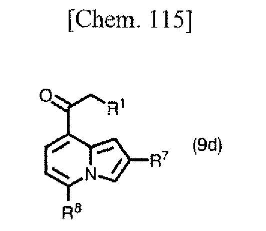

[wherein R¹, R⁷, and R⁸ are as defined above]

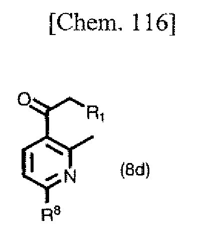

[wherein R¹ and R⁸ are as defined above].

The reaction is preferably carried out to heating under reflux by reacting both the compounds at 60°C to 80°C using ethyl acetate as a solvent, and then changing the solvent into benzene, toluene, or xylene, and preferably toluene, with the addition of 1,8-diazabicyclo[5,4,0]undec-7-ene.

In Synthesis Pathway G, a compound represented by the general formula (10d-1) can be prepared by treating the compound represented by the general formula (9d) with a base, followed by reacting with the compound represented by the general formula (16) (Step G-2-1):

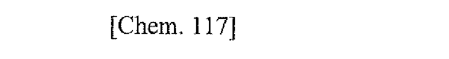

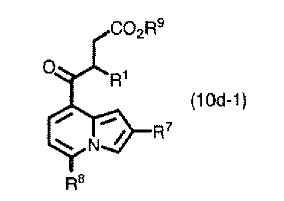

[wherein R¹, R⁷, R⁸, and R⁹ are as defined above].

The reaction can be carried out in the same manner as in Step D-2-1.

In Synthesis Pathway G, a compound represented by the general formula (10d-2) can be prepared by halogenating the compound represented by the general formula (9d) (Step G-2-2):

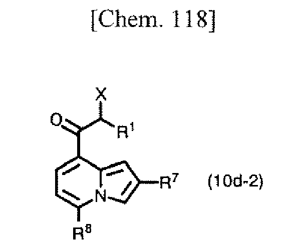

[wherein R¹, R⁷, R⁸ and X are as defined above].

The reaction can be carried out in the same manner as in Step D-2-2.

In Synthesis Pathway G, a compound represented by the general formula: (11d-1) can be prepared by allowing the compound represented by the general formula (10d-2) to act on the compound represented by the general formula (17) in the presence of a base (Step G-3-2):

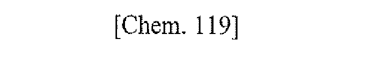

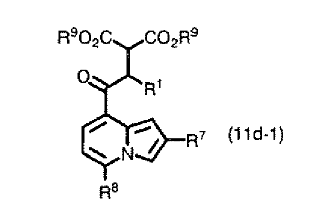

[wherein R¹, R⁷, R⁸, and R⁹ are as defined above].

The reaction can be carried out in the same manner as in Step D-3-2.

In Synthesis Pathway G, a compound represented by the general formula (10d-3) can be prepared by allowing the compound represented by the general formula (9d) to act on the compound represented by the general formula (18) in the presence of a base (Step G-2-3):

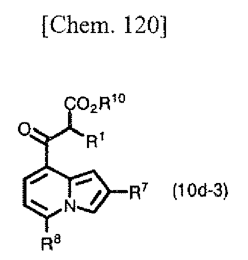

[wherein R¹, R⁷, R⁸, and R¹⁰ are as defined above].

The reaction can be carried out in the same manner as in Step D-2-3.

In Synthesis Pathway G, a compound represented by the general formula (11d-2) can be prepared by reacting the compound represented by the general formula (10d-3) with the compound represented by the general formula (16) in the presence of a base (Step G-3-3):

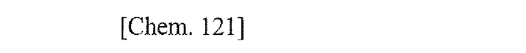

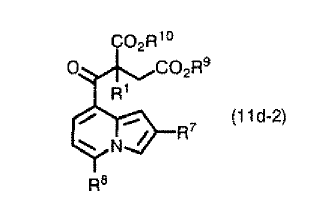

[wherein R¹, R⁷, R⁸, R⁹, and R¹⁰ are as defined above].

The reaction can be carried out in the same manner as in Step D-3-3.

In Synthesis Pathway G, the compound represented by the general formula (3d) can be prepared by hydrolyzing the compound represented by the general formula (10d-1) and then allowing a hydrazine derivative to react therewith, or by directly allowing a hydrazine derivative to react therewith (Step G-3-1).

The reaction can be carried out in the same manner as in Step D-3-1.

Further, the compound represented the general formula (3d) can also be prepared by hydrolyzing the compound represented by the general formula (11d-1) and then allowing a hydrazine derivative to react therewith (Step G-4-1).

The reaction can be carried out in the same manner as in Step D-4-1.

Further, the compound represented by the general formula (3d) can also be prepared by hydrolyzing the compound represented by the general formula (11d-2) and then allowing a hydrazine derivative to react therewith (Step G-4-2).

The reaction can be carried out in the same manner as in Step D-4-2.

In Synthesis Pathway G, the compound represented by the general formula (9d) can also be prepared via Synthesis Pathway G' below.

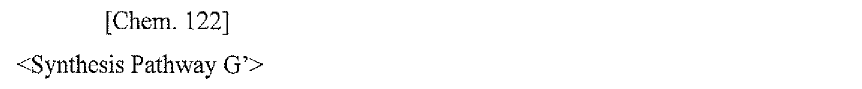

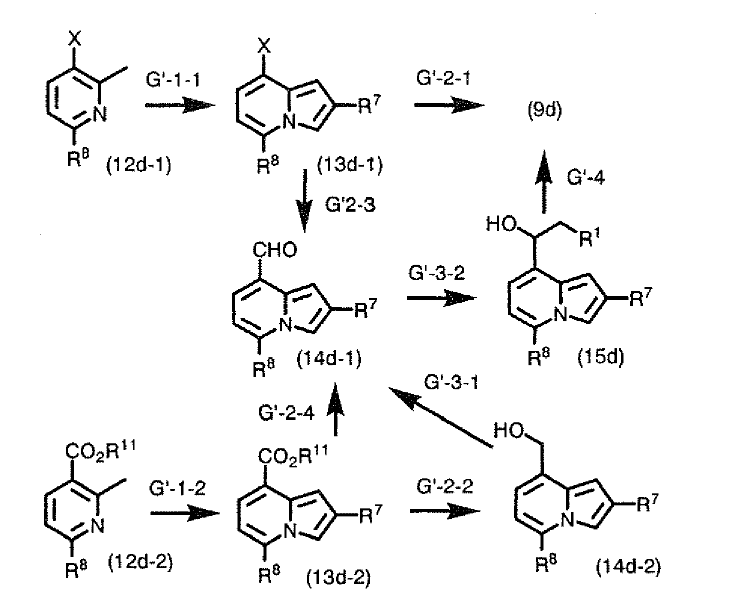

In Synthesis Pathway G', a compound represented by the general formula (13d-1) or a compound represented by the general formula (13d-2) can be prepared by reacting the compound represented by the general formula (12d-1) or the compound represented by the general formula (12d-2) with the compound represented by the general formula (22), respectively (Step G'-1-1 and G'-1-2):

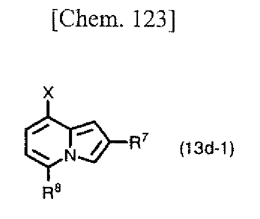

[wherein R⁷, R⁸, and X are as defined above]

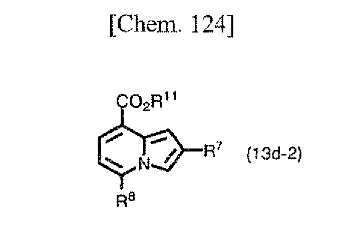

[wherein R⁷, R⁸, and R¹¹ are as defined above]

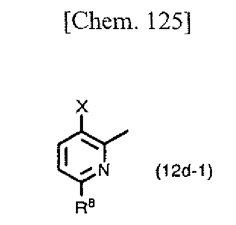

[wherein R⁸ and X are as defined above]

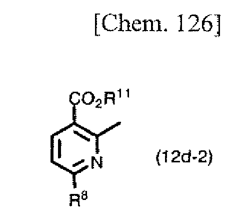

[wherein R⁸ and R¹¹ are as defined above].

The reaction can be carried out in the same manner as in Step G-1.

In Synthesis Pathway G', a compound represented by the general formula (14d-2) can be prepared by reducing the compound represented by the general formula (13d-2) (Step G'-2-2):

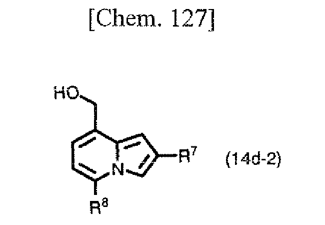

[wherein R⁷ and R⁸ are as defined above].

The reaction can be carried out in the same manner as in Step E'-3-4.

In Synthesis Pathway G', a compound represented by the general formula (14d-1) can be prepared by oxidizing the compound represented by the general formula (14d-2) (Step G'-3-1):

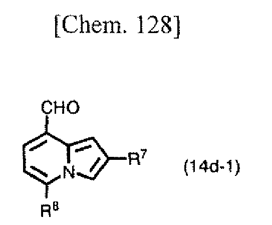

[wherein R⁷ and R⁸ are as defined above].

The reaction can be carried out in the same manner as in Step E'-4-1.

Further, the compound represented by the general formula (14d-1) can be prepared by treating the compound represented by the general formula (13d-1) with an organometalic reagent, followed by reacting with DMF or formic ester (Step G'-2-3).

The reaction can be carried out in the same manner as in Step E'-3-2.

Further, the compound represented by the general formula (14d-1) can also be prepared by reducing the compound represented by the general formula (13d-2) (Step G'-2-4).

The reaction can be carried out in the same manner as in Step E'-3-3.

In Synthesis Pathway G', a compound represented by the general formula (15d) can be prepared by reacting the compound represented by the general formula (14d-1) with the compound represented by the general formula (20) (Step G'-3-2):

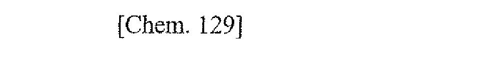

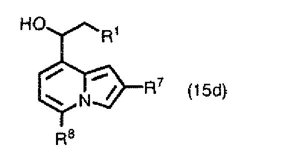

[wherein R¹, R⁷, and R⁸ are as defined above].

The reaction can be carried out in the same manner as in Step E'-4-2.

In Synthesis Pathway G', the compound represented by the general formula (9d) can be prepared by oxidizing the compound represented by the general formula (15d) (Step G'-4).

The reaction can be carried out in the same manner as in Step E'-4-1.

Further, the compound represented by the general formula (9d) can be prepared by treating the compound represented by the general formula (3d-1) with an organometalic reagent, followed by reacting with the compound represented by the general formula (12) or the compound represented by the general formula (13) (Step G'-2-1).

The reaction can be carried out in the same manner as in Step D-1-1.

In Synthesis Pathways G and G', the compound represented by the general formula (9d), wherein R⁸ is an alkoxy group having 1 to 6 carbon atoms, that is, a compound represented by the general formula (9d-1) can also be prepared by two methods as shown in Synthesis Pathway G" below:

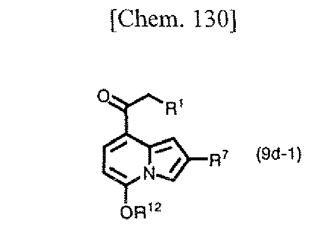

[wherein R¹, R⁷, and R¹² are as defined above].

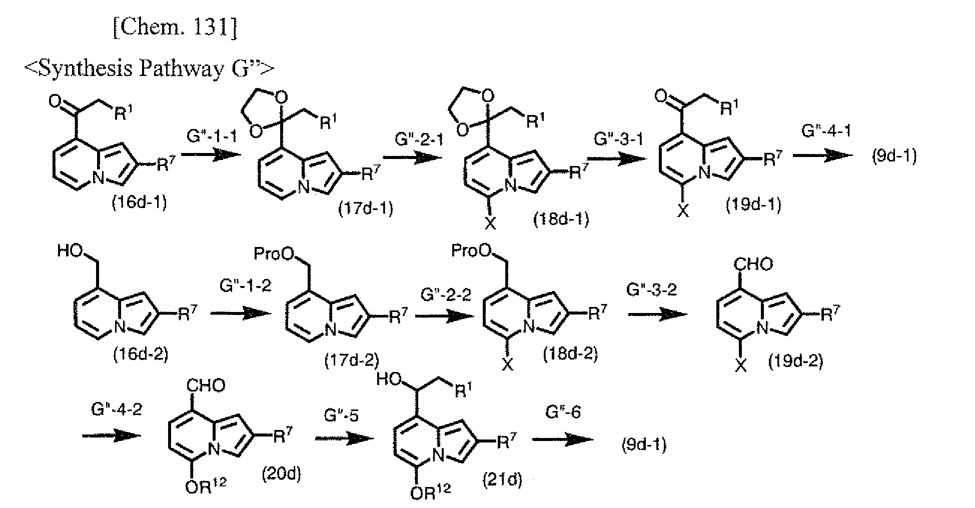

In Synthesis Pathway G", a compound represented by the general formula (17d-1) can be prepared by reacting the compound of the general formula (9d), wherein R⁸ is a hydrogen atom, that is, a compound represented by the general formula (16d-1) with ethylene glycol (Step G"-1-1):

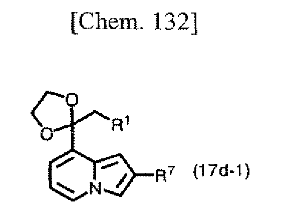

[wherein R¹ and R⁷ are as defined above].

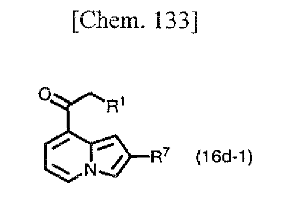

[wherein R¹ and R⁷ are as defined above].

The reaction can be carried out in the same manner as in Step E"-1-1.

In Synthesis Pathway G", a compound represented by the general formula (18d-1) can be prepared by halogenating the compound represented by the general formula (17d-1) (Step G"-2-1):

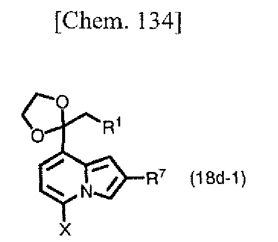

[wherein R¹, R⁷, and X are as defined above].

The reaction can be carried out in the same manner as in Step E"-2-1.

In Synthesis Pathway G", a compound represented by the general formula (13d-1) can be prepared by deprotecting the compound represented by the general formula (18d-1) (Step G"-3-1):

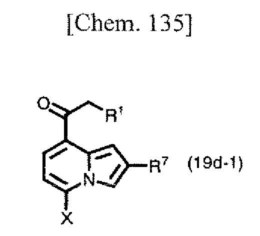

[wherein R¹, R⁷, and X are as defined above].

The reaction can be carried out in the same manner as in Step E"-3-1.

In Synthesis Pathway G", a compound represented by the general formula (17d-2) can be prepared by subjecting the compound of the general formula (14d-2), wherein R⁸ is a hydrogen atom, that is, a compound represented by the general formula (16d-2) to various reactions for introducing a protecting group (Step G"-1-2):

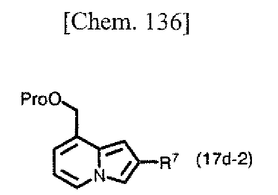

[wherein R⁷ and Pro are as defined above]

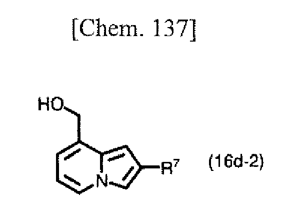

[wherein R⁷ is as defined above].

The reaction can be carried out in the same manner as in Step E"-1-2.

In Synthesis Pathway G", a compound represented by the general formula (18d-2) can be prepared by halogenating the compound represented by the general formula (17d-2) (Step G"-2-2):

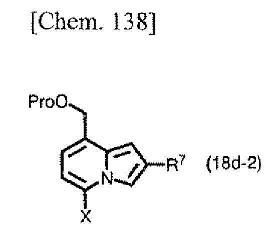

[wherein R⁷, X, and Pro are as defined above].

The reaction can be carried out in the same manner as in Step E"-2-1.

In Synthesis Pathway G", a compound represented by the general formula (19d-2) can be prepared by deprotecting and oxidizing the compound represented by the general formula (18d-2) (Step G"-3-2):

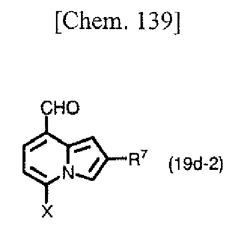

[wherein R⁷ and X are as defined above].

The reaction can be carried out in the same manner as in Step E"-3-2.

In Synthesis Pathway G", a compound represented by the general formula (20d) can be prepared by reacting the compound represented by the general formula (19d-2) with the compound represented by the general formula (21) (Step G"-4-2):

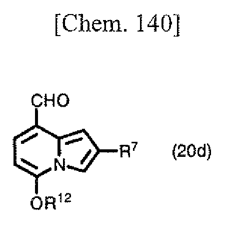

[wherein R⁷ and R¹² are as defined above].

The reaction can be carried out in the same manner as in Step E"-4-2.

In Synthesis Pathway G", a compound represented by the general formula (21d) can be prepared by reacting the compound represented by the general formula (20d) with the compound represented by the general formula (20) (Step G"-5):

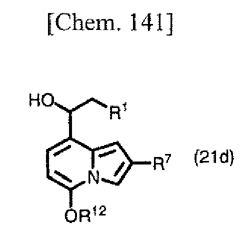

[wherein R¹, R⁷, and R¹² are as defined above].

The reaction can be carried out in the same manner as in Step E'-4-2.

In Synthesis Pathway G", the compound represented by the general formula (9d-1) can be prepared by oxidizing the compound represented by the general formula (21d) (Step G"-6).

The reaction can be carried out in the same manner as in Step E'-4-1.

Further, the compound represented by the general formula (9d-1) can be prepared by reacting the compound represented by the general formula (19d-1) with the compound represented by the general formula (21) (Step G"-4-1).

The reaction can be carried out in the same manner as in Step E"-4-2.
The compound represented by the general formula (3), wherein Heterocycle 1 is a benzofuran ring or a benzothiophene ring, and

[Chem. 142] - - -

is a single bond, that is, a compound represented by the general formula (3e) can be prepared via Synthesis Pathway H below:

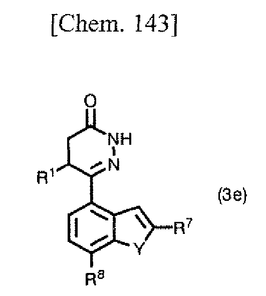

[wherein Y represents an oxygen atom or a sulfur atom, and R¹, R⁷, and R⁸ are as defined above].

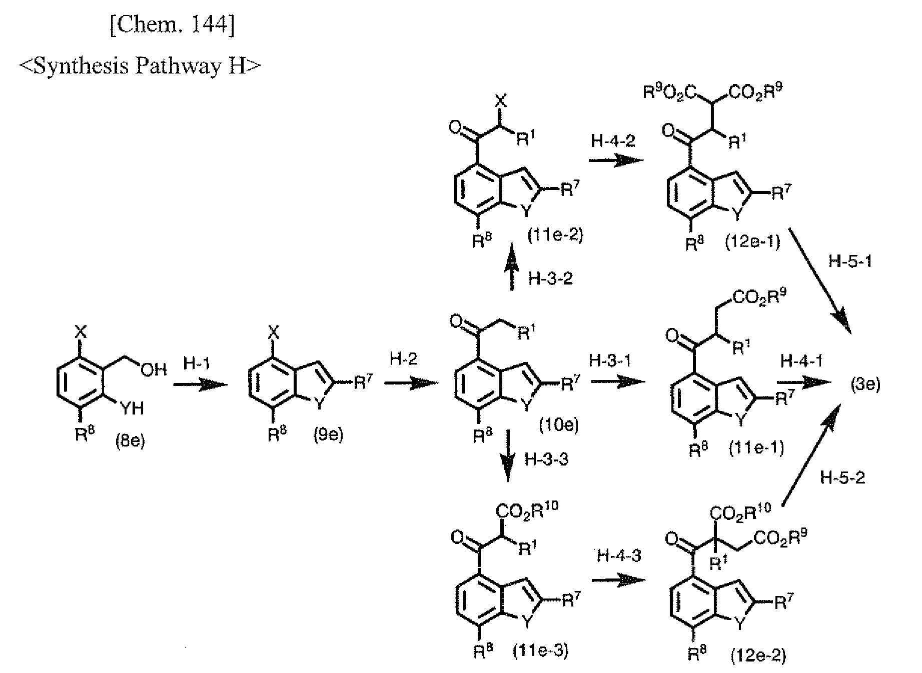

In Synthesis Pathway H, a compound represented by the general formula (9e) can be prepared by reacting the compound represented by the general formula (8e) with triphenylphosphonium bromide, followed by reacting with the compound represented by the general formula (19) (Step H-1):

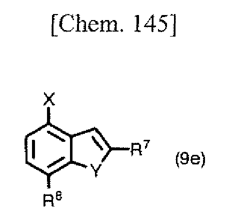

[wherein R⁷, R⁸, X, and Y are as defined above]

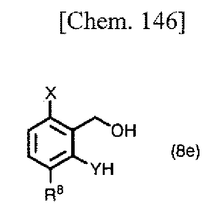

[wherein R⁸, X, and Y are as defined above].

The reaction is preferably carried out by performing the reaction to heating under reflux with the addition of triphenylphosphonium bromide, using a solvent such as acetonitrile, THF, 1,4-dioxane, ethyl acetate, and the like, and preferably acetonitrile, and then changing the reaction solvent to toluene, benzene, or xylene, and preferably toluene performing the reaction to heating under reflux with the addition of triethylamine and the compound represented by the general formula (19).

In Synthesis Pathway H, a compound represented by the general formula (10e) can be prepared by treating the compound represented by the general formula (9e) with an organometalic reagent, followed by reacting with the compound represented by the general formula (12) or the compound represented by the general formula (13) (Step H-2):

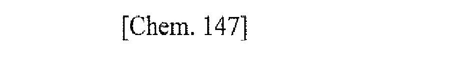

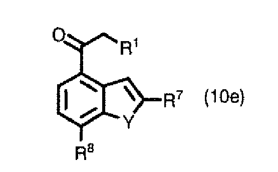

[wherein R¹, R⁷, R⁸, and Y are as defined above].

The reaction can be carried out in the same manner as in Step D-1-1.

In Synthesis Pathway H, a compound represented by the general formula (11e-1) can be prepared by treating the compound represented by the general formula (10e) with a base, followed by reacting with the compound represented by the general formula (16) (Step H-3-1):

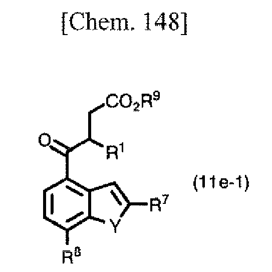

[wherein R¹, R⁷, R⁸, R⁹, and Y are as defined above].

The reaction can be carried out in the same manner as in Step D-2-1.

In Synthesis Pathway H, a compound represented by the general formula (11e-2) can be prepared by halogenating the compound represented by the general formula (10e) (Step H-3-2):

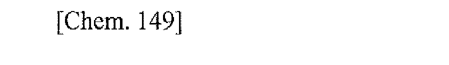

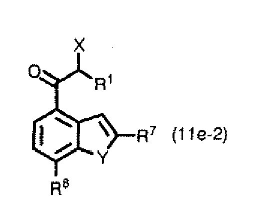

[wherein R¹, R⁷, R⁸, X, and Y are as defined above].

The reaction can be carried out in the same manner as in Step D-2-2.

In Synthesis Pathway H, a compound represented by the general formula (12e-1) can be prepared by allowing the compound represented by the general formula (11e-2) to act on the compound represented by the general formula (17) in the presence of a base (Step H-4-2):

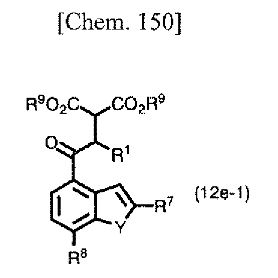

[wherein R¹, R⁷, R⁸, R⁹, and Y are as defined above].

The reaction can be carried out in the same manner as in Step D-3-2.

In Synthesis Pathway H, a compound represented by the general formula (11e-3) can be prepared by allowing the compound represented by the general formula (10e) to act on the compound represented by the general formula: (18) in the presence of a base (Step H-3-3):

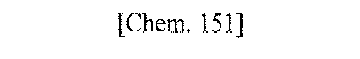

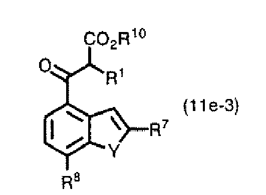

[wherein R¹, R⁷, R⁸, R¹⁰, and Y are as defined above].

The reaction can be carried out in the same manner as in Step D-2-3.

In Synthesis Pathway H, a compound represented by the general formula (12e-2) can be prepared by reacting the compound represented by the general formula (11e-3) with the compound represented by the general formula (16) in the presence of a base (Step H-4-3):

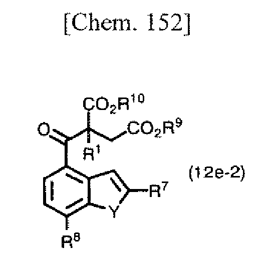

[wherein R¹, R⁷, R⁸, R⁹, R¹⁰, and Y are as defined above].

The reaction can be carried out in the same manner as in Step D-3-3.

In Synthesis Pathway H, the compound represented by the general formula (3e) can be prepared by hydrolyzing the compound represented by the general formula (11e-1) and then allowing a hydrazine derivative to react therewith, or by directly allowing a hydrazine derivative to react therewith (Step H-4-1).

The reaction can be carried out in the same manner as in Step D-3-1.

Further, the compound represented by the general formula (3e) can also be prepared by hydrolyzing the compound represented by the general formula (12e-1) and then allowing a hydrazine derivative to react therewith (Step H-5-1).

The reaction can be carried out in the same manner as in Step D-4-1.

Further, the compound represented by the general formula (3e) can also be prepared by hydrolyzing the compound represented by the general formula (12e-2) and then allowing a hydrazine derivative to react therewith (Step H-5-2).

The reaction can be carried out in the same manner as in Step D-4-2.

The compound represented by the general formula (3), wherein Heterocycle 1 is a benzoxazole ring or a benzothiazole ring and is linked at its position 7, and

[Chem. 153] - - -

is a single bond, that is, a compound represented by the general formula (3f) can be prepared via Synthesis Pathway J below:

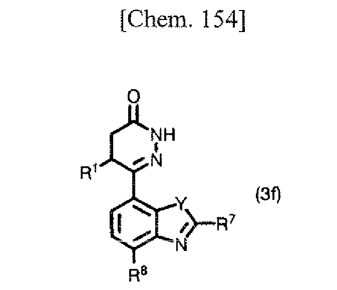

[wherein R¹, R⁷, R⁸, and Y are as defined above].

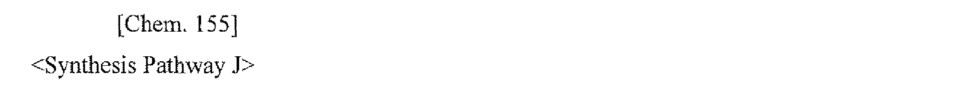

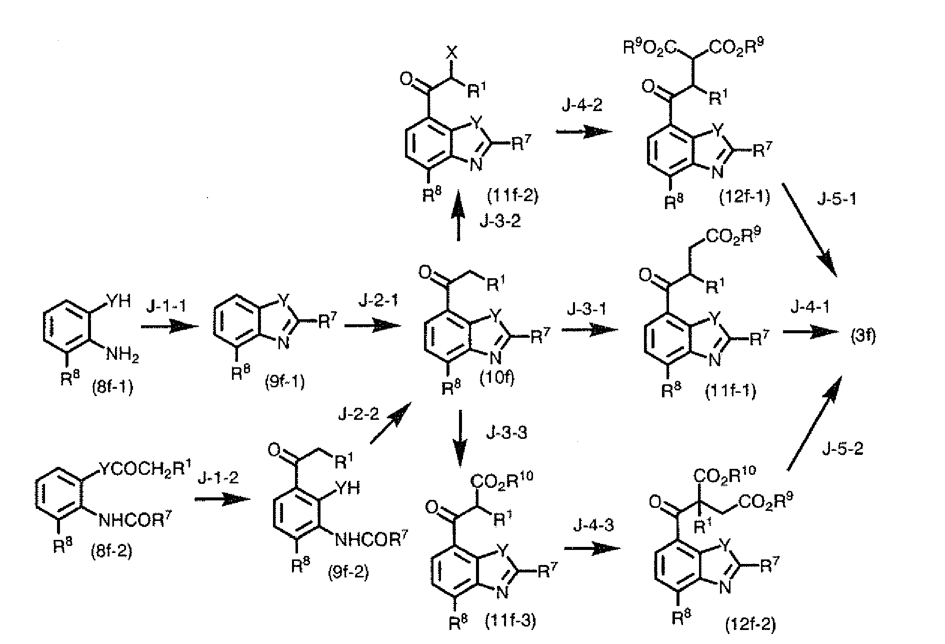

In Synthesis Pathway J, a compound represented by the general formula (9f-1) can be prepared by reacting the compound represented by the general formula (8f-1) with the compound represented by the general formula (19)(step J-1-1):

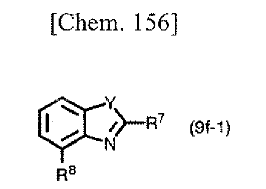

[wherein R⁷, R⁸, and Y are as defined above]

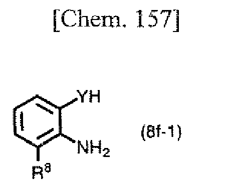

[wherein R⁸ and Y are as defined above].

The reaction is preferably carried out to heating under reflux while dehydrating, using a solvent such as toluene, benzene, xylene, and the like, and preferably toluene, with the addition of paratoluenesulfonic acid, pyridinium paratoluenesulfonate, or the like as a catalyst.

In Synthesis Pathway J, a compound represented by the general formula (9f-2) can be prepared by treating the compound represented by the general formula (8f-2) with a Lewis acid (Step J-1-2):

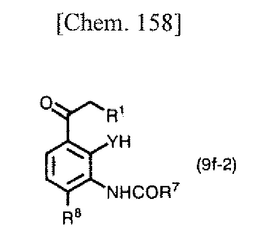

[wherein R¹, R⁷, R⁸, and Y are as defined above]

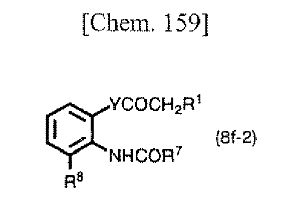

[wherein R¹, R⁷, R⁸, and Y are as defined above].

The reaction can be carried out at room temperature to 80°C using a solvent such as benzene, nitromethane, nitrobenzene, chlorobenzene, dichlorobenzene, methylene chloride, and the like, and preferably nitromethane and using a Lewis acid such as titanium tetrachloride, aluminum chloride, tin chloride, and the like, and preferably titanium tetrachloride.

In Synthesis Pathway J, a compound represented by the general formula (10f) can be prepared by reacting the compound represented by the general formula (9f-1) with the compound represented by the general formula (14) (Step J-2-1):

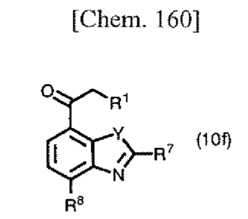

[wherein R¹, R⁷, R⁸, and Y are as defined above]

The reaction can be carried out in the same manner as in Step D-1-2.

Further, the compound represented by the general formula (10f) can also be prepared by dehydrating the compound represented by the general formula (9f-2) (Step J-2-2).

The reaction is preferably carried out to heating under reflux while dehydrating, using a solvent such as toluene, benzene, xylene, and the like, and preferably toluene, with the addition of paratoluenesulfonic acid, pyridinium, paratoluenesulfonate, or the like as a catalyst.

In Synthesis Pathway J, a compound represented by the general formula (11f-1) can be prepared by treating the compound represented by the general formula (10f) with a base, followed by reacting with the compound represented by the general formula (16) (Step J-3-1):

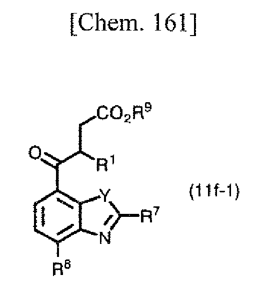

[wherein R¹, R⁷, R⁸, R⁹, and Y are as defined above].

The reaction can be carried out in the same manner as in Step D-2-1.

In Synthesis Pathway J, a compound represented by the general formula (11f-2) can be prepared by halogenating the compound represented by the general formula (10f) (Step J-3-2):

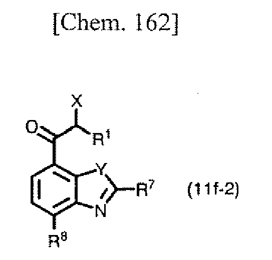

[wherein R¹, R⁷, R⁸, X, and Y are as defined above].

The reaction can be carried out in the same manner as in Step D-2-2.

In Synthesis Pathway J, a compound represented by the general formula (12f-1) can be prepared by allowing the compound represented by the general formula (11f-2) to act on the compound represented by the general formula (17) in the presence of a base (Step J-4-2):

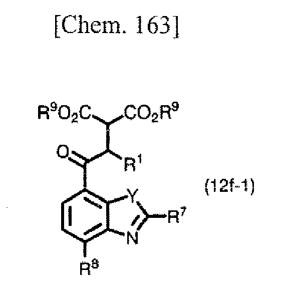

[wherein R¹, R⁷, R⁸, R⁹ and Y are as defined above].

The reaction can be carried out in the same manner as in Step D-3-2.

In Synthesis Pathway J, a compound represented by the general formula (11f-3) can be prepared by allowing the compound represented by the general formula (10f) to act on the compound represented by the general formula (18) in the presence of a base (Step J-3-3):

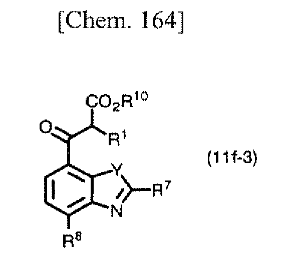

[wherein R¹, R⁷, R⁸, R¹⁰, and Y are as defined above].

The reaction can be carried out in the same manner as in Step D-2-3.

In Synthesis Pathway J, a compound represented by the general formula (12f-2) can be prepared by reacting the compound represented by the general formula (11f-3) with the compound represented by the general formula (16) in the presence of a base (Step J-4-3):

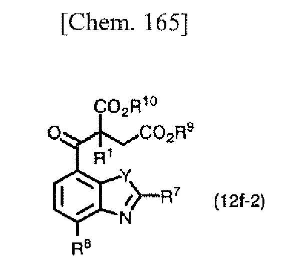

[wherein R¹, R⁷, R⁸, R⁹, R¹⁰, and Y are as defined above].

The reaction can be carried out in the same manner as in Step D-3-3.

In Synthesis Pathway J, a compound represented by the general formula (3f) can be prepared by hydrolyzing the compound represented by the general formula (11f-1) and then allowing a hydrazine derivative to react therewith, or by directly allowing a hydrazine derivative to react therewith (Step J-4-1).

The reaction can be carried out in the same manner as in Step D-3-1.

Further, the compound represented by the general formula (3f) can also be prepared by hydrolyzing the compound represented by the general formula (12f-1) and then allowing a hydrazine derivative to react therewith (Step J-5-1).

The reaction can be carried out in the same manner as in Step D-4-1.

Further, the compound represented by the general formula (3f) can also be prepared by hydrolyzing the compound represented by the general formula (12f-2), and then allowing it to act on a hydrazine derivative (Step J-5-2).

The reaction can be carried out in the same manner as in Step D-4-2. In the case using an acidic condition, the reaction can be carried out at 80°C to 100°C in hydrochloric acid or hydrobromic acid.

The compound represented by the general formula (3), wherein Heterocycle 1 is an imidazole ring and linked at its position 7, and

[Chem. 166] - - -

is a single bond, that is, a compound represented by the general formula (3g) can be prepared via Synthesis Pathway K as below:

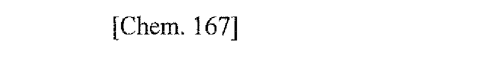

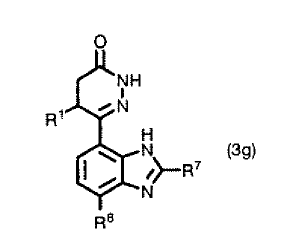

[wherein R¹, R⁷, and R⁸ are as defined above].

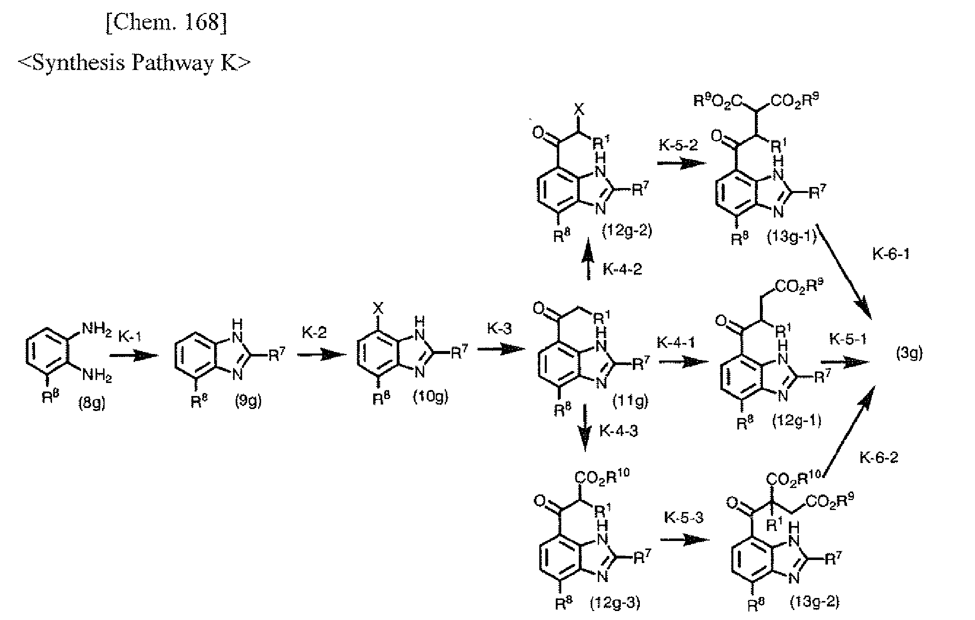

In Synthesis Pathway K, a compound represented by the general formula (9g) can be prepared by reacting the compound represented by the general formula (8g) with the compound represented by the general formula (19) (Step K-1):

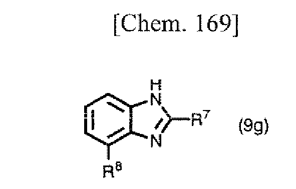

[wherein R⁷ and R⁸ are as defined above]

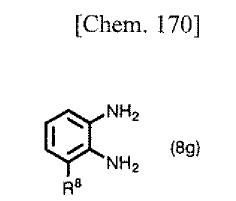

[wherein R⁸ is as defined above].

The reaction is preferably carried out to heating under reflux, using a solvent amount of the compound represented by the general formula (19).

In Synthesis Pathway K, a compound represented by the general formula (10g) can be prepared by halogenating the compound represented by the general formula (9g) (Step K-2):

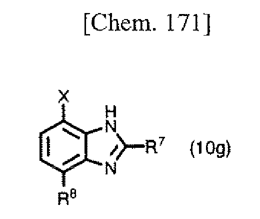

[wherein R⁷, R⁸, and X are as defined above].

The reaction can be carried out at room temperature using NCS, NBS, NIS, or the like as a halogenating agent and using methylene chloride, chloroform, carbon tetrachloride, or the like as a solvent.

In Synthesis Pathway K, a compound represented by the general formula (11 g) can be prepared by treating the compound represented by the general formula (10g) with an organometalic reagent, followed by reacting with the compound represented by the general formula (12) or the compound represented by the general formula (13) (Step K-3):

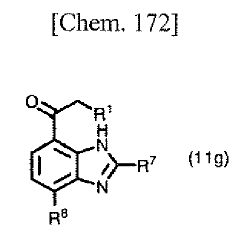

[wherein R¹, R⁷, and R⁸ are as defined above].

The reaction can be carried out in the same manner as in Step D-1-1.

In Synthesis Pathway K, a compound represented by the general formula (12g-1) can be prepared by treating the compound represented by the general formula (11g) with a base, followed by reacting with the compound represented by the general formula: (16) (Step K-4-1):

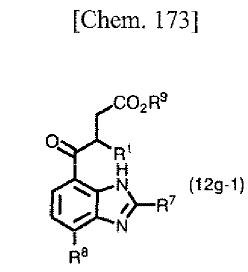

[wherein R¹, R⁷, R⁸, and R⁹ are as defined above].

The reaction can be carried out in the same manner as in Step D-2-1.

In Synthesis Pathway K, a compound represented by the general formula (12g-2) can be prepared by halogenating the compound represented by the general formula (11g) (Step K-4-2):

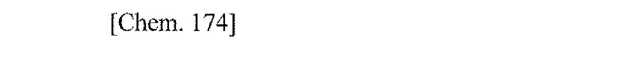

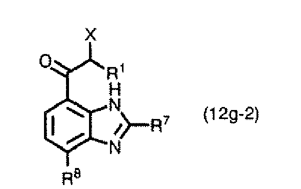

[wherein R¹, R⁷, R⁸, and X are as defined above].

The reaction can be carried out in the same manner as in Step D-2-2.

In Synthesis Pathway K, a compound represented by the general formula (13g-1) can be prepared by allowing the compound represented by the general formula (12g-2) to act on the compound represented by the general formula (17) in the presence of a base (Step K-5-2):

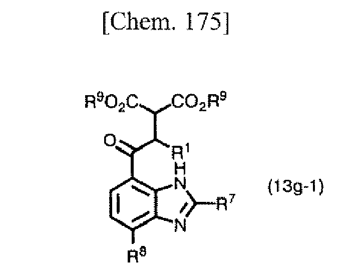

[wherein R¹, R⁷, R⁸, and R⁹ are as defined above].

The reaction can be carried out in the same manner as in Step D-3-2.

In Synthesis Pathway K, a compound represented by the general formula (12g-3) can be prepared by allowing the compound represented by the general formula (11g) to act on the compound represented by the general formula (18) in the presence of a base (Step K-4-3):

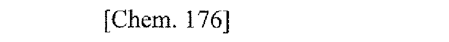

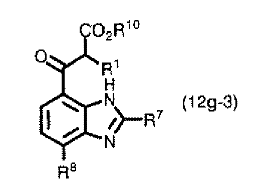

[wherein R¹, R⁷, R⁸, and R¹⁰ are as defined above].

The reaction can be carried out in the same manner as in Step D-2-3.

In Synthesis Pathway K, a compound represented by the general formula (13g-2) can be prepared by reacting the compound represented by the general formula (12g-3) with the compound represented by the general formula (16) in the presence of a base (Step K-5-3):

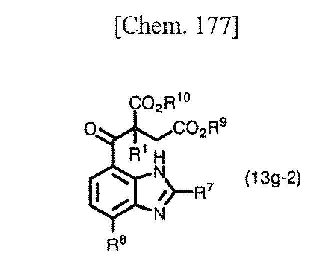

[wherein R¹, R⁷, R⁸, R⁹, and R¹⁰ are as defined above].

The reaction can be carried out in the same manner as in Step D-3-3.

In Synthesis Pathway K, a compound represented by the general formula (3g) can be prepared by hydrolyzing the compound represented by the general formula (12g-1) and then allowing a hydrazine derivative to react therewith, or by directly allowing a hydrazine derivative to react therewith (Step K-5-1).

The reaction can be carried out in the same manner as in Step D-3-1.

Further, the compound represented by the general formula (3g) can also be prepared by hydrolyzing the compound represented by the general formula (13g-1) and then allowing a hydrazine derivative to react therewith (Step K-6-1).

The reaction can be carried out in the same manner as in Step D-4-1.

Further, the compound represented by the general formula (3g) can also be prepared by hydrolyzing the compound represented by the general formula (13g-2) and then allowing a hydrazine derivative to react therewith (Step K-6-2).

The reaction can be carried out in the same manner as in Step D-4-2.

The compound represented by the general formula (3), wherein Heterocycle 1 is a benzoxazole ring or a benzothiazole ring and linked at its position 4, and

[Chem. 178] - - -

is a single bond, that is, a compound represented by the general formula (3h) can be prepared via Synthesis Pathway L below:

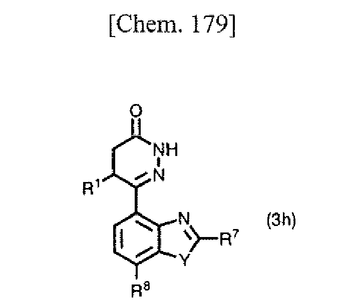

[wherein R¹, R⁷, R⁸, and Y are as defined above].

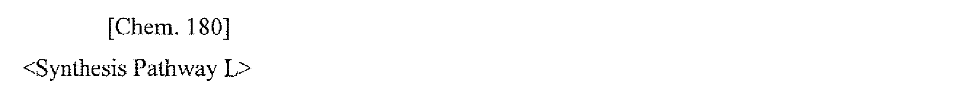

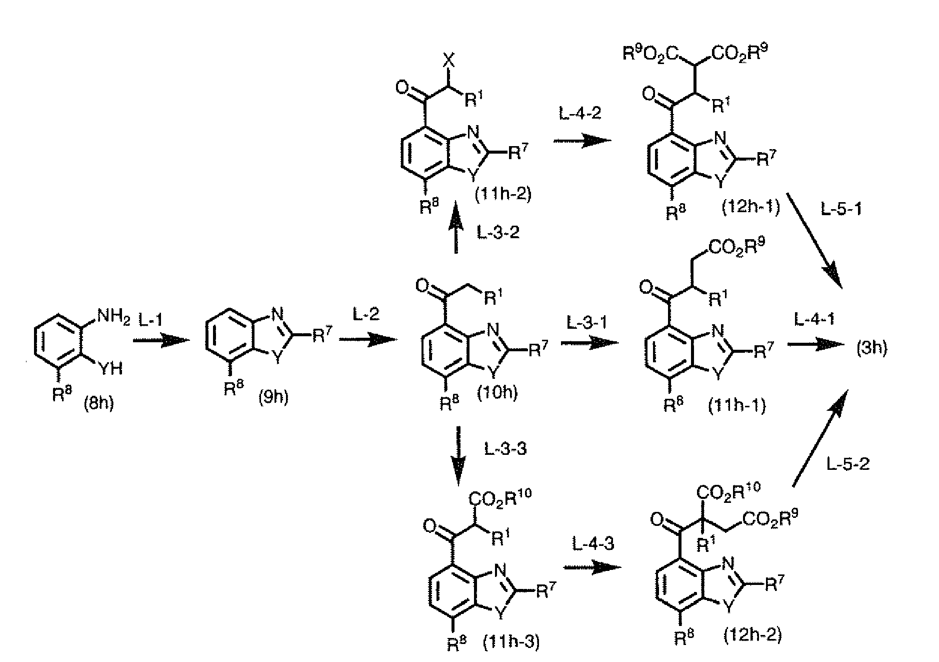

In Synthesis Pathway L, a compound represented by the general formula (9h) can be prepared by reacting the compound represented by the general formula (8h) with the compound represented by the general formula (19) (Step L-1):

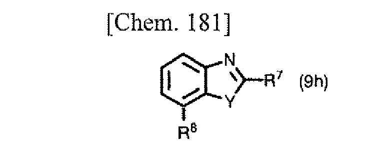

[wherein R⁷, R⁸, and Y are as defined above].

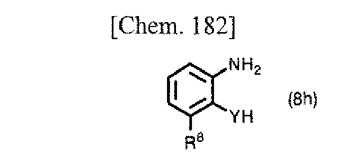

[wherein R⁸ and Y are as defined above].

The reaction can be carried out in the same manner as in Step J-1-1.

In Synthesis Pathway L, a compound represented by the general formula (10h) can be prepared by reacting the compound represented by the general formula (9h) with the compound represented by the general formula (14) (Step L-2):

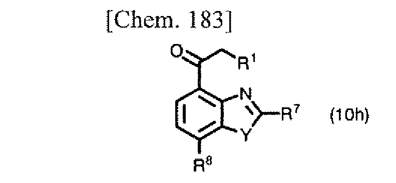

[wherein R¹, R⁷, R⁸, and Y are as defined above].

The reaction can be carried out in the same manner as in Step D-1-2.

In Synthesis Pathway L, a compound represented by the general formula (11h-1) can be prepared by treating the compound represented by the general formula (10h) with a base, followed by reacting with the compound represented by the general formula (16) (Step L-3-1):

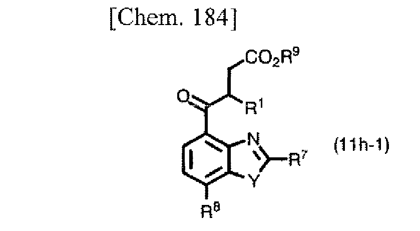

[wherein R¹, R⁷, R⁸, R⁹ and Y are as defined above].

The reaction can be carried out in the same manner as in Step D-2-1.

In Synthesis Pathway L, a compound represented by the general formula (11h-2) can be prepared by halogenating the compound represented by the general formula (10h) (Step L-3-2):

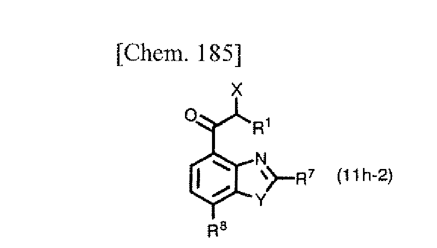

[wherein R¹, R⁷, R⁸, X, and Y are as defined above].

The reaction can be carried out in the same manner as in Step D-2-2.

In Synthesis Pathway L, a compound represented by the general formula (12h-1) can be prepared by allowing the compound represented by the general formula (11h-2) to act on the compound represented by the general formula (17) in the presence of a base (Step L-4-2):

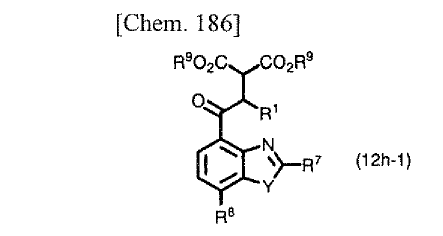

[wherein R¹, R⁷, R⁸, R⁹, and Y are as defined above].

The reaction can be carried out in the same manner as in Step D-3-2.

In Synthesis Pathway L, a compound represented by the general formula (11h-3) can be prepared by allowing the compound represented by the general formula (10h) to act on the compound represented by the general formula (18) in the presence of a base (Step L-3-3):

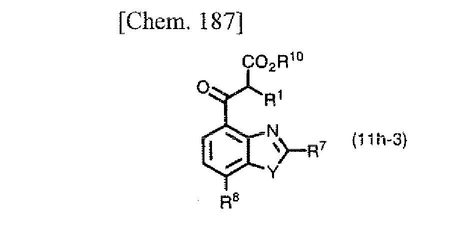

[wherein R¹, R⁷, R⁸, R¹⁰, and Y are as defined above].

The reaction can be carried out in the same manner as in Step D-2-3.

In Synthesis Pathway L, a compound represented by the general formula (12h-2) can be prepared by reacting the compound represented by the general formula (11h-3) with the compound represented by the general formula (16) in the presence of a base (Step L-4-3):

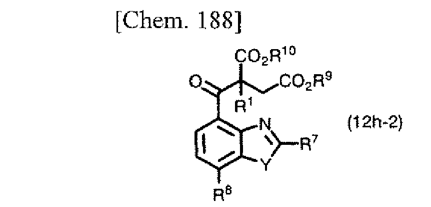

[wherein R¹, R⁷, R⁸, R⁹, R¹⁰, and Y are as defined above].

The reaction can be carried out in the same manner as in Step D-3-3.

In Synthesis Pathway L, a compound represented by the general formula (3h) can be prepared by hydrolyzing the compound represented by the general formula (11h-1) and then allowing a hydrazine derivative to react therewith, or by directly allowing a hydrazine derivative to react therewith (Step L-4-1).

The reaction can be carried out in the same manner as in Step D-3-1.

Further, the compound represented by the general formula (3h) can also be prepared by hydrolyzing the compound represented by the general formula (12h-1) and then allowing a hydrazine derivative to react therewith (Step L-5-1).

The reaction can be carried out in the same manner as in Step D-4-1.

Further, the compound represented by the general formula (3h) can also be prepared by hydrolyzing the compound represented by the general formula (12h-2) and then allowing a hydrazine derivative to react therewith (Step L-5-2).

The reaction can be carried out in the same manner as in Step D-4-2.

The compound represented by the general formula (3), wherein

[Chem. 189] - - -

is a double bond, that is, a compound represented by the general formula (3j)

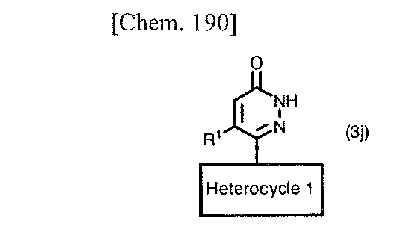

[wherein R¹ and Heterocycle 1 are as defined above] can be prepared by oxidizing the compounds represented by the general formulae (3a-1 to 3h), that is, the compound represented by the general formula (3k):

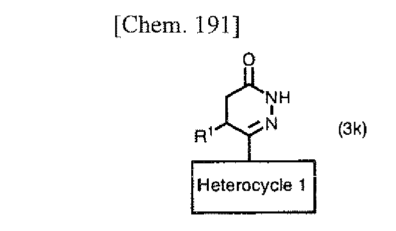

[wherein R¹ and Heterocycle 1 are as defined above].

The reaction can be carried out by allowing bromine to react at 50°C to 60°C in acetic acid as a solvent, or by reacting with copper (II) chloride at room temperature to under heating in acetonitrile. Further, the synthesis can be carried out by allowing m-nitrobenzenesodium sulfonate to act thereon at room temperature to heating temperature under reflux in an aqueous sodium hydroxide solution.

Further, a compound represented by the general formula (3j) can also be prepared via Synthesis Pathway M below.

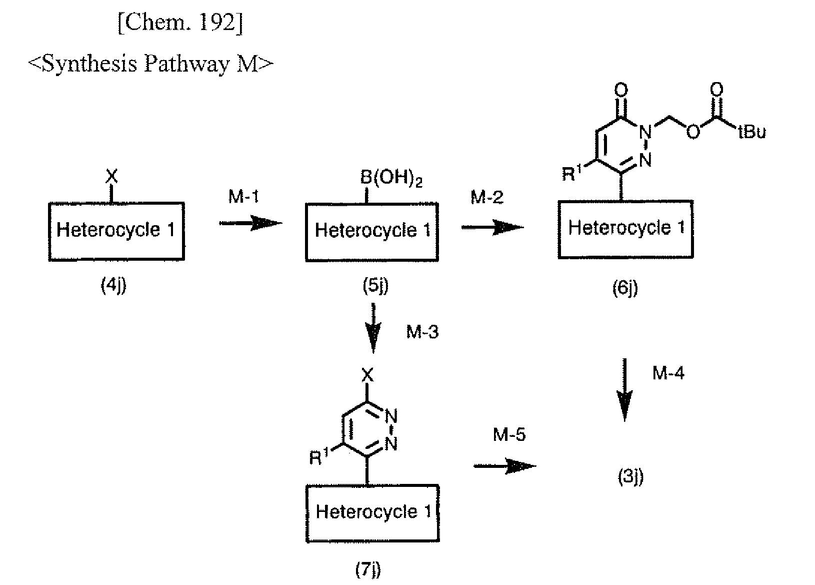

In Synthesis Pathway M, a compound represented by the general formula (5j) can be prepared by treating the compound represented by the general formula (4j) with an organometalic reagent, followed by reacting with the compound represented by the general formula (23) (Step M-1):

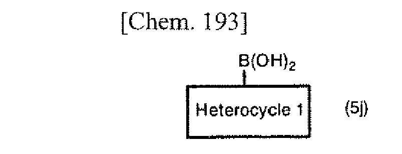

[wherein Heterocycle 1 is as defied above].

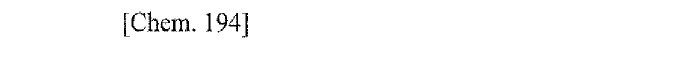

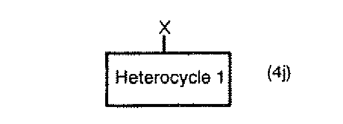

[wherein X and Heterocycle 1 are as defined above]

[Chem. 195] **B(OR)₃** (23)

[wherein R is an alkyl group having 1 to 6 carbon atoms].

The reaction is preferably carried out by allowing the compound represented by the general formula (4j) to act on LDA, n-butyl lithium, s-butyl lithium, or t-butyl lithium at -78°C in THF, or by adding magnesium, heating it under reflux, followed by reacting with the compound represented by the general formula (23) at -78°C to room temperature.

In Synthesis Pathway M, the compound represented by the general formula (6j) can be prepared by reacting the compound represented by the general formula (5j) with the compound represented by the general formula (24) (Step M-2):

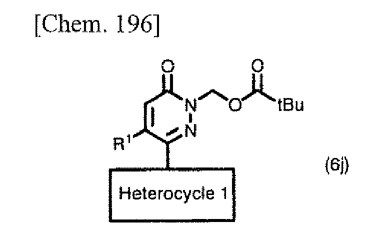

[wherein R¹ and Heterocycle 1 are as defined above]

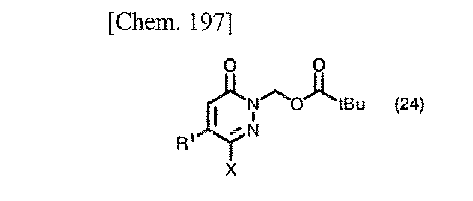

[wherein R¹ and X are as defined above].

The reaction is preferably carried out at 80°C to heating temperature under reflux in a solvent such as THF, benzene, toluene, xylene, 1,4-dioxane, and the like, using sodium carbonate, or cesium carbonate as a base, in the presence of a palladium catalyst such as tetrakistriphenyl phosphine palladium, and the like.

In Synthesis Pathway M, the compound represented by the general formula (7j) can be prepared by reacting the compound represented by the general formula (5j) with the compound represented by the general formula (25) (Step M-3):

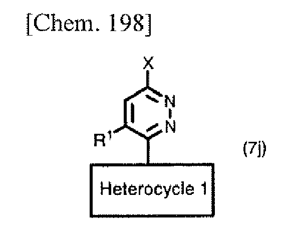

[wherein R¹, X, and Heterocycle 1 are as defined above]

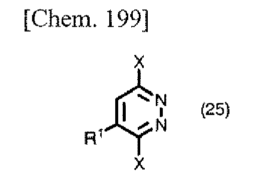

[wherein R¹ and X are as defined above].

The reaction can be carried out in the same manner as in Step M-2.

In Synthesis Pathway M, the compound represented by the general formula (3j) can be prepared by hydrolyzing the compound represented by the general formula (7j) (Step M-5).

The reaction is preferably carried out by heating to 80°C to 90°C in acetic acid.

Further, the compound represented by the general formula (3j) can also be prepared by hydrolyzing the compound represented by the general formula (6j) (Step M-4).

For the reaction, the synthesis can be carried out by allowing aqueous ammonia to react in a solvent such as methanol, ethanol, THF, and the like.

The compound represented by the general formula (3k) can also be prepared by reducing the compound represented by the general formula (3j).

The reaction is preferably carried out at 80°C to 90°C in acetic acid as a solvent with the addition of zinc.

The compound represented by the general formula (3), wherein

[Chem. 200] - - -

is a double bond and R¹ is a hydrogen atom, that is, a compound represented by the general formula (3m) can be prepared by reacting the compound represented by the general formula (4m) with glyoxylic acid, and then treating it with a hydrazine derivative.

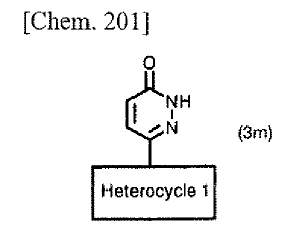

[wherein Heterocycle 1 is as defined above]

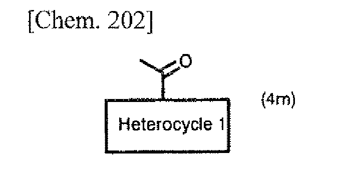

[wherein Heterocycle 1 is as defined above].

The reaction is preferably carried out at room temperature, and preferably 50°C to 80°C, with the addition of glyoxylic acid and an aqueous sodium hydroxide solution or an aqueous potassium hydroxide solution, using methanol or ethanol as a solvent. The subsequent cyclization reaction can be carried out by performing a reaction with hydrazine, hydrazine hydrochloride, or hydrazine acetate at room temperature or heating to reflux, using benzene, toluene, ethanol, or acetic acid, and preferably ethanol, as a reaction solvent.

### EXAMPLES

Hereinbelow, the present invention will be described with reference to specific Examples, but the present invention is not limited to these Examples.

### <Examples 1>

### 6-Amino-5-methoxy-2-picoline

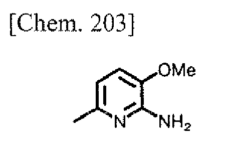

To a solution of 3-hydroxy-6-methyl-2-nitropyridine (9.76 g)in DMF (120 mL) were added potassium carbonate (14.0 g) and iodomethane (5.91 mL), followed by stirring at room temperature for 2 hours. To the reaction liquid was added water, followed by extraction with ethyl acetate, and the extract was then washed with water and a saturated brine solution, and then dried over anhydrous sodium sulfate. The extract was concentrated and then purified by silica gel column chromatography (hexane:ethyl acetate=4:1) to obtain a methyl ether form (10.1 g). To a solution thereof in ethyl acetate (300 mL) was added 10 % palladium-carbon (1.00 g), followed by stirring at room temperature for 4 hours under a hydrogen atmosphere. The insoluble materials were removed by filtration through Celite and the filtrate was concentrated to obtain the desired product (8.28 g) as a colorless powder. ¹H NMR (CDCl₃, 400 MHz): δ 2.33 (3H, s), 3.81 (3H, s), 4.59 (2H, brs), 6.45 (1H, d, J=7.9 Hz), 6.82 (1H, d, J=7.9 Hz).

### <Example 2>

### 6-di-(t-Butoxycarbonyl)amino-5-methoxy-2-picoline

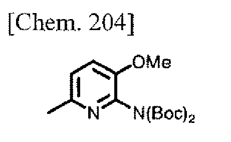

To a solution of the compound of Example 1 (3.00 g) in acetonitrile (100 mL) were added di-t-butyldicarbonate (28.4 g), triethylamine (4.39 g), and 4-dimethyl aminopyridine (100 mg), followed by stirring at room temperature for 8 hours. The reaction liquid was concentrated and extracted with ethyl acetate (500 mL), and the extract was washed with water and a saturated brine solution, and then dried over anhydrous sodium sulfate. The extract was concentrated and then purified by silica gel column chromatography (hexane:ethyl acetate=3:1) to obtain the desired product (5.80 g) as a colorless powder. ¹H NMR (CDCl₃, 400 MHz): δ 1.41 (18H, s), 2.48 (3H, s), 3.81 (3H, s), 7.07 (1H, d, J=8.6 Hz), 7.14 (1H, d, J=8.6 Hz).

### <Example 3>

### 6-di-(t-Butoxycarbonyl)amino-6-bromomethyl-3-methoxypyridine

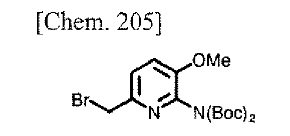

To a solution of the Example 2 compound (6.34 g) in carbon tetrachloride (50 mL) were added NBS (3.67 g) and benzoyl peroxide (20 mg), followed by heating under reflux for 4 hours under an argon atmosphere. The insoluble materials were removed by filtration and the filtrate was concentrated. The residue was purified by recrystallization (ethyl acetate/hexane) to obtain the desired product (6.33 g) as a colorless powder.
¹H NMR (CDCl₃, 400 MHz): δ 1.40 (18H, s), 3.86 (3H, s), 4.53 (2H, s), 7.21 (1H, d, J=8.6 Hz), 7.37 (1H, d, J=8.6 Hz).

### <Example 4>

### 6-di-(t-Butoxycarbonyl)amino-6-formyl-3-methoxypyridine

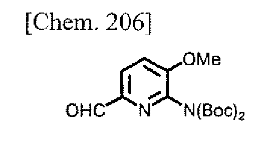

To N-methyl morpholine-N-oxide (3.55 g) and Molecular Sieve 4 A powders (5.00 g) was added acetonitrile (80 mL), and a solution of the compound in Example 3 (6.33 g) in acetonitrile (20 mL) was added thereto at room temperature under an argon atmosphere, followed by stirring for 4 hours. The reaction liquid was filtered through a silica gel pad and the filtrate was concentrated. The residue was purified by silica gel column chromatography (hexane:ethyl acetate=5: 1→3:1) to obtain the desired product (3.70 g) as a colorless powder. ¹H NMR (CDCl₃, 400 MHz): δ 1.42 (18H, s), 3.96 (3H, s), 7.35 (1H, d, J=8.5 Hz), 8.00 (1H, d, J=8.5 Hz), 9.94 (1H, s).

### <Example 5>

### 6-di-(t-Butoxycarbonyl)amino-5-methoxy-2-picolinic acid

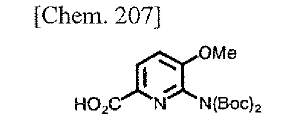

To a solution of the Example 4 compound (2.76 g) in t-butanol (80 mL) were added sodium chlorite (2.48 g), sodium dihydrogen phosphate dihydrate (1.22 g), 2-methyl-2-butene (3.7 mL), and water (25 mL), followed by stirring at room temperature for 4 hours. It was weakly acidified with 0.5 mol/L hydrochloric acid and extracted with ethyl acetate (500 mL). The extract was washed with water and a saturated brine, and then dried over anhydrous sodium sulfate, The extract was concentrated to obtain the desired product (2.95 g) as a colorless powder.
¹H NMR (CDCl₃, 400 MHz): δ 1.41 (18H, s), 3.97 (³H, s), 7.42 (1H, d, J=8.6 Hz), 8.22 (1H, d, J=8.6 Hz), 10.24 (1H, brs).

### <Example 6>

### 6-di-(t-Butoxycarbonyl)amino-5-methoxy-2-picolinic acid methoxymethyl amide

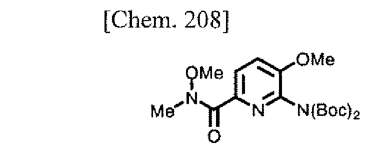

To a solution of the compound of Example 5 (1.62 g) in DMF (50 mL) were added 1-ethyl-3-(3-dimethylaminopropyl)-carbodiimide hydrochloride (1.27 g), N,O-dimethylhydroxylamine hydrochloride (0.644 g), 1-hydroxybenzotriazole (1.01 g), and diisopropyl ethylamine (3.45 mL), followed by stirring at room temperature for 2 hours. Water (300 mL) was added thereto, followed by extraction with ethyl acetate (500 mL). The extract was washed with water and saturated brine, and then dried over anhydrous sodium sulfate. The extract was concentrated and then purified by silica gel column chromatography (hexane:ethyl acetate= 1:1→ethyl acetate alone) to obtain the desired product (1.60 g) as a colorless amorphous.
¹H NMR (CDCl₃, 400 MHz): δ 1.40 (18H, s), 3.42 (3H, brs), 3.81 (3H, s), 3.90 (3H, s), 7.29 (1H, d, J=8.6 Hz), 7.82 (1H, brs).

### <Example 7>

### 3-Amino-4-methoxyacetophenone

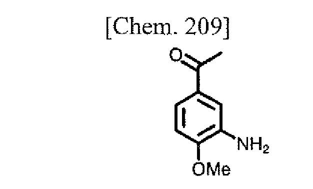

Paramethoxyacetophenone (15.0 g) was dissolved in concentrated sulfuric acid (100 mL), and a solution of sodium nitrite (9.34 g) in 50% sulfuric acid (50 mL) was added thereto at 0°C, followed by stirring at 0°C for 10 minutes. Ice water was added thereto and the precipitated solid was collected by filtration. The obtained solid was suspended in ethanol (500 mL), and 25% palladium-carbon (2.00 g) was added thereto, followed by stirring at room temperature for 1 hour under hydrogen atmosphere. Acetic acid (20 mL) was added thereto, followed by further stirring for 7 hours. The insoluble materials were filtered through Celite, and the solvent of the filtrate was then evaporated under reduced pressure. The residue was dissolved in ethyl acetate, neutralized using a saturated aqueous sodium hydrogen carbonate solution and potassium carbonate, and extracted three times with ethyl acetate, and the combined organic layer was washed with saturated brine, dried over sodium sulfate, and then filtered. The solvent of the filtrate was evaporated under reduced pressure and then purified by silica gel column chromatography (hexane:ethyl acetate=2:1→1:1) to obtain the desired product (6.58 g) as a colorless powder.
¹H NMR (CDCl₃, 400 MHz): δ 2.56 (3H, s), 3.95 (3H, s), 3.95 (2H, brs), 6.82 (1H, d, J=7.9 Hz), 7.37 (1H, d, J=2.4 Hz), 7.42 (1H, dd, J=7.9, 2.4 Hz).

### <Example 8>

### 2-Ethyl-8-methoxyquinoline

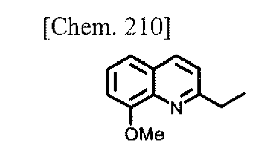

8-Hydroxy-2-methylquinoline (7.00 g) was dissolved in THF (100 mL), and tetrabutyl ammonium bromide (700 mg), iodomethane (8.20 mL), and a 50% aqueous sodium hydroxide solution (8.8 mL) were added thereto in this order, followed by stirring at room temperature for 6 hours. After evaporating THF under reduced pressure, the residue was extracted three times with ethyl acetate, and the combined extracted layer was washed with saturated brine and then dried over sodium sulfate. The solvent was evaporated under reduced pressure and then purified by silica gel column chromatography (hexane:ethyl acetate=1:2) to obtain 8-methoxy-2-methylquinoline (7.29 g) as a colorless powder.

The obtained 8-methoxy-2-methylquinoline (7.29 g) was dissolved in THF (210 mL) under an argon atmosphere, and a solution (2.71 mol/L, 17.1 mL) of n-butyl lithium in hexane was added thereto at -78°C, followed by stirring at 0°C for 30 minutes. Iodomethane (2.88 mL) was added thereto at -78°C, followed by stirring at room temperature for 3.5 hours. To the reaction liquid was added a saturated aqueous ammonium chloride solution, followed by extraction three times with ethyl acetate, and the combined extracted layer was washed with saturated brine and then dried over sodium sulfate. The solvent was evaporated under reduced pressure and then purified by silica gel column chromatography (hexane:ethyl acetate=2:1) to obtain the desired product (7.3 6 g) as a colorless powder.
¹H NMR (CDCl₃, 400 MHz): δ 1.40 (3H, t, J=7.3 Hz), 3.08 (2H, q, J=7.3 Hz), 4.08 (3H, s), 7.03 (1H, dd, J=7.3, 1.2 Hz), 7.34-7.40 (3H, m), 8.05 (1H, d, J=8.6 Hz).

### <Example 9>

### 8-Methoxyquinoline

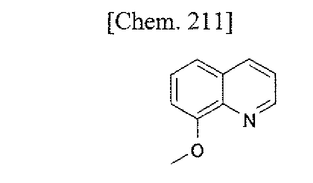

To a solution of 8-hydroxy quinoline (4.00 g) in DMF (60 mL) was added sodium hydride (60% content, 1.21 g) at 0°C, followed by stirring at room temperature for 1 hour. To the reaction liquid was added methyl iodide (2.57 mL), followed by stirring for 18 hours, and the solvent was the evaporated under reduced pressure. To the residue was added water, followed by extraction with ethyl acetate, the extracted layer was dried over anhydrous sodium sulfate, and the solvent was evaporated. The residue was purified by silica gel column chromatography (hexane:ethyl acetate=2:3) to obtain the desired product (3.50 g) as a colorless powder.
EIMS (+): 159 [M]⁺.
¹H NMR (CDCl₃, 400 MHz): δ 4.10 (3H, s), 7.06 (1H, dd, J=7.9, 1.2 Hz), (7.39-7.49 (3H, m), 8.14 (1H, dd, J=8.6, 1.8 Hz), 8.93 (1H, dd, J=4.3, 1.8 Hz).

### <Example 10>

### 8-Methoxy-2-trifluoromethylquinolin-4-one

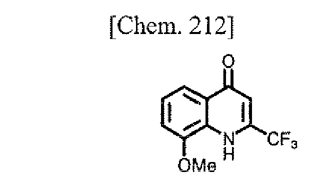

2-Methoxyaniline (5.00 mL) was dissolved in diphenyl ether (100 mL), and ethyl 3-trifluoromethylpropionate (8.10 g) was added thereto, followed by stirring at 100°C for 1 hour and at 250°C for 1 hour. After leaving to be cooled, hexane was added thereto, and the precipitated crystal was collected by filtration to obtain the desired product (9.78 g) as a yellow powder.
¹H NMR (CDCl₃, 400 MHz): δ 4.05 (3H, s), 6.63 (1H, s), 7.13 (1H, dd, J=8.2, 1.2 Hz), 7.34 (1H, dd, J=8.2, 8.2 Hz), 7.90 (1H, dd, J=8.2, 1.2 Hz), 8.71 (1H, brs).

### <Example 11>

### 4-Chloro-8-methoxy-2-trifluoromethylquinoline

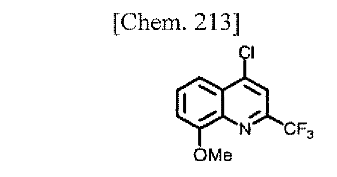

The compound of Example 10 (5.0 g) was dissolved in phosphorous oxychloride (100 mL) under an argon atmosphere, followed by stirring for 2 hours under the condition of heating to reflux. The phosphorous oxychloride was evaporated under reduced pressure, and a saturated aqueous sodium hydrogen carbonate solution was added thereto, followed by extraction three times with ethyl acetate. The combined extracted layer was washed with saturated brine and then dried over sodium sulfate. After evaporating the solvent under reduced pressure, it was purified by silica gel column chromatography (hexane:ethyl acetate=10:1) to obtain the desired product (5.17 g) as a colorless powder. ¹H NMR (CDCl₃, 400 MHz): δ 4.12 (3H, s), 7.20 (1H, d, J=7.9 Hz), 7.70 (1H, dd, J=7.9, 7.9 Hz), 7.85-7.87 (2H, m).

### <Example 12>

### 8-Methoxy-2-trifluoromethylquinoline

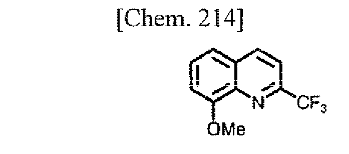

The compound of Example 11 (5.17 g) was dissolved in ethanol (100 mL), and 25% palladium-carbon (500 mg) was added thereto, followed by replacing with hydrogen and then stirring at room temperature for 2 hours. The reaction liquid was filtered, and the solvent of the filtrate was evaporated under reduced pressure and then purified by silica gel column chromatography (hexane:ethyl acetate=15:1) to obtain 8-methoxy-2-trifluoromethyl-1,2,3,4-tetrahydroquinoline (3.60 g) as a yellow oil and the desired product (814 mg) as a colorless powder. 8-Methoxy-2-trifluoromethyl-1,2,3,4-tetrahydroquinoline (3.60 g) was dissolved in acetic acid (70 mL), and bichromate potassium (2.75 g) was added thereto, followed by stirring at room temperature for 1 hour and at 90°C for 3 hours. It was neutralized with an aqueous sodium hydroxide solution and then extracted three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=15:1) to obtain the desired product (2.24 g) as a colorless powder.
¹H NMR (CDCl₃, 400 MHz): δ 4.11 (3H, s), 7.15 (1H, d, J=8.6 Hz), 7.47 (1H, d, J=8.6 Hz), 7.60 (1H, d, J=8.6 Hz), 7.77 (1H, d, J=8.6 Hz), 8.33 (1H, d, J=8.6 Hz).

### <Example 13>

### 5-Bromo-2-ethyl-8-methoxyquinoline

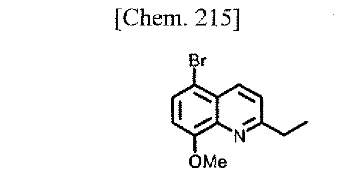

The compound of Example 8 (7.36 g) was dissolved in methanol (80 mL), and bromine (2.10 mL) was added thereto, followed by stirring at room temperature for 45 minutes. A saturated aqueous sodium hydrogen carbonate solution was added thereto, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=2:1) to obtain the desired product (9.55 g) as a yellow powder.
¹H NMR (CDCl₃, 400 MHz): δ 1.41 (3H, t, J=7.3 Hz), 3.12 (2H, q, J=7.3 Hz), 4.08 (3H, s), 6.92 (1H, d, J=8.6 Hz), 7.47 (1H, d, J=8.6 Hz), 7.66 (1H, d, J=8.6 Hz), 8.42 (1H, d, J= 8.6 Hz).

### <Example 14>

### 5-Bromo-8-methoxyquinoline

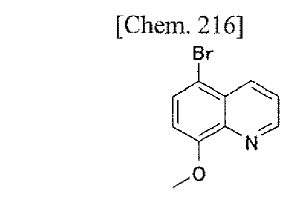

To a solution of the compound of Example 9 (1.22 g) in methanol (60 mL) was added bromine (0.425 mL) at 0°C, followed by stirring at room temperature for 2 hours. The reaction liquid was poured into a mixed liquid of a 5% aqueous sodium thiosulfate solution and a 5% aqueous sodium hydrogen carbonate solution, followed by extraction with ethyl acetate, and the extracted layer was dried over anhydrous sodium sulfate, and the solvent was evaporated. The residue was purified by silica gel column chromatography (hexane:ethyl acetate=1:2) to obtain the desired product (1.64 g) as a colorless powder. EIMS (+): 237 [M]⁺.
¹NMR (CDCl₃, 400 MHz): δ 4.10 (3H, s), 6.95 (1H, d, J=8.6 Hz), 7.56 (1H, dd, J=8.6, 4.3 Hz), 7.75 (1H, d, J=8.6 Hz), 8.51 (1H, dd, J=8.6, 1.8 Hz), 8.96 (1H, dd, J=4.3, 1.8 Hz).

### <Example 15>

### 5-Bromo-8-methoxy-2-trifluoromethylquinoline

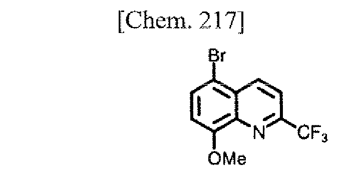

The compound of Example 12 (3.05 g) was dissolved in methanol (30 mL), and bromine (0.763 mL) was added thereto, followed by stirring at room temperature for 50 minutes. A saturated aqueous sodium hydrogen carbonate solution was added thereto, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=15:1) to obtain the desired product (3.86 g) as a red powder.
¹H NMR (CDCl₃, 400 MHz): δ 4.10 (3H, s), 7.03 (1H, d, J=8.6 Hz), 7.86 (1H, d, J=8.6 Hz), 7.87 (1H, d, J=8.6 Hz), 8.71 (1H, d, J=8.6 Hz).

### <Example 16>

### 5-Bromo-8-methoxy-2-methylquinoline

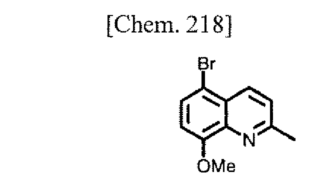

8-Methoxy-2-methylquinoline (7.92 g) was dissolved in methanol (80 mL), and bromine (2.37 mL) was added dropwise thereto under ice cooling, followed by stirring at room temperature for 1.5 hours. To the reaction liquid was added a saturated aqueous sodium thiosulfate solution and further added a saturated aqueous sodium hydrogen carbonate solution, followed by evaporating methanol under reduced pressure. This aqueous solution was extracted with ethyl acetate, and the organic layer was washed with water and saturated brine in this order, and then dried over anhydrous sodium sulfate. After evaporating the solvent, the desired product (47.6 g) was obtained as a yellow powder.
¹H NMR (CDCl₃, 400 MHz): δ 2.83 (3H, s), 4.07 (3H, s), 6.92 (1H, d, J=8.6 Hz), 7.42 (1H, d, J=8.6 Hz), 7.66 (1H, d, J=8.6 Hz), 8.38 (1H, d, J=8.6 Hz).

### <Example 17>

### 5-Bromo-8-methoxy-2-isopropylquinoline

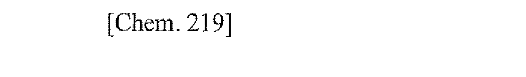

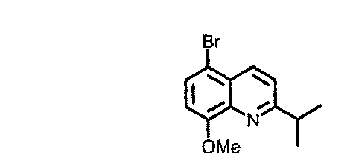

8-Methoxy-2-isopropylquinoline (4.09 g) was dissolved in methanol (35.2 mL), and bromine (1.15 mL) was added dropwise thereto under ice cooling, followed by stirring at room temperature for 2 hours. To the reaction liquid was added a saturated aqueous sodium thiosulfate solution and further added a saturated aqueous sodium hydrogen carbonate solution, followed by evaporating methanol under reduced pressure. This aqueous solution was extracted with ethyl acetate, and the organic layer was washed with water and saturated brine in this order, and then dried over anhydrous sodium sulfate. After evaporating the solvent, the residue was purified by silica gel column chromatography (hexane:ethyl acetate=2:1) to obtain the desired product (5.00 g) as a yellow powder.
¹H NMR (CDCl₃, 400 MHz): δ 1.39 (6H, d, J=8.0 Hz), 3.36-3.46 (1H, m), 4.06 (3H, s), 6.90 (1H, d, J=8.6 Hz), 7.49 (1H, d, J=8.6 Hz), 7.64 (1H, d, J=8.6 Hz), 8.43 (1H, d, J=8.6 Hz).

### <Example 18>

### 2-Ethyl-5-iodo-8-methoxyquinoline

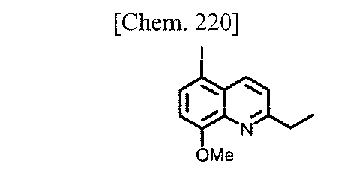

To a solution of the compound of Example 8 (3.00 g) in methylene chloride (50 mL) were added silver trifluoromethanesulfonate (4.94 g) and iodine (4.88 g), followed by stirring at room temperature for 1 hour under light shielding. To the reaction liquid was added a saturated aqueous sodium thiosulfate solution and further added a saturated aqueous sodium hydrogen carbonate solution. The insoluble materials were removed by filtration and the filtrate was then extracted with ethyl acetate (300 mL). The extract was washed with water and saturated brine, and then dried over anhydrous sodium sulfate. The extract was concentrated to obtain the desired product (5.01 g) as a pale yellow solid.
¹H NMR (CDCl₃, 400 MHz): δ 1.40 (3H, t, J=7.3 Hz), 3.11 (2H, q, J=7.3 Hz), 4.07 (3H, s), 6.82 (1H, d, J=8.6 Hz), 7.42 (1H, d, J=8.6 Hz), 7.93 (1H, d, J=8.6 Hz), 8.26 (1H, d, J=8.6 Hz).

### <Example 19>

### Ethyl 2-ethylindolidine-8-carboxylate ester

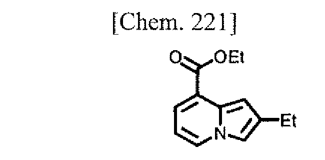

Ethyl 2-methylnicotinate (3.77 mL) was dissolved in ethyl acetate (2.5 mL), and bromomethyl ethyl ketone (2.5 mL) was added thereto, followed by stirring at 70°C for 7 hours. After evaporating the solvent under reduced pressure, it was dissolved in toluene (25 mL), and 1,8-diazabicyelo[5,4,0]undeca-7-ene (8.06 mL) was added thereto, followed by stirring for 1 hour under the condition of heating to reflux. Cold water was added thereto, followed by extracted three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=9:1) to obtain the desired product (3.00 g) as a brown oil.
¹H NMR (CDCl₃, 400 MHz): δ 1.31 (3H, t, J=7.3 Hz), 1.44 (3H, t, J=7.3 Hz), 2.75 (2H, q, J=7.3 Hz), 4.42 (2H, q, J=7.3 Hz), 6.46 (1H, dd, J=7.3, 7.3 Hz), 6.96 (1H, s), 7.21 (1H, s), 7.54 (1H, d, J=7.3 Hz), 8.00 (1H, d, J=7.3 Hz).

### <Example 20>

### 2-Ethylindolidine-8-carboxylic acid methoxymethyl amide

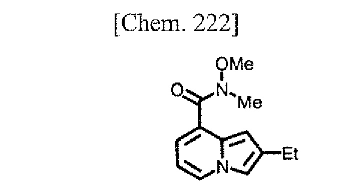

The compound of Example 19 (3.00 g) was dissolved in ethanol (100 mL), and water (50 mL) and potassium hydroxide (2.31 g) were added thereto, followed by stirring for 2.5 hours under the condition of heating to reflux. 1 mol/L Hydrochloric acid was added thereto to adjust the pH to 4-5, followed by extraction three times with ethyl acetate, and the combined extracted layer was washed with saturated brine, dried over sodium sulfate, and then filtered. The solvent of the filtrate was evaporated under reduced pressure to obtain a carboxylic acid (2.52 g) as a yellow powder. The obtained carboxylic acid (2.52 g) was dissolved in DMF (130 mL), and diisopropyl ethylamine (10.4 mL), 1-ethyl-3-(3-dimethylaminopropyl)-carbodiimide hydrochloride (3.83 g), 1-hydroxybenzotriazole (2.70 g), and N,O-dimethylhydroxylamine hydrochloride (1.95 g) were added thereto, followed by stirring at room temperature for 5 hours. To the reaction liquid was added water, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=3:2) to obtain the desired product (2.76 g) as a yellow oil.
¹H NMR (CDCl₃, 400 MHz): δ 1.28 (3H, t, J=7.3 Hz), 2.71 (2H, q, J=7.3 Hz), 3.35 (3H, s), 3.66 (3H, s), 6.39 (1H, s), 6.42 (1H, dd, J=6.7, 6.7 Hz), 6.80 (1H, d, J=6.7 Hz), 7.18 (1H, s), 7.86 (1H, d, J=6.7 Hz).

### <Example 21>

### Ethyl 2-aminonicotinate ester

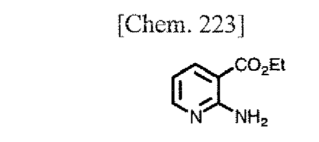

2-Aminonicotinic acid (24.8 g) was dissolved in acetone (540 mL), and iodoethane (43.1 mL) and potassium carbonate (124 g) were added thereto, followed by stirring for 16 hours under the condition of heating to reflux, and iodoethane (29.0 mL) was added thereto, by further stirring for 15 hours. After removing the insoluble materials by filtration, the solvent of the filtrate was evaporated under reduced pressure, and the obtained residue was recrystallized from ethyl acetate to obtain the desired product (17.3 g). The mother liquid was purified by silica gel column chromatography (hexane:ethyl acetate=2:1→1:1) to obtain the desired product (1.60 g), and a combined product thereof (18.9 g) as yellow powders. ¹H NMR (CDCl₃, 400 MHz): δ 1.38 (3H, t, J=7.3 Hz), 4.34 (2H, q, J=7.3 Hz), 6.62 (1H, dd, J=7.9, 4.9 Hz), 8.13 (1H, dd, J=7.9, 1.8 Hz), 8.21 (1H, dd, J=4.9, 1.8 Hz).

### <Example 22>

### N-Amino-2-amino-3-ethoxycarbonylpyridiniummesitylensulfonate

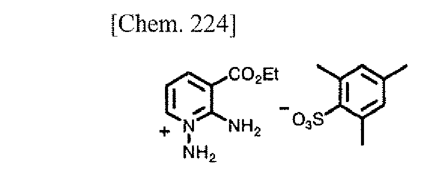

Ethyl mesitylsulfonylacetohydroxamate ester (28.3 g) was dissolved in 1,4-dioxane (40 mL), and 70% perchloric acid (14 mL) was added thereto at 0°C, followed by stirring for 30 minutes. To the reaction liquid was added cold water, and the precipitated solid was then collected by filtration and dissolved in methylene chloride. After removing the aqueous layer by a liquid separation operation, the methylene chloride layer was washed with saturated brine, dried over anhydrous sodium sulfate, and filtered. The filtrate was added to a solution of the compound of Example 21 (13.8 g) in methylene chloride (50 mL) at 0°C, followed by stirring at room temperature for 1 hour. The solvent was evaporated under reduced pressure, and diethyl ether was added thereto. The precipitated crystal was collected by filtration to obtain the desired product (29.0 g) as a yellow powder.
¹H NMR (DMSO-d₆, 400 MHz): δ 1.34 (3H, t, J= 7.3 Hz), 2.17 (3H, s), 2.50 (6H, s), 4.37 (2H, q, J=7.3 Hz), 6.74 (2H, s), 6.96 (2H, brs), 7.00 (1H, t, J=6.7 Hz), 8.41 (1H, dd, J=6,7, 1.2 Hz), 8.53 (1H, d, J=6.7 Hz), 8.75 (2H, brs).

### <Example 23>

### Ethyl 2-trifluoromethyl-[1,2,4]triazolo[1,5-a]pyridine-8-carboxylate ester

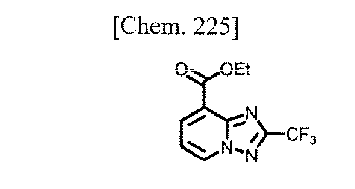

The compound of Example 22 (10.0 g) was dissolved in toluene (75 mL), and triethylamine (12.5 mL) and trifluoroacetic anhydride (5.60 mL) were added thereto, followed by stirring for 13 hours under the condition of heating to reflux. After evaporating the solvent under reduced pressure, a saturated aqueous sodium hydrogen carbonate solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=3:2) to obtain the desired product (5.34 g) as a colorless powder.
¹H NMR (CDCl₃, 400 MHz): δ 1.47 (3H, t, J=7.3 Hz), 4.54 (2H, q, J=7.3 Hz), 7.30 (1H, dd, J=7.3, 7.3 Hz), 8.39 (1H, dd, J=7.3, 1.2 Hz), 8.81 (1H, dd, J=7.3, 1.2 Hz).

### <Example 24>

### 8-t-Butyldimethylsiloxymethyl-2-trifluoromethyl-[1,2,4]triazolo[1,5-a]pyridine

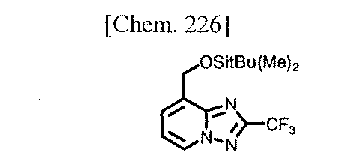

The compound of Example 23 (5.03 g) was dissolved in THF (150 mL) under an argon atmosphere, and a solution (0.95 mol/L, 40.9 mL) of diisobutylaluminum hydride in hexane was slowly added thereto at -10°C. 1 mol/L Hydrochloric acid was added thereto, followed by extraction three times with ethyl acetate, and the combined extracted layer was washed with saturated brine, dried over anhydrous sodium sulfate, and then filtered. The solvent of the filtrate was evaporated under reduced pressure, and the residue was dissolved in DMF (100 mL) under an argon atmosphere, and imidazole (3.30 g) and chloro-t-butyldimethylsilane (3.51 g) were added thereto at 0°C, followed by stirring at room temperature for 1 hour. To the reaction liquid was added water, followed by extraction three times with acetic acid, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=30:1) to obtain the desired product (5.90 g) as a colorless powder.
¹H NMR (CDCl₃, 400 MHz): δ 0.17 (6H, s), 0.99 (9H, s), 5.17 (2H, s), 7.22 (1H, dd, J=6.7, 6.7 Hz), 7.80 (1H, dd, J=6.7, 1.2 Hz), 8.53 (1H, dd, J=6.7, 1.2 Hz).

### <Example 25>

### 8-t-Butyldimethylsiloxymethyl-5-iodo-2-trifluoromethyl-[1,2,4]triazoto[1,5-a]pyridine

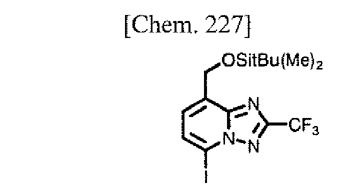

The compound of Example 24 (5.90 g) was dissolved in THF (120 mL) under an argon atmosphere, and a solution (2.71 mol/L, 7.23 mL) of n-butyl lithium in hexane was added thereto at -78°C, followed by stirring for 30 minutes. 1,2-Diiodoethane (5.52 g) was added thereto, followed by stirring at -78°C for 2.5 hours. A saturated aqueous sodium hydrogen carbonate solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=30:1) to obtain the desired product (7.64 g) as a yellow powder.
¹H NMR (CDCl₃, 400 MHz): δ 0.17 (6H, s), 0.96 (9H, s), 5.14 (2H, d, J=1.2 Hz), 7.55 (1H, dt, J= 1.2, 7.9 Hz), 7.68 (1H, d, J=7.9 Hz).

### <Example 26>

### 5-Methoxy-2-trifluoromethyl-[1,2,4]triazolo[1,5-a]pyridine-8-carboaldehyde

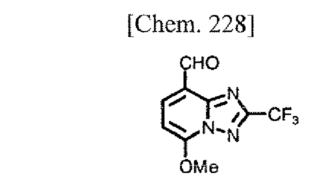

The compound of Example 25 (7.64 g) was dissolved in THF (100 mL) under an argon atmosphere, and a solution (1.0 mol/L, 33.4 mL) of tetrabutyl ammonium fluoride in THF was added thereto at 0°C, followed by stirring at room temperature for 1 hour. To the reaction liquid was added water, followed by extraction three times with acetic acid, and the combined extracted layer was washed with saturated brine, dried over anhydrous sodium sulfate, and then filtered. The solvent of the filtrate was evaporated under reduced pressure and then to the residue were added methylene chloride (150 mL) and active manganese dioxide (14.5 g), followed by stirring at 60°C for 5 hours. The insoluble materials were removed by filtration through Celite, the solvent of the filtrate was evaporated under reduced pressure, and the residue was then dissolved in methanol (100 mL) under an argon atmosphere. Sodium methoxide (3.61 g) was added thereto, followed by stirring for 2 hours under the condition of heating to reflux. A saturated aqueous ammonium chloride solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=1:1→2:3) to obtain the desired product (1.43 g) as a yellow powder.
¹H NMR (CDCl₃, 400 MHz): δ 4.34 (3H, s), 6.66 (1H, d, J=7.9 Hz), 8.36 (1H, d, J=7.9 Hz), 10.59 (1H, s).

### <Example 27>

### Ethyl 2-ethyl-[1,2,4]triazolo[1,5-a]pyridine-8-carboxylate ester

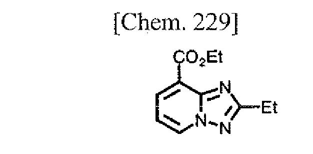

The compound of Example 22 (10.0 g) was dissolved in toluene (75 mL), and propionic anhydride (5.0 mL) and triethylamine (12.5 mL) were added thereto, followed by stirring for 14 hours under the condition of heating to reflux. After evaporating the solvent under reduced pressure, a saturated aqueous sodium hydrogen carbonate solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=3:2→1:1→1:3) to obtain the desired product (4.57 g) as a yellow powder.
EIMS (+): 219 [M]⁺.
¹H NMR (CDCl₃, 400 MHz): δ 1.45 (3H, t, J=7.3 Hz), 1.45 (3H, t, J=7.3 Hz), 3.04 (2H, q, J=7.3 Hz), 4.51 (2H, q, J=7.3 Hz), 7.03 (1H, dd, J=7.3, 7.3 Hz), 8.21 (1H, dd, J=7.3, 1.2 Hz), 8.68 (1H, dd, J=7.3, 1.2 Hz).

### <Example 28>

### 2-Ethyl-[1,2,4]triazolo[1,5-a]pyridine-8-carboxylic acid methoxymethyl amide

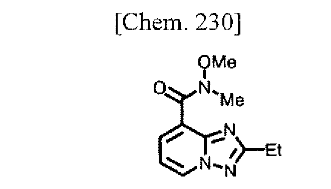

The compound of Example 27 (5.55 g) was dissolved in ethanol (200 mL) and water (100 mL), and potassium hydroxide (4.26 g) was added thereto, followed by stirring for 4 hours under the condition of heating to reflux. 1 mol/L Hydrochloric acid was added thereto to adjust the pH to 4, followed by extraction three times with chloroform, and the combined extracted layer was washed with saturated brine, dried over anhydrous sodium sulfate, and then filtered. After evaporating the solvent of the filtrate under reduced pressure, the residue was dissolved in DMF (250 mL), and diisopropyl ethylamine (19.8 mL), 1-ethyl-3-(3-dimethylaminopropyl)-carbodiimide hydrochloride (7.28 g), 1-hydroxybenzotriazole (5.13 g), and N,O-dimethylhydroxylamine hydrochloride (3.71 g) were added thereto, followed by stirring at room temperature for 5 hours. Methylene chloride (150 mL) and 4-dimethyl aminopyridine (600 mg) were added thereto, followed by stirring at room temperature for 10 hours. 1 mol/L Hydrochloric acid was added thereto to adjust the pH to 4, followed by extraction three times with chloroform, and the combined extracted layer was washed with saturated brine, dried over anhydrous sodium sulfate, and then filtered. After evaporating the solvent of the filtrate under reduced pressure, the residue was dissolved in DMF (250 mL), diphenylphosphoryl azide (6.00 mL), triethylamine (11.6 mL), and 4-dimethyl aminopyridine (600 mg) were added thereto, followed by stirring at room temperature for 25 hours. 1 mol/L Hydrochloric acid was added thereto to adjust the pH to 4, followed by extraction three times with chloroform, and the combined extracted layer was washed with saturated brine, dried over anhydrous sodium sulfate, and then filtered. After evaporating the solvent of the filtrate under reduced pressure, the residue was purified by silica gel column chromatography (ethyl acetate:methanol=20:1) and further by silica gel column chromatography (ethyl acetate) to obtain the desired product (1.37 g) as a yellow oil.
¹H NMR (CDCl₃, 400 MHz): δ 1.42 (3H, t, J=7.3 Hz), 2.98 (2H, q, J=7.3 Hz), 3.42 (3H, s), 3.64 (3H, s), 7.00 (1H, dd, J=6.7, 6.7 Hz), 7.60 (1H, dd, J=6.7, 1.2 Hz), 8.56 (1H, dd, J=6.7, 1.2 Hz).

### <Example 29>

### N-Amino-2-amino-3-bromo-6-methoxypyridiniummesitylensulfonate

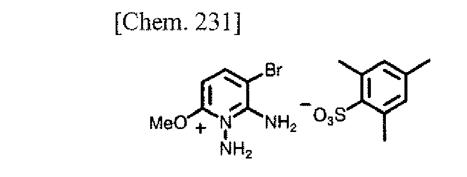

Ethyl mesitylsulfonylacetohydroxamate ester (25.3 g) was dissolved in 1,4-dioxane (35 mL), and 70% perchloric acid (13 mL) was added thereto at 0°C, followed by stirring for 30 minutes. To the reaction liquid was added cold water, and the precipitated solid was then collected by filtration and dissolved in methylene chloride. After removing the aqueous layer by a liquid separation operation, the methylene chloride layer was washed with saturated brine, dried over anhydrous sodium sulfate, and filtered. The filtrate was added to a solution of 2-amino-3-bromo-6-methoxypyridine (15.0 g) in methylene chloride (1.00 mL) at 0°C, followed by stirring at room temperature for 1 hour. The solvent was evaporated under reduced pressure, and diethyl ether was added thereto. The precipitated crystal was collected by filtration to obtain the desired product (27.3 g) as a yellow powder.
¹H NMR (CDCl₃, 400 MHz): δ 2.14 (3H, s), 2.47 (6H, s), 4.06 (3H, s), 5.73 (1H, s), 6.28 (2H, s), 6.45 (1H, d, J=8.6 Hz), 6.71 (2H, s), 8.20 (1H, d, J=8.6 Hz), 8.40 (2H, s).

### <Example 30>

### 8-Bromo-5-methoxy-2-trifluoromethyl-[1,2,4]triazolo[1,5-a]pyridine

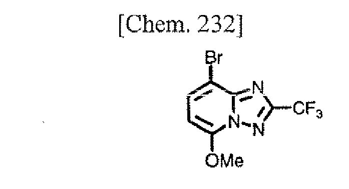

The compound of Example 29 (13.0 g) was dissolved in methanol (100 mL), and a mixture of triethylamine (13.0 mL), trifluoroacetic anhydride (6.6 mL), and methanol (20 mL) was added thereto at 0°C, followed by stirring at room temperature for 17.5 hours. To the reaction liquid was added water, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=1:1) to obtain the desired product (6.73 g) as a brown powder.
EIMS (+): 295 [M]⁺.
¹H NMR (CDCl₃, 400 MHz): δ 4.23 (3H, s), 6.41 (1H, d, J=7.9 Hz), 7.87 (1H, d, J=7.9 Hz).

### <Example 31>

### 3-Bromo-2-hydroxy methyl-6-methoxyphenol

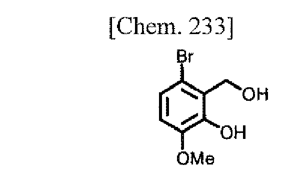

6-Bromo-2-hydroxy-3-methoxybenzaldehyde (1.00 g) was dissolved in methanol (30 mL), and sodium borohydride (164 mg) was added thereto under stirring with ice cooling. After stirring at room temperature for 4 hours, a diluted hydrochloric acid was added thereto, followed by extraction with ethyl acetate. It was washed with water and saturated brine, and then dried over anhydrous sodium sulfate, and the solvent was evaporated under reduced pressure to obtain the desired product (911 mg) as a pale yellow powder.
¹H NMR (CDCl₃, 400 MHz): δ 3.39 (3H, s), 4.91 (2H, s), 6.27 (1H, s), 6.70 (1H, d, J=8.6 Hz), 7.07 (1H, d, J=8.6 Hz).

### <Example 32>

### (6-Bromo-2-hydroxy-3-methoxyphenyl)methyltriphenylphosphonium bromide

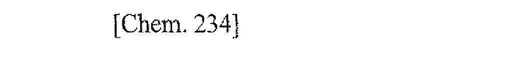

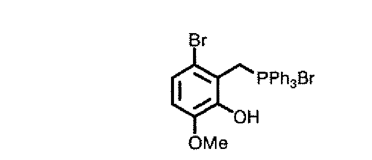

The compound of Example 31 (910 mg) was dissolved in acetonitrile (10 mL), and triphenyl phosphine hydrobromide (1.47 g) was added thereto, followed by heating to reflux for 5 hours. A half of the solvent was evaporated under reduced pressure, and ethyl acetate (50 mL) was added thereto. The precipitated crystal was collected by filtration and then dried to obtain the desired product (2.20 g) as a pale yellow powder.
¹H NMR (DMSO-d₆, 400 MHz): δ 3.63 (3H, s), 4.81 (2H, d, J=14.1 Hz), 6.81 (1H, dd, J=8.6, 1.8 Hz), 6.90 (1H, dd, J=8.6, 0.6 Hz), 7.52-7.72 (12H, m), 7.80-7.84 (3H, m), 9.80 (1H, s).

### <Example 33>

### 4-Bromo-2-trifluoromethyl-7-methoxybenzofuran

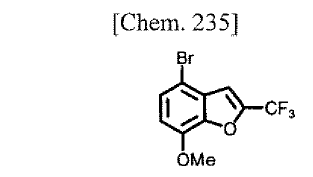

The compound of Example 32 (2.20 g) was suspended in toluene (20 mL) under an argon gas atmosphere, and anhydrous trifluoroacetic acid (0.612 mL) and triethylamine (1.64 mL) were added thereto, followed by heating to reflux for 5 hours. To the reaction liquid was added water, followed by extraction with ethyl acetate, the extracted layer was washed with saturated brine, and dried over anhydrous sodium sulfate, and the solvent was then was evaporated under reduced pressure. The residue was purified by silica gel column chromatography (hexane:ethyl acetate=10:1) to obtain the desired product (1.01 g) as a pale yellow powder.
¹H NMR (CDCl₃, 400 MHz): δ 4.01 (3H, s), 6.82 (1H, d, J=8.6 Hz), 7.20-7.21 (1H, m), 7.38 (1H, d, J=8.6 Hz).

### <Example 34>

### O-(3-Bromo-2-formyl-6-methoxy)phenyldimethyl thiocarbamate

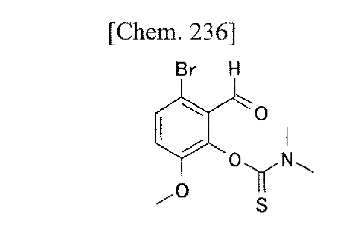

To a solution of 6-bromo-2-hydroxy-3-methoxybenzaldehyde (231 mg) in DMF (4.0 mL) were added triethylene diamine (224 mg) and dimethylthiocarbamoyl chloride (247 mg), followed by stirring at room temperature for 12 hours. The solvent was evaporated under reduced pressure and then to the residue was added water, followed by extraction with ethyl acetate. The extracted layer was dried over anhydrous magnesium sulfate and the solvent was evaporated. The residue was washed with isopropyl ether to obtain the desired product (258 mg) as a pale yellow powder.
EIMS (+): 317 [M+].
¹H NMR (CDCl₃, 400 MHz): δ 3.40 (3H, s), 3.45 (3H, s), 3.86 (3H, s), 7.05 (1H, d, J=8.6 Hz), 7.51 (1H, d, J=8.6 Hz), 10.20 (1H, s).

### <Example 35>

### S-(3-Bromo-2-formyl-6-methoxy)phenyldimethyl thiocarbamate

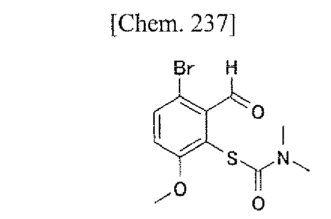

A solution of the compound of Example 34 (5.78 g) in diphenylether (57 mL) was stirred at 200°C for 30 minutes. The reaction liquid was cooled and then purified by silica gel column chromatography (hexane:ethyl acetate=1:1) to obtain the desired product (3.28 g) as a pale brown powder.
EIMS (+): 317 [M+].
¹H NMR (CDCl₃, 400 MHz): δ 3.00 (3H, brs), 3.16 (3H, brs), 3.89 (3H, s), 6.97 (1H, d, J=9.2 Hz), 7.64 (1H, d, J=9.2 Hz), 10.25 (1H, s).

### <Example 36>

### (6-Bromo-2-mercapto-3-methoxy)phenyl methanol

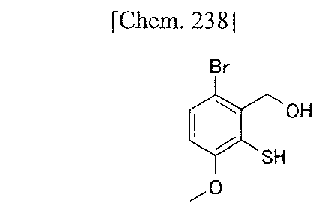

The compound of Example 35 (2.44 g) was suspended in isopropyl alcohol (60 mL), and 1 mol/L sodium hydroxide (15.3 mL) was added thereto, followed by stirring at 60°C for 30 minutes. The solvent was concentrated under reduced pressure, acidified by the addition of 5% hydrochloric acid, and then extracted with ethyl acetate. The extracted layer was washed with saturated brine and then dried over anhydrous magnesium sulfate, and the solvent was evaporated under reduced pressure. The residue was dissolved in methanol (60 mL), and sodium borohydride (580 mg) was added thereto under ice cooling, followed by stirring at room temperature for 30 minutes. The solvent was concentrated under reduced pressure, acidified by the addition of 5% hydrochloric acid, and then extracted with ethyl acetate. The extracted layer was washed with saturated brine and then dried over anhydrous magnesium sulfate, and the solvent was evaporated under reduced pressure to obtain the desired product (1.95 g) as a pale purple oil.
EIMS (+): 248 [M]⁺.
¹H NMR (CDCl₃, 400 MHz): δ 1.99 (1H, brs), 3.90 (3H, s), 4.46 (1H, s), 4.93 (2H, s), 6.71 (1H, d, J=8.6 Hz), 7.33 (1H, d, J=8.6 Hz).

### <Example 37>

### 4-Bromo-7-methoxy-2-trifluoromethylbenzo[b]thiophene

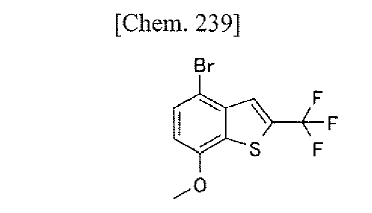

The compound of Example 36 (1.95 g) was dissolved in acetonitrile (15 mL), and triphenylphosphonium bromide (2.90 g) was added thereto, followed by heating to reflux for 17 hours. The solvent was concentrated under reduced pressure and then washed with ethyl acetate to obtain a colorless powder (4.39 g). To the obtained solid (4.35 g) were added toluene (60 mL), anhydrous trifluoroacetic acid (1.18 mL) and triethylamine (3.17 mL), followed by heating to reflux for 3 hours. To the reaction liquid was added water, followed by extraction with ethyl acetate, the extracted layer was washed with saturated brine and then dried over anhydrous magnesium sulfate, and the solvent was evaporated under reduced pressure. The residue was purified by silica gel column chromatography (hexane:ethyl acetate=10:1) to obtain the desired product (2.00 g) as a colorless powder.
EIMS (+): 310 [M]⁺.
¹H NMR (CDCl₃, 400 MHz): δ 4.00 (3H, s), 6.75 (1H, d, J=8.6 Hz), 7.53 (1H, d, J=8.6 Hz), 7.79 (1H, q, J=1.2 Hz).

### <Example 38>

### 2-di-(t-Butoxycarbonyl)amino-3-methoxy-6-propionylpyridine

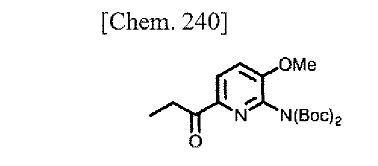

To a solution of the compound of Example 6 (1.60 g) in THF (25 mL) was added dropwise a 0.97 mol/L ethyl magnesium bromide-THF solution (12.0 mL) at -78°C under an argon atmosphere, followed by stirring at -78°C for 30 minutes and then slowly returning to room temperature. To the reaction liquid was added a saturated aqueous ammonium chloride solution, followed by extraction with ethyl acetate (150 mL). The extract was washed with water and saturated brine, and then dried over anhydrous sodium sulfate. The solvent was evaporated under reduced pressure, the obtained residue was dissolved in acetonitrile (50 mL), di-t-butyldicarbonate (3.39 g), triethylamine (787 mg) and 4-dimethyl aminopyridine (20 mg), were added thereto, followed by stirring at room temperature for 2 hours. The reaction liquid was concentrated and the residue was then purified by silica gel column chromatography (hexane:ethyl acetate=3:1→ethyl acetate) to obtain the desired product (1.26 g) as a pale yellow powder.
¹H NMR (CDCl₃, 400 MHz): δ 1.19 (3H, t, J=7.3 Hz), 1.41 (18H, s), 3.14 (2H, q, J=7.3 Hz), 3.92 (3H, s), 7.29 (1H, d, J=8.6 Hz), 8.06 (1H, d, J=8.6 Hz).

### <Example 39>

### 5-Acetyl-4-chloro-8-methoxy-2-trifluoromethylquinoline

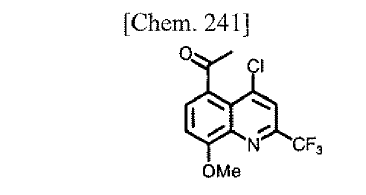

The compound of Example 7 (6.58 g) and ethyl 3-trifluoromethylpropionate (7.94 g) were dissolved in diphenyl ether (80 mL), followed by stirring at 100°C for 1.5 hours and at 250°C for 1 hour. Hexane was added thereto, and the precipitated solid was collected by filtration. The obtained solid was dissolved in phosphorous oxychloride (40 mL), followed by stirring for 1 hour under the condition of heating to reflux. After evaporating phosphorous oxychloride under reduced pressure, to the residue was added a saturated aqueous sodium hydrogen carbonate solution, followed by extraction three times with ethyl acetate. The combined organic layer was washed with saturated brine, dried over anhydrous sodium sulfate, and then filtered. The solvent of the filtrate was evaporated under reduced pressure and then purified by silica gel column chromatography (hexane:ethyl acetate=4:1→2:1) to obtain the desired product (3.98 g) as a brown powder.
¹H NMR (CDCl₃, 400 MHz): δ 2.67 (3H, s), 4.13 (3H, s), 7.14 (1H, d, J=7.9 Hz), 7.60 (1H, d, J=7.9 Hz), 7.91 (1H, s).

### <Example 40>

### 5-Acetyl-8-methoxy-2-trifluoromethylquinoline

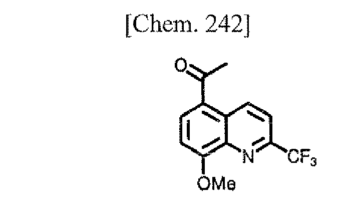

The compound of Example 39 (3.98 g) was dissolved in ethanol (75 mL) and THF (50 mL), and triethylamine (9.10 mL) and 5% palladium carbon (400 mg) were added thereto, followed by stirring at room temperature for 3.5 hours under a hydrogen atmosphere. The insoluble materials were removed by filtration through Celite, the solvent of the filtrate was then evaporated under reduced pressure, and the residue was purified by silica gel column chromatography (hexane:ethyl acetate=4:1→1:1) to obtain the desired product (1.83 g) as a yellow powder.
¹H NMR (CDCl₃, 400 MHz): δ 2.75 (3H, s), 4.18 (3H, s), 7.12 (1H, d, J=8.6 Hz), 7.89 (1H, d, J=9.2 Hz), 8.25 (1H, d, J=8.6 Hz), 9.73 (1H, d, J=9.2 Hz).

### <Example 41>

### 2-Ethyl-8-methoxy-5-propionylquinoline

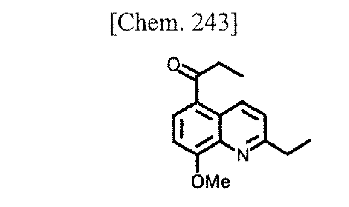

The compound of Example 13 (4.00 g) was dissolved in THF (150 mL) under an argon atmosphere, and a solution (2.71 mol/L, 6.1 mL) of n-butyl lithium in hexane was added thereto at -78°C, followed by stirring for 1 hour. Propionic anhydride (1.53 mL) was added thereto at the same temperature, followed by stirring for 2.5 hours, then warming to room temperature, and followed by further stirring for 1.5 hours. A saturated aqueous ammonium chloride solution was added thereto, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=1:1) to obtain the desired product (2.08 g) as a yellow powder.
¹H NMR (CDCl₃, 400 MHz): δ 1.27 (3H, t, J=7.3 Hz), 1.40 (3H, t, J=7.3 Hz), 3.05-3.11 (4H, m), 4.14 (3H, s), 7.01 (1H, d, J=8.6 Hz), 7.48 (1H, d, J=8.6 Hz), 8.03 (1H, d, J=8.6 Hz), 9.28 (1H, d, J=8.6 Hz).

### <Example 42>

### 2-Ethyl-8-methoxy-5-propionylquinoline (2)

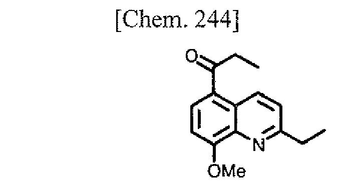

Aluminum, chloride (214 mg) and 1,2,4-trichlorobenzene (1.0 mL) were mixed, and propionyl chloride (0.163 mL) was added thereto at room temperature. The compound of Example 8 (100 mg) was added thereto, followed by stirring at an outer temperature of 70°C for 1 hour. 1 mol/L Hydrochloric acid was added thereto, followed by washing with ethyl acetate. The organic layer was extracted with 1 mol/L hydrochloric acid. The aqueous layer was combined, neutralized with sodium acetate, and then extracted twice with ethyl acetate. The extracted layer was washed with saturated brine, dried over anhydrous sodium sulfate, and concentrated under reduced pressure. The residue was treated with silica gel column chromatography (hexane:ethyl acetate=1:1) to obtain the desired product (70.9 mg) as a pale yellow powder.

### <Example 43>

### 2-Ethyl-8-methoxy-5-propionylquinoline

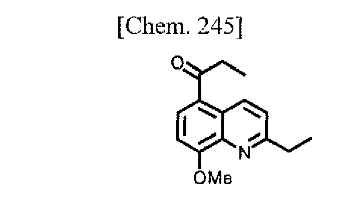

2-Amino-4-propionylanisole (24.6 g), sodium iodide (20.6 g), 1-butanol (246 mL), and concentrated hydrochloric acid (246 mL) were mixed, and trans-1-heptenal (57.8 g) was added dropwise for 50 minutes while heating at 140°C, followed by heating to reflux at 140°C for 3 hours. To the residue obtained by evaporating the reaction liquid under reduced pressure were added ethyl acetate (500 mL) and water (250 mL), followed by liquid separation. The organic layer was extracted with water (250 mL), combined with the above aqueous layer, and adjusted to pH 8 with a 1 mol/L sodium hydroxide solution. The precipitated solid was collected by filtration and washed with water (75.0 mL). The obtained solid was dissolved in ethyl acetate (100 mL), and silica gel (1.23 g) was added thereto, followed by separation by filtration and concentration under reduced pressure. The residue was added with ethanol (98.5 mL), heated, and dissolved at 50°C, and water (98.5 mL) was then added thereto, followed by leaving to be cooled at room temperature. The precipitated solid was collected by filtration and washed with ethanol:water=1:5 (98.5 mL). It was dried at 60°C under reduced pressure to obtain the desired product (14.1 g) as a brown powder.

### <Example 44>

### 5-Acetyl-2-ethyl-8-methoxyquinoline

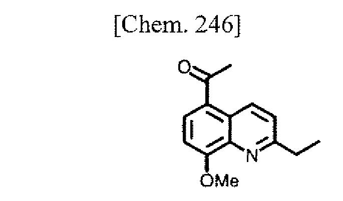

To a solution of the compound of Example 18 (4.07 g) in THF (50 mL) was added dropwise a 2.0 mol/L isopropyl magnesium chloride-THF solution (7.76 mL) at 0°C under an argon atmosphere, followed by stirring at 0°C for 30 minutes. Acetic anhydride (1.83 mL) was added thereto at 0°C, followed by slowly warming to room temperature. To the reaction liquid was added a saturated aqueous ammonium chloride solution, followed by extraction with ethyl acetate (100 mL). The extract was washed with water, a saturated aqueous sodium hydrogen carbonate solution, and saturated brine, and then dried over anhydrous sodium sulfate. The extract was concentrated and then purified by recrystallization (ethyl acetate-hexane) to obtain the desired product (2.12 g) as an orange powder.
¹H NMR (CDCl₃, 400 MHz): δ 1.40 (3H, t, J=7.9 Hz), 2.71 (3H, s), 3.08 (2H, q, J=7.9 Hz), 4.15 (3H, s), 7.02 (1H, d, J=8.6 Hz), 7.50 (1H, d, J=9.2 Hz), 8.07 (1H, d, J=8.6 Hz), 9.37 (1H, d, J=9.2 Hz).

### <Example 45>

### 8-Methoxy-5-propionyl-2-trifluoromethylquinoline

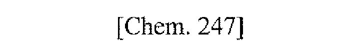

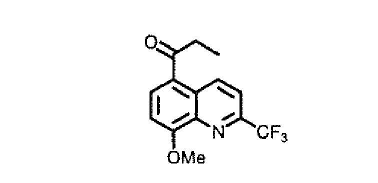

The compound of Example 15 (3.86 g) was dissolved in THF (100 mL) under an argon atmosphere, and a solution (2.71 mol/L, 5.2 mL) of n-butyl lithium in hexane was added thereto at -78°C, followed by stirring for 1 hour. Propionic anhydride (3.5 mL) was added thereto at the same temperature, followed by stirring for 3 hours. Then, a saturated aqueous ammonium chloride solution was added thereto, followed by extraction three times with ethyl acetate. The combined extracted layer was washed with saturated brine, dried over sodium sulfate, and then filtered. After evaporating the solvent of the filtrate under reduced pressure, the residue was purified by silica gel column chromatography (hexane:ethyl acetate=5:1) to obtain the desired product (1.21 g) as a yellow powder.
¹H NMR (CDCl₃, 400 MHz): δ 1.29 (3H, t, J=7.3 Hz), 3.11 (2H, q, J=7.3 Hz), 4.18 (3H, s), 7.12 (1H, d, J=8.6 Hz), 7.88 (1H, d, J=8.6 Hz), 8.23 (1H, d, J=8.6 Hz), 9.65 (1H, d, J=8.6 Hz).

### <Example 46>

### 8-Methoxy-5-propionylquinoline

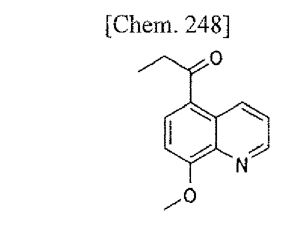

To a solution of 8-hydroxy quinoline (30.0 g) in 1,2-dichloroethane (207 mL) were added aluminum chloride (68.9 g) and propionyl chloride (19.9 mL), followed by stirring at 70°C for 3 hours. The reaction liquid was poured into 5% hydrochloric acid (1.0 L), followed by stirring for 30 minutes, and adjustment to pH 4 with sodium acetate, and the organic layer was collected by separation. The aqueous layer was extracted with chloroform, combined with the above organic layer, and dried over anhydrous sodium sulfate. The solvent was evaporated under reduced pressure to obtain crude 8-hydroxy-5-propionylquinoline as a pale yellow amorphous powder. To this amorphous powder were added THF (500 mL), a 50% aqueous sodium hydroxide solution, tetra-n-butylammoniumbromide (3.00 g), and methyl iodide (38.6 mL), followed by stirring at room temperature for 29 hours. After concentrating the reaction liquid under reduced pressure, to the residue was added ice water (1.0 L), and the precipitated solid was collected by filtration. This solid was dissolved in ethyl acetate and dried over anhydrous sodium sulfate, and the solvent was evaporated. To the residue was added ethyl acetate (1.0 L), followed by warming, and the insoluble materials were removed by filtration. The filtrate was concentrated and the precipitated solid was collected by filtration to obtain the desired product (23.2 g) as a pale yellow powder.
EIMS (+): 215 [M]⁺.
¹H NMR (CDCl₃, 400 MHz): δ 1.28 (3H, t, J=7.3 Hz), 3.10 (2H, q, J=7.3 Hz), 4.10 (3H, s), 7.04 (1H, d, J=7.9 Hz), 7.55 (1H, dd, J=8.6, 4.3 Hz), 8.11 (1H, d, J=7.9 Hz), 8.96 (1H, dd, J=4.3, 1.8 Hz), 9.39 (1H, dd, J=8.6, 1.8 Hz).

### <Example 47>

### 8-Methoxy-2-methyl-5-propionylquinoline

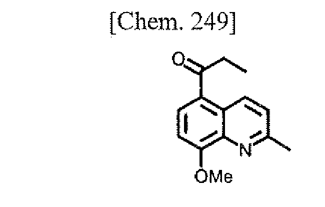

The compound of Example 16 (5.55 g) was dissolved in THF (220 mL) under an argon gas atmosphere, and an n-butyl lithium/hexane solution (1.58 mol/L, 15.3 mL) was added dropwise thereto at -78°C, followed by stirring at the same temperature for 5 minutes. Thereafter, propionic anhydride (4.86 mL) was added thereto at -78°C, followed by stirring at -78°C for 10 minutes. To the reaction liquid was added a saturated aqueous ammonium chloride solution, followed by extraction with ethyl acetate. The organic layer was washed with water and saturated brine in this order, and then dried over anhydrous sodium sulfate. The solvent was evaporated under reduced pressure, and the residue was then purified by silica gel column chromatography (hexane:ethyl acetate= 1:1→1:2) to obtain the desired product (2.39 g) as a yellow powder.
¹H NMR (CDCl₃, 400 MHz): δ 1.27 (3H, t, J=7.3 Hz), 2.80 (3H, s), 3.08 (2H, q, J=7.3 Hz), 4.14 (3H, s), 7.01 (1H, d, J=8.6 Hz), 7.44 (1H, d, J=9.2 Hz), 8.04 (1H, d, J=8.6 Hz), 9.26 (1H, d, J=9.2 Hz).

### <Example 48>

### 8-Methoxy-5-propionyl-2-isopropylquinoline

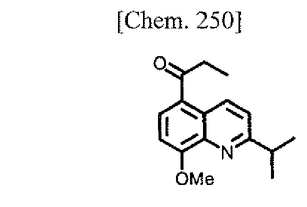

The compound of Example 17 (3.57 g) was dissolved in THF (120 mL) under an argon gas atmosphere, and an n-butyl lithium/hexane solution (1.60 mol/L, 8.06 mL) was added dropwise thereto at -78°C, followed by stirring at the same temperature for 5 minutes. Thereafter, propionic anhydride (2.61 mL) was added thereto at -78°C, followed by stirring at -78°C for 25 minutes. To the reaction liquid was added a saturated aqueous ammonium chloride solution, followed by extraction with ethyl acetate. The organic layer was washed with water and saturated brine in this order, and then dried over anhydrous sodium sulfate. The solvent was evaporated under reduced pressure, and the residue was then purified by silica gel column chromatography (hexane:ethyl acetate=3: 1) to obtain the desired product (1.20 g) as a colorless powder.
¹H NMR (CDCl₃, 400 MHz): δ 1.27 (3H, t, J=7.3 Hz), 1.39 (6H, d, J=7.3 Hz), 3.08 (2H, q, J=7.3 Hz), 3.31-3.41 (1H, m), 4.14 (3H, s), 7.01 (1H, d, J=8.6 Hz), 7.52 (1H, d, J=9.2 Hz), 8.03 (1H, d, J=8.6 Hz), 9.29 (1H, d, J=9.2 Hz).

### <Example 49>

### 2-Ethyl-8-propionylindolidine

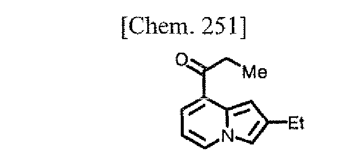

The compound of Example 20 (2.76 g) was dissolved in THF (60 mL) under an argon atmosphere, and a solution (0.97 mol/L, 36.7 mL) of ethyl magnesium bromide in THF was added thereto at 0°C, followed by stirring at room temperature for 1.5 hours. A saturated aqueous ammonium chloride solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=10:1) to obtain the desired product (1.76 g) as a yellow oil.
¹H NMR (CDCl₃, 400 MHz): δ 1.28 (3H, t, J=7.3 Hz), 1.33 (3H, t, J=7.3 Hz), 2.77 (2H, q, J=7.3 Hz), 3.04 (2H, q, J=7.3 Hz), 6.50 (1H, dd, J=6.7, 6.7 Hz), 7.19 (1H, s), 7.21 (1H, s), 7.43 (1H, d, J=6.7 Hz), 8.04 (1H, d, J=6.7 Hz).

### <Example 50>

### 2-Ethyl-8-(2-ethyl-[1,3]dioxolan-2-yl)indolidine

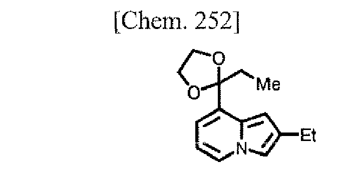

The compound of Example 49 (1.98 g) was dissolved in benzene (100 mL), and ethylene glycol (10 mL) and paratoluenesulfonic acid monohydrate (187 mg) were added thereto, followed by stirring for 9 hours with the equipment of a Dean-stark device to heating under reflux. A saturated aqueous sodium hydrogen carbonate solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=10:1) to obtain the desired product (2.02 g) as a yellow oil.
¹H NMR (CDCl₃, 400 MHz): δ 0.89 (3H, t, J=7.3 Hz), 1.30 (3H, t, J=7.3 Hz), 2.12 (2H, q, J=7.3 Hz), 2.72 (2H, q, J=7.3 Hz), 3.83-3.87 (2H, m), 4.02-4.06 (2H, m), 6.36 (1H, dd, J=6.7, 6.7 Hz), 6.54 (1H, s), 6.72 (1H, d, J=6.7 Hz), 7.13 (1H, s), 7.77 (1H, d, J=6.7 Hz).

### <Example 51>

### 2-Ethyl-8-(2-ethyl-[1,3]dioxolan-2-yl)-5-iodoindolidine

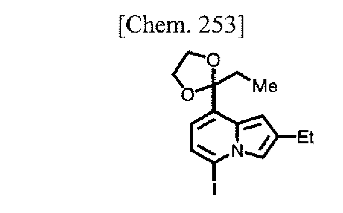

The compound of Example 50 (2.02 g) and N,N,N',N'-tetramethylethylene diamine (6.21 mL) were dissolved in THF (80 mL) under an argon atmosphere, and a solution (2.71 mol/L, 3.34 mL) of n-butyl lithium in hexane was added thereto at -40°C, followed by stirring at -40°C for 2 hours. 1,2-Diiodoethane (2.55 g) was added thereto at -40°C, followed by slowly warming to room temperature and stirring for 16 hours. A saturated aqueous ammonium chloride solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=30:1→15:1) to obtain the desired product (787 mg) as a yellow oil. EIMS (+): 371 [M]⁺.
¹H NMR (CDCl₃, 400 MHz): δ 0.89 (3H, t, J=7.3 Hz), 1.32 (3H, t, J=7.3 Hz), 2.11 (2H, q, J=7.3 Hz), 2.75 (2H, q, J=7.3 Hz), 3.81-3.85 (2H, m), 4.02-4.04 (2H, m), 6.51 (1H, d, J=7.3 Hz), 6.85 (1H, s), 6.96 (1H, d, J=7.3 Hz), 7.42 (1H, s).

### <Example 52>

### 2-Ethyl-5-methoxy-8-propionylindolidine

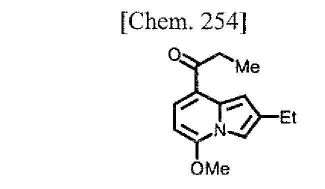

The compound of Example 51 (787 mg) was dissolved in acetone (10 mL), and water (5.0 mL) and paratoluenesulfonic acid monohydrate (40.0 mg) were added thereto, followed by stirring at 80°C for 2 hours. A saturated aqueous sodium hydrogen carbonate solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=30:1) to obtain 2-ethyl-5-iodo-8-proplonylindolidine (578 mg) as a yellow oil.

The obtained 2-ethyl-5-iodo-8-propionylindolidine (577 mg) was dissolved in methanol (17 mL) under an argon atmosphere, and sodium methoxide (381 mg) was added thereto, followed by stirring for 6 hours to heating under reflux. A saturated aqueous ammonium chloride solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=3;2) to obtain the desired product (392 mg) as a yellow powder.
¹H NMR (CDCl₃, 400 MHz): δ 1.24 (3H, t, J=7.3 Hz), 1.31 (3H, t, J=7.3 Hz), 2.75 (2H, q, J=7.3 Hz), 2.98 (2H, q, J=7.3 Hz), 4.11 (3H, s), 5.80 (1H, d, J=7.9 Hz), 7.22 (1H, s), 7.29 (1H, s), 7.57 (1H, d, J=7.9 Hz).

### <Example 53>

### 2-Ethyl-8-propionyl-[1,2,4]triazolo[1,5-a]pyridine

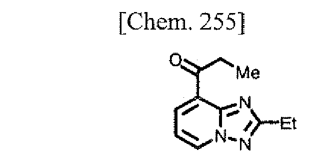

The compound of Example 28 (1.37 g) was dissolved in THF (50 mL) under an argon atmosphere, and a solution (0.97 mol/L, 12.0 mL) of ethyl magnesium bromide in THF was added thereto at 0°C, followed by stirring at room temperature for 1.5 hours. A saturated aqueous sodium hydrogen carbonate solution was added thereto, followed by extraction three times with ethyl acetate, and the combined extracted layer was washed with saturated brine, dried over anhydrous sodium sulfate, and then filtered. The solvent was evaporated under reduced pressure to obtain the desired product (1.06 g) as a yellow powder. ¹H NMR (CDCl₃, 400 MHz): δ 1.27 (3H, t, J=7.3 Hz), 1.46 (3H, t, J=7.3 Hz), 3.00 (2H, q, J=7.3 Hz), 3.53 (2H, q, J=7.3 Hz), 7.06 (1H, dd, J=7.3, 7.3 Hz), 8.16 (1H, dd, J=7.3, 1.2 Hz), 8.67 (1H, dd, J=7.3, 1.2 Hz).

### <Example 54>

### 2-Ethyl-8-(2-ethyl-[1,3]dioxolan-2-yl)-5-iodo-[1,2,4]triazolo[1,5-a]pyridine

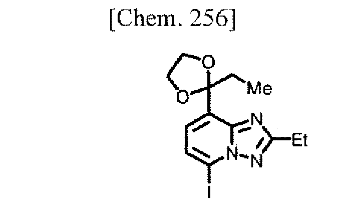

The compound of Example 53 (1.06 g) was dissolved in benzene (50 mL), and ethylene glycol (10 mL) and paratoluenesulfonic acid monohydrate (105 mg) were added thereto, followed by stirring for 11 hours with the equipment of a Dean-stark device to heating under reflux. A saturated aqueous sodium hydrogen carbonate solution was added thereto, followed by extraction three times with ethyl acetate, and the combined extracted layer was washed with saturated brine, dried over sodium sulfate, and then filtered. After evaporating the solvent of the filtrate under reduced pressure, it was purified by silica gel chromatography (hexane:ethyl acetate=1:1) to obtain 2-ethyl-8-(2-ethyl-[1,3]dioxolan-2-yl)-[1,2,4]triazolo[1,5-a]pyridine (772 mg) as a yellow oil.

The obtained 2-ethyl-8-(2-ethyl-[1,3]dioxolan-2-y1)-[1,2,4]triazolo[1,5-a]pyridine (772 mg) was dissolved in THF (20 mL) under an argon atmosphere, and a solution (2.71 mol/L, 1.50 mL) of n-butyl lithium in hexane was added thereto at -78°C, followed by stirring for 30 minutes. Then, 1,2-diiodoethane (1.14 g) was added thereto at the same temperature, followed by stirring for 2 hours. A saturated aqueous sodium hydrogen carbonate solution was added thereto, followed by extraction three times with ethyl acetate, and the combined extracted layer was washed with saturated brine, dried over anhydrous sodium sulfate, and then filtered. After evaporating the solvent under reduced pressure, the residue was purified by silica gel column chromatography (hexane:ethyl acetate=2:1) to obtain the desired product (961 mg) as a yellow oil.
¹H NMR (CDCl₃, 400 MHz): δ 0.88 (3H, t, J=7.3 Hz), 1.42 (3H, t, J=7.3 Hz), 2.35 (2H, q, J=7.3 Hz), 3.02 (2H, q, J=7.3 Hz), 3.90-3.92 (2H, m), 4.10-4.13 (2H, m), 7.32 (1H, d, J=7.3 Hz), 7.40 (1H, d, J=7.3 Hz).

### <Example 55>

### 2-Ethyl-5-methoxy-8-propionyl-[1,2,4]triazolo[1,5-a]pyridine

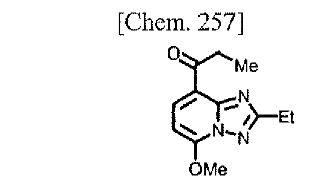

The compound of Example 54 (960 mg) was dissolved in acetone (20 mL) and water (10 mL), and paratoluenesulfonic acid monohydrate (48.9 mg) was added thereto, followed by stirring at 60°C to 80°C for 6 hours. A saturated aqueous sodium hydrogen carbonate solution was added thereto, followed by extraction three times with ethyl acetate, and the combined extracted layer was washed with saturated brine, dried over anhydrous sodium sulfate, and then filtered. After evaporating the solvent of the filtrate under reduced pressure, the residue was dissolved in methanol (25 mL) under an argon atmosphere, and sodium methoxide (544 mg) was added thereto, followed by stirring for 2 hours with heating under reflux under an argon atmosphere. To the reaction liquid was added water, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=3:2→1:2) to obtain the desired product (83.7 mg) as a yellow powder.
¹H NMR (CDCl₃, 400 MHz): δ 1.26 (3H, t, J=7.3 Hz), 1.46 (3H, t, J=7.3 Hz), 3.01 (2H, q, J=7.3 Hz), 3.49 (2H, q, J=7.3 Hz), 4.25 (3H, s), 6.39 (1H, d, J=8.6 Hz), 8.29 (1H, d, J=8.6 Hz).

### <Example 56>

### 8-(1-Hydroxypropyl)-5-methoxy-2-trifluoromethyl-[1,2,4]triazolo[1,5-a]pyridine

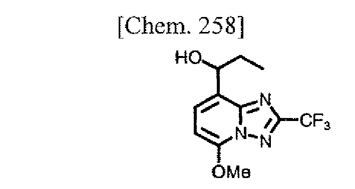

The compound of Example 26 (682 g) was dissolved in THF (25 mL) under an argon atmosphere, and a solution (0.97 mol/L, 3.44 mL) of ethyl magnesium bromide in THF was added thereto at -78°C, followed by stirring at room temperature for 3 hours. A saturated aqueous sodium hydrogen carbonate solution was added thereto, followed by extraction three times with ethyl acetate, and the combined extracted layer was washed with saturated brine, dried over anhydrous sodium sulfate, and then filtered. The solvent was evaporated under reduced pressure to obtain the desired product (492 mg) as a yellow powder.
¹H NMR (CDCl₃, 400 MHz): δ 1.00 (3H, t, J=7.3 Hz), 1.96-2.03 (2H, m), 3.02 (1H, brs), 4.21 (3H, s), 5.05 (1H, brs), 6.44 (1H, d, J=7.9 Hz), 7.60 (1H, d, J=7.9 Hz).

### <Example 57>

### 5-Methoxy-8-propionyl-2-trifluoromcthyl-[1,2,4]triazolo[1,5-a]pyridine

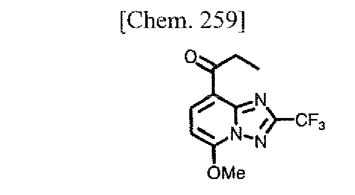

The compound of Example 56 (492 mg) was dissolved in DMSO (9.0 mL) under an argon atmosphere, and triethylamine (2.50 mL) and a sulfur trioxide-pyridine complex (1.42 g) were added thereto, followed by stirring at room temperature for 1 hour. Water was added thereto, and the insoluble materials were collected by filtration to obtain the desired product (276 mg). On the other hand, the filtrate was extracted three times with ethyl acetate, and the combined extracted layer was washed with saturated brine, dried over anhydrous sodium sulfate, and then filtered. After evaporating the solvent under reduced pressure, the residue was purified by silica gel column chromatography (hexane:ethyl acetate=3:2) to obtain the desired product (66.9 mg), from which a white powder (343 mg) was obtained by combination.
0
¹H NMR (CDCl₃, 400 MHz): δ 1.27 (3H, t, J=7.3 Hz), 3.50 (2H, q, J=7.3 Hz), 4.30 (3H, s), 6.60 (1H, d, J=8.6 Hz), 8.47 (1H, d, J=8.6 Hz).

### <Example 58>

### 7-Methoxy-4-propionyl-2-trifluoromethylbenzofuran

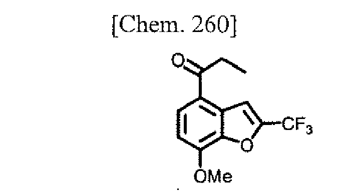

The compound of Example 33 (500 mg) was dissolved in THF (10 mL) under an argon gas atmosphere, and an n-butyl lithium hexane solution (1.54 mol/L, 1.21 mL) was added dropwise thereto at -78°C, followed by stirring for 5 minutes. To this was added N,N-dimethyl propionic acid amide (513 mg), followed by stirring at room temperature for 30 minutes. A saturated aqueous ammonium chloride solution was added thereto, followed by extraction with ethyl acetate, the extracted layer was washed with saturated brine and dried over anhydrous sodium sulfate, and the solvent was then evaporated under reduced pressure. The residue was purified by silica gel column chromatography (hexane:ethyl acetate=20:1→10:1) to obtain the desired product (162 mg) as a colorless powder.
¹H NMR (CDCl₃, 400 MHz): δ 1.26 (3H, t, J=7.3 Hz), 3.05 (2H, q, J=7.3 Hz), 4.10 (3H, s), 6.93 (1H, d, J=8.6 Hz), 7.90 (1H, d, J=8.6 Hz), 8.01 (1H, d, J=1.2 Hz).

### <Example 59>

### 7-Methoxy-4-propionyl-2-trifluoromethylbenzo[b]thiophene

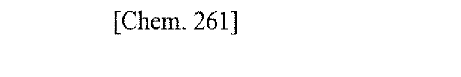

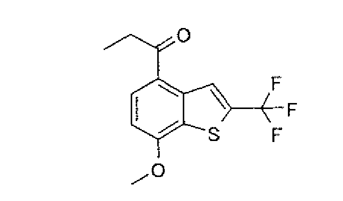

To a solution of the compound of Example 37 (1.70 g) in THF (27 mL) was added an n-butyl lithium (1.58 mol/L hexane solution, 3.80 mL) at -78°C, followed by stirring at the same temperature for 30 minutes, and then N,N-dimethyl propionamide (1.20 mL) was added thereto, followed by stirring at room temperature for 30 minutes. To the reaction liquid was added a saturated aqueous ammonium chloride solution, followed by extraction with ethyl acetate, the organic layer was washed with saturated brine and then dried over anhydrous magnesium sulfate, and the solvent was evaporated. The residue was purified by silica gel column chromatography (hexane:ethyl acetate=4: 1) to obtain the desired product (243 mg) as a white powder.
EIMS (+): 288 [M]⁺_{,}
¹H NMR (CDCl₃, 400 MHz): δ 1.27 (3H, t, J=7.3 Hz), 3.07 (2H, q, J=7.3 Hz), 4.09 (3H, s), 6.89 (1H, d, J=8.6 Hz), 8.05 (1H, d, J=8.6 Hz), 8.80 (1H, q, J=1.2 Hz).

### <Example 60>

### Ethyl 4-[2-di-(t-butoxycarbonyl)amino-3-methoxypyridin-6-yl]-3-methyl-4-oxobutyrate ester

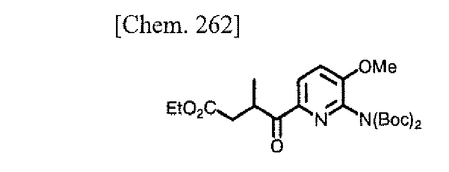

To a solution of the compound of Example 38 (108 mg) in THF (5 mL) was added dropwise a lithium hexamethyl disilazane-THF solution (1.0 mol/L, 0.312 mL) at 0°C under an argon atmosphere, followed by stirring at 0°C for 30 minutes. Bromoethyl acetate (0.394 mL) was added dropwise thereto at -78°C, followed by stirring at room temperature for 30 minutes. A saturated aqueous ammonium chloride solution was added thereto, followed by extraction with ethyl acetate (50 mL), and the extract was washed with water and saturated brine, and then dried over anhydrous sodium sulfate. The extract was concentrated and then purified by silica gel column chromatography (hexane:ethyl acetate= 3:1→ethyl acetate) to obtain the desired product (84.0 mg) as a pale yellow oil.
¹H NMR (CDCl₃, 400 MHz): δ 1.20 (3H, t, J=7.3 Hz), 1.23 (3H, d, J=7.3 Hz), 1.40 (18H, s), 2.48 (1H, dd, J=16.5, 6.1 Hz), 2.89 (1H, dd, J=16.5, 8.6 Hz), 3.93 (3H, s), 4.03-4.16 (2H, m), 4.25-4.37 (1H, m), 7.30 (1H, d, J=8.6 Hz), 8.07 (1H, d, J=8.6 Hz).

### <Example 61>

### t-Butyl 4-(2-ethyl-8-methoxyquinolin-5-yl)-3-methyl-4-oxobutyrate ester

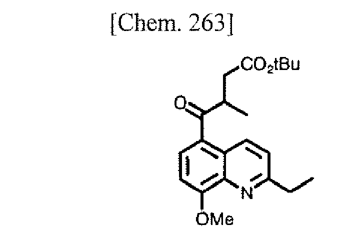

The compound of Example 41 (1.00 g) was dissolved in THF (40 mL) under an argon atmosphere, and a solution (1.0 mol/L, 4.52 mL) of lithium hexamethyl disilazane in THF was added thereto at -78°C, followed by stirring at -78°C to 0°C for 40 minutes. t-Butyl bromoacetate (0.784 mL) was added thereto at 0°C, followed by stirring at room temperature for 17 hours. Water was added thereto, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=1:1) to obtain the desired product (889 mg) as a yellow oil.
¹H NMR (CDCl₃, 400 MHz): δ 1.21 (3H, d, J=7.3 Hz), 1.33 (9H, s), 1.40 (3H, t, J=7.3 Hz), 2.40 (1H, dd, J=16.8, 5.2 Hz), 2.92 (1H, dd, J=16.8, 9.2 Hz), 3.07 (2H, q, J=7.3 H), 3.93-3.95 (1H, m), 4.14 (3H, s), 7.03 (1H, d, J=8.6 Hz), 7.46 (1H, d, J=8.6 Hz), 8.09 (1H, d, J=8.6 Hz), 9.01 (1H, d, J=8.6 Hz).

### <Example 62>

### t-Butyl 4-(8-methoxy-2-methylquinolin-5-yl)-3-methyl-4-oxobutyrate ester

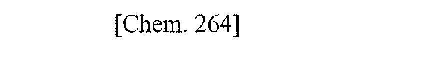

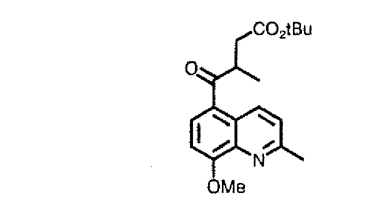

The compound of Example 47 (5.19 g) was dissolved in THF (220 mL) and hexamethyl phosphoric acid triamide (22 mL) under an argon gas atmosphere, and a lithium bistrimethylsilyl amide/THF solution (1.00 mol/L, 24.9 mL) was added dropwise thereto at 0°C, followed by stirring at 0°C for 30 minutes. Thereafter, t-butyl bromoacetate (4.32 mL) was added thereto at 0°C, followed by stirring for 2 hours while warming to room temperature. To the reaction liquid was added a saturated aqueous ammonium chloride solution, followed by extraction with ethyl acetate. The organic layer was washed with water and saturated brine, and then dried over anhydrous sodium sulfate, and the solvent was evaporated under reduced pressure. To the residue was added ethyl acetate again, followed by washing with water and saturated brine, and then drying over anhydrous sodium sulfate. After evaporating the solvent under reduced pressure, the desired product (8.68 g) was obtained as a yellow powder. This was used for the reaction described in Example 75 without purification.

### <Example 63>

### t-Butyl 4-(8-methoxy-2-isopropylquinolin-5-yl)-3-methyl-4-oxobutyrate ester

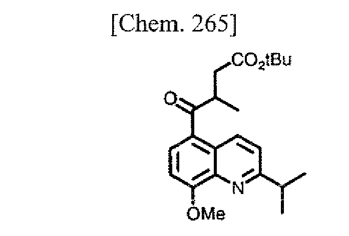

The compound of Example 48 (1.20 g) was dissolved in THF (46 mL) and hexamethyl phosphoric acid triamide (4.6 mL) under an argon gas atmosphere, and a lithium bistrimethylsilyl amide/THF solution (1.00 mol/L, 24.9 mL) was added dropwise thereto at -78°C, followed by stirring for 30 minutes while slowly warming to 0°C. Thereafter, t-butyl bromoacetate (0.89 mL) was added thereto at 0°C, followed by stirring for 3 hours while warming to room temperature. To the reaction liquid was added a saturated aqueous ammonium chloride solution, followed by extraction with ethyl acetate, and the organic layer was washed with water and saturated brine, and then dried over anhydrous sodium sulfate. The residue was purified by silica gel column chromatography (hexane:ethyl acetate=3:1) to obtain the desired product (1.61 g) as a pale yellow powder.
¹H NMR (CDCl₃, 400 MHz): δ 1.23 (3H, t, J=7.3 Hz), 1.39 (6H, d, J=6.7 Hz), 1.39, (9H, s), 2.41 (1H, dd, J=16.6, 5.5 Hz), 2.92 (1H, dd, J=16.6, 9.2 Hz), 3.31-3.42 (1H, m), 3.89-3.99 (1H, m), 4.14 (3H, s), 7.04 (1H, d, J=8.6 Hz), 7.49 (1H, d, J=9.2 Hz), 8.09 (1H, d, J=8.6 Hz), 9.02 (1H, d, J=9.2 Hz).

### <Example 64>

### t-Butyl 4-(8-methoxy-2-trifluoromethylquinolin-5-yl)-4-oxobutyrate ester

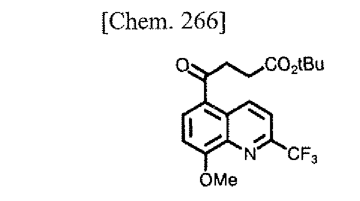

The compound of Example 40 (1.83 g) was dissolved in THF (50 mL) under an argon atmosphere, and a solution (1.0 mol/L, 7.48 mL) of lithiumhexadisilazane in THF was added thereto at 0°C, followed by stirring for 20 minutes. t-Butyl bromoacetate (1.50 mL) was added thereto, followed by stirring at room temperature for 3 hours. A saturated aqueous ammonium chloride solution was added thereto, followed by extraction three times with ethyl acetate, and the combined organic layer was washed with saturated brine, dried over sodium sulfate, and then filtered. The solvent of the filtrate was evaporated under reduced pressure and then purified by silica gel column chromatography (hexane:ethyl acetate=4:1) to obtain the desired product (405 mg) as a yellow powder.
¹H NMR (CDCl₃, 400 MHz): δ 1.46 (9H, s), 2.75 (2H, t, J=6.7 Hz), 3.34 (2H, t, J=6.7 Hz), 4.18 (3H, s), 7.12 (1H, d, J=8.6 Hz), 7.87 (1H, d, J=9.2 Hz), 8.29 (1H, d, J=8.6 Hz), 9.59 (1H, d, J=9.2 Hz).

### <Example 65>

### t-Butyl 4-(2-ethyl-5-methoxyindolidin-8-yl)-3-methyl-4-oxobutyrate ester

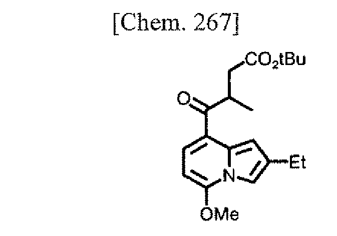

The compound of Example 52 (392 mg) was dissolved in THF (12 mL) under an argon atmosphere, and a solution (1.0 mol/L, 1.86 mL) of lithium hexamethyl disilazane in THF was added thereto at -78°C, followed by stirring at -78°C to 0°C for 1 hour. t-Butyl bromoacetate (0.322 mL) was added thereto at 0°C, followed by stirring at room temperature for 1.5 hours. A saturated aqueous ammonium chloride solution was added thereto, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (hexane:ethyl acetate=4:1) to obtain the desired product (455 mg) as a yellow powder.
¹H NMR (CDCl₃, 400 MHz): δ 1.22 (3H, d, J=6.7 Hz), 1.30 (3H, t, J=7.3 Hz), 1.39 (9H, s), 2.36 (1H, dd, J=16.2, 6.4 Hz), 2.74 (2H, q, J=7.3 Hz), 2.86 (1H, dd, J=16.2, 7.6 Hz), 3.89-3.95 (1H, m), 4.12 (3H, s), 5.82 (1H, d, J=7.9 Hz), 7.2 (1H, d, J=1.8 Hz), 7.28 (1H, d, J=1.8 Hz), 7.66 (1H, d, J=7.9 Hz).

### <Example 66>

### t-Butyl 4-(7-methoxy-2-trifluoromethylbenzofuran-4-yl)-3-methyl-4-oxobutyrate ester

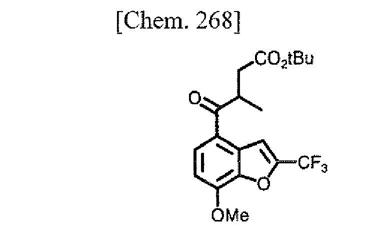

The compound of Example 58 (445 mg) was suspended in THF (15 mL) under an argon gas atmosphere, and a solution (1 mol/L, 1.79 mL) lithium hexamethyl disilazane in THF was added dropwise at -78°C, followed by slowly warming. When all of the insoluble materials were dissolved, t-butyl bromoacetate (0.359 mL) was added thereto, followed by warming to room temperature. A saturated aqueous ammonium chloride solution was added thereto, followed by extraction with ethyl acetate and washing with saturated brine. It was dried over anhydrous sodium sulfate, the solvent was then evaporated, and the residue was purified by silica gel column chromatography (hexane:ethyl acetate=10:1→6:1) to obtain the desired product (282mg) as a colorless powder.
¹H NMR (CDCl₃, 400 MHz): δ 1.23 (3H, d, J=7.0 Hz), 1.38 (9H, s), 2.40 (1H, dd, J=16.4, 5.5 Hz), 2.90 (1H, dd, J=16.4, 8.6 Hz), 3.92-3.97 (1H, m), 4.11 (3 H, s), 6.96 (1H, d, J=8.3 Hz), 7.98 (1H, d, J=8.3 Hz), 7.99 (1H, s).

### <Example 67>

### 4-(8-Methoxy-2-methylquinolin-5-yl)-4-oxobutyric acid

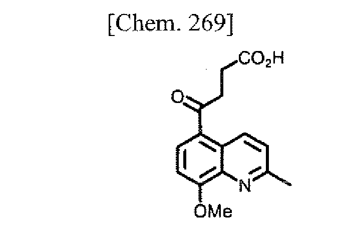

The compound of Example 16 (7.24 g) was dissolved in THF (290 mL) under an argon gas atmosphere, and an n-butyl lithium/hexane solution (1.58 mol/L, 20.1 mL) was added dropwise thereto at -78°C, followed by stirring at the same temperature for 5 minutes. Thereafter, a succinic anhydride (4.33 g)/THF (92 mL) solution was added thereto at -78°C, followed by stirring at -78°C for 30 minutes. To the reaction liquid was added a saturated aqueous ammonium chloride solution, followed by making the liquid property alkaline (pH 11) with the addition of a 10% aqueous sodium hydroxide solution and extraction with a 5% aqueous sodium hydroxide solution. The liquid property of the obtained aqueous layer was made acidic (pH 4) by the addition of concentrated hydrochloric acid, and the precipitate was collected by filtration to obtain the desired product (2.65 g) as a yellow powder.
¹H NMR (CDCl₃, 400 MHz): δ 2.79 (3H, s), 2.86 (2H, t, J=6.8 Hz), 3.40 (2H, t, J=6.8 Hz), 4.14 (3H, s), 7.02 (1H, d, J=8.6 Hz), 7.44 (1H, d, J=9.2 Hz), 8.11 (1H, d, J=8.6 Hz), 9.27 (1H, d, J=9.2 Hz).

### <Example 68>

### 4-(8-Methoxy-2-isopropylquinolin-5-yl)-4-oxobutyric acid

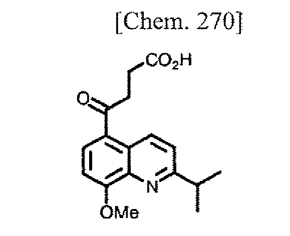

The compound of Example 17 (1.77 g) was dissolved in THF (58 mL) under an argon gas atmosphere, and an n-butyl lithium/hexane solution (1.60 mol/L, 3.99 mL) was added dropwise thereto at -78°C, followed by stirring at the same temperature for 5 minutes. Thereafter, a solution of succinic anhydride (1.40 g) in THF (22 mL) was added at once at -78°C, followed by stirring at -78°C for 30 minutes. To the reaction liquid was added a saturated aqueous ammonium chloride solution, followed by making the liquid property alkaline (pH 11) by the addition of a 10% aqueous sodium hydroxide solution and extracting with a 5% aqueous sodium hydroxide solution. The liquid property of the obtained aqueous layer was made acidic (pH 4) by the addition of concentrated hydrochloric acid, followed by extraction with ethyl acetate, and the extracted layer was washed with saturated brine and then dried over anhydrous sodium sulfate. The solvent was evaporated to obtain the desired product (1.14 g) as a yellow powder in the amorphous state.
¹H NMR (CDCl₃, 400 MHz): δ 1.38 (6H, d, J=7.3 Hz), 2.86 (2H, t, J=6.7 Hz), 3.32-3.41 (3H, m), 4.13 (3H, s), 7.01 (1H, d, J=8.6 Hz), 7.51 (1H, d, J=9.2 Hz), 8.11 (1H, d, J=8.6 Hz), 9.31 (1H, d, J=9.2 Hz).

### <Example 69>

### 6-(2-Amino-3-methoxypyridin-6-yl)-4,5-dihydro-5-methyl-3-(2H)-pyridazinone

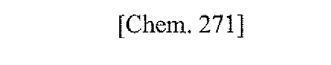

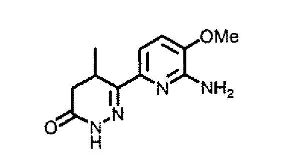

To a solution of the compound of Example 60 (733 mg) in methanol (30 mL) was added thereto a 4 mol/L aqueous potassium hydroxide solution (1.57 mL), followed by stirring at 60°C for 4 hours. The reaction liquid was concentrated, and then the residue was acidified with a 1 mol/L hydrochloric acid aqueous solution and extracted with ethyl acetate (150mL). The organic layer was washed with water and saturated brine, and then dried over anhydrous sodium sulfate. The solvent was concentrated under reduced pressure, and the residue was then dissolved in ethanol (50 mL), and hydrazine monohydrate (0.228 mL) was added thereto, followed by heating to reflux for 3 hours. The reaction liquid was concentrated, the residue was then dissolved in ethyl acetate (100 mL), and filtered through NH type silica gel (Chromatorex: registered trademark), and the filtrate was concentrated under reduced pressure. To the residue was added 10% hydrochloric acid-methanol (100 mL), followed by leaving to stand at room temperature overnight, and the reaction liquid was then concentrated. The residue was dissolved in methylene chloride (15 mL), and trifluoroacetic acid (15 mL) was added thereto, followed by leaving to stand at room temperature overnight. The reaction liquid was concentrated, and neutralized with a saturated aqueous sodium hydrogen carbonate solution. The precipitated crystal was collected by filtration and the filtrate was extracted with chloroform (200 mL). The extract was washed with saturated brine and then dried over anhydrous sodium sulfate, and the solvent was concentrated under reduced pressure, and combined with the crystal that had been first collected by filtration to obtain the desired product (328 mg) as a colorless powder. ¹H NMR (CDCl₃, 400 MHz): δ 1.21 (3H, d, J=7.3 Hz), 2.43 (1H, d, J=16.5 Hz), 2.69 (1H, dd, J=6.7, 16.5 Hz), 3.61-3.73 (1H, m), 3.88 (3H, s), 5.04 (2H, brs), 6.93 (1H, d, J=7.9 Hz), 7.21 (1H, d, J=7.9 Hz), 9.17 (1H, brs).

### <Example 70>

### 6-(5-Methoxy-2-trifluoromethylimidazo[1,2-a]pyridin-8-yl)-4,5-dihydro-5-methyl-3-(2H)-pyridazinone

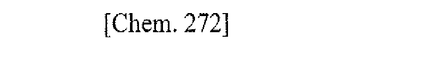

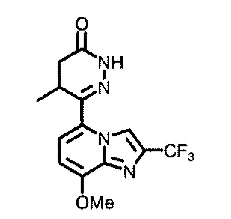

To a solution of the compound of Example 69 (328 mg) in ethanol (50 mL) was added 3-bromo-1,1,1-trifluoroacetone (535 mg), followed by stirring at 70°C for 5 hours. Then, 3-bromo-1,1,1-trifluoroacetone (535 mg) was added thereto, followed by stirring at 75°C for 72 hours. The reaction liquid was concentrated, the residue was extracted with chloroform (300 mL), and the extract was washed with 1 mol/L aqueous hydrochloric acid solution, a saturated aqueous sodium hydrogen carbonate solution, washed with saturated brine, and then dried over anhydrous sodium sulfate. The solvent was concentrated under reduced pressure and purified by recrystallization (chloroform-diisopropyl ether) to obtain the desired product (87.1 mg) as a colorless powder.
¹H NMR (CDCl₃, 400 MHz): δ 1.35 (3H, d, J=7.3 Hz), 2.58 (1H, dd, J=17.1, 1.2 Hz), 2.80 (1H, dd, J=17.1, 6.7 Hz), 3.37-3.48 (1H, m), 4.11 (3H, s), 6.66 (1H, d, J=7.9 Hz), 7.26 (2H, d, J=7.9 Hz), 8.77 (1H, brs), 9.34 (1H, s).
EIMS (+): 326 [M]⁺.
Elemental analysis: Found value C 51.34%, H 3.95%, N 17.04%, Calculated value as C₁₄H₁₃F₃N₄O₂ C 51.54%, H 4.00%, N 17.17%.

### <Example 71>

### 6-(8-Methoxy-2-trifluoromethylquinolin-5-yl)-4,5-dihydro-3-(2H)-pyridazinone

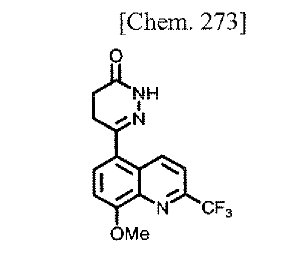

The compound of Example 64 (404 mg) was dissolved in methylene chloride (10 mL), and trifluoroacetic acid (4.0 mL) was added thereto, followed by stirring at room temperature for 1.5 hours. After evaporating the solvent under reduced pressure, the residue was dissolved in ethanol (10 mL), and hydrazine monohydrate (0.152 mL) was added thereto, followed by stirring for 1 hour to heating under reflux. After evaporating the solvent under reduced pressure, the residue was purified by silica gel column chromatography (ethyl acetate) to obtain the desired product (286 mg) as a yellow powder.
¹H NMR (CDCl₃, 400 MHz): δ 2.73 (2H, t, J=7.9 Hz), 3.12 (2H, t, J=7.9 Hz), 4.15 (3H, s), 7.1 (1H, d, J=8.6 Hz), 7.70 (1H, d, J=8.6 Hz), 7.83 (1H, d, J=8.6 Hz), 8.61 (1H, s), 9.17 (1H, d, J=8.6 Hz).

### <Example 72>

### 6-(2-Ethyl-8-methoxyquinolin-5-yl)-4,5-dihydro-5-methyl-3-(2H)-pyridazinone

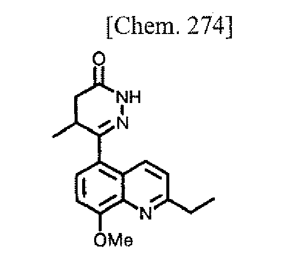

The compound of Example 61 (889 mg) was dissolved in methylene chloride (25 mL), and trifluoroacetic acid (10 mL) was added thereto, followed by stirring at room temperature for 3 hours. After evaporating the solvent under reduced pressure, the residue was dissolved in ethanol (25 mL), and acetic acid (0.94 mL) and hydrazine monohydrate (0.36 mL) were added thereto, followed by stirring for 7 hours to heating under reflux. To the reaction liquid was added water, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (ethyl acetate) to obtain the desired product (229 mg) as a yellow amorphous.
HREIMS (+): 297.1491 (Calculated value as C₁₇H₁₉N₃O₂ 297.1477).
¹H NMR (CDCl₃, 400 MHz): δ 1.21 (3H, d, J=7.3 Hz), 1.41 (3H, t, J=7.3 Hz), 2.54 (1H, dd, J=17.1, 3.1 Hz), 2.89 (1H, dd, J=17.1, 6.9 Hz), 3.09 (2H, q, J=7.3 Hz), 3.25-3.29 (1H, m), 4.12 (3H, s), 7.05 (1H, d, J=8.6 Hz), 7.43 (1H, d, J=8.6 Hz), 7.49 (1H, d, J=8.6 Hz), 8.56 (1H, brs), 8.57 (1H, d, J=8.6 Hz).

### <Example 73>

### 6-(8-Methoxy-2-trifluoromethylquinolin-5-yl)-4,5-dihydro-5-methyl-3-(2H)-pyridazinone

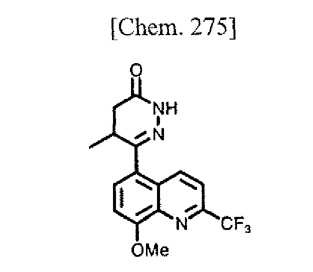

The compound of Example 45 (800 mg) was dissolved in THF (28 mL) under an argon atmosphere, and a solution (1.00 mol/L, 3.38 mL) of lithium hexamethyl disilazane in THF was added thereto at 0°C, followed by stirring at the same temperature for 30 minutes. t-Butyl bromoacetate (0.620 mL) was added thereto, followed by stirring at room temperature for 3 hours. To the reaction liquid was added water, followed by extraction three times with ethyl acetate, and the combined extracted layer was washed with saturated brine, dried over sodium sulfate, and then filtered. After evaporating the solvent under reduced pressure, the residue was dissolved in methylene chloride (20 mL), and trifluoroacetic acid (10 mL) was added thereto, followed by stirring at room temperature for 5 hours. After evaporating the solvent under reduced pressure, the residue was dissolved in ethanol (25 mL), and acetic acid (1.07 mL) and hydrazine monohydrate (0.410 mL) were added thereto, followed by stirring for 14 hours to heating under reflux. To the reaction liquid was added water, followed by extraction three times with ethyl acetate, and the combined extracted layer was washed with saturated brine, dried over sodium sulfate, and then filtered. After evaporating the solvent of the filtrate under reduced pressure, the residue was purified by silica gel column chromatography (ethyl acetate) to obtain the desired product (293 mg) as a yellow powder. Elemental analysis: Found value C 56.67%, H 4.13%, N 12.36%, Calculated value as C₁₆H₁₄F₃N₃O₂ C 56.97%, H 4.18%, N 12.46%.
¹H NMR (CDCl₃, 400 MHz): δ 1.24 (3H, d, J=7.3 Hz), 2.58 (1H, dd, J=16.8, 3.4 Hz), 2.90 (1H, dd, J=16.8, 6.7 Hz), 3.29-3.32 (1H, m), 4.15 (3H, s), 7.16 (1H, d, J=8.3 Hz), 7.70 (1H, d, J=8.3 Hz), 7.82 (1H, d, J=9.2 Hz), 8.65 (1H, brs), 8.97 (1H, d, J=9.2 Hz).

### <Example 74>

### 6-(8-Methoxyquinolin-5-yl)-4,5-dihydro-5-methyl-3-(2H)-pyridazinone

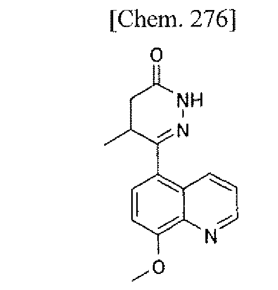

To a solution of the compound of Example 46 (600 mg) in THF (25.0 mL) was added hexamethyl phosphoric acid triamide (2.5 mL), and then a solution (1.00 mol/L, 3.34 mL) of hexamethyl disilazane in THF was added thereto at 0°C, followed by stirring at 0°C for 30 minutes. Then, t-butyl bromoacetate (0.617 mL) was added thereto, followed by stirring at room temperature for 2 hours. To the reaction liquid was added a saturated aqueous ammonium chloride solution, followed by extraction with ethyl acetate, the organic layer was washed with water, and dried over anhydrous sodium sulfate, and the solvent was evaporated. The residue was dissolved in methylene chloride (6 mL), and trifluoroacetic acid (6 mL) was added thereto, followed by stirring at room temperature for 1 hour. Then, the solvent and the like were evaporated under reduced pressure. To the residue was added ethanol (25 mL), and hydrazine monohydrate (0.541 mL), followed by heating to reflux for 4 hours. The reaction liquid was concentrated to 5 mL, and then to the residue was added ice water, followed by extraction with methanol-containing chloroform
(chloroform:methanol=9:1). The extracted layer was dried over anhydrous sodium sulfate and the solvent was evaporated under reduced pressure. The residue was purified by silica gel column chromatography (ethyl acetate:methanol=9:1) to obtain the desired product (377 mg) as a colorless powder.
Elemental analysis: Found value C 66.75%, H 5.64%, N 15.43%, Calculated value as C₁₅H₁₅N₃O₂ C 66.90%, H 5.61%, N 15.60%.
EIMS (+): 269 [M]⁺.
¹H NMR (CDCl₃, 400 MHz): δ 1.22 (3H, d, J=7.3 Hz), 2.56 (1H, dd, J=17.1, 3.7 Hz), 2.90 (1H, dd, J=17.1, 6.7 Hz), 3.25-3.33 (1H, m), 4.14 (3H, s), 7.08 (1H, d, J=7.9 Hz), 7.51 (1H, dd, J=8.6, 4.3 Hz), 7.57 (1H, d, J=7.9 Hz), 8.65 (1H, s), 8.68 (1H, dd, J=8.6, 1.8 Hz), 8.98 (1H, dd, J=4.3, 1.8 Hz).

### <Example 75>

### 6-(8-Methoxy-2-methylquinolin-5-yl)-4,5-dihydro-5-methyl-3-(2H)-pyridazinone

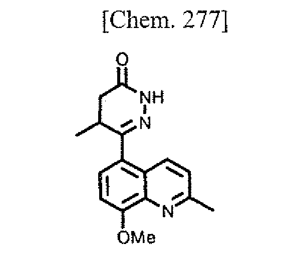

The compound of Example 62 (8.64 g) was dissolved in methylene chloride (220 mL), and trifluoroacetic acid (110 mL) was added thereto, followed by stirring at room temperature for 2.5 hours. After evaporating the solvent under reduced pressure, the residue was azeotroped twice with toluene. To the residue were added ethanol (220 mL) and hydrazine monohydrate (4.39 mL), followed by stirring for 27 hours to heating under reflux. After evaporating the solvent under reduced pressure, water was added thereto, followed by extraction with ethyl acetate. The organic layer was washed with water and saturated brine, and then dried over anhydrous sodium sulfate. After evaporating the solvent under reduced pressure, the residue was purified by silica gel column chromatography (ethyl acetate) to obtain the desired product (1.90 g) as a pale yellow powder.
Elemental analysis: Found value C 67.37%, H 6.04%, N 14.41%, Calculated value as C₁₆H₁₇N₃O₂ · 1/5H₂O C 66.98%, H 6.11%, N 14.64%.
EIMS (+): 283 [M]⁺.
¹H NMR (CDCl₃, 400 MHz): δ 1.21 (3H, t, J=7.4 Hz), 2.55 (1H, dd, J=17.1, 3.0 Hz), 2.81 (3H, s), 2.89 (1H, dd, J=17.1, 7.3 Hz), 3.24-3.32 (1H, m), 4.12 (3H, s), 7.05 (1H, d, J=8.6 Hz), 7.38 (1H, d, J=8.6 Hz), 7.50 (1H, d, J=8.6 Hz), 8.54 (1H, d, J=8.6 Hz), 8.59 (1H, brs).

### <Example 76>

### 6-(8-Methoxy-2-methylquinolin-5-yl)-4,5-dihydro-3-(2H)-pyridazinone

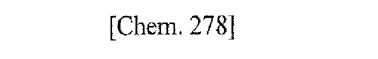

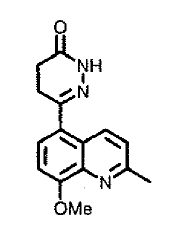

The compound of Example 67 (2.65 g) was dissolved in ethanol (280 mL), and hydrazine monohydrate (4.19 mL) was added thereto, followed by stirring for 6.5 hours to heating under reflux. To the reaction liquid was added water, followed by extraction with chloroform and drying over anhydrous magnesium sulfate. After evaporating the solvent under reduced pressure, the residue was purified by silica gel column chromatography (ethyl acetate:methanol=10:1) to obtain the desired product (2.78 g) as a yellow powder.
¹H NMR (CDCl₃, 400 MHz): δ 2.70 (2H, dd, J=8.6, 8.0 Hz), 2.81 (3H, s), 3.08 (2H, dd, J=8.6, 8.0 Hz), 4.12 (3H, s), 7.05 (H, d, J=8.6 Hz), 7.39 (1H, d, J=8.6 Hz), 7.51 (1H, d, J=8.6 Hz), 8.59 (1H, brs), 8.74 (1H, d, J=8.6 Hz).

### <Example 77>

### 6-(8-Methoxy-2-isopropylquinolin-5-yl)-4,5-dihydro-5-methyl-3-(2H)-pyridazinone

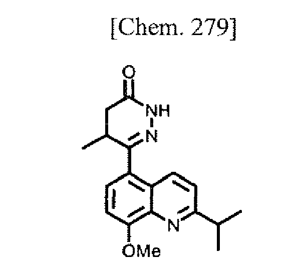

The compound of Example 63 (1.61 g) was dissolved in methylene chloride (220 mL), and trifluoroacetic acid (21 mL) was added thereto, followed by stirring at room temperature for 3.5 hours. After evaporating the solvent under reduced pressure, the residue was azeotroped twice with toluene. To the residue were added ethanol (43 mL) and hydrazine monohydrate (0.87 mL), followed by stirring for 4 hours to heating under reflux. After evaporating the solvent under reduced pressure, to the residue was added water, followed by extraction with ethyl acetate. The organic layer was washed with water and saturated brine, and then dried over anhydrous sodium sulfate. Alter evaporating the solvent under reduced pressure, the residue was purified by silica gel column chromatography (ethyl acetate) to obtain the desired product (645 mg) as a white solid.
Elemental analysis: Found value C68.34%, H 6.67%, N 13.06%, Calculated value as C₁₈H₂₁N₃O₂ · 1/3H₂O C68.12%, H 6.88%, N 13.24%.
EIMS (+): 311 [M]⁺.
¹H NMR (CDCl₃, 400 MHz): δ 1.21 (3H, t, J=7.4 Hz), 1.39 (3H, d, J=6.7 Hz), 1.40 (3H, d, J=6.7 Hz), 2.55 (1H, dd, J=17.1, 3.0 Hz), 2.89 (1H, dd, J=17.1, 6.7 Hz), 3.23-3.32 (1H, m), 3.34-3.45 (1H, m), 4.12 (3H, s), 7.05 (1H, d, J=8.6 Hz), 7.46 (1H, d, J=8.6 Hz), 7.51 (1H. d, J=8.0 Hz), 8.58 (1H, d, J=8.6 Hz), 8.63 (1H, brs).

### <Example 78>

### 6-(8-Methoxy-2-isopropylquinolin-5-yl)-4,5-dihydro-3-(2H)-pyridazinone

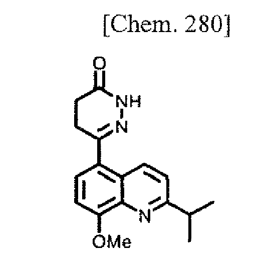

The compound of Example 68 (1.14 g) was dissolved in ethanol (38 mL), and hydrazine monohydrate (0.55 mL) was added thereto, followed by stirring for 2.5 hours to heating under reflux. After evaporating the solvent of the reaction liquid under reduced pressure, the residue was purified by silica gel column chromatography (ethyl acetate) to obtain the desired product (795 mg) as a colorless powder.
¹H NMR (CDCl₃, 400 MHz): δ 1.39(6H, d, J=6.7 Hz), 2.70 (2H, dd, J=8.6, 6.7 Hz), 3.08 (2H, dd, J=8.6, 6.7 Hz), 3.34-3.44 (1H, m), 4.12 (3H, s), 7.04 (1H, d, J=8.6 Hz), 7.47 (1H, d, J=9.2 Hz), 7.51 (1H, d, J=8.6 Hz), 8.66 (1H, brd, J=14.7 Hz), 8.77 (1H, d, J=9.2 Hz).

### <Example 79>

### 6-(2-Ethyl-5-methoxyindolidin-8-yl)-4,5-dihydro-5-methyl-3-(2H)-pyridazinone

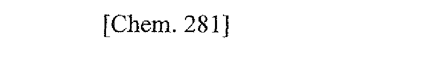

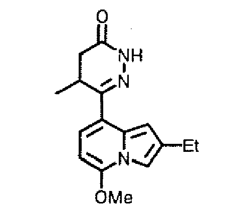

The compound of Example 65 (455 mg) was dissolved in methylene chloride (15 mL), and trifluoroacetic acid (7.0 mL) was added thereto, followed by stirring at room temperature for 1.5 hours. After evaporating the solvent under reduced pressure, the residue was dissolved in ethanol (13 mL), and acetic acid (0.499 mL) and hydrazine monohydrate (0.192 mL) were added thereto, followed by stirring for 8 hours to heating under reflux. To the reaction liquid was added water, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=1:1) to obtain the desired product (110 mg) as a yellow powder.
Elemental analysis: Found value C 67.10%, H 6.76%, N 14.47%, Calculated value as C₁₆H₁₉N₃O₂ C 67.35%, H 6.71%, N 14.73%.
¹H NMR (CDCl₃, 400 MHz): δ 1.14 (3H, d, J=7.3 Hz), 1.28 (3H, t, J=7.3 Hz), 2.29 (1H, d, J=16.5 Hz), 2.68-2.77 (3H, m), 3.49-3.52 (1H, m), 4.15 (3H, s), 6.13 (1H, d, J=7.3 Hz), 7.09 (1H, s), 7.30 (1H, d, J=7.3 Hz), 7.33 (1H, s), 10.96 (1H, s).

### <Example 80>

### 6-(2-Ethyl-5-methoxy-[1,2,4]triazolo[1,5-a]pyridin-8-yl)-4,5-dihydro-5-methyl-3-(2H)-pyridazinone

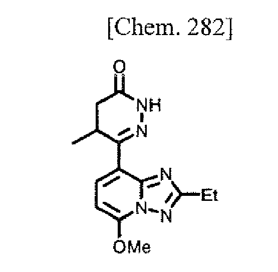

The compound of Example 55 (83.7 mg) was dissolved in THF (3.6 mL) under an argon atmosphere, and a solution (1.0 mol/L, 0.466 mL) of lithium hexamethyl disilazane in THF was added thereto at 0°C, followed by stirring for 1 hour. t-Butyl bromoacetate (0.790 mL) was added thereto, followed by stirring at room temperature for 2 hours. To the reaction liquid was added water, followed by extraction three times with ethyl acetate, and the combined extracted layer was washed with saturated brine, dried over anhydrous sodium sulfate, and then filtered. After evaporating the solvent of the filtrate under reduced pressure, the residue was dissolved in methylene chloride (4.0 mL), and trifluoroacetic acid (2.0 mL) was added thereto, followed by stirring at room temperature for 2 hours. After evaporating the solvent under reduced pressure, the residue was dissolved in ethanol (3.6 mL), and acetic acid (0.181 mL) and hydrazine monohydrate (0.696 mL) were added thereto, followed by stirring for 5.5 hours under the condition of heating to reflux. To the reaction liquid was added water, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=2:3) and washed with diisopropyl ether to obtain the desired product (17.4 mg) as a yellow powder.
HREIMS (+): 287.1377 (Calculated value as C₁₄H₁₇N₅O₂ 287.1382).
¹H NMR (CDCl₃, 400 MHz): δ 1.26 (3H, d, J=7.3 Hz), 1.44 (3H, t, J=7.3 Hz), 2.52 (1H, dd, J=17.1, 1.8 Hz), 2.84 (1H, dd, J=17.1, 7.0 Hz), 2.99 (2H, q, J=7.3 Hz), 4.22 (3H, s), 4.27-4.32 (1H, m), 6.35 (1H, d, J=8.6 Hz), 7.96 (1H, d, J= 8.6 Hz), 8.51 (1H, brs).

### <Example 81>

### 6-(5-Methoxy-2-trifluoromethyl-[1,2,4]triazolo[1,5-a]pyridin-8-yl)-4,5-dihydro-5-methyl-3-(2H)-pyridazinone

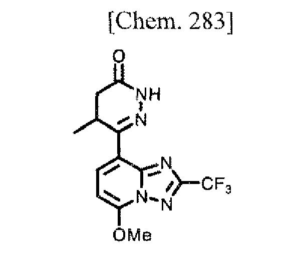

The compound of Example 57 (342 mg) was dissolved in THF (13 mL) under an argon atmosphere, and a solution (1.0 mol/L, 1.34 mL) of lithium hexamethyl disilazane in THF was added thereto at -78°C, followed by stirring for 30 minutes. t-Butyl bromoacetate (0.277 mL) was added thereto, followed by stirring at room temperature for 1 hour. To the reaction liquid was added water, followed by extraction three times with ethyl acetate, and the combined extracted layer was washed with saturated brine, dried over anhydrous sodium sulfate, and then filtered. After evaporating the solvent of the filtrate under reduced pressure, the residue was dissolved in methylene chloride (15 mL), and trifluoroacetic acid (5.0 mL) was added thereto, followed by stirring at room temperature for 2 hours. After evaporating the solvent under reduced pressure, the residue was dissolved in ethanol (10 mL), and acetic acid (0.472 mL) and hydrazine monohydrate (0.182 mL) were added thereto, followed by stirring for 7 hours under the condition of heating to reflux. To the reaction liquid was added water, followed by extraction three times with ethyl acetate, and the combined extracted layer was washed with saturated brine, dried over anhydrous sodium sulfate, and then filtered. After evaporating the solvent of the filtrate under reduced pressure, the residue was purified by silica gel column chromatography (hexane:ethyl acetate=3:1) and washed with diisopropyl ether to obtain the desired product (166 mg) as a yellow powder.
Elemental analysis: Found value C 47.73%, H 3.82%, N 21.10%, Calculated value as C₁₃H₁₂F₃N₅O₂ C 47.71%, H 3.70%, N 21.40%.
¹H NMR (CDCl₃, 400 MHz): δ 1.27 (3H, d, J=7.3 Hz), 2.54 (1H, dd, J=17.1, 1.8 Hz), 2.84 (1H, dd, J=17.1, 6.7 Hz), 4.27 (3H, s), 4.27-4.31 (1H, m), 6.56 (1H, d, J=8.2 Hz), 8.19 (1H, d, J=8.2 Hz), 8.58 (1H, brs).

### <Example 82>

### 6-(7-Methoxy-2-trifluoromethylbenzofuran-4-yl)-4,5-dihydro-5-methyl-3-(2H)-pyridazinone

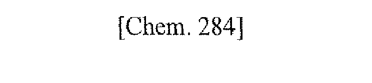

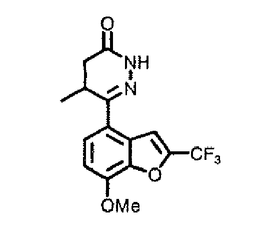

The compound of Example 66 (300 mg) was dissolved in methylene chloride (5 mL), and trifluoroacetic acid (2 mL) was added thereto under stirring with ice cooling. After stirring at room temperature overnight, the solvent and trifluoroacetic acid were evaporated under reduced pressure to obtain a red powder of a carboxylic acid product. This was dissolved in ethanol (10 mL), and acetic acid (0.294 mL) and hydrazine monohydrate (0.113 mL) were added thereto, followed by heating to reflux for 2 hours. To the reaction liquid was added water, and the precipitated crystal was collected by filtration, dissolved in ethyl acetate, and then dried over anhydrous sodium sulfate. After evaporating the solvent under reduced pressure, the residue was purified by silica gel column chromatography (ethyl acetate), suspended in hexane, and then collected by filtration to obtain the desired product (208 mg) as a colorless powder.
HREIMS (+): 326.0886 (Calculated value as C₁₅H₁₃F₃N₂O₃ 326.0878).
Elemental analysis: Found value C 55.13%, H 4.02%, N 8.42%, Calculated value as C₁₅H₁₃F₃N₂O₃ C 55.22%, H 4.02%, N 8.59%.
¹H NMR (CDCl₃, 400 MHz): δ 1.30 (3H, d, J=7.3 Hz), 2.52 (1H, dd, J=16.8, 0.6 Hz), 2.77 (1H, dd, J=16.8, 6.7 Hz), 3.40-3.48 (1H, m), 4.07 (3H, s), 6.93 (1H, d, J=8.3 Hz), 7.45 (1H, d, J=8.3 Hz), 7.89-7.90 (1H, m), 8.56 (1H, s).

### <Example 83>

### 6-(7-Methoxy-2-trifluoromethylbenzo[b]thiophen-4-yl)-4,5-dihydro-5-methyl-3-(2H)-pyridazinone

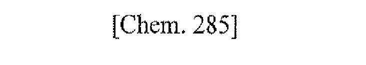

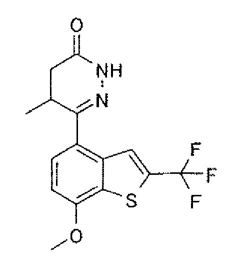

To a solution of the compound of Example 59 (230 mg) in THF (8.0 mL) was added a solution (1 mol/L, 0.878 mL) of hexamethyl disilazane in THF at -78°C, followed by stirring at 0°C for 5 minutes, and then t-butyl bromoacetate (0.176 mL) was added thereto at -78°C, followed by stirring at room temperature for 1 hour. To the reaction liquid was added a saturated aqueous ammonium chloride solution, followed by extraction with ethyl acetate, and the organic layer was dried over anhydrous magnesium sulfate. The residue obtained by evaporating the solvent under reduced pressure was purified by silica gel column chromatography (hexane:ethyl acetate=4:1) to obtain an ester form (309 mg) as a colorless oil. The obtained oil (292 mg) was dissolved in methylene chloride (5 mL), and trifluoroacetic acid (2 mL) was added thereto, followed by stirring at room temperature for 2 hours and then evaporating the solvent and the like under reduced pressure. To the residue were added ethanol (10 mL), acetic acid (0.274 mL) and hydrazine monohydrate (0.106 mL), followed by heating to reflux for 4 hours. To the reaction liquid was added water, followed by extraction with ethyl acetate, the extracted layer was washed with saturated brine and then dried over anhydrous magnesium sulfate, and the solvent was evaporated. The residue was purified by silica gel column chromatography (hexane:ethyl acetate=1:2) to obtain the desired product (197 mg) as a colorless powder.
Elemental analysis: Found value C 52.46%, H 3.76%, N 8.12%, Calculated value as C₁₅H₁₃F₃N₂O₂S C 52.62%, H 3.83%, N 8.18%.
EIMS (+): 342 [M]⁺.
¹H NMR (CDCl₃, 400 MHz): δ 1.30 (3H, d, J=7.3 Hz), 2.53 (1H, dd, J=17.1, 1.8 Hz), 2.80 (1H, dd, J=17.1, 6.7 Hz), 3.37-3.47 (1H, m), 4.06 (3H, s), 6.90 (1H, d, J=8.6 Hz), 7.58 (1H, d, J=8.6 Hz), 8.54 (1H, q, J=1.2 Hz), 8.62 (1H, s).

### <Example 84>

### 6-Chloro-5-methyl-2H-pyridazin-3-one

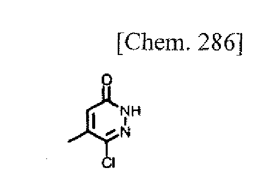

3,6-Dichloro-4-methylpyridazine (30.6 g) was added to glacial acetic acid (800 mL), followed by stirring at 110 to 115°C for 4 hours. Acetic acid was concentrated under reduced pressure, and then to the obtained residue was added a saturated aqueous sodium hydrogen carbonate solution (200 mL) to adjust the pH of the reaction liquid to 6, followed by stirring vigorously at room temperature. After extraction with methylene chloride, the extract was washed with saturated brine and dried over magnesium sulfate. The solvent was evaporated under reduced pressure, and the residue was purified by silica gel column chromatography (1% methanol/dichloromethane→5% methanol/dichloromethane) to obtain the desired product (5.00 g) as a colorless crystal.
¹H NMR (CDCl₃-CD₃OD, 200 MHz): δ 2.20 (3H, s), 2.90 (1H, brs), 6.82 (1H, s).
¹³C-NMR (CDCl₃, 50 MHz): δ 19.8, 129.6, 141.2, 144.8, 161.9.

### <Example 85>

### 5-(6-Chloropyridazin-3-yl)-8-methoxyquinoline

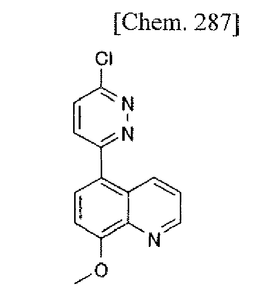

A solution of the compound of Example 14 (100 mg), bis(pinacolate)diborone (128 mg), potassium 2-ethylhexanoate (115 mg), and a [1,1'-bis(diphenylphosphino)ferrocene]dichloropalladium (II) methylene chloride complex (34.3 mg) in 1,4-dioxane (4.0 mL) was stirred at 80°C for 2 hours under an argon atmosphere. To the reaction liquid were added 1,4-dioxane (4.0 mL), 3,6-dichloropyridazine (188 mg), a 2 mol/L aqueous sodium carbonate solution, and tetrakis(triphenylphosphine)palladium (24.3 mg), followed by stirring at 100°C for 4 hours. The reaction liquid was purified by silica gel column chromatography (ethyl acetate) to obtain the desired product (65.0 mg) as a pale yellow powder.
EIMS (+): 271 [M]⁺.
¹H NMR (CDCl₃, 400 MHz): δ 4.17 (3H, s), 7.18 (1H, d, J=7.9 Hz), 7.50 (1H, dd, J=4.3, 8.6 Hz), 7.66 (1H, d, J=8.6 Hz), 7.68 (1H, d, J=7.9 Hz), 7.72 (1H, d, J=8.6 Hz), 8.61 (1H, dd, J=8.6, 1.8 Hz), 9.00 (1H, dd J=4.3, 1.8 Hz).

### <Example 86>

### 8-(6-Chloropyridazin-3-yl)-5-methoxy-2-trifluoromethyl-[1,2,4]triazolo[1,5-a]pyridine

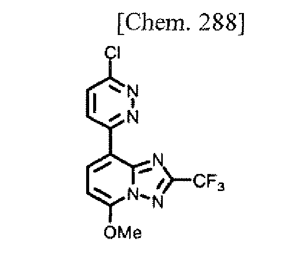

The compound of Example 30 (3.50 g), bis(pinacolate)diborone (3.60 g), a [1,1'-bis(diphenylphosphino)ferrocene]dichloropalladium (II) methylene chloride complex (964 mg), and potassium 2-ethylhexanoate (3.23 g) were dissolved in 1,4-dioxane (100 mL), followed by stirring at 80°C for 2 hours under an argon atmosphere. The reaction liquid was cooled to room temperature, and a solution of 3,6-dichlaropyridazine (5.27 g) and tetrakistriphenyl phosphine palladium (1.36 g) in 1,4-dioxane (50 mL) was then added thereto, followed by adding a 2.0 mol/L aqueous sodium carbonate solution and stirring at 100°C for 4.5 hours. To the reaction liquid was added water, followed by extraction three times with ethyl acetate, and the combined extracted layer was washed with saturated brine and then dried over anhydrous sodium sulfate. After evaporating the solvent under reduced pressure, the residue was purified by silica gel column chromatography (hexane:ethyl acetate=1:1→2:3) to obtain the desired product (644 mg) as a yellow powder.
¹H NMR (CDCl₃, 400 MHz): δ 4.32 (3H, s), 6.73 (1H, d, J=8.6 Hz), 7.68 (1H, d, J=9.2 Hz), 9.05 (1H, d, J=8.6 Hz), 9.16 (1H, d, J=9.2 Hz).

### <Example 87>

### 4-(6-Chloropyridazin-3-yl)-7-methoxybenzofuran

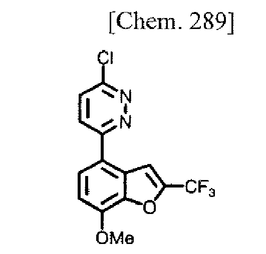

The compound of Example 33 (2.00 g), bis(pinacolate)diborone (2.07 g), a [1,1'-bis(diphenylphosphino)ferrocene]dichloropalladium (II) methylene chloride complex (831 mg), and potassium 2-ethylhexanoate (1.85 g) were dissolved in 1,4-dioxane (50.0 mL) under an argon gas atmosphere, followed by stirring at 80°C for 2 hours. After evaporating the solvent under reduced pressure, the residue was purified by silica gel column chromatograph (hexane:ethyl acetate=19:1) to obtain a pale yellow powder (2.87 g). The obtained powder (2.87 g), 3,6-dichloropyridazine (3.03 g), and tetrakistriphenyl phosphine palladium (784 mg) were dissolved in 1,4-dioxane (50.0 mL) under an argon gas atmosphere, and a 2.00 mol/L aqueous sodium carbonate solution (17.0 mL) was added thereto, followed by stirring at 100°C for 4 hours. To the reaction liquid was added water, followed by extraction with ethyl acetate, and the organic layer was washed with water and saturated brine, and then dried over anhydrous sodium sulfate. After evaporating the solvent under reduced pressure, the residue was purified by silica gel column chromatography (hexane:ethyl acetate=1:1) to obtain the desired product (1.38 g) as a colorless powder.
EIMS (+): 328 [M]⁺.
¹H NMR (CDCl₃, 400 MHz): δ 4.11 (3H, s), 7.04 (1H, d, J=8.6 Hz), 7.61 (1H, d, J=8.2 Hz), 7.67 (1H, d, J=8.6 Hz), 7.87 (1H, d, J=8.2 Hz), 8.04 (1H, s).

### <Example 88>

### 6-(8-Methoxyquinolin-5-yl)-3-(2H)-pyridazinone

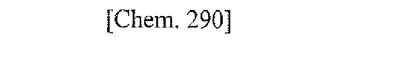

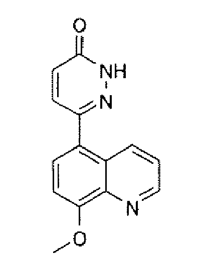

To the compound of Example 85 (400 mg) was added acetic acid (50 mL), followed by stirring at 100°C for 7 hours. To the residue obtained by evaporating acetic acid under reduced pressure was added a saturated aqueous sodium hydrogen carbonate solution, followed by extraction with ethyl acetate:methanol=9:1, and the solvent of the extracted layer was evaporated under reduced pressure. The residue was purified by silica gel column chromatography (chloroform:methanol=9:1) to obtain the desired product (351 mg) as a pale brown powder.
HREIMS (+): 253.0849 (Calculated value as C₁₄H₁₁N₃O₂ 253.0851).
¹H NMR (DMSO-d₆, 400 MHz): δ 4.01 (3H, s), 7.02 (1H, dd, J=9.8, 1.8 Hz), 7.27 (1H, d, J=8.6 Hz), 7.58 (1H, dd, J=8.6, 4.3 Hz), 7.68 (1H, d, J=8.6 Hz), 7.73 (1H, d, J=9.8 Hz), 8.51 (1H, dd, J=8.6, 1.8 Hz), 8.88 (1H, dd, J=4.3, 1.8 Hz), 13.25 (1H, brs).

### <Example 89>

### 6-(5-Methoxy-2-trifluoromethyl-[1,2,4]triazolo[1,5-a]pyridin-8-yl)-3-(2H)-pyridazinone

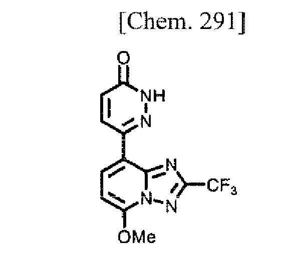

The compound of Example 86 (46.9 mg) was dissolved in acetic acid (1.5 ml), followed by stirring for 2.5 hours to heating under reflux. To the reaction liquid was added water, followed by extraction three times with ethyl acetate, and the combined extracted layer was washed with saturated brine and then dried over anhydrous sodium sulfate. The residue obtained by evaporating the solvent under reduced pressure was purified by silica gel column chromatography (hexane:ethyl acetate=2:3→ethyl acetate alone) to obtain the desired product (23.3 mg) as a colorless powder.
HRESIMS (+): 312.07044 (Calculated value as C₁₂H₉F₃N₅O₂ 312.07083).
¹H NMR (CDCl₃, 400 MHz): δ 4.29 (3H, s), 6.64 (1H, d, J=8.6 Hz), 7.14 (1H, d, J-9.8 Hz), 8.37 (1H, d, J=8.6 Hz), 8.84 (1H, d, J=9.8 Hz), 10.81 (1H, s).

### <Example 90>

### 6-(7-Methoxy-2-trifluoromethylbenzofuran-4-yl)-3-(2H)-pyridazinone

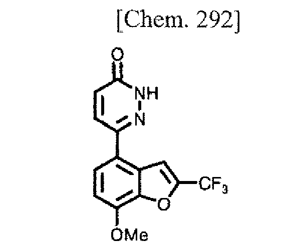

The compound of Example 87 (1.38 g) was dissolved in acetic acid (30.0 mL), followed by stirring for 2 hours to heating under reflux. To the reaction liquid was added water, and the resulting solid was collected by filtration, and washed with water. The obtained solid was dissolved in ethyl acetate, washed with a saturated aqueous sodium hydrogen carbonate solution and saturated brine, and dried over anhydrous sodium sulfate. After evaporating the solvent under reduced pressure, the desired product (1.18 g) was obtained as a colorless powder.
HREIMS (+): 310.0572 (Calculated value as C₁₄H₉F₃N₂O₃ 310.0565).
¹H NMR (CDCl₃, 400 MHz): δ 4.09 (3H, s), 6.99 (1H, d, J=8.6 Hz), 7.10 (1H, d, J=8.0 Hz), 7.51 (1H, d, J=8.6 Hz), 7.78 (1H, d, J=8.2 Hz), 7.81 (1H, s), 10.6 (1H, brs).

### <Example 91>

### 2,2-Dimethyl propionic acid-3-chloro-4-methyl-6-oxo-6H-pyridazin-1-yl methyl ester

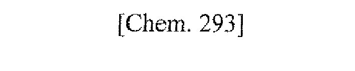

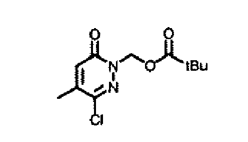

The compound of Example 84 (2.15 g) was dissolved in DMF (70 mL) under an argon atmosphere, and potassium carbonate (4.11 g) was added thereto, followed by stirring at 40 to 50°C for 20 minutes, and then chloromethyl pivalate ester (2.60 mL) was added thereto at room temperature, followed by stirring at room temperature for 18 hours. The solvent was evaporated under reduced pressure, and the residue was dissolved in ethyl acetate, washed with saturated brine, and then dried over magnesium sulfate. The solvent was concentrated and the residue was then purified by silica gel column chromatography (ethyl acetate:petroleum ether=3:7) to obtain the desired product (3.22 g) as a colorless powder.
¹H NMR (CDCl₃, 200 MHz): δ 1.35 (9H, s), 2.40 (3H, s), 6.07 (2H, s), 6.82 (1H, s). ¹³C-NMR (CDCl₃, 50 MHz): δ 19.6, 26.9, 38.8, 72.6, 129.8, 140.3, 144.2, 159.2, 177.2.

### <Example 92>

### 6-(8-Methoxy-2-trifluoromethylquinolin-5-yl)-3-(2H)-pyridazinone

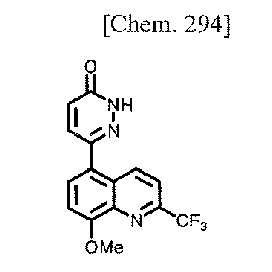

The compound of Example 71 (285 mg) was dissolved in a 0.5 mol/L aqueous sodium hydroxide solution (10 mL), and sodium m-nitrobenzene sulfonate (219 mg) was added thereto, followed by stirring for 7 hours to heating under reflux. 1 mol/L Hydrochloric acid was added there until the solution turned acidic, followed by extraction three times with ethyl acetate-THF (10-1), and the combined organic layer was washed with saturated brine and then dried over anhydrous sodium sulfate. The solvent was evaporated under reduced pressure and then purified by silica gel column chromatography (ethyl acetate) to obtain the desired product (134 mg) as a colorless powder.
Elemental analysis: Found value C 55.93%, H 3.24%, N 12.76%, Calculated value as C₁₅H₁₀F₃N₃O₂ C 56.08%, H 3.14%, N 13.08%.
¹H NMR (CDCl₃, 400 MHz): δ 4.05 (3H, s), 7.04 (1H, d, J=9.8 Hz), 7.42 (1H, d, J=8.6 Hz), 7.76 (1H, d, J=9.8 Hz), 7.86 (1H, d, J=8.6 Hz), 8.01 (1H, d, J=8.6 Hz), 8.84 (1H, d, J=8.6 Hz), 13.30 (1H, brs).

### <Example 93>

### 6-(8-Methoxy-2-methylquinolin-5-yl)-3-(2H)-pyridazinone

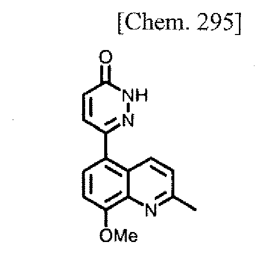

The compound of Example 76 (2.78 g) was dissolved in a 0.5 mol/L aqueous sodium hydroxide solution, and sodium m-nitrobenzene sulfonate (2.56 g) was added thereto, followed by stirring for 2 hours to heating under reflux. To the reaction liquid was added concentrated hydrochloric acid under ice cooling, followed by making the liquid property acidic (pH 4), and the precipitate was collected by filtration. The precipitate was dissolved in methanol-containing chloroform (chloroform:methanol=9:1), washed with water, and dried over anhydrous magnesium sulfate. After evaporating the solvent, the residue was washed with ethyl acetate to obtain the desired product (2.28 g) as a colorless powder.
Elemental analysis: Found value 65.67%, H 4.94%, N 15.07%, Calculated value as C₁₅H₁₃N₃O₂ · 1/3H₂O C 65.92%, H 5.04%, N 15.38%.
EIMS (+): 267 [M]⁺.
¹H NMR (CDCl₃, 400 MHz): δ 2.65 (3H, s), 3.99 (3H, s), 7.01 (1H, d, J=9.8 Hz), 7.23 (1H, d, J=7.4 Hz), 7.46 (1H, d, J=8.6 Hz), 7.57 (1H, d, J=7.4 Hz), 7.71 (1H, d, J=9.8 Hz), 8.40 (1H, d, J=8.6 Hz).

### <Example 94>

### 6-(8-Methoxy-2-isopropylquinolin-5-yl)-3-(2H)-pyridazinone

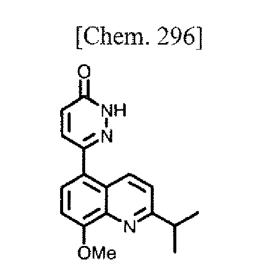

The compound of Example 78 (752 mg) was dissolved in a 0.5 mol/L aqueous sodium hydroxide solution, and sodium m-nitrobenzene sulfonate (627 mg) was added thereto, followed by stirring for 6.5 hours to heating under reflux. To the reaction liquid was added 1 mol/L hydrochloric acid under ice cooling, followed by neutralization and extraction with methanol-containing chloroform (chloroform:methanol=9:1), the extracted layer was dried over anhydrous magnesium sulfate, and the solvent was evaporated under reduced pressure. The residue was purified by silica gel column chromatography (ethyl acetate→ethyl acetate:methanol=10:1) to obtain the desired product (600 mg) as a colorless powder.
Elemental analysis: Found value C 69.27%, H 5.82%, N 14.26%, Calculated value as C₁₇H₁₇N₃O₂ C 69.14%, H 5.80%, N 14.23%.
EIMS (+): 295 [M]⁺.
¹H NMR (CDCl₃, 400 MHz): δ 1.40 (6H, d, J=7.3 Hz), 3.35-3.46 (1H, m), 4.13 (3H, s), 7.10 (1H, d, J=8.6 Hz), 7.12 (1H, d, J=9.8 Hz), 7.46 (1H, dd, J=9.8, 1.2 Hz), 7.52 (1H, d, J=8.6 Hz), 7.58 (1H, d, J=9.8 Hz), 8.46 (1H, d, J=9.2 Hz), 11.90-12.13 (1H, brm).

### <Example 95>

### 6-(2-Ethyl-8-methoxyquinolin-5-yl)-3-(2H)-pyridazinone

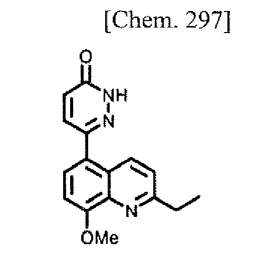

To a solution of the compound of Example 44 (1.00 g) in methanol (20 mL) was added benzyl triethyl ammonium chloride (9.93 mg), and a mixed liquid of glyoxylic acid monohydrate (602 mg) and a 1 mol/L aqueous sodium hydroxide solution (8.72 mL) was added dropwise thereto while stirring at 60°C. After stirring at 60°C for 6 hours and concentrating a part of the reaction liquid, the residue was washed with diethyl ether (50 mL). The aqueous layer was adjusted to pH 4 to 5 with a 0.5 mol/L aqueous hydrochloric acid solution and extracted with chloroform (100 mL×5). The extract was washed with saturated brine and then dried over anhydrous sodium sulfate. After concentrating the solvent under reduced pressure, the residue was dissolved in ethanol (20 mL), and hydrazine monohydrate (1.06 mL) was added thereto, followed by heating to reflux for 6 hours. The reaction liquid was concentrated, alkalified with a 1 mol/L aqueous sodium hydroxide solution, and washed with diethyl ether (50 mL). The aqueous layer was neutralized with 0.5 mol/L hydrochloric acid and extracted with chloroform (200 mL). The extract was washed with saturated brine and then dried over anhydrous sodium sulfate. The extract was concentrated under reduced pressure and then purified by recrystallization (chloroform-diisopropyl ether) to obtain the desired product (454 mg) as a brown powder.
¹H NMR (CDCl₃, 400 MHz): δ 1.41 (3H, t, J=7.9 Hz), 3.10 (2H, q, J=7.9 Hz), 4.13 (3H, s), 7.10 (1H, d, J=7.9 Hz), 7.12 (1H, d, J=9.2 Hz), 7.43 (1H, d, J=9.2 Hz), 7.52 (1H, d, J=7.9 Hz), 7.58 (1H, d, J=9.2 Hz), 8.43 (1H, d, J=9.2 Hz), 11.75 (1H, brs).

### <Examples 96 and 97>

### (+)-6-(2-Ethyl-8-methoxyquinolin-5-yl)-4,5-dihydro-5-methyl-3-(2H)-pyridazinone and (-)-6-(2-ethyl-8-methoxyquinolin-5-yl)-4,5-dihydro-5-methyl-3-(2H)-pyridazinone

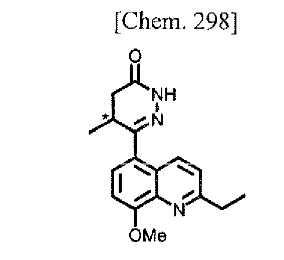

The compound of Example 72 was subjected to optical resolution by high performance liquid chromatography (Daicel CHIRALPAK IA column, Eluent: hexane:ethyl acetate=10:90, Flow rate: 3.0 mL/min, and Detection: UV 293 nm) to obtain a (+) form of Example 96 from the former eluted portion and a (-) form of Example 97 from the latter eluted portion, as a colorless powder, respectively.
Example 96: [α]_{D}²³=+111 (c 0.56, CHCl₃)
Example 97: [α]_{D}²³=-115 (c 0.60, CHCl₃)

### <Example 98>

### 6-Acetyl-2-(di-t-butoxycarbonylamino)-3-methoxypyridine

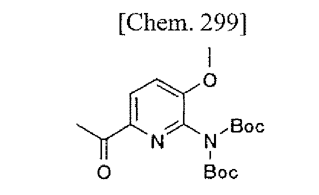

To a solution of the compound of Example 6 (4.13 g) in THF (100 mL) was added a methyl magnesium bromide-THF solution (0.82 mol/L, 36.7 mL) at -78°C under an argon atmosphere, followed by slowly warming to room temperature. To the reaction liquid was added a saturated aqueous ammonium chloride solution, followed by extraction with ethyl acetate (300 mL), and the extract was washed with water and saturated brine, and then dried over anhydrous sodium sulfate. The solvent was concentrated under reduced pressure, the residue was then dissolved in acetonitrile (100 mL), and di-tert-butyldicarbonate (8.73 g), triethylamine (2.23 g), and N,N-azxainapyridine (400 mg) were added thereto, followed by stirring at room temperature for 4 hours. The reaction liquid was concentrated under reduced pressure and then extracted with ethyl acetate (300 mL), and the extract was washed with water and saturated brine, and then dried over anhydrous sodium sulfate. The solvent was concentrated under reduced pressure, and the residue was then purified by silica gel column chromatography (hexane:ethyl acetate=4:1→2:1) to obtain the desired product (3.56 g) as a brown oil.
¹H NMR (CDCl₃, 400 MHz): δ 1.41 (18H, s), 2.64 (3H, s), 3.93 (3H, s), 7.30 (1H, d, J=8.6 Hz), 8.06 (1H, d, J=8.6 Hz).

### <Example 99>

### 6-(2-1-Butoxycarbonylamino-3-methoxypyridin-6-yl)-3-(2H)-pyridazinone

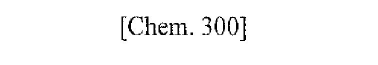

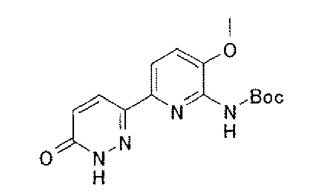

To a solution of the compound of Example 98 (1.63 g) in methanol (50 mL) was added triethylbenzyl ammonium chloride (10.1 mg), and a 1 mol/L aqueous sodium hydroxide solution (11.1 mL) of glyoxylic acid monohydrate was added thereto at 60°C, followed by stirring at 60°C for 4 hours. The reaction liquid was concentrated under reduced pressure and washed with diethyl ether (30 mL). The aqueous layer was adjusted to pH 3 to 4 with 0.5 mol/L hydrochloric acid and extracted with ethyl acetate (100 mL), and the extract was washed with water and saturated brine, and then dried over anhydrous sodium sulfate. The solvent was concentrated under reduced pressure, and the residue was then dissolved in ethanol (50 mL), and hydrazine monohydrate (1.08 mL) was added thereto, followed by reflux for 8 hours. The reaction liquid was concentrated and extracted with ethyl acetate (200 mL), and the extract was washed with water and saturated brine, and then dried over anhydrous sodium sulfate. The solvent was concentrated under reduced pressure and the residue was purified by silica gel column chromatography (hexane:ethyl acetate=3:2) to obtain the desired product (146 mg) as a pale yellow powder.
¹H NMR (CDCl₃, 400 MHz): δ 1.57 (9H, s), 3.94 (3H, s), 7.04 (1H, d, J=9.8 Hz), 7.18 (1H, d, J=8.6 Hz), 7.38 (1H, brs), 7.75 (1H, d, J=8.6 Hz), 8.49 (1H, d, J=9.8 Hz), 10.41 (1H, brs).

### <Example 100>

### 6-(8-Methoxy-2-trifluoromethyl-imidazo[1,2-a]pyridin-5-yl)-3-(2H)-pyridazinone

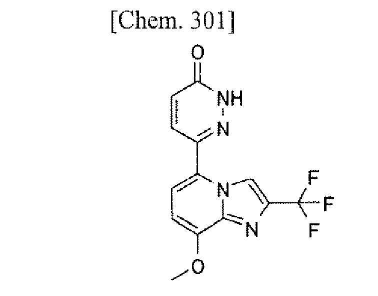

To a solution of the compound of Example 99 (146 mg) in dichloromethane (5 mL) was added thereto trifluoroacetic acid (5 mL), followed by stirring at room temperature for 3 hours. The reaction liquid was concentrated under reduced pressure and then neutralized with a saturated aqueous sodium hydrogen carbonate solution, and the precipitated crystal was collected by filtration. The obtained crystal was dissolved in ethanol (10 mL), and 3-bromo-1,1,1-trifluoroacetone (0.238 mL) was added thereto, followed by heating to reflux for 3 6 hours. The reaction liquid was concentrated under reduced pressure, neutralized with a saturated aqueous sodium hydrogen carbonate solution, and then extracted with ethyl acetate (80 mL). The extract was washed with 0.5 mol/L hydrochloric acid, a saturated aqueous sodium hydrogen carbonate solution, and saturated brine, and then dried over anhydrous sodium sulfate. The solvent was concentrated under reduced pressure and the residue was washed with diisopropyl ether to obtain the desired product (31.0 mg) as a pale yellow powder.
ESIMS (+): 311 [M+H]⁺.
HRESIMS (+) 311.07842 (Calculated value as C₁₃H₉F₃N₄O₂ 311.07558).
¹H NMR (CDCl₃, 400 MHz): δ 4.11 (3H, s), 6.69 (1H, d, J=7.9 Hz), 7.15 (1H, d, J=9.8 Hz), 7.21 (1H, d, J=7.9 Hz), 7.75 (1H, d, J=9.8 Hz), 8.97 (1H, s), 10.80 (1H, brs).

### <Example 101>

### N-t-Butoxycarbonyl-3-methoxy-2-nitroaniline

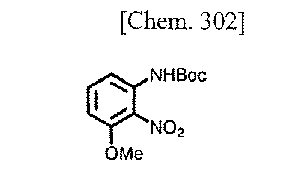

3-Methoxy-2-nitrobenzoic acid (10.0 g) was dissolved in t-butanol (50.0 mL), and diphenylphosphoryl azide (11.5 mL) and triethylamine (7.40 mL) were added thereto, followed by stirring for 10 hours to heating under reflux. After evaporating the solvent, the residue was diluted with ethyl acetate, washed with a saturated aqueous sodium hydrogen carbonate solution and saturated brine, and then dried over anhydrous sodium sulfate. After evaporating the solvent, the resulting solid was suspended in hexane and collected by filtration to obtain the desired product (13.3 g) as a yellow powder.
¹H NMR (CDCl₃, 400 MHz): δ 1.50 (9H, s), 3.90 (3H, s), 6.71 (1H, dd, J=8.6, 1.2 Hz), 7.39 (1H, dd, J=8.6, 8.6 Hz), 7.55 (1H, brs), 7.77 (1H, dd, J=8.6, 1.2 Hz).

### <Example 102>

### 3-Methoxy-2-nitroaniline

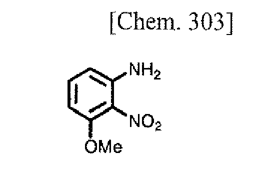

The compound of Example 1.01 (13.3 g) was dissolved in methylene chloride (100 mL), and trifluoroacetic acid (20.0 mL) was added thereto, followed by stirring at room temperature for 4 hours. After evaporating the solvent, the residue was dissolved in ethyl acetate and poured into a saturated aqueous sodium hydrogen carbonate solution. The organic layer was collected by separation, washed with saturated brine, and then dried over anhydrous sodium sulfate. After evaporating the solvent, the resulting solid was suspended in hexane and collected by filtration to obtain the desired product (7.55 g) as a yellow powder. ¹H NMR (CDCl₃, 400 MHz): δ 3.88 (3H, s), 6.31 (1H, dd, J=8.6, 1.2 Hz), 7.36 (1H, dd, J=8.6, 1.2 Hz), 7.16 (1H, dd, J=8.6, 8.6 Hz).

### <Example 103>

### 2-Amino-3-methoxyaniline

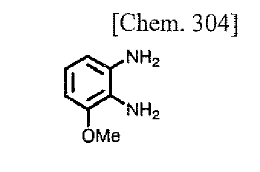

The compound of Example 102 (7.75 g) was dissolved in ethyl acetate (100 mL) and ethanol (100 mL), a few droplets of acetic acid was added thereto, and 10% palladium-activated carbon (775 mg) was added thereto, followed by stirring at room temperature for 11 hours under hydrogen atmosphere. The insoluble materials were removed by filtration through Celite and the solvent of the filtrate was then evaporated to obtain the desired product (6.49 g) as a yellowish brown oil. This was used in the reaction described in Example 104 without purification.

### <Example 104>

### 4-Methoxy-2-trifluoromethyl-1H-benzimidazole

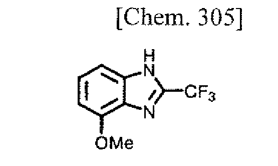

The compound of Example 103 (6.49 g) was dissolved in trifluoroacetic acid (75.0 mL) under ice cooling, followed by stirring for 5 hours to heating under reflux. After evaporating the solvent, the residue was dissolved in ethyl acetate and poured into a saturated aqueous sodium hydrogen carbonate solution. The organic layer was collected by separation, washed with saturated brine, and then dried over anhydrous sodium sulfate. After evaporating the solvent, the resulting solid was suspended in hexane and collected by filtration to obtain the desired product (8.69 g) as a yellowish brown powder.
¹H NMR (CD₃OD, 400 MHz): δ 4.01 (3H, s), 6.89 (1H, d, J=8.0 Hz), 7.24 (1H, d, J=8.0 Hz), 7.32 (1H, dd, J=8.0, 8.0 Hz).

### <Example 105>

### 7-Bromo-4-methoxy-2-trifluoromethyl-1H-benzimidazole

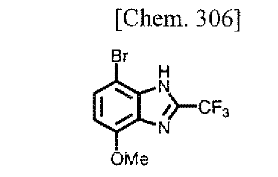

The compound of Example 104 (5.54 g) was dissolved in chloroform (130 mL), and NBS (5.02 g) was added thereto, followed by stirring at room temperature for 2 hours. To the reaction liquid was added a saturated aqueous sodium hydrogen carbonate solution, and the organic layer was collected by separation and then dried over anhydrous sodium sulfate. After evaporating the solvent, the residue was purified by silica gel column chromatography (hexane:ethyl acetate=4:1) to obtain the desired product (47.2 mg) as a pale brown powder. ¹H NMR (CDCl₃, 400 MHz): δ 4.00 (3H, s), 6.71 (1H, d, J=8.6 Hz), 7.46 (1H, d, J=8.6 Hz), 10.1 (1H,brs).

### <Example 106>

### 3-Methoxy-2-nitrophenol

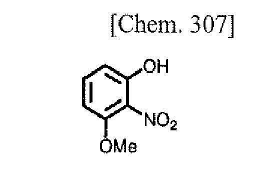

To a solution of 2-nitroresoreinol (25.0 g) in DMF (500 mL) were added potassium carbonate (6.8 g) and methyl iodide (11.0 mL), followed by stirring at room temperature for 7 hours. The solvent was evaporated under reduced pressure, and then to the residue was added water, followed by washing with ethyl acetate. The aqueous layer was acidified with hydrochloric acid and extracted with ethyl acetate. The extracted layer was washed with saturated brine and dried over anhydrous sodium sulfate, and the solvent was then evaporated under reduced pressure. The residue was purified by silica gel column chromatography (hexane:ethyl acetate=2:1) to obtain the desired product (8.78 g) as a yellow oil.
¹H NMR (CDCl₃, 400 MHz): δ 3.95 (3H, s), 6.54 (1H, dd, J=8.6, 1.2 Hz), 6.71 (1H, dd, J=8.6, 1.2 Hz), 7.40 (1H, t, J=8.6 Hz), 10.22 (1H, s).

### <Example 107>

### 2-Amino-3-methoxyphenol

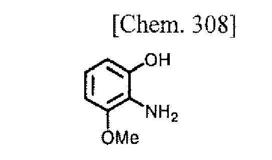

To 10%-palladium carbon (1.00 g) were added ethanol (250 mL) and the compound of Example 106 (8.78 g), followed by stirring at room temperature for 2.5 hours under hydrogen atmosphere. The insoluble materials were removed by filtration through Celite and the solvent of the filtrate was evaporated under reduced pressure to obtain the desired product (7.09 g) as a yellow powder.
EIMS (+): 139 [M]⁺
¹H NMR (CDCl₃, 400 MHz): δ 3.85 (3H, s), 6.46 (1H, d, J=7.9 Hz), 6.48 (1H, d, J=7.9 Hz), 7.40 (1H, t, J=8.6 Hz), 10.22 (1H, s).

### <Example 108>

### (3-Methoxy-2-propionylaminophenyl)propionate

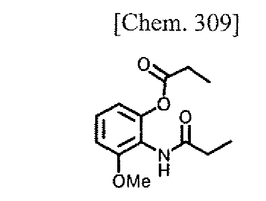

To a solution of the compound of Example 107 (1.35 g) in methylene chloride (50 mL) were added triethylamine (4.06 mL) and propionylchloride (1.86 mL), followed by stirring at room temperature for 2 hours. To the reaction liquid was added ice water, the organic layer was collected by separation, and the aqueous layer was extracted with methylene chloride. The organic layer was combined and dried over anhydrous magnesium sulfate, the solvent was evaporated under reduced pressure, and the residue was purified by silica gel column chromatography (hexane:ethyl acetate=1:2) to obtain the desired product (1.86 g) as a reddish brown powder.
¹H NMR (CDCl₃, 400 MHz): δ 1.24 (3H, t, J=7.3 Hz), 1.24 (3H, brs), 2.39 (2H, brs), 2.56 (2H, q, J=7.3 Hz), 3.84 (3H, s), 6.65 (1H, brs), 6.77 (1H, d, J=8.6 Hz), 6.81 (1H, d, J=7.9 Hz), 7.20-7.26 (1H, brm).

### <Example 109>

### N-(2-hydroxy-6-methoxy-3-propionylphenyl)propionamide

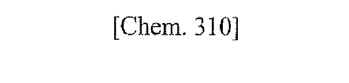

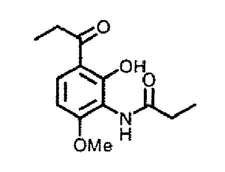

To a solution of the compound of Example 108 (4.00 g) in nitromethane (159 mL) was added titanium tetrachloride (5.25 mL), followed by stirring at room temperature for 14 hours. The reaction liquid was added to a mixed liquid of ice water (300 mL)and concentrated hydrochloric acid (10 mL), followed by extraction with ethyl acetate. The organic layer was dried over anhydrous magnesium sulfate, the solvent was then evaporated, and the residue was washed with isopropyl ether to obtain the desired product (3.39 g) as a pale yellow powder.
ESIMS (+): 251 [M]⁺.
¹H NMR (CDCl₃, 400 MHz): δ 1.23 (3H, t, J=7.3 Hz), 1.25 (3H, brs), 2.45 (2H, brs), 2.98 (2H, q, J=7.3 Hz), 3.91 (3H, s), 6.51 (1H, d, J=9.2 Hz), 6.62 (1H, brs), 7.71 (1H, d, J=9.2 Hz), 12.89 (1H, brs).

### <Example 110>

### 4-Methoxy-2-trifluoromethylbenzoxazole

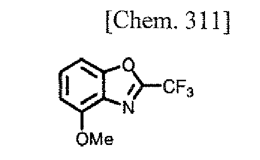

To a solution of the compound of Example 107 (2.00 g) in toluene (53 mL) was added anhydrous trifluoroacetic acid (2.20 mL), followed by heating to reflux for 5 hours. To the reaction liquid was added p-toluene sulfonic acid monohydrate (274 mg), followed by heating to reflux for 2 hours with the equipment of a Dean-Stark trap. After leaving the reaction liquid to be cooled, a saturated sodium hydrogen carbonate solution was added thereto, followed by extraction with ethyl acetate. The organic layer was dried over anhydrous magnesium sulfate, the solvent was then evaporated under reduced pressure, and the residue was purified by silica gel column chromatography (hexane:ethyl acetate=4:1 to obtain the desired product (1.84 g) as a pale yellow powder.
EIMS (+): 217 [M]⁺.
¹H NMR (CDCl₃, 400 MHz): δ 4.08 (3H, s), 6.90 (1H, d, J=7.9 Hz), 7.25 (1H, d, J=8.6 Hz), 7.46 (1H, dd, J=8.6, 7.9 Hz).

### <Example 111>

### 4-Methoxy-2-trifluoromethylbenzothiazole

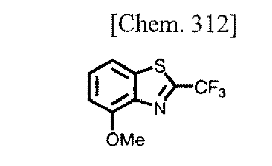

2-Amino-4-methoxybenzothiazole (26.2 g) and a 60% aqueous sodium hydroxide solution were stirred under heating at 150°C for 22 hours. After cooling, ice was added thereto, followed by adjusting to pH 5 with concentrated hydrochloric acid, and the precipitate was removed by filtration. Then, the aqueous layer was extracted with toluene. The organic layer was dried over magnesium sulfate and then concentrated to a viscous oil (2.68 g). On the other hand, to the precipitate as above was added a saturated aqueous sodium hydrogen carbonate solution, followed by extraction with toluene, and the organic layer was dried over magnesium sulfate and then concentrated to obtain a viscous oil in the similar manner. The obtained oil was combined, dissolved in trifluoroacetic acid (96 mL) and trimethylsilyl polyphosphate ester (53 mL), and reacted at 95°C for 6 hours. After cooling, the reaction liquid was added with water, adjusted to pH 8 with an aqueous sodium hydroxide solution, and extracted with methylene chloride. The organic layer was dried over magnesium sulfate and then concentrated, and the residue was purified by silica gel column chromatography (hexane:ethyl acetate=3:1) to obtain the desired product (3.46 g) as a white powder.
ELMS (+): 233 [M]⁺.
¹H NMR (CDCl₃, 400 MHz): δ 4.09 (3H, s), 7.01 (1H, dd, J=7.6, 1.5 Hz), 7.50-7.55 (2H, m).

### <Example 112>

### 7-Bromo-4-methoxy-2-trifluoromethylbenzothiazole

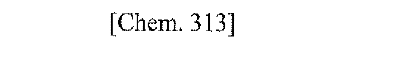

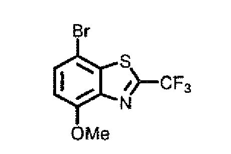

To a solution of the compound of Example 111 (3.45 g) in acetic acid (25 mL) was added dropwise a 1 mol/L bromine-acetic acid solution (15.6 mL) at room temperature, followed by stirring under heating at 75°C for 6 hours. After evaporating acetic acid, the residue was dissolved in ethyl acetate, washed with a saturated aqueous sodium bicarbonate solution, and then dried over magnesium sulfate. The solvent was concentrated under reduced pressure and the residue was purified by silica gel column chromatography (hexane:ethyl acetate=9: 1) to obtain the desired product (8.43 g) as a colorless powder.
¹H NMR (CDCl₃, 200 MHz): δ 4.06 (3H, s), 6.91 (1H, d, J=8.5 Hz), 7.59 (1H, d, J=8.5 Hz).

### <Example 113>

### 4-Methoxy-7-propionyl-2-trifluoromethyl-1H-benzimidazole

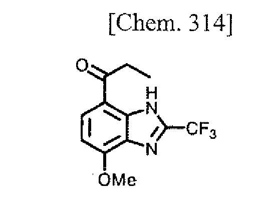

The compound of Example 105 (800 mg) was dissolved in THF (20.0 mL) under an argon gas atmosphere, and a 1.58 mol/L n-butyl lithium/hexane solution (3.90 mL) was added dropwise thereto at -78°C, followed by stirring for 1 hour as it was. Thereafter, N,N-dimethyl propionamide (0.890 mL) was added thereto at the same temperature, followed by stirring for 3 hours while warming to room temperature. To the reaction liquid was added a saturated aqueous ammonium chloride solution, followed by extraction with ethyl acetate. The organic layer was washed with water and saturated brine, and then dried over anhydrous sodium sulfate. The solvent was evaporated under reduced pressure, and the residue was then purified by silica gel column chromatography (chloroform) to obtain the desired product (342 mg) as a white powder.
EIMS (+): 272 [M]⁺.
¹H NMR (CDCl₃, 400 MHz): δ 1.29 (3H, t, J=7.3 Hz), 3.08 (2H, q, J=7.3 Hz), 4.15 (3H, s), 6.80 (1H, d, J=8.6 Hz), 7.96 (1H, d, J=8.6 Hz), 11.4 (1H, brs).

### <Example 114>

### 2-Ethyl-4-methoxy-7-propionylbenzoxazole

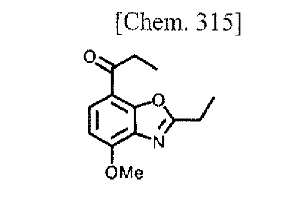

To a solution of the compound of Example 109 (3.39 g) in toluene (135 mL) was added paratoluenesulfonic acid monohydrate (257 mg), followed by heating to reflux for 8 hours with the equipment of a Dean-Stark trap. The reaction liquid was left to be cooled, and a saturated aqueous sodium hydrogen carbonate solution was then added thereto, followed by extraction with ethyl acetate. The organic layer was dried over anhydrous sodium sulfate, the solvent was evaporated under reduced pressure, and the residue was purified by silica gel column chromatography (hexane:ethyl acetate=1:1) to obtain the desired product (3.00 g) as a pale yellow powder.
EIMS (+): 233 [M]⁺.
¹H NMR (CDCl₃, 400 MHz): δ 1.27 (3H, d, J=7.3 Hz), 1.49 (3H, d, J=7.3 Hz), 3.03 (2H, q, J=7.3 Hz), 3.15 (2H, q, J=7.3 Hz), 4.09 (3H, s), 6.84 (1H, d, J=8.6 Hz), 7.94 (1H, d, J=8.6 Hz).

### <Example 115>

### 4-Methoxy-7-propionyl-2-trifluoromethylbenzoxazole

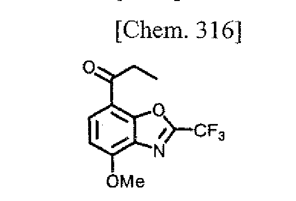

Aluminum chloride (2.21 g) was suspended in methylene chloride (55 mL), propionylchloride (1.45 mL) was added thereto, and then the compound of Example 110 (1.20 g) was added thereto, followed by stirring at room temperature for 3 days. To the reaction liquid was added 5% hydrochloric acid (30 mL), the organic layer was then collected by separation, and the aqueous layer was extracted with methylene chloride. The organic layer was combined and dried over anhydrous magnesium sulfate, the solvent was then evaporated and the residue was purified by silica gel column chromatography (hexane:ethyl acetate=4:1) to obtain the desired product (955 mg) as a pale yellow powder.
EIMS (+): 273 [M]⁺.
¹H NMR (CDCl₃, 400 MHz): δ 1.29 (3H, t, J=7.3 Hz), 3.17 (2H, q, J=7.3 Hz), 4.15 (3H, s), 6.98 (1H, d, J=8.6 Hz), 8.17 (1H, d, J=8.6 Hz).

### <Example 116>

### 7-Methoxy-4-propionyl-2-trifluoromethylbenzoxazole

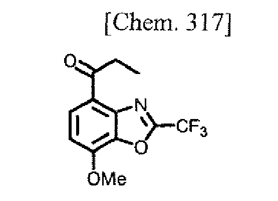

To titanium tetrachloride (1.0 mol/L methylene chloride solution, 2.90 mL) was added propionyl chloride (0.254 mL), and a solution of 7-methoxy-2-trifluoromethylbenzoxazole (420 mg) in methylene chloride (5 mL) was then added thereto, followed by stirring at room temperature for 2 days. To the reaction liquid was added 5% hydrochloric acid (30 mL), the organic layer was then collected by separation, and the aqueous layer was extracted with methylene chloride. The organic layer was combined and dried over anhydrous magnesium sulfate. Then, after evaporating the solvent, the residue was purified by silica gel column chromatography (hexane:ethyl acetate=5:1) to obtain the desired product (422 mg) as a colorless powder.
EIMS (+): 273 [M]⁺.
¹H NMR (CDCl₃, 400 MHz): δ 1.26 (3H, t, J=7.3 Hz), 3.39 (2H, q, J=7.3 Hz), 4.11 (3H, s), 7.08 (1H, d, J=8.6 Hz), 8.13 (1H, d, J=8.6 Hz).

### <Example 117>

### 4-Methoxy-7-propionyl-2-trifluoromethylbenzothiazole

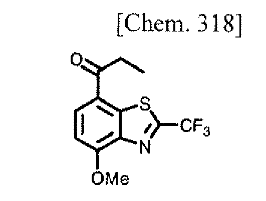

Titanium tetrachloride (7.46 mL) was dissolved in nitromethane (40 mL) under an argon atmosphere, and propionyl chloride (5.91 ml) was added thereto. Then, the compound of Example 111 (3.93 g) dissolved in nitromethane (30 mL) was added thereto, followed by stirring at room temperature for 30 minutes and at 75°C for 6 hours. Water was added thereto, followed by extraction three times with ethyl acetate, and the combined organic layer was washed with saturated brine, dried over sodium sulfate, and then filtered. After evaporating the solvent of the filtrate under reduced pressure, the residue was purified by silica gel column chromatography (hexane:ethyl acetate=9:1→4:1) to obtain the desired product (2.19 g) as a yellow powder.
¹H NMR (CDCl₃, 400 MHz): δ 1.31 (3H, t, J=7.3 Hz), 3.14 (2H, q, J=7.3 Hz), 4.18 (3H, s), 7.08 (1H, d, J=8.6 Hz), 8.20 (1H, d, J=8.6 Hz).

### <Example 118>

### 7-(2-Bromopropionyl)-4-methoxy-2-trifluoromethylbenzoxazole

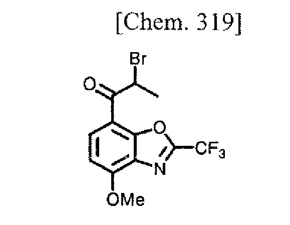

To a solution of the compound of Example 115 (92.0 mg) in ethyl acetate (4.2 mL) was added copper (II) bromide (150 mg), followed by stirring at 40°C for 2 hours. The insoluble materials were removed by filtration through Celite, followed by washing with ethyl acetate. The filtrate and the washing liquid were combined, the solvent was evaporated, and the residue was purified by silica gel column chromatography (hexane:ethyl acetate=3:1) to obtain the desired product (72.3 mg) as a pale yellow powder.
EIMS (+): 351 [M]⁺.
¹H NMR (CDCl₃, 400 MHz): δ 1.97 (3H, d, J=6.7 Hz), 4.18 (3H, s), 5.45 (1H, q, J=6.7 Hz), 7.03 (1H, d, J=8.6 Hz), 8.25 (1H, d, J=8.6 Hz).

### <Example 119>

### 4-(2-Bromopropionyl)-7-methoxy-2-trifluoromethylbenzoxazole

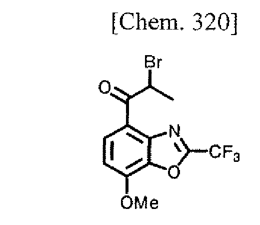

To a solution of the compound of Example 116 (20.0 mg) in ethyl acetate (1.0 mL) was added copper (II) bromide (32.7 mg), followed by heating to reflux for 1 hour. The insoluble materials were removed by filtration through Celite and the residue was washed with ethyl acetate. The filtrate and the washing liquid were combined, the solvent was evaporated, and the residue was purified by silica gel column chromatography (hexane:ethyl acetate=3:1) to obtain the desired product (24.2 mg) as a colorless powder.
EIMS (+): 351 [M]⁺.
¹H NMR (CDCl₃, 400 MHz): δ 1.95 (3H, d, J=6.7 Hz), 4.13 (3H, s), 6.20 (1H, q, J=6.7 Hz), 7.12 (1H, d, J=8.6 Hz), 8.22 (1H, d, J=8.6 Hz).

### <Example 120>

### t-Butyl 4-(4-methoxy-2-trifluoromethyl-1H-benzimidazol-7-yl)-3-methyl-4-oxobutyrate ester

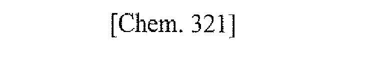

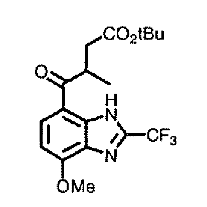

The compound of Example 113 (1.19 g) was dissolved in THF (40.0 mL) under an argon gas atmosphere, and a lithium bistrimethylsilylamide/THF solution (1.00 mol/L, 10.1 mL) was added dropwise thereto at -78°C, followed by stirring for 2 hours while slowly warming to -20°C. Thereafter, t-butyl bromoacetate (0.970 mL) was added thereto at -78°C, followed by stirring for 2.5 hours while warming to room temperature. To the reaction liquid was added a saturated aqueous ammonium chloride solution, followed by extraction with ethyl acetate. The organic layer was washed with water and saturated brine, and then dried over anhydrous sodium sulfate. After evaporating the solvent under reduced pressure, the desired product (1.93 g) was obtained as a yellow powder. It was used in the reaction described in Example 122 without purification.

### <Example 121>

### 4-(4-Methoxy-2-trifluoromethyl-1H-benzimidazol-7-yl)-3-methyl-4-oxobutyric acid

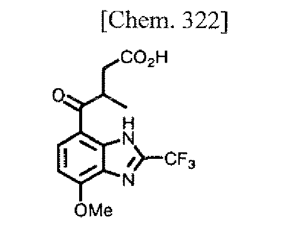

The compound of Example 120 (1.93 g, crude) was dissolved in methylene chloride (20.0 mL), and trifluoroacetic acid (10.0 mL) was added thereto, followed by stirring at room temperature for 2 hours. After evaporating the solvent under reduced pressure, the residue was azeotroped twice with ethanol to obtain the desired product (2.13 g, crude) as a yellowish brown amorphous. It was used in the reaction described in Example 122 as it was without purification.

### <Example 122>

### 6-(4-Methoxy-2-trifluoromethyl-1H-benzimidazol-7-yl)-4,5-dihydro-5-methyl-3-(2H)-pyridazinone

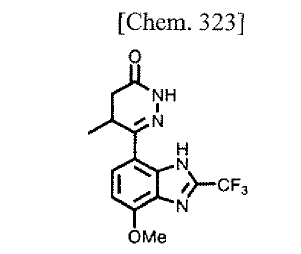

The compound of Example 121 (2.13 g, crude) was dissolved in ethanol (30.0 mL), and hydrazine monohydrate (0.540 mL) was added thereto, followed by heating to reflux for 1 hour. After evaporating the solvent under reduced pressure, to the residue was added water, followed by extraction with ethyl acetate. The organic layer was washed with water and saturated brine, and then dried over anhydrous sodium sulfate. After evaporating the solvent under reduced pressure, the residue was purified by silica gel column chromatography (hexane:ethyl acetate=1:2) to obtain the desired product (974 mg) as a pale yellow powder. HREIMS (+): 326.0961 (Calculated value as C₁₄H₁₃F₃N₄O₂ 326.2780). ¹H NMR (CDCl₃, 400 MHz): δ 1.33 (3H, d, J=7.3 Hz), 2.57 (1H, d, J=17.1 Hz), 2.79 (1H, dd, J=6.7, 17.1 Hz), 3.50-3.57 (1H, m), 4.11 (3H, s), 6.81 (1H, d, J=8.6 Hz), 7.55 (1H, d, J=8.6 Hz), 8.79 (1H, brs), 11.3 (1H, brs).

### <Example 123>

### 6-(2-Ethyl-4-methoxybenzooxazol-7-yl)-4,5-dihydro-5-methyl-3-(2H)-pyridazinone

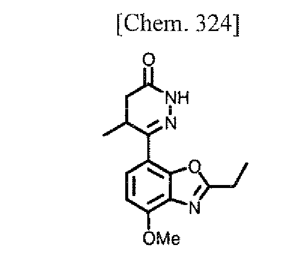

To a solution of the compound of Example 114 (946 mg) in THF (40 mL) was added a solution (1.00 mol/L, 4.26 mL) of hexamethyl disilazane in THF, followed by stirring for 15 minutes under ice cooling, and then t-butyl bromoacetate (0.719 mL) was added thereto, followed by stirring at 0°C for 1 hour. To the reaction liquid was added a saturated aqueous ammonium chloride solution, followed by extraction with ethyl acetate, and the organic layer was dried over anhydrous magnesium sulfate. Then, the solvent was evaporated under reduced pressure and the residue was purified by silica gel column chromatography (hexane:ethyl acetate=2:1) to obtain a crude ester form (1.20 g) as a pale yellow oil. The obtained oil was dissolved in methylene chloride (8 mL), and trifluoroacetic acid (4 mL) was added thereto, followed by stirring at room temperature for 3 hours. Then, the solvent and the like were evaporated under reduced pressure. To the residue were added toluene (40 mL), t-butyl carbazate (1.61 g), and paratoluenesulfonic acid monohydrate (771 mg), followed by reflux for 3 hours with the equipment of a Dean-Stark trap. The solvent of the reaction liquid was evaporated under reduced pressure and the residue was purified by silica gel column chromatography (hexane:ethyl acetate=1:3) to obtain the desired product (259 mg) as a pale yellow powder.
Elemental analysis: Found value C 62.54%, H 5.90%, N 14.63%, Calculated value as C₁₅H₁₇N₃O₃ C 62.71%, H 5.96%, N 14.63%.
EIMS (+): 287 [M]⁺.
¹H NMR (CDCl₃, 400 MHz): δ 131 (3H, d, J=7.3 Hz), 1.47 (3H, t, J=7.3 Hz), 2.52 (1H, dd, J=17.1, 1.2 Hz), 2.79 (1H, dd, J=17.1, 6.7 Hz), 3.00 (2H, q, J=7.3 Hz), 3.57-3.64 (1H, m), 4.06 (3H, s), 6.83 (1H, d, J=8.6 Hz), 7.65 (1H, d, J=8.6 Hz), 8.62 (1H, s).

### <Example 124>

### 6-(4-Methoxy-2-trifluoromethylbenzooxazol-7-yl)-4,5-dihydro-5-methyl-3-(2H)-pyridazinone

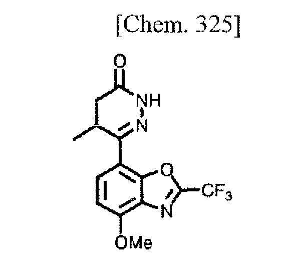

To a solution ofdi-t-butyl malonate (856 mg) in DMF (15 mL) was added 60% sodium hydride (119 mg), followed by stirring at room temperature for 30 minutes. To the reaction liquid was added a solution of the compound of Example 118 (541 mg) in DMF (15 mL) under ice cooling, followed by stirring for 1 hour. The reaction liquid was poured into a saturated aqueous ammonium chloride solution, followed by extraction with ethyl acetate. The organic layer was dried over anhydrous magnesium sulfate, the solvent was then evaporated, and the residue was purified by silica gel column chromatography (hexane:ethyl acetate=3:1) to obtain a crude butylester form (780 mg) as a pale yellow powder in the amorphous state. The obtained powder in the amorphous state was dissolved in methylene chloride (10 mL), and trifluoroacetic acid (5 mL) was added thereto, followed by stirring at room temperature for 1 hour. Then, the solvent and the like were evaporated under reduced pressure. To the residue was added xylene (20 mL), followed by stirring at 150°C for 2 hours. Then, t-butyl carbazate (618 mg) and paratoluenesulfonic acid monohydrate (296 mg) were added thereto, followed by heating to reflux for 3 hours with the equipment of a Dean-Stark trap. The solvent of the reaction liquid was evaporated under reduced pressure and the residue was purified by silica gel column chromatography (hexane:acetone=3:2) to obtain the desired product (157 mg) as a colorless powder.
Elemental analysis: Found value C 51.08%, H 3.69%, N 12.68%, Calculated value as C₁₄H₁₂F₃N₃O₃ C 51.38%, H 3.70%, N 12.84%.
FABMS (+): 328 [M+H]⁺.
¹H NMR (CDCl₃, 400 MHz): δ 1.32 (3H, d, J=7.3 Hz), 2.55 (1H, dd, J=17.1, 1.8 Hz), 2.80 (1H, dd, J=17.1, 6.7 Hz), 3.51-3.59 (1H, m), 4.12 (3H, s), 6.96 (1H, d, J=9.2 Hz), 7.86 (1H, d, J=9.2 Hz), 8.65 (1H, s).

### <Example 125>

### 6-(7-Methoxy-2-trifluoromethylbenzooxazol-4-yl)-4,5-dihydro-5-methyl-3-(2H)-pyridazinone

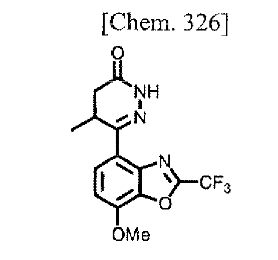

To a solution of di-t-butyl malonate (1.08 g) in DMF (20 mL) was added 60% sodium hydride (150 mg), followed by stirring at room temperature for 30 minutes. To the reaction liquid was added a solution of the compound of Example 119 (684 mg) in DMF (5 mL) under ice cooling, followed by stirring for 1 hour. The reaction liquid was poured into a saturated aqueous ammonium chloride solution, followed by extraction with ethyl acetate. The organic layer was dried over anhydrous magnesium sulfate, the solvent was then evaporated, and the residue was purified by silica gel column chromatography (hexane:ethyl acetate=4:1) to obtain a t-butylester form (793 mg) as a colorless oil. The obtained oil was dissolved in methylene chloride (10 mL), and trifluoroacetic acid (5 mL) was added thereto, followed by stirring at room temperature for 30 minutes. Then, the solvent and the like were evaporated under reduced pressure. To the residue was added xylene (30 mL), followed by stirring at 150°C for 2 hours. Then, t-butyl carbazate (646 mg) and paratoluenesulfonic acid monohydrate (310 mg) were added thereto, followed by heating to reflux for 2 hours with the equipment of a Dean-Stark trap. The solvent of the reaction liquid was evaporated, and the residue was purified by silica gel column chromatography (hexane:ethyl acetate=2:3) and then washed with isopropyl ether to obtain the desired product (285 mg) as a colorless powder.
Elemental analysis: Found value C 51.54%, H 3.65%, N 12.88%, Calculated value as C₁₄H₁₂F₃N₃O₃ C 51.38%, H 3.70%, N 12.84%.
EIMS (+): 327 [M]⁺.
¹H NMR (CDCl₃, 400 MHz): δ 1.27 (3H, d, J=7.3 Hz), 2.53 (1H, dd, J=17.1, 1.2 Hz), 2.82 (1H, dd, J=17.1, 7.3 Hz), 4.06-4.16 (1H, m), 4.09 (3H, s), 7.06 (1H, d, J=8.6 Hz), 7.91 (1H, d, J=8.6 Hz), 8.57 (1H, s).

### <Example 126>

### 6-(4-Methoxy-2-trifluoromethylbenzothiazol-7-yl)-4,5-dihydro-5-methyl-3-(2H)-pyridazinone

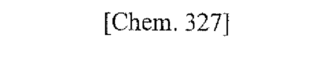

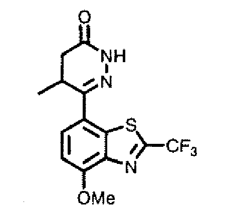

The compound of Example 117 (2.10 g) was suspended in THF (70 mL) under an argon atmosphere, and a solution (1.0 mol/L, 7.99 mL) of lithium hexamethyl disilazane in THF was added thereto at 0°C, followed by stirring at 0°C for 30 minutes, and then t-butyl bromoacetate (1.38 mL) was added thereto, followed by stirring at room temperature for 4 hours. To the reaction liquid was added a saturated aqueous ammonium chloride solution, THF was evaporated under reduced pressure, and the residue was then extracted three times with ethyl acetate. The extracted layer was washed with saturated brine and then dried over anhydrous sodium sulfate. After evaporating the solvent under reduced pressure, the residue was purified by silica gel column chromatography (hexane:ethyl acetate=6:1→5:1) to obtain an ester form. The obtained ester form was dissolved in methylene chloride (20 mL), and trifluoroacetic acid (10 mL) was added thereto, followed by leaving to stand at room temperature for 1.5 hours. After evaporating the solvent under reduced pressure, to the residue was added a saturated aqueous potassium carbonate solution, followed by washing with ethyl acetate. To the aqueous layer was added 1 mol/L hydrochloric acid to adjust the pH to 4, followed by extraction three times with ethyl acetate, and the combined organic layer was washed with saturated brine and then dried over anhydrous sodium sulfate. The solvent was evaporated under reduced pressure, the obtained residue were dissolved in ethanol (40 mL), and hydrazine monohydrate (1.06 mL) was added thereto, followed by stirring for 17 hours to heating under reflux. To the reaction liquid was added water, and the precipitated solid was collected by filtration and dissolved in acetone. The insoluble materials were removed by filtration, the solvent of the filtrate was evaporated under reduced pressure, and the residue was then washed with diisopropyl ether to obtain the desired product (460 mg) as a yellow powder.
Elemental analysis: Found value C 48.61%, H 3.36%, N 12.11%, Calculated value as C₁₄H₁₂F₃N₃O₂S · 1/10 H₂O C 48.72%, H 3.56%, N 12.24%.
¹H NMR (CDCl₃, 400 MHz): δ 1.34 (3H, d, J=7.3 Hz), 2.59 (1H, d, J=17.1 Hz), 2.81 (1H, dd, J=17.1, 6.7 Hz), 3.55-3.58 (1H, m), 4.16 (3H, s), 7.09 (1H, d, J=8.6 Hz), 7.76 (1H, d, J=8.6 Hz), 8.68 (1H, s).

### <Examples 127>

### 6-(4-Methoxy-2-trifluoromethylbenzothiazol-7-yl)-5-methyl-3-(2H)-pyridazinone

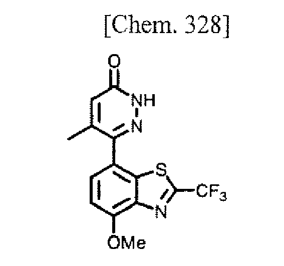

To a solution of the compound of Example 112 (2.53 g) in THF (60 mL) was added a solution (2.5 mol/L, 3.60 mL) of n-butyl lithium in hexane at -78°C, and after 1.5 hours, trimethyl borate (4.50 mL) was added thereto. After slowly warming to room temperature and stirring overnight, the solvent was evaporated under reduced pressure. To the residue were added water and ethyl acetate, and concentrated hydrochloric acid was added thereto, followed by vigorously stirring. The organic layer was dried over magnesium sulfate and concentrated, and the residue was then purified by silica gel column chromatography (ethyl acetate→ethyl acetate:methanol=95:5) to obtain a boric acid form (659 mg). A mixed liquid of the obtained boric acid form, the compound of Example 28 (842 mg), tetrakistriphenyl phosphine palladium (231 mg), THF (80 mL), and a 2 mol/L-aqueous sodium carbonate solution (12 mL) was stirred at 90°C for 24 hours. To the reaction liquid was added water, followed by extraction with ethyl acetate, and then the extracted layer was dried over magnesium sulfate. The solvent was concentrated under reduced pressure, and the residue was then purified by silica gel column chromatography (hexane:ethyl acetate=1:1) to obtain a colorless powder (770 mg). The obtained powder (770 mg) was dissolved in methanol (50 mL), and concentrated aqueous ammonia (50 mL) and chloroform (15 mL) were added thereto, followed by stirring at room temperature for 2 days. The solvent was concentrated and the residue was then purified by silica gel column chromatography (chloroform:methanol=95:5) to obtain the desired product (347 mg) as a colorless powder. ¹H NMR (CDCl₃, 200 MHz): δ 2.37 (3H, d, J=1.2 Hz), 4.13 (3H, s), 6.11 (2H, s), 6.88 (1H, q, J=1.2 Hz), 7.34 (1H, d, J=8.5 Hz), 7.93 (1H, d, J=8.5 Hz).

### <Example 128>

### 7-(6-Chloropyridazin-3-yl)-4-methoxy-2-trifluoromethylbenzothiazole

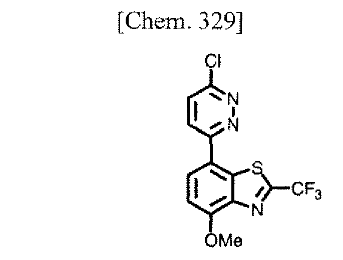

The compound of Example 112 (400 mg), bis(pinacolate)diborone (411 mg), [1,1'-bis(diphenylphosphino)ferrocene]dichloropalladium (II) methylene chloride complex (110 mg), and potassium 2-ethylhexanoate (370 mg) were dissolved in 1,4-dioxane (10 mL), followed by stirring at 80°C for 1.5 hours under an argon atmosphere. The reaction liquid was cooled to room temperature, and then a solution of 3,6-dichloropyridazine (603 mg) and tetrakistriphenyl phosphine palladium (78.0 mg) in 1,4-dioxane, (3.0 mL) was added thereto. Then, a 2.0 mol/L aqueous sodium carbonate solution (8.1 mL) was added thereto, followed by stirring at 100°C for 3 hours. To the reaction liquid was added water, followed by extraction three times with ethyl acetate, and the combined extracted layer was washed with saturated brine and then dried over anhydrous sodium sulfate. After evaporating the solvent under reduced pressure, the residue was purified by silica gel column chromatography (hexane:ethyl acetate=1:1→1:2) to obtain the desired product (166 mg) as a yellow solid. EIMS (+): 345 [M]⁺.
¹H NMR (CDCl₃, 400 MHz): δ 4.18 (3H, s), 7.16 (1H, d, J=8.6 Hz), 7.65 (1H, d, J=9.2 Hz), 8.05 (1H, d, J=8.6 Hz), 8.09 (1H, d, J=9.2 Hz).

### <Example 129>

### 6-(4-Methoxy-2-trifluoromethylbenzothiazol-7-yl)-3-(2H)-pyridazinone

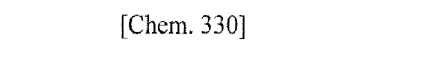

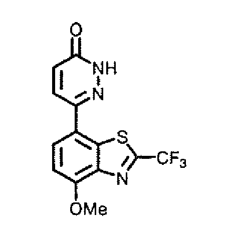

The compound of Example 128 (165 mg) was dissolved in acetic acid (5.0 mL), followed by stirring for 1.5 hours to heating under reflux. To the reaction liquid was added a saturated aqueous sodium hydrogen carbonate solution, followed by extraction three times with ethyl acetate, and the combined extracted layer was washed with saturated brine and then dried over anhydrous sodium sulfate. The solvent was evaporated under reduced pressure and the residue was purified by silica gel column chromatography (ethyl acetate) to obtain the desired product (73.8 mg) as a white solid.
Elemental analysis: Found value C 47.98%, H 2.62%, N 12.65%, Calculated value as C₁₃H₈F₃N₃O₂S C 47.71 %, H 2.46%, N 12.84%.
¹H NMR (DMSO-d₆, 400 MHz): δ 4.08 (3H, s), 7.10 (1H, dd, J=9.8, 2.4 Hz), 7.37 (1H, d, J=8.6 Hz), 8.32 (1H, d, J=8.6 Hz), 8.38 (1H, d, J=9.8 Hz), 13.39 (1H, s).

### <Example 130>

### (3-Methoxy-2-propionylamino)phenol

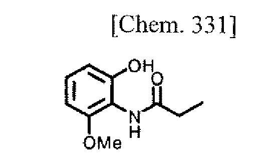

The compound of Example 107 (3.00 g) was suspended in toluene (80 mL), and anhydrous propionic acid (3.00 mL) was added thereto, followed by stirring at room temperature for 1 hour. To the reaction liquid was added water, followed by extraction with ethyl acetate, the extracted layer was washed with a 3% aqueous sodium hydrogen carbonate solution and saturated brine in this order and then dried over anhydrous magnesium sulfate, and the solvent was evaporated under reduced pressure to obtain the desired product (4.17 g) as a pale yellow liquid.
¹H NMR (CDCl₃, 400 MHz): δ 1.30 (3H, d, J=7.6 Hz), 2.54 (2H, q, J=7.6 Hz), 3.87 (3H, s), 6.44 (1H, dd, J=7.9, 1.2 Hz), 6.66 (1H, dd, J=8.6, 1.2 Hz), 7.04 (1H, dd, J=8.6, 7.9 Hz), 7.88 (1H, s), 9.99 (1H, s).

### <Example 131>

### 2-Ethyl-4-methoxybenzoxazole

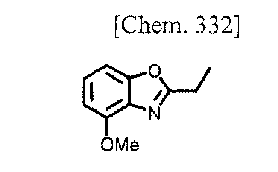

To a solution of the compound of Example 130 (1.44 g) in toluene (70 mL) was added paratoluenesulfonic acid monohydrate (140 mg), followed by heating to reflux for 6.5 hours with the equipment of a Dean-Stark trap. The reaction liquid was left to be cooled, and a saturated aqueous sodium hydrogen carbonate solution was then added thereto, followed by extraction with ethyl acetate. The organic layer was dried over anhydrous sodium sulfate, the solvent was evaporated under reduced pressure, and the residue was purified by silica gel column chromatography (hexane:ethyl acetate=8:1) to obtain the desired product (1.12 g) as a colorless oil.
CIMS (+): 178 [M+H]⁺.
¹H NMR (CDCl₃, 400 MHz): δ 1.45 (3H, d, J=7.7 Hz), 2.95 (2H, q, J=7.7 Hz), 4.02 (3H, s), 6.76 (1H, d, J=7.9 Hz), 7.10 (1H, d, J=7.9 Hz), 7.22 (1H, dd, J=7.9, 7.9 Hz).

### <Example 132>

### 4-Bromo-2-ethyl-4-methoxybenzoxazole

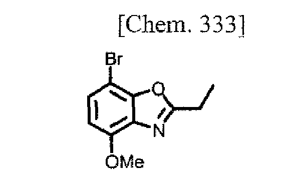

To a solution of the compound of Example 131 (100 mg) in acetonitrile (5.0 mL) was added N-bromosuccinimide (111 mg) at 0°C, followed by stirring at room temperature for 1 hour and then reflux for 1 hour. After leaving to be cooled, water was poured into the reaction liquid, followed by extraction with ethyl acetate, the extracted layer was then dried over anhydrous sodium sulfate, and the solvent was evaporated under reduced pressure. The residue was purified by silica gel column chromatography (hexane:ethyl acetate=2:1) to obtain the desired product (98.3 g) as a colorless liquid.
¹H NMR (CDCl₃, 400 MHz): δ 1.47 (3H, t, J=7.6 Hz), 2.98 (2H, q, J=7.6 Hz), 4.01 (3H, s), 6.69 (1H, d, J=8.6 Hz), 7.34 (1H, J=8.6 Hz).

### <Example 133>

### 2-Ethyl-4-methoxy-7-(4,4,5,5-tetramethyl-[1,3,2]dioxaboran-2-yl)benzoxazole

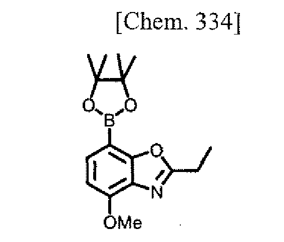

A solution of the compound of Example 132 (100 mg), bis(pinacolate)diborone (109 mg), potassium 2-ethylhexanoate (85.4 mg), and [1,1'-bis(diphenylphosphino)ferrocene]diehloropalladium (II) dichloromethane complex (15.9 mg) in 1,4-dioxane (2.0 mL) was stirred at 80°C for 1.5 hours under an argon atmosphere. After leaving to be cooled, to the reaction liquid was added water, followed by extraction with ethyl acetate, the extracted layer was dried over anhydrous sodium sulfate, and the solvent was evaporated. The residue was purified by silica gel column chromatography (hexane:ethyl acetate=3.5:1) to obtain the desired product (71.2 mg) as a colorless liquid.
EIMS (+): 303 [M]⁺.
¹H NMR (CDCl₃, 400 MHz): δ 1.37 (12H, s), 1.46 (3H, t, J=7.6 Hz), 3.00 (2H, q, J=7.6 Hz), 4.03 (3H, s), 6.77 (1H, d, J=8.6 Hz), 7.67 (1H, d, J=8.6 Hz).

### <Example 134>

### 7-(6-Chloropyridazin-3-yl)-2-ethyl-4-methoxybenzoxazole

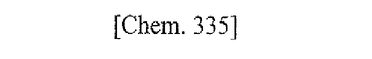

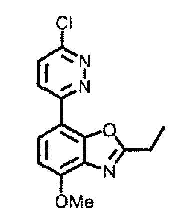

To a solution of the compound of Example 133 (50.0 mg) in 1,4-dioxane (2.0 mL) were added 3,6-dichloropyridazine (71.7 mg), a 2 mol/L aqueous sodium carbonate solution (0.247 mL), and tetrakis(triphenyl phosphine) palladium (24.3 mg) under an argon atmosphere, followed by stirring at 80°C for 2 hours. After leaving to be cooled, to the reaction liquid was added water, followed by extraction with ethyl acetate, the extracted layer was dried over anhydrous magnesium sulfate, and the solvent was evaporated. The residue was purified by silica gel column chromatography (hexane:ethyl acetate=1:1) to obtain the desired product (34.1 mg) as a white solid.
¹H NMR (CDCl₃, 400 MHz): δ 1.50 (3H, t, J=7.6 Hz), 3.03 (2H, q, J=7.6 Hz), 4.10 (3H, s), 6.96 (1H, d, J=9.2 Hz), 7.60 (1H, d, J=9.2 Hz), 8.25 (1H, d, J=9.2 Hz), 8.35 (1H, d, J=9.2 Hz).

### <Example 135>

### 6-(2-Ethyl-4-methoxybenzooxazol-7-yl)-3-(2H)-pyridazinone

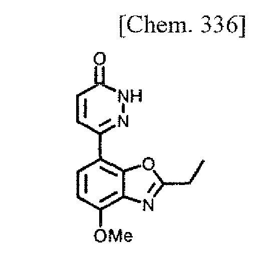

To the compound of Example 134 (24.0 mg) was added acetic acid (2.0 mL), followed by stirring at 100°C for 1 hour. To the residue obtained by evaporating acetic acid under reduced pressure was added a saturated aqueous sodium hydrogen carbonate solution, followed by extraction with chloroform, the extracted layer was dried over anhydrous magnesium sulfate, and the solvent was then evaporated under reduced pressure. The residue was purified by silica gel column chromatography (ethyl acetate) to obtain the desired product (15.8 mg) as a white solid.
Elemental analysis: Found value C 61.51%, H 4.73%, N 15.28%, Calculated value as C₁₄H₁₃N₃O₃ C 61.99%, H 4.83%, N 15.49%.
¹H NMR (CDCl₃, 400 MHz): δ 1.49 (3H, t, J=7.6 Hz), 3.02 (2H, q, J=7.6 Hz), 4.08 (3H, s), 6.88 (1H, d, J=8.6 Hz), 7.11 (1H, d, J=9.8 Hz), 7.79 (1H, d, J=8.6 Hz), 8.10 (1H, d, J=9.8 Hz), 11.33(1H, brs).

### <Example 136>

### 4-Methoxy-2-trifluoromethyl-1H-benzimidazol-7-yl boric acid

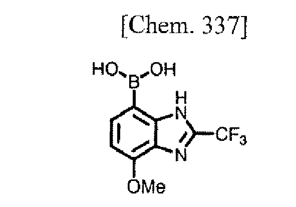

The compound of Example 105 (5.00 mg) was dissolved in tetrahydrofuran (17.0 mL) under an argon gas atmosphere, and a 1.60 mol/L n-butyl lithium/hexane solution (2.40 mL) was added dropwise thereto at -78°C, followed by stirring as it was for 1 hour. Thereafter, trimethyl borate (1.00 mL) was added thereto at once at the same temperature, followed by stirring for 3 hours while warming to room temperature. To the reaction liquid was added 3.00 mol/L hydrochloric acid, followed by stirring at room temperature for 30 minutes, and extracting three times with ethyl acetate. The organic layer was washed with saturated brine and then dried over anhydrous sodium sulfate. After evaporating the solvent, the desired product (197 mg) was obtained as a pale yellow powder. It was used in the reaction described in Example 137 without purification.

### <Example 137>

### 7-(6-Chloropyridazin-3-yl)-4-methoxy-2-trifluoromethyl-1H-benzimidazole

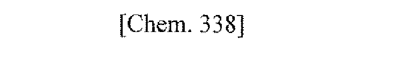

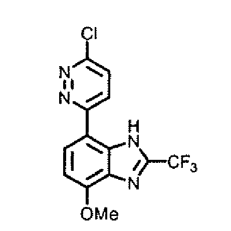

The reaction was carried out in the same manner as in Example 87 using the compound of Example 136 and 3,6-dichloropyridazine to obtain the desired product as a pale yellow powder.
EIMS (+): 328 [M]⁺.
¹H NMR (CDCl₃, 400 MHz): δ 4.15 (3H, s), 6.90 (1H, d, J=8.0 Hz), 7.63 (1H, d, J=9.2 Hz), 7.85 (1H, d, J=8.0 Hz), 8.06 (1H, d, J=9.2 Hz), 12.01 (1H, brs).

### <Example 138>

### 6-(4-Methoxy-2-trifluoromethyl-1H-benzimidazol-7-yl)-3-(2H)-pyridazinone

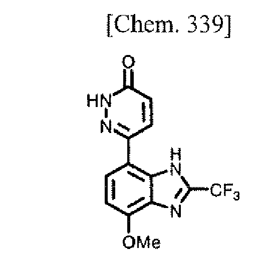

The reaction was carried out in the same manner as in Example 88 using the compound of Example 137 to obtain the desired product as a colorless powder.
HREIMS (+): 310.0671 (Calculated value as C₁₃H₉F₃N₄O₂ 310.0678).
¹H NMR (CD₃OD, 400 MHz): δ 4.07 (3H, s), 7.00 (1H, d, J=8.6 Hz), 7.10 (1H, d, J=9.8 Hz), 7.84 (1H, d, J=8.6 Hz), 8.40 (2H, brs).

### <Example 139>

### 4-(4-Methoxy-2-trifluoromethyl-1H-benzimidazol-7-yl)-4-oxobutyricacid

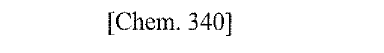

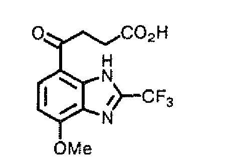

The compound of Example 105 (2.44 g) was dissolved in tetrahydrofuran (70.0 mL) under an argon gas atmosphere, and a 1.60 mol/L n-butyl lithium/hexane solution (11.9 mL) was added dropwise thereto at -78°C, followed by stirring as it was for 1 hour. Thereafter, a solution of succinic anhydride (2.48 g) in tetrahydrofuran (30.0 mL) was added thereto at once at the same temperature, followed by stirring for 1.5 hours while warming to room temperature. To the reaction liquid was added 1.00 mol/L hydrochloric acid, followed by extraction three times with ethyl acetate. The organic layer was washed with saturated brine and then dried over anhydrous sodium sulfate. After evaporating the solvent, the desired product (4.17 g, crude) was obtained as a pale red powder. It was used in the reaction described in Example 140 as it was without purification.

### <Example 140>

### 6-(4-Methoxy-2-trifluoromethyl-1H-benzimidazol-7-yl)-4,5-dihydro-3-(2H)-pyridazinone

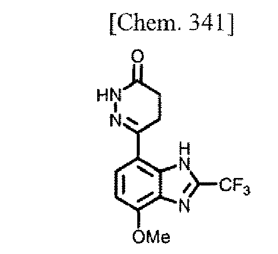

The compound of Example 139 (4.17 g) was dissolved in ethanol (60.0 mL), and hydrazine monohydrate (600 µL) was added thereto, followed by stirring for 2.5 hours to heating under reflux. Thereafter, to the reaction liquid was added hydrazine monohydrate (600 µL), followed by further stirring for 1.5 hours to heating under reflux. After evaporating the solvent, to the residue was added water, and the resulting solid was collected by filtration, washed with water and a small amount of ethyl acetate, and dried to obtain the desired product (1.24 g) as a colorless powder.
HREIMS (+): 312.0819 (Calculated value as C₁₃H₁₁F₃N₄O₂ 312.0834).
¹H NMR (CDCl₃, 400 MHz): δ 2.69 (2H, t, J=8.6 Hz), 3.13 (2H, t, J=8.6 Hz), 4.11 (3H, s), 6.80 (1H, d, J=8.0 Hz), 7.52 (1H, d, J=8.0 Hz), 8.59 (1H, brs), 11.24 (1H, brs).

### <Example 141>

### 6-(7-Methoxy-1-methoxymethyl-2-trifluoromethyl-1H-benzimidazol-4-yl)-4,5-dihydro-3-(2H)-pyridazinone

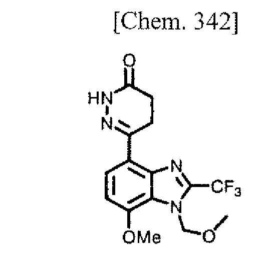

The reaction was carried out in the same manner as in Example 158 using the compound of Example 140 to obtain the desired product as a colorless powder.
EIMS (+): 356 [M]⁺.
¹H NMR (CDCl₃, 400 MHz): δ 2.64-2.69 (2H, m), 3.37 (3H, s), 3.46-3.51 (2H, m), 4.05 (3H, s), 5.91 (2H, s), 6.92 (1H, d, J=8.6 Hz), 7.70 (1H, d, J=8.6 Hz), 8.51 (1H, brs).

### <Example 142>

### 6-(7-Methoxy-1-methoxymethyl-2-trifluoromethyl-1H-benzimidazol-4-yl)-3-(2H)-pyridazinone

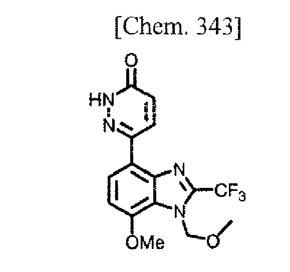

The reaction was carried out in the same manner as in Example 94 using the compound of Example 141 to obtain the desired product as a colorless powder.
EIMS (+): 354 [M]⁺.
¹H NMR (CDCl₃, 400 MHz): δ 3.39 (3H, s), 4.07 (3H, s), 5.94 (2H, s), 6.98 (1H, d, J=8.6 Hz), 7.09 (1H, d, J=9.8 Hz), 7.88 (1H, d, J=8.6 Hz), 8.74 (1H, d, J=9.8 Hz), 10.62 (1H, brs).

### <Example 143>

### 4-(6-Chloropyridazin-3-yl)-7-methoxy-2-trifluoromethylbenzo[b]thiophene

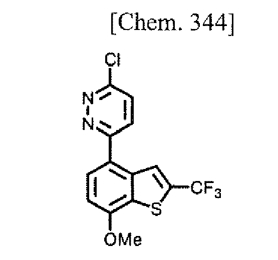

The reaction was carried out in the same manner as in Example 85 using the compound of Example 37 to obtain the desired product as a colorless powder.
EIMS (+): 344 [M]⁺.
¹H NMR (CDCl₃, 400 MHz): δ 4.09 (3H, s), 7.00 (1H, d, J=8.6 Hz), 7.63 (1H, d, J=8.6 Hz), 7.69 (1H, d, J=8.6 Hz), 7.80 (1H, d, J=8.6 Hz), 8.41 (1H, d, J=1.2 Hz).

### <Example 144>

### 6-(7-Methoxy-2-trifluoromethylbenzo[b]thiophen-4-yl)-3-(2H)-pyridazinone

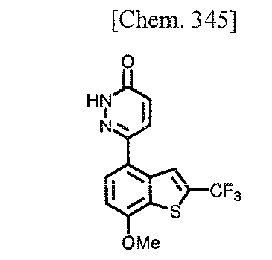

The reaction was carried out in the same manner as in Example 88 using the compound of Example 143 to obtain the desired product as a colorless powder. HREIMS (+): 326.0370 (Calculated value as C₁₄H₉F₃N₂O₂ 326.0337).
¹H NMR (DMSO-d₆, 400 MHz): δ 4.06 (3H, s), 7.03 (1H, dd, J=2.5, 9.8 Hz), 7.85 (1H, d, J=8.0 Hz), 7.99 (1H, d, J=9.8 Hz), 8.46 (1H, d, J=1.2 Hz), 13.20 (1H, brs).

### <Example 145>

### 2-(4-Bromobutyl)-6-(7-methoxy-2-trifluoromethylbenzofuran-4-yl)-3-(2H)-pyridazinone

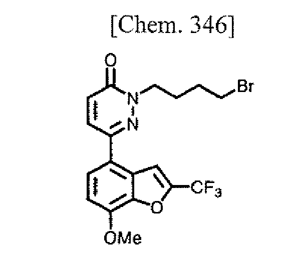

The compound of Example 90 (500 mg) was dissolved in N,N-dimethyl formamide (10.0 mL) under an argon gas atmosphere, and 60% sodium hydride (70.8 mg) was added thereto under ice cooling, followed by stirring at room temperature for 30 minutes. Thereafter, 1,4-dibromobutane (960 µL) was added thereto under ice cooling, followed by stirring at room temperature for 2.5 hours. To the reaction liquid was added a saturated aqueous ammonium chloride solution, followed by extraction with ethyl acetate. The organic layer was washed with water and saturated brine in this order, and then dried over anhydrous sodium sulfate. After evaporating the solvent, the residue was purified by silica gel column chromatography (ethyl acetate) to obtain the desired product (667 mg) as a pale yellow powder.
LRMS (+): 444 [M]⁺.
¹H NMR (CDCl₃, 400 MHz): δ 1.97-2.12 (4H, m), 3.48 (2H, t, J=6.1 Hz), 4.08 (3H, s), 4.33 (2H, t, J=6.1 Hz), 6.99 (1H, d, J=8.0 Hz), 7.05 (1H, d, J=9.8 Hz), 7.50 (1H, d, J=8.0 Hz), 7.71 (1H, d, J=9.8 Hz), 7.72 (1H, d, J=1.2 Hz).

### <Example 146>

### 2-(4-Bromobutyl)-6-(7-methoxy-1-methoxymethyl-2-trifluoromethyl-1H-benzimidazol-4-yl)-3-(2H)-pyridazinone

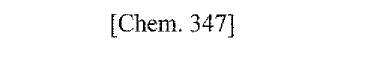

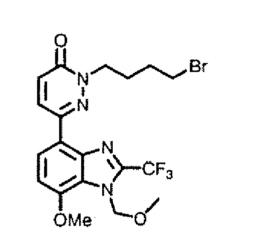

The reaction was carried out in the same manner as in Example 145 using the compound of Example 142 to obtain the desired product as a colorless powder.
EIMS (+): 489 [M+H]⁺.
¹H NMR (CDCl₃, 400 MHz): δ 1.96-2.06 (4H, m), 3.38 (3H, s), 3.49 (2H, t, J=6.7 Hz), 4.07 (3H, s), 4.31 (2H, t, J=6.7 Hz), 5.93 (2H, s), 6.99 (1H, d, J=8.6 Hz), 7.04 (1H, d, J=9.8 Hz), 7.88 (1H, d, J=8.6 Hz), 8.64 (1H, d, J=9.8 Hz).

### <Example 147>

### 2-(4-Bromobutyl)-6-(2-ethyl-8-methoxyquinolin-5-yl)-3-(2H)-pyridazinone

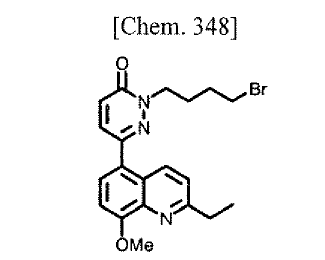

The reaction was carried out in the same manner as in Example 145 using the compound of Example 95 to obtain the desired product as a pale brown oil.
¹H NMR (CDCl₃, 400 MHz): δ 1.42 (3H, t, J=7.9 Hz), 1.94-2.04 (2H, m), 2.04-2.13 (2H, m), 3.11 (2H, q, J= 7.9 Hz), 3.48 (2H, t, J=6.7 Hz), 4.13 (3H, s), 4.31 (2H, t, J=6.7 Hz), 7.06 (1H, d, J=9.8 Hz), 7.11 (1H, d, J=7.9 Hz), 7.44 (1H, d, J=8.6 Hz), 7.49 (1H, d, J=9.8 Hz), 7.51 (1H, d, J=7.9 Hz), 8.38 (1H, d, J=8.6 Hz).

### <Example 148>

### 2-(4-Bromobutyl)-6-(8-methoxy-2-methylquinolin-5-yl)-4,5-dihydro-5-methyl-3-(2H)-pyridazinone

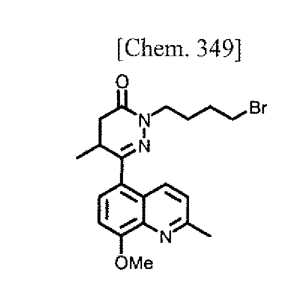

The reaction was carried out in the same manner as in Example 145 using the compound of Example 75 to obtain the desired product as a yellow oil.
¹H NMR (CDCl₃, 400 MHz): δ 1.17 (3H, t, J=7.4 Hz), 1.89-1.98 (4H, m), 2.55 (1H, dd, J=3.7, 16.5 Hz), 2.82 (3H, s), 2.86 (1H, d, J=9.8 Hz), 3.21-3.26(1H, m), 3.49 (2H, t, J=6.1 Hz), 3.77-3.83 (1H, m), 4.04-4.08 (1H, m), 4.12 (3H, s), 7.06 (1H, d, J=8.6 Hz), 7.40 (1H, d, J=8.6 Hz), 7.50 (1H, d, J=8.6 Hz), 8.57 (1H, d, J=8.6 Hz).

### <Example 149>

### 2-(4-Bromobutyl)-6-(8-methoxy-2-methylquinolin-5-yl)-3-(2H)-pyridazinone

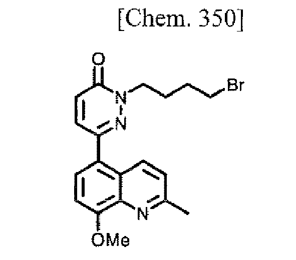

The reaction was carried out in the same manner as in Example 145 using the compound of Example 93 to obtain the desired product as a colorless amorphous.
¹H NMR (CDCl₃, 400 MHz): δ 1.97-2.00 (2H, m), 2.06-2.10 (2H, m), 2.86 (3H, s), 3.48 (2H, t, J=6.8 Hz), 4.14 (3H, s), 4.31 (2H, t, J=6.7 Hz), 7.06 (1H, d, J=9.8 Hz), 7.11 (1H, d, J=8.6 Hz), 7.40 (1H, d, J=8.6 Hz), 7.49 (1H, d, J=9.8 Hz), 7.51 (1H, d, J=8.6 Hz), 8.36 (1H, d, J=8.6 Hz).

### <Example 150>

### 2-(4-Bromobutyl)-6-(2-isopropyl-8-methoxyquinolin-5-yl)-4,5-dihydro-5-methyl-3-(2H)-pyridazinone

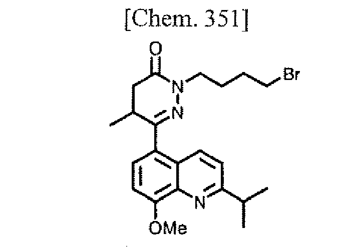

The reaction was carried out in the same manner as in Example 145 using the compound of Example 77 to obtain the desired product as a pale yellow oil.
¹H NMR (CDCl₃, 400 MHz): δ 0.17 (3H, d, J=7.4 Hz), 1.40 (6H, d, J=6.7 Hz), 1.95 (4H, m), 2.55 (1H, dd, J=16.8, 3.0 Hz), 2.85 (1H, dd, J=16.8, 6.7 Hz), 3.19-3.27 (1H, m), 3.36-3.45 (1H, m), 3.49 (2H, t, J=6.7 Hz), 3.77-3.84 (1H, m), 4.04-4.15 (1H, m), 4.12 (3H, s), 7.05 (1H, d, J=8.6 Hz), 7.48 (1H, d, J=8.6 Hz), 7.49 (1H, d, J=8.6 Hz), 8.61 (1H, d, J=8.6 Hz).

### <Example 151>

### 2-(4-Bromobutyl)-6-(2-isopropyl-8-methoxyquinolin-5-yl)-3-(2H)-pyridazinone

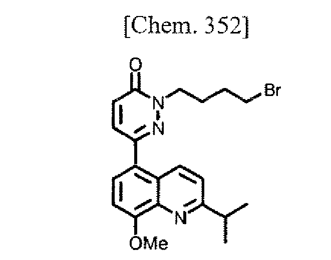

The reaction was carried out in the same manner as in Example 145 using the compound of Example 94 to obtain the desired product as a yellow oil.
¹H NMR (CDCl₃, 400 MHz): δ 1.41 (6H, d, J=7.4 Hz), 1.97-2.01 (2H, m), 2.06- 2.10 (2H, m), 3.37-3.44 (1H, m), 3.48 (2H, t, J=6.7 Hz), 4.13 (3H, s), 4.31 (2H, t, J=6.7 Hz), 7.05 (1H, d, J=9.8 Hz), 7.10 (1H, d, J=8.0 Hz), 7.47 (1H, d, J=8.6 Hz), 7.48 (1H, d, J=9.8 Hz), 7.51 (1H, d, J=8.0 Hz), 8.40 (1H, d, J=8.6 Hz).

### <Example 152>

### 2-(4-Bromobutyl)-6-(2-ethyl-8-niethoxyquinolin-5-yl)-4,5-dihydro-5-methyl-3-(2H)-pyridazinone

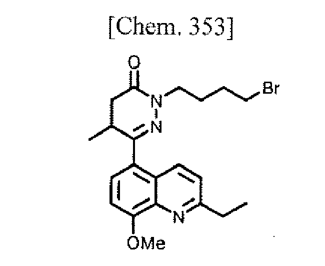

The reaction was carried out in the same manner as in Example 145 using the compound of Example 72 to obtain the desired product as a pale yellow oil.
FABMS (+): 432 [M+H]⁺.
¹H NMR (CDCl₃, 400 MHz): δ 1.17 (3H, d, J=7.3 Hz), 1.41 (3H, t, J=7.3 Hz), 1.88-1.98 (4H, m), 2.55 (1H, dd, J=16.5, 3.6 Hz), 2.86 (1H, dd, J=16.5, 6.7 Hz), 3.10 (2H, q, J=7.3 Hz), 3.20-3.27 (1H, m), 3.49 (2H, t, J=6.1 Hz), 3.76-3.84 (1H, m), 4.06-4.12 (1H, m), 4.12 (3H, s), 7.06 (1H, d, J=8.6 Hz), 7.45 (1H, d, J=8.6 Hz), 7.50 (1H, d, H=8.6 Hz), 8.60 (1H, d, J=8.6 Hz).

### <Example 153>

### 2-(4-Bromobutyl)-6-(5-methoxy-2-trifluoromethyl-[1,2,4]triazolo[1,5-a]pyridin-8-yl)-3-(2H)-pyridazinone

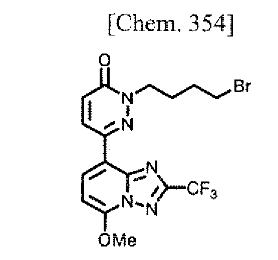

The reaction was carried out in the same manner as in Example 145 using the compound of Example 89 to obtain the desired product as a yellow powder.
¹H NMR (CDCl₃, 400 MHz): δ 1.96-2.00 (2H, m), 2.04-2.10 (2H, m), 3.49 (2H, t, J= 6.4 Hz), 4.29 (3H, s), 4.32 (2H, t, J=7.0 Hz), 6.64 (1H, d, J=8.6 Hz), 7.09 (1H, d, J=9.8 Hz), 8.36 (1H, d, J=8.6 Hz), 8.73 (1H, d, J=9.8 Hz).

### <Example 154>

### 2-(4-Bromobutyl)-6-(4-methoxy-2-trifluoromethylbenzothiazol-7-yl)-3-(2H)-pyridazinone

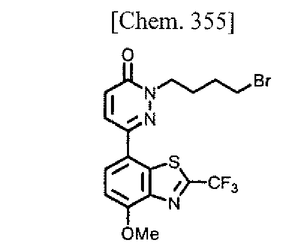

The reaction was carried out in the same manner as in Example 145 using the compound of Example 129 to obtain the desired product as a yellow powder.
EIMS (+): 461 [M]⁺.
¹H NMR (CDCl₃, 400 MHz): δ 1.99-2.04 (2H, m), 2.15-2.17 (2H, m), 3.50 (2H, t, J= 6.7 Hz), 4.16 (3H, s), 4.40 (2H, t, J=7.0 Hz), 7.10 (1H, d, J=8.6 Hz), 7.11 (1H, d, J=8.6 Hz), 7.88 (2H, d, J=8.6 Hz), 7.90 (2H, d, J=8.6 Hz).

### <Example 155>

### 2-(4-Bromobutyl)-6-(7-methoxy-2-trifluoromethylbenzofuran-4-yl)-4,5-dihydro-5-methyl-3-(2H)-pyridazinone

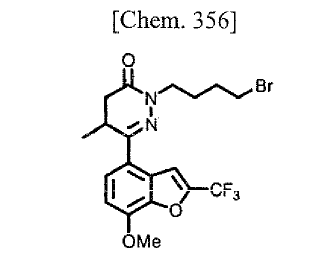

The reaction was carried out in the same manner as in Example 145 using the compound of Example 82 to obtain the desired product as a colorless powder. FABMS (+): 461 [M+H]⁺.
¹H NMR (CDCl₃, 400 MHz): δ 1.25 (3H, d, J=7.3 Hz), 1.93-1.95 (4H, m), 2.52 (1H, dd, J=16.5, 1.8 Hz), 2.73 (1H, dd, J=16.5, 6.7 Hz), 3.38-3.42 (1H, m), 3.46-3.49 (2H, m), 3.88-3.93 (1H, m), 4.02-4.07 (1H, m), 4.07 (3H, s), 6.94 (1H, d, J=8.0 Hz), 7.46(1H, d, J=8.0 Hz), 7.89 (1H, s).

### <Example 156>

### 2-(4-Bromobutyl)-6-(7-methoxy-2-trifluoromethylbenzo[b]thiophen-4-yl)-4,5-dihydro-5-methyl-3-(2H)-pyridazinone

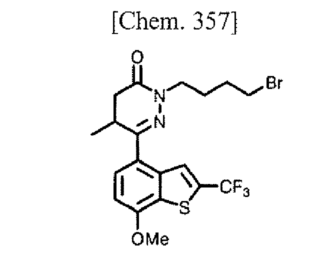

The reaction was carried out in the same manner as in Example 145 using the compound of Example 83 to obtain the desired product as a colorless powder.
FABMS (+): 477 [M+H]⁺.
¹H NMR (CDCl₃, 400 MHz): δ 1.25 (3H, d, J=7.3 Hz), 1.94-1.97 (4H, m), 2.53 (1H, dd, J=16.5, 1.8 Hz), 2.77 (1H, dd, J=16.5, 6.7 Hz), 3.36-3.40 (1H, m), 3.47(2H, t, J=6.1 Hz), 3.88-3.94 (1H, m), 4.03-4.08 (1H, m), 4.06 (3H, s), 6.90 (1H, d, J=8.6 Hz), 7.59 (1H, d, J=8.6 Hz), 8.60 (1H, s).

### <Example 157>

### 2-(4-Bromobutyl)-6-(4-methoxy-2-trifluoromethylbenzothiazol-7-yl)-4,5-dihydro-5-methyl-3-(2H)-pyridazinone

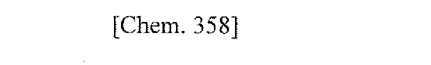

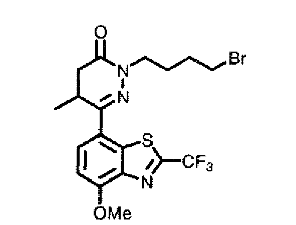

The reaction was carried out in the same manner as in Example 145 using the compound of Example 126 to obtain the desired product as a colorless powder. FABMS (+): 478 [M+H]⁺.
¹H NMR (CDCl₃, 400 MHz): δ 1.28 (3H, d, J=7.3 Hz), 1.97-2.05 (4H, m), 2.58 (1H, dd, J=16.5, 1.2 Hz), 2.76 (1H, dd, J=16.5, 6.7 Hz), 3.46 (2H, t, J=6.7 Hz), 3.46-3.53 (1H, m), 3.98-4.11 (2H, m), 4.15 (3H, s), 7.08 (1H, d, J=8.6 Hz), 7.75 (1H, d, J=8.6 Hz).

### <Examples 158>

### 6-(7-Methoxy-1-methoxymethyl-2-trifluoromethyl-1H-benzimidazol-4-yl)-4,5-dihydro-5-methyl-3-(2H)-pyridazinone

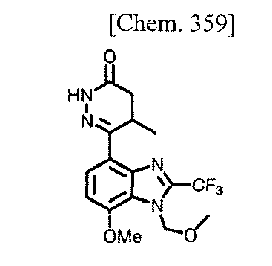

The compound of Example 122 (369 mg) was dissolved in N,N-dimethyl formamide (10.0 mL), and triethylamine (362 µL) and chloromethyl methyl ether (94.5 µL) were added thereto under ice cooling, followed by stirring as it was for 3 hours. To the reaction liquid was added water, followed by extraction with ethyl acetate. The organic layer was washed with water and saturated brine, and then dried over anhydrous sodium sulfate. After evaporating the solvent, the residue was purified by silica gel column chromatography (hexane:ethyl acetate=1:1) to obtain the desired product (228 mg) as a colorless powder. EIMS (+): 370 [M]⁺.
¹H NMR (CDCl₃, 400 MHz): δ 1.26 (3H, d, J=7.3 Hz), 2.51 (1H, dd, J=17.1, 1.8 Hz), 2.86 (1H, dd, J= 17.1, 6.7 Hz), 3.38 (3H, s), 4.05 (3H, s), 4.22-4.27 (1H, m), 5.91 (2H, s), 6.92 (1H, d, J=8.6 Hz), 7.72 (1H, d, J=8.6 Hz), 8.50 (1H, brs).

### <Example 159>

### 2-(4-Bromobutyl)-6-(7-methoxy-1-methoxymethyl-2-trifluoromethyl-1H-benzimidazol-4-yl)-4,5-dibydro-5-methyl-3-(2H)-pyridazinone

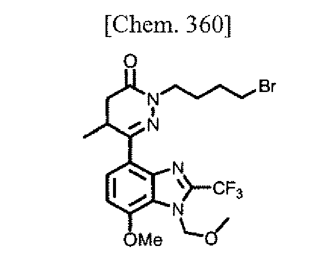

The reaction was carried out in the same manner as in Example 145 using the compound of Example 158 to obtain the desired product as a colorless oil.
FABMS (+): 505 [M+H]⁺.
¹H NMR (CDCl₃, 400 MHz): δ 1.21 (3H, d, J=7.3 Hz),1.89-1.97 (4H, m), 2.50 (1H, dd, J=16.5, 1.8 Hz), 2.81 (1H, dd, J=16.5, 6.7 Hz), 3.37 (3H, s), 3.49 (2H, t, J=6.1 Hz), 3.78-3.83 (1H, m), 4.02-4.10 (1H, m), 4.05 (3H, s), 4.17-4.22 (1H, m), 5.91 (2H, s), 6.94(1H, d, J=8.6 Hz), 7.77 (1H, d, 1=8.6 Hz).

### <Example 160>

### 2-(4-Bromobutyl)-6-(8-methoxyquinolin-5-yl)-4,5-dihydro-5-methyl-3-(2H)-pyridazinone

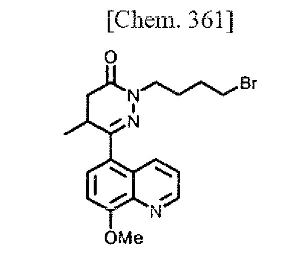

The reaction was carried out in the same manner as in Example 145 using the compound of Example 74 to obtain the desired product as a pale yellow oil.
EIMS (+): 403 [M]⁺.
¹H NMR (CDCl₃, 400 MHz): δ 1.17 (3H, d, J=6.8 Hz), 1.90-2.00 (4H, m), 2.56 (1H, dd, J=16.5, 3.0 Hz), 2.86 (1H, dd, J=16.5, 6.8 Hz), 3.20-3.30 (1H, m), 3.49 (2H, t, J=6.2 Hz), 3.78-3.85 (1H, m), 4.04-4.10 (1H, m), 4.14 (3H, s), 7.09 (1H, d, J=7.9 Hz), 7.53 (1H, dd, J=8.6, 4.3 Hz), 7.57 (1H, d, J=8.6 Hz), 8.70 (1H, dd, J=8.6, 1.8 Hz), 8.98 (1H, dd, J=4.3, 1.8 Hz).

### <Example 161>

### 2-(4-Bromobutyl)-6-(8-methoxy-2-trifluoromethylquinolin-5-yl)-3-(2H)-pyridazinone

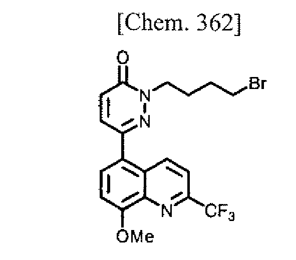

The reaction was carried out in the same manner as in Example 145 using the compound of Example 92 to obtain the desired product as a white solid.
FABMS (+): 458 [M+H]⁺.
¹H NMR (CDCl₃, 400 MHz): δ 1.97-2.17 (4H, m), 3.48 (2H, t, J=6.7 Hz), 4.17 (3H, s), 4.32 (2H, t, J=6.7 Hz), 7.10 (1H, d, J=9.2 Hz), 7.22 (1H, d, J=8.0 Hz), 7.50 (1H, d, J=9.2 Hz), 7.71 (1H, d, J=8.0 Hz), 7.84 (1H, d, J=9.2 Hz), 8.74 (1H, d, J=9.2 Hz).

### <Example 162>

### 2-(4-Bromopropyl)-6-(2-ethyl-8-methoxyquinolin-5-yl)-3-(2H)-pyridazinone

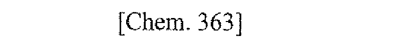

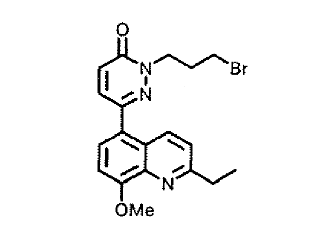

The reaction was carried out in the same manner as in Example 145 using the compound of Example 95 and 1,3-dibromopropane to obtain the desired product as a yellow powder.
¹H NMR (CDCl₃, 400 MHz): δ 1.42 (3H, t, J=7.6 Hz), 2.45-2.52 (2H, m), 3.11 (2H, m), 3.50 (2H, t, J=6.7 Hz), 4.13 (3H, s), 4.42 (2H, t, J=7.0 Hz), 7.06 (1H, d, J=9.8 Hz), 7.11 (1H, d, J=8.6 Hz), 7.44 (1H, d, J=8.6 Hz), 7.50 (1H, d, J=9.8 Hz), 7.51 (1H, d, J=8.6 Hz), 8.40 (1H, d, J=8.6 Hz).
EIMS (+): 401 [M]⁺.

### <Example 163>

### 2-(4-Bromobutyl)-6-(7-methoxy-2-trifluoromethylbenzo[b]thiophen-4-yl)-3-(2H)-pyridazinone

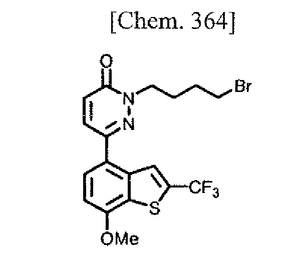

The reaction was carried out in the same manner as in Example 145 using the compound of Example 144 to obtain the desired product as a pale yellow solid.
¹H NMR (CDCl₃, 400 MHz): δ 1.96-2.03 (2H, m), 2.04-2.13 (2H, m), 3.47 (2H, t, J=6.4 Hz), 4.07 (3H, s), 4.33 (2H, t, J=7.0 Hz), 6.96 (1H, d, J=8.6 Hz), 7.06 (1H, d, J=9.8 Hz), 7.56 (1H, d, J=8.6 Hz), 7.62 (1H, d, J=3.8 Hz), 8.22 (1H, d, J=1.2 Hz).
EIMS (+): 460 [M]⁺.

### <Example 164>

### 2-(4-Bromobutyl)-6-(8-methoxy-2-trifluoromethyl-imidazo[1,2-a]pyridin-5-yl)-3-(2H)-pyridazinone

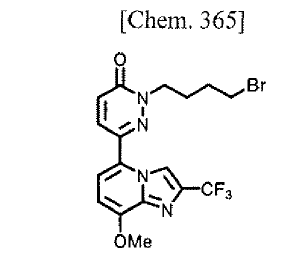

The reaction was carried out in the same manner as in Example 145 using the compound of Example 100 to obtain the desired product as a yellow solid.
¹H NMR (CDCl₃, 400 MHz): δ 1.96-2.04 (2H, m), 2.05-2.15 (2H, m), 3.47 (2H, t, J=6.4 Hz), 4.11 (3H, s), 4.35 (2H, t, J=7.0 Hz), 6.69 (1H, d, J=7.9 Hz), 7.10 (1H, d, J=9.8 Hz), 7.17 (1H, d, J=7.9 Hz), 7.66 (1H, d, J=9.8 Hz), 8.91 (1H, s).
ESIMS (+): 445 [M+H]⁺.

### <Example 165>

### 2-(4-Bromobutyl)-6-(2-ethyl-4-methoxybenzooxazol-7-yl)-3-(2H)-pyridazinone

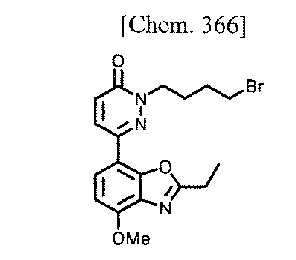

The reaction was carried out in the same manner as in Example 145 using the compound of Example 135 to obtain the desired product as a yellow oil.
¹H NMR (CDCl₃, 400 MHz): δ 1.49 (3H, t, J=7.6 Hz), 1.92-2.03 (2H, m), 2.05-2.14 (2H, m), 3.02 (2H, q, J=7.6 Hz), 3.49 (2H, t, J=6.4 Hz), 4.08 (3H, s), 4.32 (2H, t, J=6.7 Hz), 6.88 (1H, d, J=8.6 Hz), 7.05 (1H, d, 1=9.2 Hz), 7.78 (1H, d, J=8.6 Hz), 8.00 (1H, d, J=9.2 Hz).
EIMS (+): 405 [M]⁺.

### <Example 166>

### 6-(4-Methoxyphenyl)-5-methyl-4,5-dihydro-2H-pyridazin-3-one

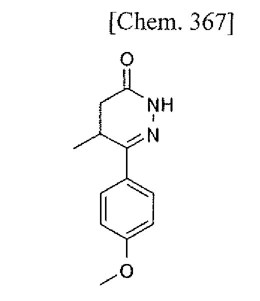

A commercially available 4-methoxypropiophenone (25.0 g) was dissolved in THF (750 mL) under an argon gas atmosphere, and a lithium bistrimethylsilyl amide (1.00 mol/L THF solution, 153 mL) was added dropwise under ice cooling, followed by stirring at the same temperature for 30 minutes. Thereafter, tert-butyl bromoacetate (33.7 mL) was added thereto at the same temperature, followed by stirring at room temperature for 3 hours. To the reaction liquid was added a saturated aqueous ammonium chloride solution, followed by extraction with ethyl acetate. The extracted layer was washed with water and saturated brine in this order, and then dried over anhydrous sodium sulfate. The solvent was evaporated under reduced pressure, and thus obtained yellowish brown oil was dissolved in acetonitrile (250 mL), montmorillonite KSF (30.0 g) was added thereto, followed by stirring for 7 hours to heating under reflux. The insoluble materials were removed by filtration and the solvent of the filtrate was evaporated under reduced pressure to obtain a yellowish brown oil. This was dissolved in ethanol (300 mL), and hydrazine monohydrate (22.0 mL) was added thereto, followed by stirring for 2.5 hours to heating under reflux. After evaporating the solvent under reduced pressure, to the residue was added ice water, and the resulting solid was collected by filtration. The obtained solid was washed with water, cold ethanol, and diisopropyl ether in this order to obtain the desired product (26.7 g) as a colorless powder. ¹H NMR (CDCl₃, 400 MHz): δ 1.25 (3H, d, J=7.3 Hz), 2.46 (1H, d, J=17.1 Hz), 2.71 (1H, dd, J=17.1, 6.7 Hz), 3.32-3.36 (1H, m), 3.85 (3H, s), 6.93-6.95 (2H, m), 7.69-7.72 (2H, m), 8.44 (1H, brs).

### <Example 167>

### 6-(4-Hydroxyphenyl)-5-methyl-4,5-dihydro-2H-pyridazin-3-one

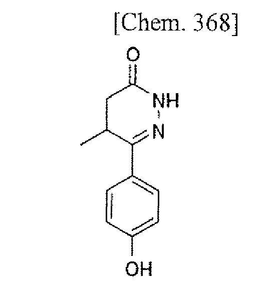

The compound of Example 166 (26.3 g) was dissolved in dichloromethane (500 mL), and aluminum chloride (323 g) was added thereto under ice cooling, followed by stirring at room temperature for 40 hours. The reaction liquid was poured into ice water, followed by extraction with THF, and the extracted layer was dried over anhydrous magnesium sulfate. After evaporating the solvent under reduced pressure, the resulting solid was suspended in diisopropyl ether and collected by filtration to obtain the desired product (20.9 g) as a pale yellow powder.
¹H NMR (DMSO-d₆, 400 MHz): δ 1.04 (3H, d, J=7.3 Hz), 2.18 (1H, d, J=15.9 Hz), 2.63 (1H, dd, J=15.9, 1.8 Hz), 3.28-3.33 (1H, m), 6.78-6.80 (2H, m), 7.59-7.63 (2H, m), 9.78 (1H, s), 10.8 (1H, s).

### <Example 168>

### 6-(4-Methoxyphenyl)-5-methyl-2H-pyridazin-3-one

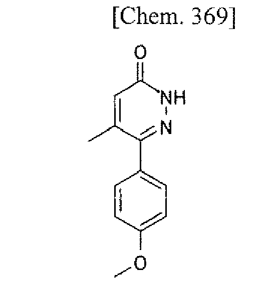

The compound of Example 166 (6.50 g) was dissolved in a 0.5 mol/L aqueous sodium hydroxide solution (350 mL), and sodium m-nitrobenzenesulfonate (6.70 g) was added thereto, followed by stirring for 4 hours under the condition of heating to reflux. The reaction liquid was neutralized with 6 mol/L hydrochloric acid, and the precipitated solid was then collected by filtration to obtain the desired product (3.90 g) as a white powder.
¹H NMR (CDCl₃, 400 MHz): δ 2.20 (3H, s), 3.86 (3H, s), 6.83 (1H, s), 6.97 (2H, d, J=8.6 Hz), 7.35 (2H, d, J=8.6 Hz).

### <Example 169>

### 6-(4-Hydroxyphenyl)-5-methyl-2H-pyridazin-3-one

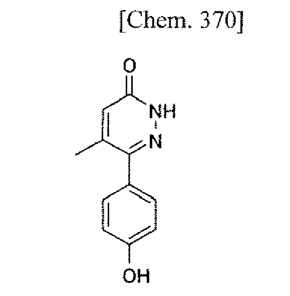

The compound of Example 168 (3.90 g) was dissolved in dichloromethane (180 mL), and aluminum chloride (24.1 g) was added thereto, followed by stirring at room temperature for 8 hours. To the reaction liquid was added water, followed by extraction with THF, the extracted layer was washed with saturated brine and then dried over anhydrous sodium sulfate, and the solvent was evaporated under reduced pressure to obtain the desired product (2.40 g) as a yellow powder.
EIMS (+): 202 [M]⁺.
¹H NMR (DMSO-d₆, 400 MHz): δ 1.89 (3H, s), 6.56 (1H, s), 6.60 (2H, d, J=8.6 Hz), 7.05 (2H, d, J=8.6 Hz).

### <Example 170>

### 6-(4-t-Butyldimethylsilyloxyphenyl)-5-methyl-2H-pyridazin-3-one

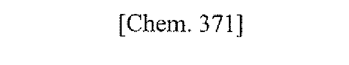

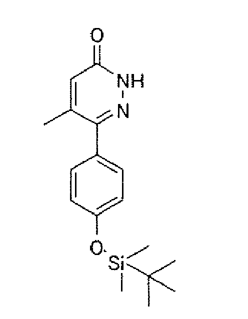

The compound of Example 169 (300 mg) was dissolved in DMF (8.0 mL), and imidazole (111 mg) and t-butyldimethylsilyl chloride (246 mg) were added thereto at 0°C, followed by stirring at room temperature for 4 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=1:1) to obtain the desired product (208 mg) as a white powder.
¹H NMR (CDCl₃, 400 MHz): δ 0.21 (6H, s), 0.95 (9H, s), 2.10 (3H, d, J=1.2 Hz), 6.79 (1H, d, J=1.2 Hz), 6.91 (2H, d, J=8.6 Hz), 7.36 (2H, d, J=8.6 Hz), 13.08 (1H, s).

### <Example 171>

### 2-t-Butoxycarbonyl-6-(4-t-butyldimethylsilyloxyphenyl)-5-methyl-2H-pyridazin-3-one

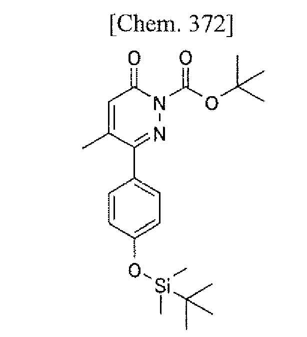

The compound of Example 170 (207 mg) was dissolved in acetonitrile (6.5 mL) under an argon atmosphere, and di-t-butyldicarbonate (170 mg) and N,N-dimethyl aminopyridine (9.50 mg) were added thereto, followed by stirring at room temperature for 3 hours. To the reaction liquid was added water, followed by extraction with ethyl acetate, and the extracted layer was washed with saturated brine and then dried over anhydrous sodium sulfate. The solvent was evaporated under reduced pressure and the obtained residue was purified by silica gel chromatography (hexane:ethyl acetate=3:1) to obtain the desired product (159 mg) as a white powder.
¹H NMR (CDCl₃, 400 MHz): δ 0.22 (6H, s), 0.99 (9H, s), 1.63 (9H, s), 2.15 (3H, d, J=1.2 Hz), 6.76 (1H, d, J=1.2 Hz), 6.89 (2H, d, J=8.6 Hz), 7.31 (2H, d, J=8.6 Hz).

### <Example 172>

### 2-t-Butoxycarbonyl-6-(4-hydroxyphenyl)-5-methyl-2H-pyridazin-3-one

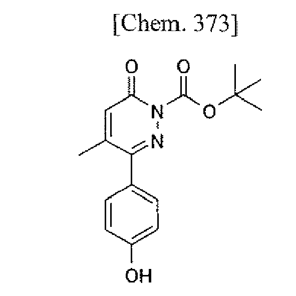

The compound of Example 171 (159 mg) was dissolved in THF (4.0 mL) under an argon atmosphere, and tetrabutyl ammonium fluoride (1.0 mol/L THF solution, 0.763 mL) was added thereto at 0°C, followed by stirring at room temperature for 40 minutes. To the reaction liquid was added a saturated aqueous ammonium chloride solution, followed by extraction with ethyl acetate, and the extracted layer was washed with saturated brine and then dried over anhydrous sodium sulfate. The solvent was evaporated under reduced pressure and the obtained residue was purified by silica gel chromatography (hexane:ethyl acetate=2:1→1:1) to obtain the desired product (32.2 mg) as a white powder.
¹H NMR (DMSO-d₆, 400 MHz): δ 1.53 (9H, s), 2.11 (3H, s), 6.83 (2H, d, J=8.6 Hz), 6.92 (1H, s), 7.30 (2H, d, J=8.6 Hz).

### <Example 173>

### t-Butyl 4-(4-methoxyphenyl)-4-oxobutanoate ester

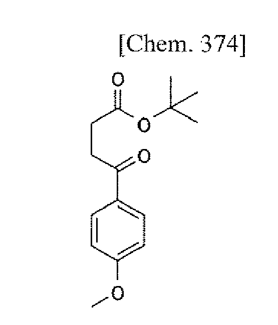

4-Methoxyacetophenone (15.0 g) was dissolved in THF (500 mL) under an argon atmosphere, and lithium hexamethyl disilazane (1.0 mol/L, THF solution, 119.9 mL) was added thereto at 0°C, followed by stirring at room temperature for 30 minutes. To the reaction liquid was added t-butyl bromoacetate (16.2 mL) at 0°C, followed by stirring at room temperature for 3 hours. Then, a saturated aqueous ammonium chloride solution was added thereto, followed by extraction with ethyl acetate. The extracted layer was washed with saturated brine and then dried over anhydrous sodium sulfate, and the solvent was evaporated under reduced pressure to obtain the desired product (27.4 g) as a red oil.
¹H NMR (CDCl₃, 400 MHz): δ 1.45 (9H, s), 2.67 (2H, t, J=6.7 Hz), 3.21 (2H, t, J=6.7 Hz), 3.87 (3H, s), 6.93(2H, d, J=8.9 Hz), 7.96 (2H, d, J=8.9 Hz).

### <Example 174>

### 6-(4-Methoxyphenyl)-4,5-dihydro-2H-pyridazin-3-one

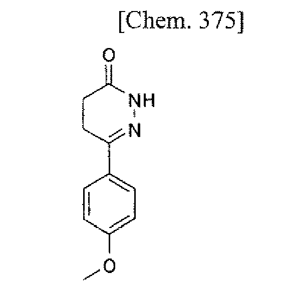

The compound of Example 173 (27.4 g) was dissolved in dichloromethane (100 mL), and trifluoroacetic acid (30 mL) was added thereto, and after leaving to stand for 16 hours, the solvent was evaporated under reduced pressure. The obtained oil was dissolved in ethanol (200 mL), and hydrazine monohydrate (14.5 mL) was added thereto, followed by stirring for 2.5 hours under the condition of heating to reflux. After evaporating the solvent under reduced pressure, the residue was washed with diethyl ether, the solid was collected by filtration to obtain the desired product (18.9 g) as a yellow powder.
EIMS (+): 204 [M]⁺.
¹H NMR (CDCl₃, 400 MHz): δ 2.60 (2H, t, J=8.3 Hz), 2.97 (2H, t, J=8.3 Hz), 3.85 (3H, s), 6.93 (2H, d, J=9.2 Hz), 7.67 (2H, d, J=9.2 Hz), 8.47 (1H, brs).

### <Example 175>

### 6-(4-Hydroxyphenyl)-4,5-dihydro-2H-pyridazin-3-one

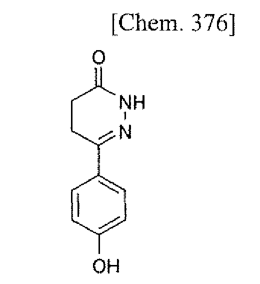

The compound of Example 174 (6.00 g) was dissolved in dichloromethane (300 mL), and aluminum chloride (78.4 g) was added thereto, followed by stirring at room temperature for 16 hours. To the reaction liquid was added water, followed by extraction with THF, the extracted layer was washed with saturated brine and then dried over anhydrous sodium sulfate, and the solvent was evaporated under reduced pressure to obtain the desired product (4.50 g) as a yellow powder.
EIMS (+): 190 [M]⁺.
¹H NMR (DMSO-d₆, 400 MHz): δ 2.38 (2H, t, J=8.3 Hz), 2.86 (2H, t, J=8.3 Hz), 6.77 (2H, d, J=8.6 Hz), 7.57 (2H, d, J=8.6 Hz), 6.77 (1H, s), 10.73 (1H, s).

### <Example 176>

### 6-(4-Hydroxyphenyl)-2H-pyridazin-3-one

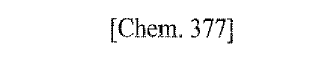

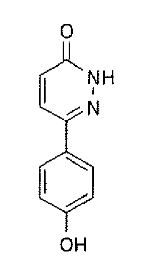

The compound of Example 175 (8.50 g) was dissolved in a 0.5 mol/L aqueous sodium hydroxide solution (500 mL), and sodium m-nitrobenzenesulfonate (10.3 g) was added thereto, followed by stirring for 1.5 hours under the condition of heating to reflux. The reaction liquid was neutralized with 6 mol/L hydrochloric acid and the precipitated solid was collected by filtration. The obtained solid was dissolved in dichloromethane (400 mL) and aluminum chloride (108 g) was added thereto, followed by stirring at room temperature for 19 hours. To the reaction liquid was added water, followed by extraction with THF, and the extracted layer was washed with saturated brine and then dried over anhydrous sodium sulfate. The residue obtained by evaporating the solvent under reduced pressure was washed with diethyl ether to obtain the desired product (3.29 g) as a yellow powder.
EIMS(+): 188 [M]⁺.
¹H NMR (DMSO-d₆, 400 MHz): δ 6.82 (2H, d, J=8.6 Hz), 6.90 (1H, d, J=9.8 Hz), 7.66 (2H, d, J=8.6 Hz), 7.92 (1H, d, J=9.8 Hz), 9.79 (1H, brs).

### <Example 177>

### 6-(4-t-Butyldimethylsilyloxyphenyl)-2H-pyridazin-3-one

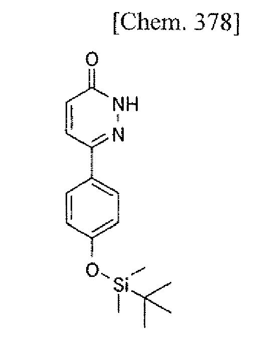

The reaction was carried out in the same manner as in Example 170 using the compound of Example 176 to obtain the desired product as a yellow powder.
¹H NMR (CDCl₃, 400 MHz): δ 0.24 (6H, s), 1.02 (9H, s), 6.93 (2H, d, J=8.6 Hz), 7.05 (1H, d, J=10.0 Hz), 7.67 (2H, d, J=8.6 Hz), 7.72 (1H, d, J=10.0 Hz).

### <Example 178>

### 2-t-Butoxycarbonyl-6-(4-t-butyidimethylsilyloxyphenyl)-2H-pyridazin-3-one

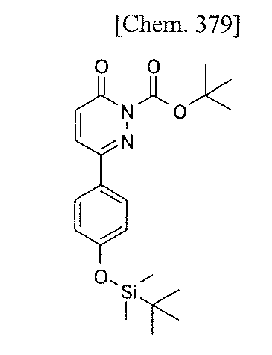

The reaction was carried out in the same manner as in Example 171 using the compound of Example 177 to obtain the desired product as a white powder.
¹H NMR (CDCl₃, 400 MHz): δ 0.22 (6H, s), 0.99 (9H, s), 1.66 (9H, s), 6.91 (2H, d, J=8.6 Hz), 6.98 (1H, d, J=9.8 Hz), 7.62 (1H, d, J=9.8 Hz), 7.69 (2H, d, J=8.6 Hz).

### <Example 179>

### 2-t-Butoxycarbonyl-6-(4-hydroxyphenyl)-2H-pyridazin-3-one

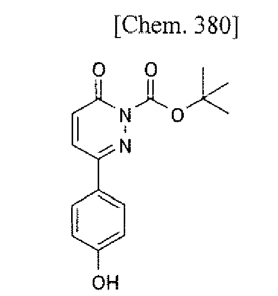

The reaction was carried out in the same manner as in Example 172 using the compound of Example 178 to obtain the desired product as a white powder.
¹H NMR (CDCl₃, 400 MHz): δ 1.55 (9H, s), 6.84 (2H, d, J=8.6 Hz), 7.05 (1H, d, J=10.4 Hz), 7.70 (2H, d, J=8.6 Hz), 8.01 (1H, d, J=10.4 Hz), 9.93 (1H, s).

### <Example 180>

### Methyl 3-(4-methoxypherlyl)-3-oxopropionate ester

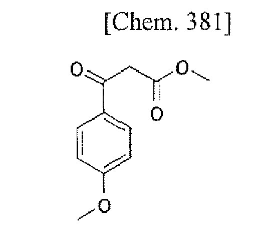

4-Methoxyacetophenone (7.00 g) was dissolved in dimethyl carbonate (100 mL), and a few droplets of 60% sodium hydride (5.60 g) and methanol were added thereto, followed by stirring for 1.5 hours under the condition of heating to reflux. To the reaction liquid was added a saturated aqueous ammonium chloride solution, followed by extraction with ethyl acetate, and the extracted layer was washed with saturated brine and then dried over anhydrous sodium sulfate. The solvent was evaporated under reduced pressure to obtain the desired product (9.70 g) as a colorless oil.
¹H NMR (CDCl₃, 400 MHz): δ 3.75 (3H, s), 3.88 (3H, s), 3.97 (2H, s), 6.95 (2H, d, J=8.9 Hz), 7.93 (2H, d, J=8.9 Hz).

### <Example 181>

### 5-(4-Methoxyphenyl)-4,4-dimethyl-2,4-dihydropyrazol-3 -one

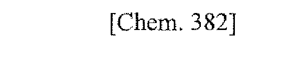

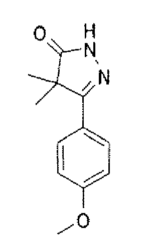

The compound of Example 180 (9.70 g) was dissolved in DMF (150 mL) under an argon atmosphere, and 60% sodium hydride (2.50 g) was added thereto at 0°C, followed by stirring at room temperature for 30 minutes. To the reaction liquid was added iodomethane (3.9 mL) at 0°C, followed by stirring at room temperature for 1 hour, 60% sodium hydride (2.50 g) was added thereto at 0°C, followed by stirring at room temperature for 30 minutes, and then iodomethane (3.9 mL) was added thereto at 0°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=10:1) to obtain a solid. The obtained solid was dissolved in ethanol (100 mL) and hydrazine monohydrate (6.70 mL) was added thereto, followed by stirring for 9 hours under the condition of heating to reflux. The solvent of the reaction liquid was evaporated under reduced pressure, and the residue was then washed with hexane to obtain the desired product (6.60 g) as a white powder.
¹H NMR (CDCl₃, 400 MHz): δ 1.51 (6H, s), 3.86 (3H, s), 6.95 (2H, d, J=9.2 Hz), 7.74 (2H, d, J=9.2 Hz).

### <Example 182>

### 5-(4-Hydroxyphenyl)-4,4-dimethyl-2,4-dihydropyrazol-3-one

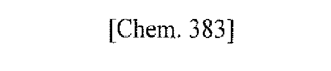

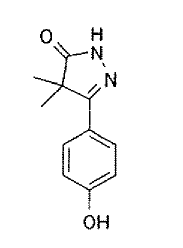

The compound of Example 181 (6.60 g) was dissolved in dichloromethane (300 mL), and aluminum chloride (80.6 g) was added thereto, followed by stirring at room temperature for 22 hours. To the reaction liquid was added water, followed by extraction with THF, and the extracted layer was washed with saturated brine and then dried over anhydrous sodium sulfate. The solvent was evaporated under reduced pressure and the residue was washed with diethyl ether to obtain the desired product (5.10 g) as a yellow powder.
¹H NMR (DMSO-d₆, 400 MHz): δ 6.93 (6H, s), 6.42 (2H, d, J=8.2 Hz), 7.26 (2H, d, J=8.6 Hz), 9.48 (1H, s), 10.91 (1H, s).

### <Example 183>

### 3,N-dimethoxy-N-methylbenzamide

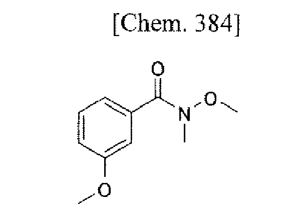

3-Methoxybenzoic acid (10.0 g) was dissolved in dichloromethane (300 mL), and N,O-dimethylhydroxylamine hydrochloride (7.00 g), triethylamine (11.9 mL), and 1-ethyl-3-(3-dimethylaminopropyl)-carbodiimide hydrochloride (18.9 g) were added thereto at 0°C, followed by stirring at room temperature for 18 hours. To the reaction liquid was added a saturated aqueous sodium hydrogen carbonate solution, followed by extraction with ethyl acetate, and the extracted layer was washed with saturated brine and then dried over anhydrous sodium sulfate. The solvent was evaporated under reduced pressure to obtain the desired product (14.5 g) as a yellow oil.
¹H NMR (CDCl₃, 400 MHz): δ 3.36 (3H, s), 3.58 (3H, s), 3.83 (3H, s), 6.98-7.01 (1H, m), 7.20-7.25 (2H, m), 7.31 (1H, t, J=7.9 Hz).

### <Example 184>

### 3-Methoxypropiophenone

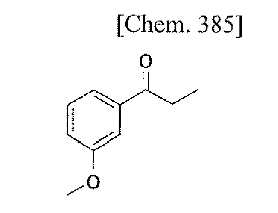

The compound of Example 183 (5.86 g) was dissolved in THF (150 mL) under an argon atmosphere, and ethyl magnesium bromide (0.96 mol/L, THF solution, 100 mL) was added thereto at 0°C, followed by stirring at room temperature for 3.5 hours. To the reaction liquid was added 1.0 mol/L hydrochloric acid, followed by extraction with ethyl acetate, and the extracted layer was washed with saturated brine and then dried over anhydrous sodium sulfate. The solvent was evaporated under reduced pressure to obtain the desired product (5.00 g) as a yellow oil.
¹H NMR (CDCl₃, 400 MHz): δ 1.23 (3H, t, J=7.3 Hz), 3.00 (2H, q, J=7.3 Hz), 3.86 (3H, s), 7.10 (1H, dd, J=7.9, 2.4 Hz), 7.37 (1H, t, J=7.9 Hz), 7.50 (1H, t, J=2.4 Hz), 7.54 (1H, d, J=7.9 Hz).

### <Example 185>

### 6-(3-Methoxyphenyl)-5-methyl-4,5-dlhydro-2H-pyridazin-3-one

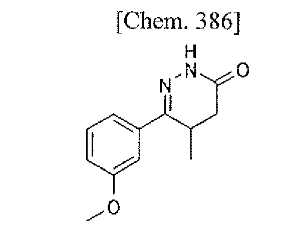

The compound of Example 184 (3.00 g) was dissolved in acetic acid (35 mL), and bromine (0.938 mL) was added thereto, followed by stirring at room temperature for 1.5 hours. The solvent of the reaction liquid was evaporated under reduced pressure, the residue was extracted with ethyl acetate, and the extracted layer was washed with 1.0 mol/L hydrochloric acid and a saturated aqueous sodium hydrogen carbonate solution in this order and then dried over anhydrous sodium sulfate. The solvent was evaporated under reduced pressure and the residue was obtained. Diethyl malonate (3.1 mL) was dissolved in DMF (20 mL) under an argon atmosphere, and 60% sodium hydride (752 mg) was added thereto at 0°C, followed by stirring at room temperature for 3 hours. Then, the previously obtained residue was dissolved in DMF (10 mL) and then added thereto, followed by stirring at 110°C for 2.5 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=8:1). The obtained oil was dissolved in 6.0 mol/L hydrochloric acid, followed by stirring for 8 hours under the condition of heating to reflux. The reaction liquid was extracted with ethyl acetate, the extracted layer was washed with saturated brine and then dried over anhydrous sodium sulfate, and the solvent was evaporated under reduced pressure. The obtained residue was dissolved in ethanol (75 mL), and hydrazine monohydrate (1.93 mL) was added thereto, followed by stirring for 5 hours under the condition of heating to reflux. The residue obtained by evaporating the solvent of the reaction liquid under reduced pressure was purified by silica gel chromatography (hexane:ethyl acetate=1:1) to obtain the desired product (1.44 g) as a yellow powder.
¹H NMR (CDCl₃, 400 MHz): δ 1.26 (3H, d, J=7.3 Hz), 2.48 (1H, d, J=17.7 Hz), 2.72 (1H, dd, J=17.7, 7.0 Hz), 3.33-3.37 (1H, m), 3.85 (3H, s), 6.96-6.98 (1H, m), 7.30-7.35 (3H, m), 8.53 (1H, brs).

### <Example 186>

### 6-(3-Hydroxyphenyl)-5-methyl-4,5-dihydro-2H-pyridazin-3-one

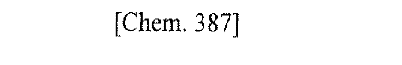

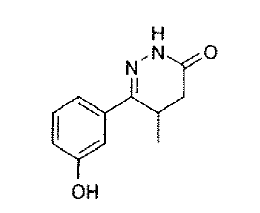

The compound of Example 185 (1.44 g) was dissolved in dichloromethane (50 mL) under an argon atmosphere, and borane tribromide (1.0 mol/L dichloromethane solution, 13.2 mL) was added thereto at 0°C, followed by stirring at room temperature for 5.5 hours. To the reaction liquid was added water, followed by extraction with THF, the extracted layer was washed with saturated brine and then dried over anhydrous sodium sulfate, and the solvent was evaporated under reduced pressure. The residue was washed with diethyl ether to obtain the desired product (630 mg) as a yellow powder. Further, the solvent of the diethyl ether washing liquid was evaporated under reduced pressure, and the residue was then purified by silica gel chromatography (hexane:ethyl acetate=4:1→1:1) to obtain the desired product (508 mg) as a white solid.
¹H NMR (DMSO-d₆, 400 MHz): δ 0.68 (3H, d, J=7.3 Hz), 1.84 (1H, d, J=15.9 Hz), 2.30 (1H, dd, J=15.9, 6.7 Hz), 2.33-2.95 (1H, m), 6.41-6.44 (1H, m), 6.80-6.87 (3H, m), 9.16 (1H, s), 10.55 (1H, s).

### <Example 187>

### Methyl 3-(3-methoxyphenyl)-2-methyl-3-oxopropionate ester

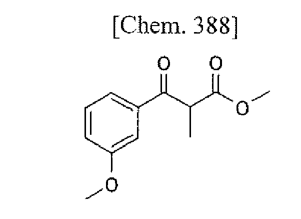

The compound of Example 184 (2.00 g) was dissolved in dimethyl carbonate (20 ml), and 60% sodium hydride (1.50 g) and a catalytic amount of methanol were added thereto, followed by stirring for 2.5 hours under the condition of heating to reflux. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=9: 1) to obtain the desired product (2.00 g) as a yellow oil.
¹H NMR (CDCl₃, 400 MHz): δ 1.50 (3H, d, J=6.7 Hz), 3.70 (3H, s), 3.86 (3H, s), 4.39 (1H, q, J=6.7 Hz), 7.14 (1H, dd, J=8.6, 2.4 Hz), 7.39 (1H, t, J=8.6 Hz), 7.51 (1H, t, J=2.4 Hz), 7.55 (1H, d, J=8.6 Hz).

### <Example 188>

### Methyl 3-(3-Methoxyphenyl)-2,2-dimethyl-3-oxopropionate ester

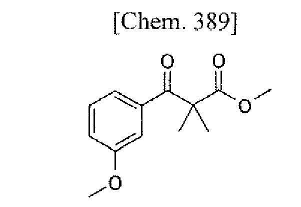

The compound of Example 187 (2.00 g) was dissolved in DMF (45 mL) under an argon atmosphere, and 60% sodium hydride (432 mg) was added thereto at 0°C, followed by stirring at room temperature for 30 minutes, and then iodomethane (0.673 mL) was added thereto at 0°C, followed by stirring at room temperature for 7 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=9:1) to obtain the desired product (1.84 g) as a colorless oil.
¹H NMR (CDCl₃, 400 MHz): δ 1.56 (6H, s), 3.65 (3H, s), 3.84 (3H, s), 7.06-7.09 (1H, m), 7.26-7.42 (3H, m).

### <Example 189>

### 5-(3-Methoxyphenyl)-4,4-dimethyl-2,4-dihydropyrazol-3-one

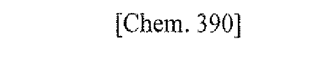

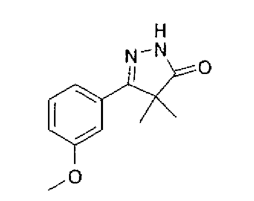

The compound of Example 188 (1.84 g) was dissolved in ethanol (50 mL), and hydrazine monohydrate (1.51 mL) was added thereto, followed by stirring for 6 hours under the condition of heating to reflux. The solvent of the reaction liquid was evaporated under reduced pressure to obtain the desired product (1.84 g) as a colorless oil.
¹H NMR (CDCl₃, 400 MHz): δ 1.53 (6H, s), 3.87 (3H, s), 6.98-7.02 (1H, m), 7.34-7.38 (3H, m), 8.56 (1H, brs).

### <Example 190>

### 5-(3-Hydroxyphenyl)-4,4-dimethyl-2,4-dihydropyrazol-3-one

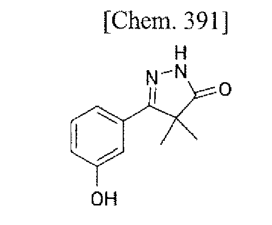

The compound of Example 189 (1.84 g) was dissolved in dichloromethane (80 mL) under an argon atmosphere, and aluminum chloride (22.7 g) was added thereto, followed by stirring for 26 hours under the condition of heating to reflux. To the reaction liquid was added water, followed by extraction with THF, and the extracted layer was washed with saturated brine and then dried over anhydrous sodium sulfate. The obtained residue was washed with diethyl ether to obtain the desired product (825 mg) as a yellow powder. Further, the solvent of the diethyl ether washing liquid was evaporated under reduced pressure, and the residue was then purified by silica gel chromatography (hexane:ethyl acetate=1:1) to obtain the desired product (290 mg) as a yellow powder.
¹H NMR (DMSO-d₆, 400 MHz): δ 1.05 (6H, s), 6.52-6.55 (1H, m), 6.92-6.97 (3H, m), 9.31 (1H, s), 11.20 (1H, s).

### <Example 191>

### 3-Fluoro-4-methoxypropiophenone

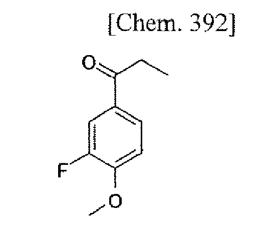

3-Fuoro-4-methoxybenzaldehyde (5.00 g) was dissolved in THF (150 mL), and ethyl magnesium bromide (0.96 mol/L THF solution 40.6 mL) was added thereto at -78°C, followed by stirring at room temperature for 5 hours. To the reaction liquid was added 1.0 mol/L hydrochloric acid, followed by extraction with ethyl acetate, and the extracted layer was washed with saturated brine and then dried over anhydrous sodium sulfate. The solvent was evaporated under reduced pressure to obtain an oil. DMSO (7.6 mL) was dissolved in dichloromethane (40 mL) under an argon atmosphere, and oxalyl chloride (6.2 mL) that had been dissolved in dichloromethane (20 mL) was added dropwise thereto at -78°C, followed by stirring at the same temperature for 15 minutes. To this reaction liquid was added dropwise a solution of the previously obtained oil in dichloromethane (40 mL) at -78°C, followed by stirring at the same temperature for 30 minutes, and then triethylamine (18 mL) was added dropwise thereto at the same temperature, followed by slowly warming to room temperature. To the reaction liquid was added water, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=15:1) to obtain the desired product (3.88 g) as a white powder.
¹H NMR (CDCl₃, 400 MHz): δ 1.22 (3H, t, J=7.3 Hz), 2.94 (2H, q, J=7.3 Hz), 3.96 (3H, s), 7.00 (1H, t, J=8.6 Hz), 7.69-7.77 (2H, m).

### <Example 192>

### 4-(3-Fluoro-4-methoxyphenyl)-3-methyl-4-oxobutanoic acid

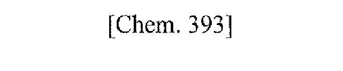

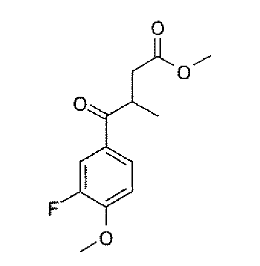

The compound of Example 191 (1.90 g) was dissolved in THF (75 mL) under an argon atmosphere, and a lithium hexamethyl disilazane (1.0 mol/L THF solution, 11.5 mL) was added thereto at -78°C, followed by stirring at room temperature for 30 minutes. To the reaction liquid was added methyl bromoacetate (0.825 mL) at -78°C, followed by stirring at room temperature for 5 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=1:1) to obtain the desired product (1.98 g) as a yellow oil.
¹H NMR (CDCl₃, 400 MHz): δ 1.22 (3H, d, J=7.3 Hz), 2.45 (1H, dd, J=16.5, 5.5 Hz), 2.95 (1H, dd, J=16.5, 8.6 Hz), 3.65 (3H, s), 3.83-3.89 (1H, m), 3.96 (3H, s), 7.02 (1H, t, J=8.2 Hz), 7.73 (1H, dd, J=11.9, 2.1 Hz), 7.79 (1H, m).

### <Example 193>

### 6-(3-Fluoro-4-methoxyphenyl)-5-methyl-4,5-dihydro-2H-pyridazin-3-one

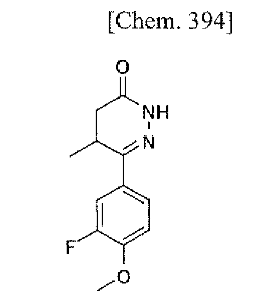

The compound of Example 192 (1.98 g) was dissolved in ethanol (50 mL), and hydrazine monohydrate (2.26 mL) and acetic acid (2.68 mmol) were added thereto, followed by stirring for 7.5 hours under the condition of heating to reflux. To the reaction liquid was added water, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=1:1) to obtain the desired product (1.70 g) as a yellow powder.
¹H NMR (CDCl₃, 400 MHz): δ 1.24 (3H, d, J=7.3 Hz), 2.48 (1H, d, J=17.1 Hz), 2.71 (1H, dd, J=17.1, 6.7 Hz), 3.26-3.33 (1H, m), 3.94 (3H, s), 6.98 (1H, t, J=8.6 Hz), 7.44 (1H, dd, J=8.6, 1.2 Hz), 7.56 (1H, dd, J=11.6, 1.2 Hz), 8.52 (1H, brs).

### <Example 194>

### 6-(3-Fluoro-4-hydroxyphenyl)-5-methyl-4,5-dihydro-2H-pyridazin-3-one

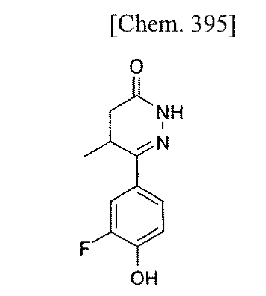

The compound of Example 193 (1.70 g) was dissolved in dichloromethane (50 mL) under an argon atmosphere, and aluminum chloride (19.2 g) was added thereto, followed by stirring at room temperature for 6 hours. To the reaction liquid was added water, followed by extraction with THF, the extracted layer was washed with saturated brine and then dried over anhydrous sodium sulfate, and the solvent was evaporated under reduced pressure. The obtained residue was washed with diethyl ether to obtain the desired product (1.33 g) as a yellow powder.
¹H NMR (DMSO-d₆, 400 MHz): δ 0.68 (3H, d, J=7.3 Hz), 1.84 (1H, d, J=16.5 Hz), 2.28 (1H, dd, J=16.5, 6.7 Hz), 2.92-3.00 (1H, m), 6.63 (1H, t, J=8.6 Hz), 7.07 (1H, dd, J=8.6, 1.0 Hz), 7.16 (1H, dd, J=13.1, 2.1 Hz), 9.91 (1H, s), 10.51 (1H, s).

### <Example 195>

### 2-Fluoro-4-methoxypropiophenone

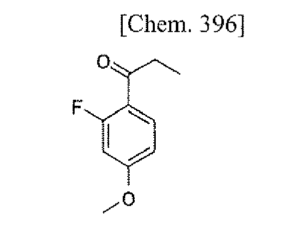

A commercially available 2-fluoro-4-methoxybenzaldehyde (5.00 g) was dissolved in THF (150 mL), and ethyl magnesium bromide (0.96 mol/L THF solution, 40.6 mL) was added thereto at -78°C, followed by stirring at room temperature for 4 hours. To the reaction liquid was added 1.0 mol/L hydrochloric acid, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=10:1) to obtain an oil. The obtained oil was dissolved in dimethyl sulfoxide (50 mL), and triethylamine (12.0 mL) and a sulfur trioxide-pyridine complex (6.80 g) were added thereto, followed by stirring at room temperature for 1 hour. To the reaction liquid was added water, followed by extraction with a mixed solvent of ethyl acetate and hexane (ethyl acetate:hexane=1:4), and the extracted layer was washed with saturated brine and then dried over anhydrous sodium sulfate. The residue obtained by evaporating the solvent under reduced pressure was purified by silica gel chromatography (hexane:ethyl acetate=9:1) to obtain the desired product (1.27 g) as a white powder.
¹H NMR (CDCl₃, 400 MHz): δ 1.19 (3H, t, J=7.3 Hz), 2.92-2.99 (2H, m), 3.86 (3H, s), 6.61 (1H, dd, J=13.4, 2.4 Hz), 6.75 (1H, dd, J=8.6, 2.4 Hz), 7.90 (1H, t, J=8.6 Hz).

### <Example 196>

### 6-(2-Fluoro-4-methoxyphenyl)-5-methyl-4,5-dihydro-2H-pyridazim-3-one

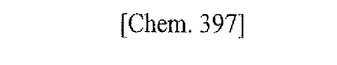

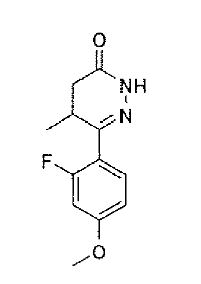

The compound of Example 195 (670 mg) was dissolved in THF (35 mL) under an argon atmosphere, and a lithium hexamethyl disilazane (1.0 mol/L, THF solution, 4.0 mL) was added thereto at 0°C, followed by stirring at room temperature for 30 minutes, and then t-butyl bromoacetate (0.591 mL) was added thereto at 0°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was dissolved in dichloromethane (10 mL), and trifluoroacetic acid (5 mL) was added thereto, followed by stirring at room temperature for 16 hours. The solvent of the reaction liquid was evaporated under reduced pressure, the residue was dissolved in ethanol (35 mL), and acetic acid (1.85 mL) and hydrazine monohydrate (0.714 mL) was added thereto, followed by stirring for 6 hours under the condition of heating to reflux. To the reaction liquid was added water, followed by extraction with ethyl acetate, and the extracted layer was washed with saturated brine and then dried over anhydrous sodium sulfate. The residue obtained by evaporating the solvent under reduced pressure was purified by NH type silica gel (Chromatorex: trademark) chromatography (hexane:ethyl acetate=1:1) to obtain the desired product (585 mg) as a yellow powder.
¹H NMR (CDCl₃, 400 MHz): δ 1.20 (3H, d, J=7.3 Hz), 2.43 (1H, dd, J=17.1, 3.0 Hz), 2.73 (1H, dd, J=17.1, 6.4 Hz), 3.27-3.31 (1H, m), 3.84 (3H, s), 6.64 (1H, dd, J=13.4, 2.4 Hz), 6.74 (1H, dd, J=8.6, 2.4 Hz), 7.55 (1H, t, J=8.6 Hz), 8.48 (1H, brs).

### <Example 197>

### 6-(2-Fluoro-4-hydroxyphenyl)-5-methyl-4,5-dihydro-2H-pyridazin-3-one

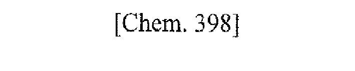

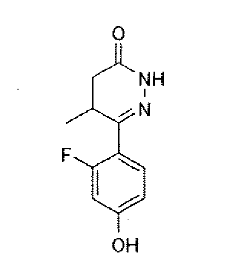

The compound of Example 196 (584 mg) was dissolved in dichloromethane (25 mL) under an argon atmosphere, and aluminum chloride (7.20 g) was added thereto, followed by stirring at room temperature for 15 hours. To the reaction liquid was added water, followed by extraction with THF, the extracted layer was washed with saturated brine and then dried over anhydrous sodium sulfate, and the solvent was evaporated under reduced pressure. The obtained residue was washed with diethyl ether to obtain the desired product (430 mg) as a yellow powder.
EIMS (+): 222 [M]⁺.
¹H NMR (DMSO-d₆, 400 MHz): δ 1.02 (3H, d, J=6.7 Hz), 2.20 (1H, dd, J=16.5, 3.0 Hz), 2.63 (1H, dd, J=17.1, 6.7 Hz), 3.08-3.13 (1H, m), 6.59 (1H, dd, J=13.4, 2.4 Hz), 6.65 (1H, dd, J=8.6, 2.4 Hz), 7.44 (1H, t, J=9.2 Hz), 10.23 (1H, s), 10.89 (1H, s).

### <Example 198>

### 4-(t-Butyldimethylsilyloxy)-3-methoxybenzaldehyde

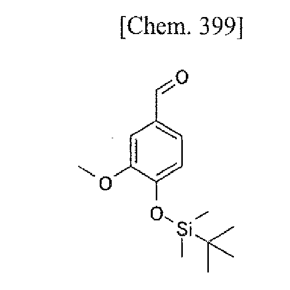

Vaniline (5.00 g) was dissolved in DMF (150 mL) under an argon atmosphere, and imidazole (3.36 g) and chloro-t-butyldimethylsilane (5.45 g) were added thereto at 0°C, followed by stirring at room temperature for 2.5 hours. The solvent of the reaction liquid was evaporated under reduced pressure and then to the residue was added water, followed by extraction with ethyl acetate. The extracted layer was washed with saturated brine and then dried over anhydrous sodium sulfate. After evaporating the solvent under reduced pressure, the residue was purified by silica gel chromatography (hexane:ethyl acetate=18:1) to obtain the desired product (8.30 g) as a colorless oil.
¹H NMR (CDCl₃, 400 MHz): δ 0.21 (6H, s), 1.02 (9H, s), 3.89 (3H, s), 6.98 (1H, d, J=7.9 Hz), 7.3 (1H, dd, J=7.9, 1.8 Hz), 7.41 (1H, d, J=1.8 Hz), 9.86 (1H, s).

### <Example 199>

### 4-(t-Butyldimethylsilyloxy)-3-methoxypropiophenone

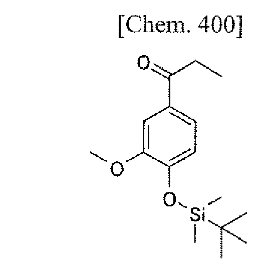

The compound of Example 198 (8.30 g) was dissolved in THF (200 mL) under an argon atmosphere, and ethyl magnesium bromide (0.97 mol/L, THF solution, 35.3 mL) was added thereto at -78°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in DMSO (150 mL), and triethylamine (43.6 mL) and a sulfur trioxide-pyridine complex (24.8 g) were added thereto, followed by stirring at room temperature for 1 hour. To the reaction liquid was added 1 mol/L hydrochloric acid until the solution turned acidic, followed by extraction three times with a mixed solvent of hexane and ethyl acetate (hexane:ethyl acetate=4:1), and the combined organic layer was washed with saturated brine and then dried over anhydrous sodium sulfate. After evaporating the solvent under reduced pressure, the residue was purified by silica gel chromatography (hexane:ethyl acetate=9:1→2:1) to obtain the desired product (4.32 g) as a yellow oil.
¹H NMR (CDCl₃, 400 MHz): δ 0.19 (6H, s), 1.0 (9H, s), 1.22 (3H, t, J=7.3 Hz), 2.97 (2H, q, J=7.3 Hz), 3.87 (3H, s), 6.88 (1H, d, J=7.9 Hz), 7.49 (1H, dd, J=7.9, 1.8 Hz), 7.54 (1H, d, J=1.8 Hz).

### <Example 200>

### Methyl 4-[4-(t-Butyldimethylsilyloxy)-3-methoxyphenyl]-3-methyl-4-oxobutanoate ester

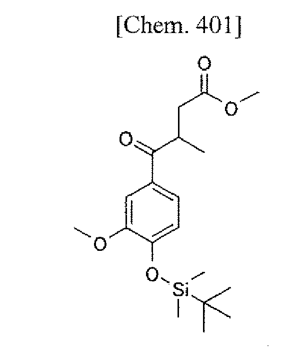

The compound of Example 199 (4.32 g) was dissolved in THF (100 mL) under an argon atmosphere, and a lithium hexamethyl disilazane (1.0 mol/L, THF solution, 16.9 mL) was added thereto at -78°C, followed by stirring at 0°C for 30 minutes, and then methyl bromoacetate (1.81 mL) was added thereto at -78°C, followed by stirring at room temperature for 2 hours. To the reaction liquid was added a saturated aqueous ammonium chloride solution, followed by extraction with ethyl acetate, and the extracted layer was washed with saturated brine and then dried over anhydrous sodium sulfate. The solvent of the filtrate was evaporated under reduced pressure, and the residue was then purified by silica gel chromatography (hexane:ethyl acetate=8:→2:1) to obtain the desired product (3.27 g) as a yellow oil.
¹H NMR (CDCl₃, 400 MHz): δ 0.18 (3H, s), 0.19 (3H, s), 1.00 (9H, s), 1.23 (3H, d, J=7.3 Hz), 2.45 (1H, dd, J=16.5, 5.8 Hz), 2.94 (1H, dd, J=16.5, 7.9 Hz), 3.65 (3H, s), 3.86 (3H, s), 3.90-3.92 (1H, m), 6.89 (1H, d, J=8.6 Hz), 7.52-7.55 (2H, m).

### <Examples 201>

### 6-(4-Hydroxy-3-methoxyphenyl)-5-methyl-4,5-dihydro-2H-pyridazin-3-one

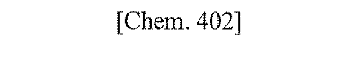

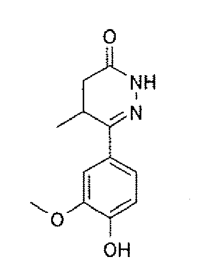

The compound of Example 200 (1.50 g) was dissolved in ethanol (40 mL), and acetic acid (0.718 mL) and hydrazine monohydrate (0.609 mL) were added thereto, followed by stirring for 2.5 hours under the condition of heating to reflux. To the reaction liquid was added water, followed by extraction with ethyl acetate, and the extracted layer was washed with saturated brine and then dried over anhydrous sodium sulfate. After evaporating the solvent under reduced pressure, the residue was dissolved in tetrahydrofuran (40 mL), and tetrabutyl ammonium fluoride (1.0 mol/L THF solution, 5.43 mL) was added thereto, followed by stirring at room temperature for 2 hours. To the reaction liquid was added 1 mol/L hydrochloric acid until the solution turned acidic, followed by extraction with ethyl acetate, and the extracted layer was washed with saturated brine and then dried over sodium sulfate. After evaporating the solvent under reduced pressure, the residue was purified by silica gel chromatography (hexane:ethyl acetate=1:3) to obtain the desired product (691 mg) as a yellow oil.
¹H NMR (CDCl₃, 400 MHz): δ 1.23 (3H, d, J=7.3 Hz), 2.38 (1H, d, J=16.5 Hz), 2.82 (1H, dd, J=16.5, 6.7 Hz), 3.53-3.54 (1H, m), 3.97 (3H, s), 6.99 (1H, d, J=8.6 Hz), 7.37 (1H, dd, J=8.6, 1.8 Hz), 7.54 (1H, d, J=1.8 Hz), 9.60 (1H, s), 10.97 (1H, s).

### <Example 202>

### 6-(3-Fluoro-4-methoxyphenyl)-4,5-dihydro-2H-pyridazin-3-one

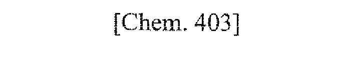

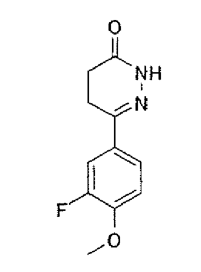

3-Fluoro-4-methoxyacetophenone (5.00 g) was dissolved in THF (150 mL) under an argon atmosphere, and lithium hexamethyl disilazane (1.0 mol/L THF solution, 31.2 mL) was added thereto at 0°C, followed by stirring at room temperature for 30 minutes, and then t-butyl bromoacetate (4.61 mL) was added thereto at 0°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in dichloromethane (30 mL), and trifluoroacetic acid (10 mL) was added thereto, followed by stirring at room temperature for 7 hours. After evaporating the solvent under reduced pressure, the residue was dissolved in ethanol (150 mL), and hydrazine monohydrate (4.33 mL) was added thereto, followed by stirring for 3 hours under the condition of heating to reflux. The reaction liquid was concentrated under reduced pressure, and the obtained residue was then purified by silica gel chromatography (hexane:ethyl acetate=1:1→ethyl acetate) to obtain the desired product (3.88 g, 57%) as a yellow powder.
¹H NMR (CDCl₃, 400 MHz): δ 2.61 (2H, t, J=7.9 Hz), 2.95 (2H, t, J=7.9 Hz), 3.93 (3H, s), 6.97 (1H, dd, J=8.6, 8.6 Hz), 7.41 (1H, dd, J=8.6, 2.4 Hz), 7.53 (1H, dd, J=12.8, 2.4 Hz), 8.57 (1H, brs).

### <Example 203>

### 6-(3-Fluoro-4-hydroxyphenyl)-4,5-dihydro-2H-pyridazin-3-one

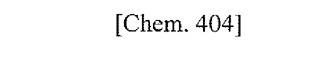

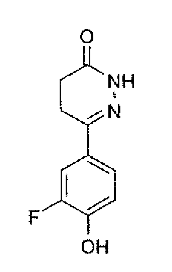

The compound of Example 202 (3.88 g) was dissolved in dichloromethane (100 mL) under an argon atmosphere, and aluminum chloride (45.3 g) was added thereto, followed by stirring at room temperature for 15 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (3.42 g) as a yellow powder.
¹H NMR (DMSO-d₆, 400 MHz): δ 2.39 (2H, t, J=8.3 Hz), 2.87 (2H, t, J=8.3 Hz), 6.96 (1H, dd, J=8.6, 8.6 Hz), 7.39 (1H, dd, J=8.6, 2.4 Hz), 7.49 (1H, dd, J=12.8, 2.4 Hz), 10.24 (1H, brs), 10.81 (1H, brs).

### <Example 204>

### t-Butyl 2,4,5-trifluorobenzoate ester

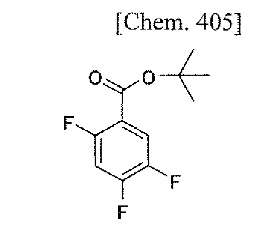

2,4,5-Trifluorobenzoic acid (5.00 g, 28.4 mmol) was dissolved in t-butanol (140 mL), and di-t-butyldicarbonate (12.4 g) and N,N-dimethyl aminopyridine (347 mg) were added thereto, followed by stirring at room temperature for 13 hours. To the reaction liquid was added ethyl acetate, and the organic layer was washed twice with 1 mol/L hydrochloric acid and twice with a saturated aqueous sodium hydrogen carbonate solution, and then dried over anhydrous sodium sulfate. After evaporating the solvent under reduced pressure, the residue was purified by silica gel chromatography (hexane:ethyl acetate=99:1) to obtain the desired product (6.04 g) as a colorless oil.
¹H NMR (CDCl₃, 400 MHz): δ 1.59 (9H, s), 6.96 (1H, ddd, J=9.9, 6.3, 3.2 Hz), 7.71 (1H, ddd, J=12.8, 6.4, 4.0 Hz).

### <Example 205>

### t-Butyl 2,5-difluoro-4-methoxybenzoate ester

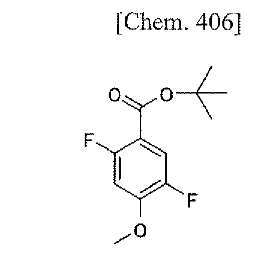

Methanol (0.996 mL) was dissolved in a 50% aqueous sodium hydroxide solution (70 mL), and a solution of tetrabutyl ammonium sulfate (2.78 g) and the compound of Example 204 (5.72 g) in toluene (170 mL) was added thereto, followed by stirring at room temperature for 1 hour. The reaction liquid was extracted with ethyl acetate, and the extracted layer was washed with saturated brine and then dried over anhydrous sodium sulfate. After evaporating the solvent under reduced pressure, the residue was purified by silica gel chromatography (hexane:ethyl acetate=20:1) to obtain the desired product (3.43 g) as a white powder.
¹H NMR (CDCl₃, 400 MHz), δ 1.58 (9H, s), 3.92 (3H, s), 6.68 (1H, dd, J=11.6, 6.7 Hz), 7.59 (1H, dd, J=11.6, 6.7 Hz).

### <Example 206>

### 2,5-Difluoro-4,N-dimethoxy-N-methylbenzamide

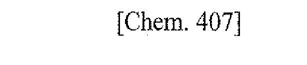

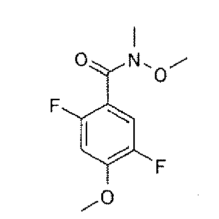

The compound of Example 205 (3.43 g) was dissolved in dichloromethane (50 mL), and trifluoroacetic acid (20 mL) was added thereto, followed by stirring at room temperature for 3 hours. The reaction liquid was concentrated under reduced pressure, the residue was then dissolved in dichloromethane (70 mL), and N,O-dimethylhydroxylamine hydrochloride (1.76 g), triethylamine (3.14 mL), and 1-ethyl-3-(3-dimethylaminopropyl)-carbodiimide hydrochloride (16.6 g) were added thereto at 0°C, followed by stirring at room temperature for 8 hours. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (ethyl acetate) to obtain the desired product (3.15 g) as a yellow oil.
¹H NMR (CDCl₃, 400 MHz): δ 3.34 (3H, s), 3.59 (3H, s), 3.91 (3H, s), 6.71 (1H, dd, J=10.4, 7.5 Hz), 7.20 (1H, dd, J=10.4, 6.1 Hz).

### <Example 207>

### 2,5-Difluoro-4-methoxyacetophenone

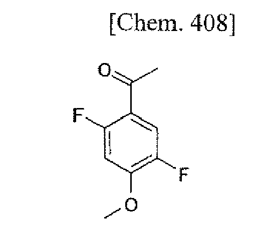

The compound of Example 206 (1.50 g) was dissolved in THF (30 mL) under an argon atmosphere, and methyl magnesium bromide (0.84 mol/L THF solution, 17.0 mL) was added thereto at 0°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=4:1) to obtain the desired product (1.12 g) as a white powder.
¹H NMR (CDCl₃, 400 MHz): δ 2.60 (3H, d, J=5.5 Hz), 3.94 (3H, s), 6.70 (1H, dd, J=11.6, 6.7 Hz), 7.64 (1H, dd, J=11.6, 6.7 Hz).

### <Example 208>

### 6-(2,5-Difluoro-4-methoxyphenyl)-4,5-dihydro-2H-pyridazin-3-one

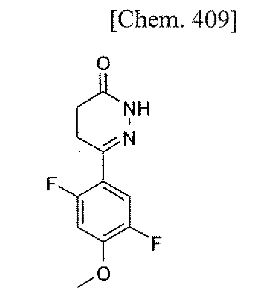

The compound of Example 207 (1.12 g) was dissolved in THF (30 mL) under an argon atmosphere, and lithium hexamethyl disilazane (1.0 mol/L THF solution, 6.32 mL) was added thereto at 0°C, followed by stirring at room temperature for 30 minutes, and then t-butyl bromoacetate (1.16 mL) was added thereto at 0°C, followed by stirring at room temperature for 5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in dichloromethane (30 mL), and trifluoroacetic acid (10 mL) was added thereto, followed by stirring at room temperature for 2.5 hours. To the reaction liquid was added potassium carbonate, and a saturated aqueous sodium hydrogen carbonate solution was added thereto, followed by washing with ethyl acetate. Then, the aqueous layer was acidified with 1.0 mol/L hydrochloric acid and extracted three times with ethyl acetate. The combined extracted layer was washed with saturated brine and then dried over anhydrous sodium sulfate, and the solvent was evaporated under reduced pressure. The residue was dissolved in ethanol (20 mL), and hydrazine monohydrate (0.564 mL) and acetic acid (1.46 mL) were added thereto, followed by stirring for 10 hours under the condition of heating to reflux. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=4:1) to obtain the desired product (478 mg) as a white powder.
¹H NMR (CDCl₃, 400 MHz): δ 2.57-2.59 (2H, m), 2.98-3.03 (2H, m), 3.91 (3H, s), 6.70 (1H, dd, J=12.2, 7.3 Hz), 7.43 (1H, dd, J=12.2, 7.3 Hz), 8.50 (1H, s).

### <Example 209>

### 6-(2,5-Difluoro-4-hydroxyphenyl)-4,5-dihydro-2H-pyridazin-3-one

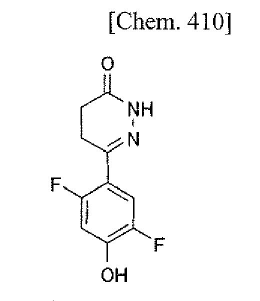

The compound of Example 208 (478 mg) was dissolved in dichloromethane (20 mL) under an argon atmosphere, and aluminum chloride (5.31 g) was added thereto, followed by stirring at room temperature for 17 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (391 mg) as a white powder.
¹H NMR (DMSO-d₆, 400 MHz): δ 2.39 (2H, t, J=8.3 Hz), 2.83-2.85 (2H, m), 6.79 (1H, dd, J=12.2, 7.3 Hz), 7.39 (1H, dd, J=12.2, 7.3 Hz), 10.76 (1H, s), 10.94 (1H, s).

### <Example 210>

### (2,3-Difluorophenoxy) triisopropylsilane

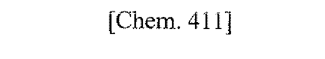

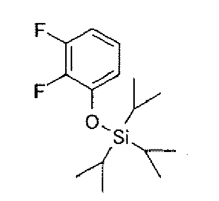

2,3-Difluorophenol (10.0 g) was dissolved in DMF (40 mL), and triisopropylsilyl chloride (16.5 mL) and imidazole (5.24 g) were added thereto, followed by stirring at room temperature for 18 hours. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=99:1) to obtain the desired product (21.0 g) as a colorless oil.
¹H NMR (CDCl₃, 400 MHz): δ 1.12 (18H, d, J=3.7 Hz), 1.27-1.32 (3H, m), 6.72-6.75 (2H, m), 6.88-6.90 (1H, m).

### <Example 211>

### 2,3-Difluoro-4-triisopropylsilyloxy benzoic acid

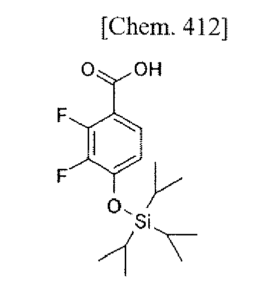

The compound of Example 210 (4.00 g) was dissolved in THF (20 mL) under an argon atmosphere, 2,2,6,6-tetramethylpiperizide (2.60 mL) was added thereto, and then n-butyl lithium (2.71 mol/L hexane solution, 5.41 mL) was added thereto at -78°C, followed by stirring at the same temperature for 2 hours. Carbon dioxide was injected into the reaction liquid at -78°C, followed by stirring for 30 minutes, and the liquid was acidified with the addition of 1 mol/L hydrochloric acid at room temperature, and extracted three times with ethyl acetate. The combined organic layer was washed with saturated brine and then dried over anhydrous sodium sulfate. After evaporating the solvent under reduced pressure, the residue was purified by silica gel chromatography (hexane:ethyl acetate=1:1) to obtain the desired product (3.94 g) as a white powder.
¹H NMR (CDCl₃, 400 MHz): δ 1.11 (18H, d, J=7.3 Hz), 1.26-1.35 (3H, m), 6.77 (1H, ddd, J=8.6, 7.3, 1.8 Hz), 7.68-7.69 (1H, m).

### <Example 212>

### 2,3-Difluoro-4-triisopropylsilyloxyacetophenone

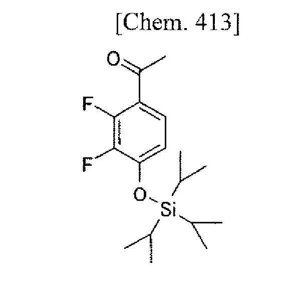

The compound of Example 211 (3.11 g) was dissolved in dichloromethane (50 mL), and N,O-dimethylhydroxylamine hydrochloride (1.10 g), triethylamine (1.97 mL), and 1-ethyl-3-(3-dimethylaminopropyl)-carbodiimide hydrochloride (2.71 g) were added thereto at 0°C, followed by stirring at room temperature for 4 hours. To the reaction liquid was added water, followed by extraction three times with ethyl acetate, and the combined organic layer was washed with saturated brine and then dried over anhydrous sodium sulfate. The solvent was evaporated under reduced pressure, the obtained residue was dissolved in tetrahydrofuran (50 mL), and methyl magnesium bromide (0.84 mol/L THF solution, 25.7 mL) was added thereto at 0°C under an argon atmosphere, followed by stirring at room temperature for 1.5 hours. To the reaction liquid was added a saturated aqueous ammonium chloride solution, followed by extraction three times with ethyl acetate, and the combined organic layer was washed with saturated brine and then dried over anhydrous sodium sulfate. After evaporating the solvent under reduced pressure, the residue was purified by silica gel chromatography (hexane:ethyl acetate=50:1) to obtain the desired product (1.80 g) as a colorless oil.
¹H NMR (CDCl₃, 400 MHz): δ 1.11 (18H, d, J=7.3 Hz), 1.25-1.34 (3H, m), 2.61 (3H, d, J=4.9 Hz), 6.76 (1H, ddd, J=9.2, 7.3, 1.8 Hz), 7.56-7.57 (1H, m).

### <Example 213>

### 6-(2,3-Difluoro-4-hydroxyphenyl)-4,5-dihydro-2H-pyridazin-3-one

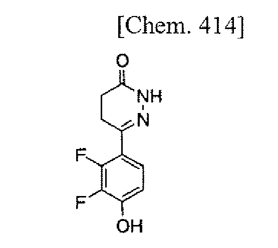

The compound of Example 212 (1.80 g) was dissolved in THF (30 mL) under an argon atmosphere, and lithium hexamethyl disilazane (1.0 mol/L THF solution, 6.30 mL) was added thereto at 0°C, followed by stirring at room temperature for 30 minutes, and then t-butyl bromoacetate (1.05 mL) was added thereto at 0°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in dichloromethane (30 mL), and trifluoroacetic acid (10 mL) was added thereto, followed by stirring at room temperature for 16 hours. To the reaction liquid was added potassium carbonate, and a saturated aqueous sodium hydrogen carbonate solution was added thereto, followed by washing with ethyl acetate. The aqueous layer was acidified with 1.0 mol/L hydrochloric acid, followed by extraction with ethyl acetate, and the extracted layer was washed with saturated brine and dried over anhydrous sodium sulfate. After evaporating the solvent under reduced pressure, the residue was dissolved in ethanol (40 mL), and hydrazine monohydrate (0.799 mL) and acetic acid (2.07 mL) were added thereto, followed by stirring for 5.5 hours under the condition of heating to reflux. To the reaction liquid was added water, followed by extraction with ethyl acetate, and the extracted layer was washed with saturated brine, dried over anhydrous sodium sulfate, and then filtered. The solvent of the filtrate was evaporated under reduced pressure, and the residue was then purified by silica gel chromatography (hexane:ethyl acetate=4:1) to obtain the desired product (432 mg) as a white powder.
¹H NMR (CDCl₃, 400 MHz): δ 2.40 (2H, t, J=8.3 Hz), 2.85 (2H, td, J=8.3, 1.6 Hz), 6.80 (1H, ddd, J=8.6, 8.6, 1.8 Hz), 7.24 (1H, ddd, J=8.6, 8.6, 2.4 Hz), 10.73 (1H, s), 10.94 (1H, s).

### <Example 214>

### Methyl 3-(3-fluoro-4-methoxyphenyl)-2-methyl-3-oxopropionate ester

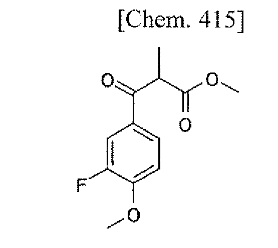

The compound of Example 191 (1.95 g) was dissolved in dimethyl carbonate (50 ml), and 60% sodium hydride (1.28 g) and a catalytic amount of methanol were added thereto, followed by stirring for 4 hours under the condition of heating to reflux. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=9:1→4:1) to obtain the desired product (2.20 g) as a yellow oil.
¹H NMR (CDCl₃, 400 MHz): δ 1.48 (3H, d, J=7.3 Hz), 3.69 (3H, s), 3.96 (3H, s), 4.32 (1H, q, J=7.3 Hz), 7.01 (1H, t, J=8.2 Hz), 7.72 (1H, dd, J=12.2, 2.1 Hz), 7.76-7.79 (1H, m).

### <Example 215>

### Methyl 3-(3-fluoro-4-methoxyphenyl)-2,2-dimethyl-3-oxopropionate ester

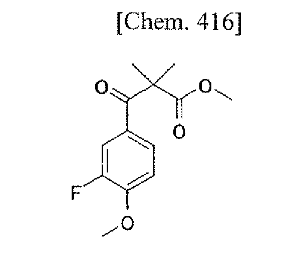

The compound of Example 214 (2.20 g) was dissolved in DMF (50 mL) under an argon atmosphere, and 60% sodium hydride (440 mg) was added thereto at 0°C, followed by stirring at room temperature for 30 minutes, and then iodomethane (0.685 mL) was added thereto at 0°C, followed by stirring at room temperature for 2 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=9:1) to obtain the desired product (2.10 g) as a colorless oil.
¹H NMR (CDCl₃, 400 MHz): δ 1.53 (6H, s), 3.66 (3H, s), 3.94 (3H, s), 6.95 (1H, t, J=8.6 Hz), 7.58-7.65 (2H, m).

### <Example 216>

### 5-(3-Fluoro-4-methoxyphenyl)-4,4-dimethyl-2,4-dihydropyrazol-3-one

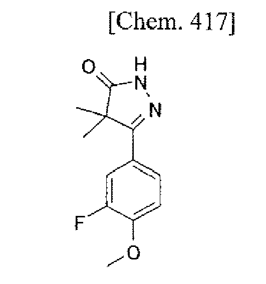

The compound of Example 215 (2.10 g) was dissolved in ethanol (40 mL), and hydrazine monohydrate (1.20 mL) was added thereto, followed by stirring for 8 hours under the condition of heating to reflux. The solvent of the reaction liquid was evaporated under reduced pressure and the residue was purified by silica gel chromatography (hexane:ethyl acetate=1:1) to obtain the desired product (1.59 g) as a white powder.
¹H NMR (CDCl₃, 400 MHz): δ 1.50 (6H, s), 3.94 (3H, s), 6.99 (1H, t, J=8.6 Hz), 7.49 (1H, d, J=8.6 Hz), 7.57 (1H, dd, J=12.2, 1.8 Hz), 8.49 (1H, brs).

### <Example 217>

### 5-(3-Fluoro-4-hydroxyphenyl)-4,4-dimethyl-2,4-dihydropyrazol-3-one

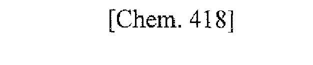

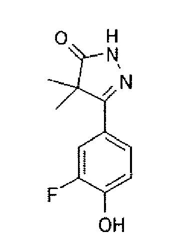

The compound of Example 216 (1.59 g) was dissolved in dichloromethane (50 mL) under an argon atmosphere, and aluminum chloride (17.9 g) was added thereto, followed by stirring at room temperature for 6 hours. To the reaction liquid was added water, followed by extraction with THF, the extracted layer was washed with saturated brine and then dried over anhydrous sodium sulfate, and the solvent was evaporated under reduced pressure. The obtained residue was washed with diethyl ether to obtain the desired product (1.30 g) as a yellow powder.
¹H NMR (DMSO-d₆, 400 MHz): δ 1.32 (6H, s), 7.00 (1H, t, J=8.9 Hz), 7.45 (1H, dd, J=7.9, 2.1 Hz), 7.52 (1H, dd, J=12.5,2.1 Hz), 10.37 (1H, s), 11.41 (1H, s).

### <Example 218>

### Methyl 3-(2-fluoro-4-methoxyphenyl)-2,2-dimethyl-3-oxopropionate ester

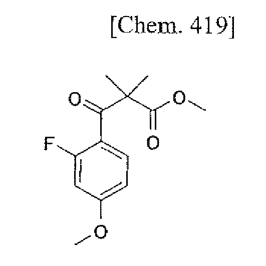

Diisopropyl amine (1.48 mL) was dissolved in THF (20 mL) under an argon atmosphere, and an n-butyl lithium (2.71 mol/L hexane solution, 3.87 mL) was added thereto at -78°C, followed by stirring at 0°C for 25 minutes, and then methyl isobutyrate (1.12 mL, 9.73 mmol) was added thereto at -78°C, followed by stirring at room temperature for 30 minutes. To the reaction liquid was added a solution of 2-fluoro-4-methoxybenzaldehyde (1.25 g) in THF (20 mL) at -78°C, followed by stirring at 0°C for 4 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=4:1). The obtained compound was dissolved in DMSO (40 mL), and triethylamine (11.2 mL) and a sulfur trioxide-pyridine complex (6.40 g) was added thereto, followed by stirring at room temperature for 1 hour. To the reaction liquid was added water, followed by extraction three times with a mixed solvent of hexane and ethyl acetate (ethyl acetate:hexane=1:4), and the combined extracted layer was washed with saturated brine and then dried over anhydrous sodium sulfate. The residue obtained by evaporating the solvent under reduced pressure was purified by silica gel chromatography (hexane:ethyl acetate=9:1) to obtain the desired product (1.23 g) as a yellow oil.
¹H NMR (CDCl₃, 400 MHz): δ 1.49 (6H, s), 3.68 (3H, s), 3.85 (3H, s), 6.57 (1H, dd, J=13.4, 2.4 Hz), 6.76 (1H, dd, J=9.2, 2.4 Hz), 7.86 (1H, t, J=9.2 Hz).

### <Example 219>

### 5-(2-Fluoro-4-hydroxyphenyl)-4,4-dimethyl-2,4-dihydropyrazol-3-one

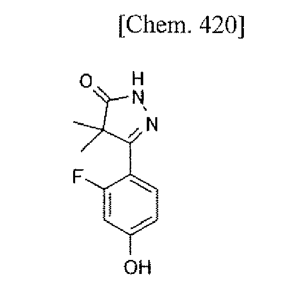

The compound of Example 218 (1.23 g) was dissolved in ethanol (25 mL), and acetic acid (2.43 mL) and hydrazine monohydrate (940 mL) were added thereto, followed by stirring for 8 hours under the condition of heating to reflux. The residue obtained by concentrating the reaction liquid under reduced pressure was purified by NH type silica gel chromatography (hexane:ethyl acetate4:1). The obtained powder was dissolved in dichloromethane (40 mL), and aluminum chloride (10.9 g) was added thereto, followed by stirring at room temperature for 10 hours. To the reaction liquid was added water, followed by extraction with THF, and the extracted layer was washed with saturated brine and then dried over anhydrous sodium sulfate. The residue obtained by evaporating the solvent under reduced pressure was washed with diethyl ether to obtain the desired product (295 mg) as a white powder.
¹H NMR (DMSO-d₆, 400 MHz): δ 1.19 (6H, s), 6.61 (1H, dd, J=13.1, 2.4 Hz), 6.66 (1H, dd, J=9.2, 2.4 Hz), 7.50 (1H, t, J=8.6 Hz), 10.32 (1H, s), 11.42 (1H, s).

### <Example 220>

### 2,5-Difluoro-4-methoxybenzyl alcohol

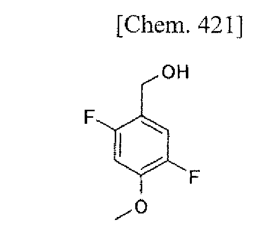

The compound of Example 205 (8.10 g) was dissolved in dichloromethane (100 mL), and trifluoroacetic acid (20 mL) was added thereto, followed by stirring at room temperature for 6 hours. The reaction liquid was concentrated under reduced pressure, and the residue was then dissolved in THF (250 mL) under an argon atmosphere, and a borane-tetrahydrofuran complex (1.0 mol/L THF solution, 42.6 mL) was added thereto at 0°C, followed by stirring at room temperature for 8 hours. To the reaction liquid was added ice water, followed by extraction with ethyl acetate, the extracted layer was washed with saturated brine and then dried over anhydrous sodium sulfate, and the solvent was evaporated under reduced pressure to obtain the desired product (5.74 g, 93%) as a white powder. ¹H NMR (CDCl₃, 400 MHz): δ 3.88 (3H, s), 4.67 (2H, s), 6.70 (1H, dd, J=11.0, 6.7 Hz), 7.15 (1H, dd, J=11.3, 6.7 Hz).

### <Example 221>

### 2,5-Difluoro-4-methoxybenzaldehyde

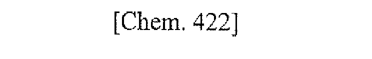

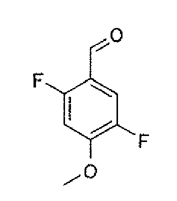

The compound of Example 220 (5.74 g) was dissolved in dichloromethane (300 mL), and active manganese dioxide (28.7 g) was added thereto, followed by stirring at 60°C for 3.5 hours. The insoluble materials were removed by filtration through Celite, and the solvent of the filtrate was evaporated under reduced pressure to obtain the desired product (5.48 g) as a yellow powder.
¹H NMR (CDCl₃, 400 MHz): δ 3.97 (3H, s), 6.74 (1H, dd, J=11.0, 6.7 Hz), 7.57 (1H, dd, J=111.0, 6.7 Hz), 10.21 (1H, d, J=3.1 Hz).

### <Example 222>

### Methyl 3-(2,5-difluoro-4-methoxyphenyl)-3-hydroxy-2,2-dimethyl propionate ester

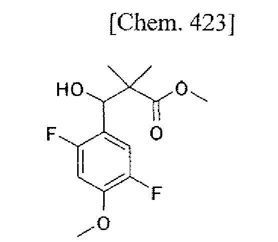

The compound of Example 221 (2.94 g) was dissolved in diethyl ether (150 mL) under an argon atmosphere, and dimethylketene methyltrimethylsilyl acetal (5.22 mL) and a boron trifluoride-diethyl ether complex (3.26 mL) were added thereto at room temperature, followed by shirring at room temperature for 1.5 hours. To the reaction liquid was added a 10% aqueous sodium hydroxide solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=6:1) to obtain the desired product (4.24 g) as a yellow powder.
¹H NMR (CDCl₃, 400 MHz): δ 1.14 (6H, s), 3.36 (1H, brs), 3.74 (3H, s), 3.87 (3H, s), 5.19 (1H, s), 6.64 (1H, dd, J=11.3, 7.0 Hz), 7.14 (1H, dd, J=12.2, 7.0 Hz).

### <Example 223>

### 5-(2,5-Difluoro-4-methoxyphenyl)-4,4-dimethyl-2,4-dihydropyrazol-3-one

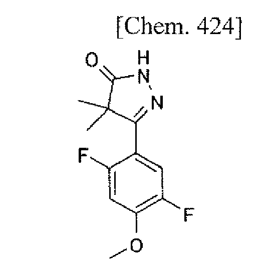

The compound of Example 222 (4.24 g) was dissolved in DMSO (100 mL) under an argon atmosphere, and triethylamine (22.9 mL) and a sulfur trioxide-pyridine complex (13.1 g) was added thereto, followed by stirring at room temperature for 1 hour. To the reaction liquid was added 1 mol/L hydrochloric acid, followed by extraction three times with a mixed solvent of hexane and ethyl acetate (ethyl acetate:hexane=1:4), and the combined organic layer was washed with saturated brine and then dried over anhydrous sodium sulfate. After evaporating the solvent under reduced pressure, the residue was purified by silica gel chromatography (hexane:ethyl acetate=7:1) to obtain a yellow oil. The obtained oil was dissolved in ethanol (100 mL), and acetic acid (10.3 mL) and hydrazine monohydrate (0.399 mL) were added thereto, followed by stirring for 8 hours under the condition of heating to reflux. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=3:1→2:1) to obtain the desired product (1.01 g) as a white powder.
¹H NMR (CDCl₃, 400 MHz): δ 1.42 (6H, s), 3.93 (3H, s), 6.74 (1H, dd, J=12.2, 7.3 Hz), 7.51 (1H, dd, J=11.6, 6.7 Hz), 8.66 (1H, s).

### <Example 224>

### 5-(2,5-Difluoro-4-hydroxyphenyl)-4,4-dimethyl-2,4-dihydropyrazol-3-one

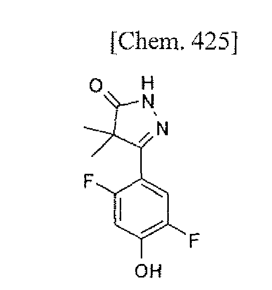

The compound of Example 223 (1.01 g) was dissolved in dichloromethane (40 mL) under an argon atmosphere, and aluminum chloride (11.9 g) was added thereto, followed by stirring at room temperature for 7 hours. To the reaction liquid was added ice water, followed by extraction with THF, and the extracted layer was washed with saturated brine and then dried over anhydrous sodium sulfate. After evaporating the solvent under reduced pressure, the residue was dissolved in dichloromethane (40 mL) again, and aluminum chloride (11.9 g) was added thereto, followed by stirring at room temperature for 22 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (430 mg) as a white powder.
¹H NMR (DMSO-d₆, 400 MHz): δ 1.21 (3H, s), 1.21 (3H, s), 6.84 (1H, dd, J=12.2, 7.3 Hz), 7.46 (1H, dd, J=12.2, 7.3 Hz), 10.92 (1H, brs), 11.54 (1H, s).

### <Example 225>

### 2,3-Difluoro-4-triisopropylsilyloxybenzaldehyde

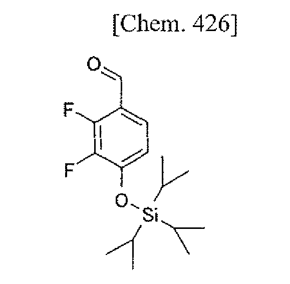

The compound of Example 211 (3.31 g) was dissolved in THF (75 mL) under an argon atmosphere, and a borane-tetrahydrofuran complex (1.0 mol/L THF solution, 12.0 mL) was added thereto at 0°C, followed by stirring at room temperature for 24 hours. To the reaction liquid was added ice water, followed by extraction with ethyl acetate, and the extracted layer was washed with saturated brine and then dried over anhydrous sodium sulfate. After evaporating the solvent under reduced pressure, the residue was dissolved in chloroform (75 mL), and active manganese dioxide (8.70 g) was added thereto, followed by stirring at 60°C for 6 hours. The insoluble materials were removed by filtration through Celite, and the solvent of the filtrate was evaporated under reduced pressure to obtain the desired product (3.09 g) as a yellow oil.
¹H NMR (CDCl₃, 400 MHz): δ 1.11 (18H, d, J=7.3 Hz), 1.29-1.32 (3H, m), 6.80 (1H, ddd, J=8.6, 7.3, 1.8 Hz), 7.53 (1H, ddd, J=8.6, 7.3, 2.4 Hz), 10.19 (1H, s).

### <Example 226>

### 2,3-Difluoro-4-methoxybenzaldehyde

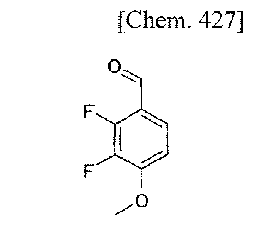

The compound of Example 225 (3.09 g) was dissolved in THF (50 mL) under an argon atmosphere, and tetrabutyl ammonium fluoride (1.0 mol/L THF solution, 12.8 mL) was added thereto, followed by stirring at room temperature for 20 minutes. To the reaction liquid was added 1.0 mol/L hydrochloric acid, followed by extraction with ethyl acetate, and the extracted layer was washed with saturated brine and then dried over anhydrous sodium sulfate. After evaporating the solvent under reduced pressure, the residue was dissolved in acetone (50 mL) and DMF (20 mL), and potassium carbonate (2.72 g) and iodomethane (0.918 mL) were added thereto, followed by stirring at room temperature for 5.5 hours. The insoluble materials were removed by filtration through Celite, the filtrate was concentrated under reduced pressure, and the obtained residue was purified by silica gel chromatography (hexane:ethyl acetate=3:1) to obtain the desired product (1.30 g) as a white powder.
¹H NMR (CDCl₃, 400 MHz): δ 4.00 (3H, s), 6.86 (1H, ddd, J=9.2, 6.7, 1.8 Hz), 7.64 (1H, ddd, J=9.2, 7.3, 1.8 Hz), 10.20 (1H, s).

### <Example 227>

### Methyl 3-(2,3-difluoro-4-methoxyphenyl)-3-hydroxy-2,2-dimethyl propionate ester

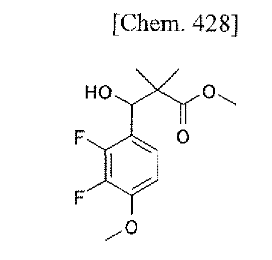

The compound of Example 226 (1.30 g) was dissolved in diethyl ether (70 mL) under an argon atmosphere, and dimethylketene methyltrimethylsilyl acetal (2.30 mL) and a boron trifluoride-diethyl ether complex (1.44 mL) were added thereto at room temperature, followed by stirring at room temperature for 1 hour. To the reaction liquid was added a 10% aqueous sodium hydroxide solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=3:1) to obtain the desired product (1.98 g) as a white powder.
¹H NMR (CDCl₃, 400 MHz): δ 1.15 (3H, s), 1.16 (3H, d, J=2.4 Hz), 3.37 (1H, d, J=4.9 Hz), 3.75 (3H, s), 3.91 (3H, s), 5.20 (1H, d, J=4.9 Hz), 6.79 (1H, ddd, J=8.6, 7.9, 2.4 Hz), 7.13 (1H, ddd, J=8.6, 7.3, 2.4 Hz).

### <Example 228>

### Methyl 3-(2,3-difluoro-4-methoxyphenyl)-2,2-dimethyl-3-oxopropionate ester

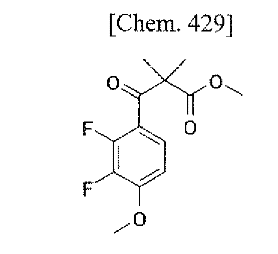

The compound of Example 227 (1.98 g) was dissolved in DMSO (50 mL) under an argon atmosphere, and triethylamine (10.1 mL) and a sulfur trioxide-pyridine complex (5.75 g) were added thereto, followed by stirring at room temperature for 1 hour. To the reaction liquid was added 1 mol/L hydrochloric acid, followed by extraction three times with a mixed solvent of hexane and ethyl acetate (ethyl acetate:hexane=1:4), and the combined organic layer was washed with saturated brine and then dried over anhydrous sodium sulfate. After evaporating the solvent under reduced pressure, the residue was purified by silica gel chromatography (hexane:ethyl acetate=6:1) to obtain the desired product (1.24 g) as a yellow oil.
¹H NMR (CDCl₃, 400 MHz): δ 1.50 (6H, s), 3.70 (3H, s), 3.96 (3H, s), 6.82 (1H, ddd, J=9.2, 7.3, 1.8 Hz), 7.63 (1H, ddd, J=9.2, 7.3, 2.4 Hz).

### <Example 229>

### 5-(2,3-Difluoro-4-methoxyphenyl)-4,4-dimethyl-2,4-dihydropyrazol-3-one

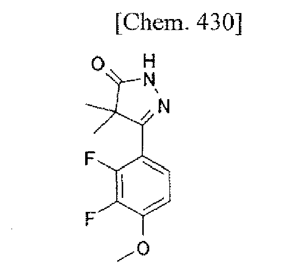

The compound of Example 228 (1.12 g) was dissolved in ethanol (20 mL), and acetic acid (1.55 mL) and hydrazine monohydrate (0.60 mL) were added thereto, followed by stirring for 10 hours under the condition of heating to reflux. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=2:1) to obtain the desired product (425 mg) as a white powder.
¹H NMR (CDCl₃, 400 MHz): δ 1.42 (3H, s), 1.43 (3H, s), 3.97 (3H, s), 6.82 (1H, ddd, J=9.2, 7.3, 1.8 Hz), 7.40 (1H, ddd, J=9.2, 7.3, 2.4 hz), 8.64 (1H, s).

### <Example 230>

### 5-(2,3-Difluoro-4-hydroxyphenyl)-4,4-dimethyl-2,4-dihydropyrazol-3-one

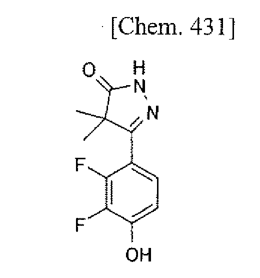

The compound of Example 229 (425 mg) was dissolved in dichloromethane (15 mL) under an argon atmosphere, and aluminum chloride (4.45 g) was added thereto, followed by stirring at room temperature for 15 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (210 mg) as a white powder.
¹H NMR (DMSO-d₆, 400 MHz): δ 1.21 (6H, s), 6.83-6.84 (1H, m), 7.31 (1H, ddd, J=9.2, 8.6, 1.8 Hz), 10.87 (1H, s), 11.53 (1H, s).

### <Example 231>

### Methyl 3-(4-fluoro-3-methoxyphenyl)-3-hydroxy-2,2-dimethyl propionate ester

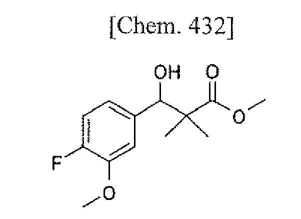

Diisopropyl amine (2.96 mL) was dissolved in THF (40 mL) under an argon atmosphere, and n-butyl lithium (2.71 mol/L hexane solution, 7.79 mL) was added thereto at -78°C, followed by stirring at 0°C for 30 minutes, and then methyl isobutyrate ester (2.33 mL) was added thereto at -78°C, followed by stirring at room temperature for 1 hour. To the reaction liquid was added a solution of 4-fluoro-3-methoxybenzaldehyde (2.50 g) in THF (40 mL) at -78°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel column chromatography (hexane:ethyl acetate=4:1) to obtain the desired product (4.26 g) as a colorless oil.
¹H NMR (CDCl₃, 400 MHz): δ 1.11 (3H, s), 1.15 (3H, s), 3.15 (1H, d, J=3.1 Hz), 3.73 (3H, s), 3.88 (3H, s), 4.86 (1H, d, J=3.1 Hz), 6.78-6.81 (1H, m), 6.95-7.03 (2H, m).

### <Example 232>

### Methyl 3-(4-fluoro-3-methoxyphenyl)-2,2-dimethyl-3-oxopropionate ester

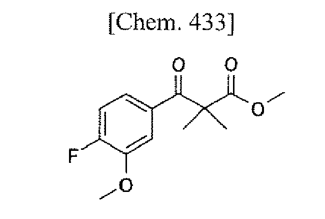

The compound of Example 231 (4.26 g) was dissolved in DMSO (80 mL) under an argon atmosphere, and triethylamine (23.2 mL) and a sulfur trioxide-pyridine complex (13.2 g) were added thereto, followed by stirring at room temperature for 1 hour. To the reaction liquid was added water, followed by extraction three times with a mixed solvent of hexane and ethyl acetate (hexane:ethyl acetate=4:1), and the combined organic layer was washed with saturated brine and then dried over anhydrous sodium sulfate. After evaporating the solvent under reduced pressure, the residue was purified by silica gel column chromatography (hexane:ethyl acetate=30:1→4:1) to obtain the desired product (2.50 g) as a colorless oil. ¹H NMR (CDCl₃, 400 MHz): δ 1.55 (6H, s), 3.65 (3H, s), 3.92 (3H, s), 7.08 (1H, dd, J=10.4, 8.6 Hz), 7.33-7.37 (1H, m), 7.57 (1H, dd, J=2.4, 8.6 Hz).

### <Example 233>

### 5-(4-fluoro-3-methoxyphenyl)-4,4-dimethyl-2,4-dihydropyrazol-3-one

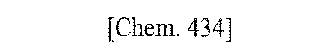

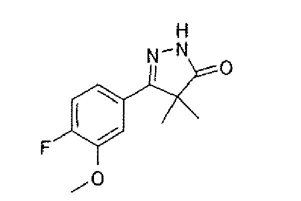

The compound of Example 232 (2.50 g) was dissolved in ethanol (50 mL), and hydrazine monohydrate (1.28 mL) was added thereto, followed by stirring for 5 hours under the condition of heating to reflux. The solvent of the reaction liquid was evaporated under reduced pressure to obtain the desired product (2.23 g) as a white powder.
¹H NMR (CDCl₃, 400 MHz): δ 1.52 (6H, s), 3.94 (3H, s), 7.11 (1H, dd, J=11.0, 8.6 Hz), 7.21-7.25 (1H, m), 7.52 (1H, dd, J=8.6, 2.4 Hz), 8.95 (1H, brs).

### <Example 234>

### 5-(4-Fluoro-3-hydroxyphenyl)-4,4-dimethyl-2,4-dihydropyrarol-3-one

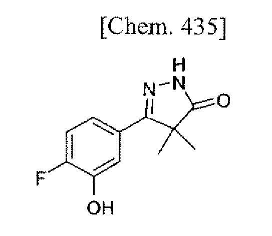

The compound of Example 233 (2.23 g) was dissolved in dichloromethane (50 mL) under an argon atmosphere, and boron tribromide (1.0 mol/L dichloromethane solution, 14.2 mL) was added thereto at 0°C, followed by stirring at 0°C for 1 hour and then stirring at room temperature for 4 hours. To the reaction liquid was added water, followed by extraction three times with a mixed solvent of ethyl acetate and THF (ethyl acetate:THF=1:1), and the combined organic layer was washed with saturated brine and then dried over anhydrous sodium sulfate. After evaporating the solvent under reduced pressure, the obtained residue was washed with diethyl ether to obtain the desired product (1.91 g) as a yellow powder.
¹H NMR (DMSO-d₆, 400 MHz): δ 132 (6H, s), 7.12-7.21 (2H, m), 7.43 (1H, dd, J=8.9, 2.1 Hz), 10.09 (1H, s), 11.48 (1H, s).

### <Example 235>

### 2-Fluoro-3-methoxybenzaldehyde

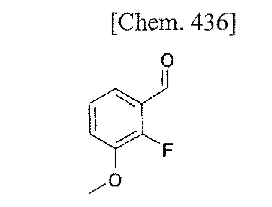

2-Fluoroanisole (2.24 mL) was dissolved in THF (25 mL) under an argon atmosphere, and an n-butyl lithium (2.71 mol/L hexane solution, 7.38 mL) and N,N,N', N", N"-pentamethyldiethylene triamine (4.20 mL) were added thereto at -78°C, followed by stirring at -78C for 2 hours. Then, DMF (2.01 mL) was added thereto at -78°C, followed by stirring at room temperature for 1.5 hours. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel column chromatography (hexane:ethyl acetate=15:1) to obtain the desired product (2.36 g) as a yellow powder.
¹H NMR (CDCl₃, 400 MHz): δ 3.94 (3H, s), 7.16-7.44 (3H, m), 10.39 (1H, s).

### <Example 236>

### Methyl 3-(2-fluoro-3-methoxyphenyl)-3-hydroxy-2,2-dimethyl propionate ester

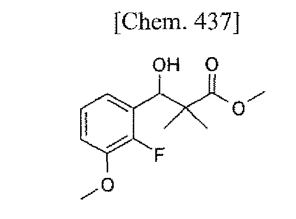

Diisopropyl amine (2.48 mL) was dissolved in THF (40 mL) under an argon atmosphere, and n-butyl lithium (2.71 mol/L hexane solution, 6.52 mL) was added thereto at -78°C, followed by stirring at 0°C for 30 minutes, and then methyl isobutyrate ester (1.87 mL) was added thereto at -78°C, followed by stirring at room temperature for 1 hour. To the reaction liquid was added a solution of the compound of Example 235 (2.50 g) in THF (40 mL) at -78°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel column chromatography (hexane:ethyl acetate=10:1→5:1) to obtain the desired product (1.73 g) as a yellow oil.
¹H NMR (CDCl₃, 400 MHz): δ 1.16 (3H, s), 1.17 (3H, d, J=2.4 Hz), 3.33 (1H, d, J=3.7 Hz), 3.74 (3H, s), 3.88 (3H, s), 5.30 (1H, d, J=3.7 Hz), 6.89 (1H, td, J=7.9, 1.8 Hz), 7.00-7.07 (2H, m).

### <Example 237>

### Methyl 3-(2-fluoro-3-methoxyphenyl)-2,2-dimethyl-3-oxopropionate ester

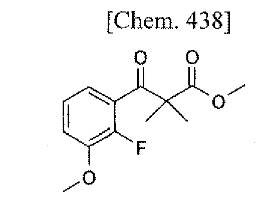

The compound of Example 236 (1.73 g) was dissolved in DMSO (35 mL) under an argon atmosphere, and triethylamine (9.43 mL) and a sulfur trioxide-pyridine complex (5.40 g) were added thereto, followed by stirring at room temperature for 1 hour. To the reaction liquid was added water, followed by extraction three times with a mixed solvent of hexane and ethyl acetate (hexane:ethyl acetate=4:1), and the combined organic layer was washed with saturated brine and then dried over anhydrous sodium sulfate. After evaporating the solvent under reduced pressure, the residue was purified by silica gel column chromatography (hexane:ethyl acetate=1:1) to obtain the desired product (970 mg) as a yellow oil. ¹H NMR (CDCl₃, 400 MHz): δ 1.50 (6H, s), 3.71 (3H, s), 3.90 (3H, s), 7.08-7.15 (2H, m), 7.19-7.23 (1H, m).

### <Example 238>

### 5-(2-Fluoro-3-hydroxyphenyl)-4,4-dirmethyl-2,4-dihydropyrazol-3-one

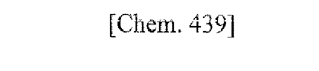

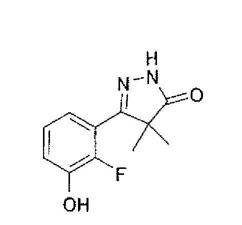

The compound of Example 237 (970 mg) was dissolved in ethanol (30 mL), and hydrazine monohydrate (0.552 mL) was added thereto, followed by stirring for 11 hours under the condition of heating to reflux. After evaporating the solvent of the reaction liquid under reduced pressure, the residue was dissolved in dichloromethane (30 mL), and boron tribromide (1.0 mol/L dichloromethane solution, 8.40 mL) was added thereto at 0°C under an argon atmosphere, followed by stirring at room temperature for 1 hour. To the reaction liquid was added water, followed by extraction with THF, and the extracted layer was washed with saturated brine and then dried over anhydrous sodium sulfate. After evaporating the solvent under reduced pressure, the obtained residue was washed with diethyl ether to obtain the desired product (601 mg) as a yellow powder.
¹H NMR (DMSO-d₆, 400 MHz): δ 1.24 (6H, s), 7.01-7.09 (3H, m), 10.09 (1H, s), 11.61 (1H, s).

### <Example 239>

### (2-Bromoethoxy)-t-butyldiphenylsilane

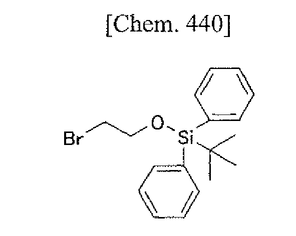

2-Bromoethanol (3.00 mL) was dissolved in DMF (40.0 mL) under an argon gas atmosphere, and imidazole (4.32 g) and tert-butyl diphenylsilyl chloride (11.6 mL) were added thereto under ice cooling, followed by stirring at room temperature for 1.5 hours. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate=50:1) to obtain the desired product (15.1 g) as a colorless oil.
¹H NMR (CDCl₃, 400 MHz): δ 1.07 (9H, s), 3.42 (2H, t, J=6.1 Hz), 3.92 (2H, t, J=6.1 Hz), 7.39-7.44 (6H, m), 7.66-7.68 (4H, m).

### <Example 240>

### 6-4-[2-(t-Butyldiphenylsilyloxy)ethoxy]phenyl]-5-methyl-4,5-dihydro-2H-pyridazin-3-one

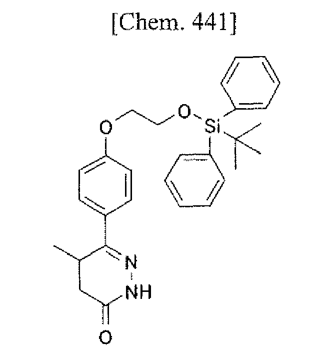

The compound of Example 167 (3.00 g) was dissolved in DMF (50.0 mL) under an argon gas atmosphere, and the compound of Example 239 (5.87 g) and potassium carbonate (4.06 g) were added thereto, followed by stirring at room temperature for 1.5 hours and at 60°C for 6.5 hours. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate= 2:1) to obtain the desired product (5.73 g) as a colorless powder.
¹H NMR (CDCl₃, 400 MHz): δ 1.69 (9H, s), 1.25 (3H, d, J=7.3 Hz), 2.46 (1H, d, J=17.1 Hz), 2.71 (1H, dd, J=17.1, 7.3 Hz), 3.31-3.35 (1H, m), 4.01 (2H, t, J=5.5 Hz), 4.12 (2H, t, J=5.5 Hz), 6.89-6.91 (2H, m), 7.37-7.44 (6H, m), 7.66-7.72 (6H, m), 8.48 (1H, brs).

### <Example 241>

### 2-t-Butoxycarbonyl-6-[4-[2-(t-butyldiphenylsilyloxy)ethoxy]phenyl]-5-methyl-4,5-dihydro-2H-pyridazin-3-one

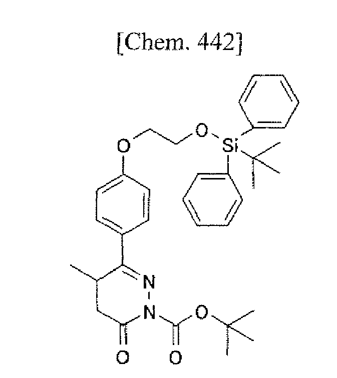

The compound of Example 240 (5.73 g) was dissolved in acetonitrile (100 mL), and di-tert-butyl-di-carbonate (3.08 g) and a catalytic amount of 4-dimethyl aminopyridine were added thereto, followed by stirring at room temperature for 2 hours under an argon atmosphere. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate= 4:1) to obtain the desired product (6.90 g) as a colorless amorphous.
¹H NMR (CDCl₃, 400 MHz): δ 1.07 (9H, s), 1.25 (3H, d, J=6.7 Hz), 1.66 (9H, s), 2.57 (1H, dd, J=16.5, 1.2 Hz), 2.78 (1H, dd, J=16.5, 6.7 Hz), 3.35-3.38 (1H, m), 4.01 (2H, t, J=5.5 Hz), 4.12 (2H, t, J=5.5 Hz), 6.89-6.91 (2H, m), 7.37-7.44 (6H, m), 7.69-7.72 (4H, m), 7.75-7.78 (2H, m).

### <Example 242>

### 2-t-Butoxycarbonyl-6-[4-(2-hydroxyethoxy)phenyl]-5-methyl-4,5-dihydro-2H-pyridazin-3-one

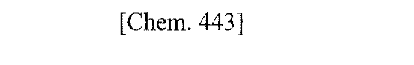

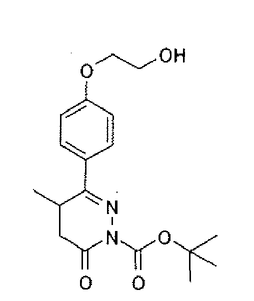

The compound of Example 241 (6.90 g) was dissolved in THF (60.0 mL) under an argon gas atmosphere, and tetrabutyl ammonium fluoride (1.00 mol/L THF solution, 14.0 mL) was added thereto under ice cooling, followed by stirring at room temperature for 4 hours. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate=1:2) to obtain the desired product (3.33 g) as a colorless powder.
¹H NMR (CDCl₃, 400 MHz): δ 1.25 (3H, d, J=7.3 Hz), 1.62 (9H, s), 2.57 (1H, dd, J=16.5, 1.8 Hz), 2.78 (1H, dd, J=16.5, 6.7 Hz), 3.33-3.40 (1H, m), 3.98-4.01 (2H, m), 4.11-4.15 (2H, m), 6.95-697 (2H, m), 7.78-7.81 (2H, m).

### <Example 243>

### 2-t-Butoxycarbonyl-6-[4-(2-iodoethoxy)phenyl]-5-methyl-4,5-dihydro-2H-pyridazin-3-one

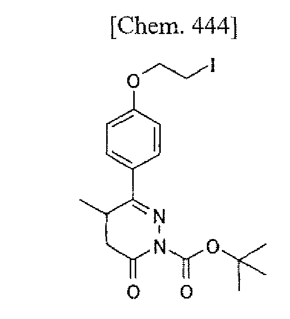

The compound of Example 242 (500 mg) was dissolved in THF (10.0 mL), and imidazole (147 mg), triphenyl phosphine (567 mg), and iodine (402 mg) were added thereto, followed by stirring at room temperature for 1 hour. To the reaction liquid was added a saturated aqueous sodium thiosulfate solution, followed by extraction with ethyl acetate, and the extracted layer was washed with water and saturated brine in this order, and then dried over anhydrous sodium sulfate. After evaporating the solvent under reduced pressure, the residue was purified by silica gel column chromatography (hexane:ethyl acetate= 2:1) to obtain the desired product (645 mg) as a colorless powder.
¹H NMR (CDCl₃, 400 MHz): δ 1.25 (3H, d, J=7.3 Hz), 1.62 (9H, s), 2.57 (1H, dd, J=16.5, 1.8 Hz), 2.78 (1H, dd, J=16.5, 6.7 Hz), 3.35-3.39 (1H, m), 3.44 (2H, t, J=6.1 Hz), 4.30 (2H, t, J=6.1 Hz), 6.92-6.95 (2H, m), 7.78-7.81 (2, m).

### <Example 244>

### 3-Methoxy-4-methoxymethyloxybenzaldehyde

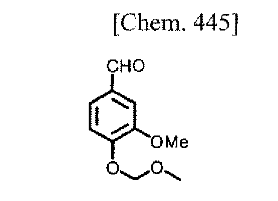

Vaniline (7.00 g) was dissolved in dichloromethane (200 mL) under an argon atmosphere, and diisopropyl ethylamine (16.0 mL) and chloromethylmethylether (4.54 mL) were added thereto at 0°C, followed by stirring at room temperature for 4 hours. To the reaction liquid was added water, followed by extraction three times with ethyl acetate, and the combined organic layer was washed with saturated brine and then dried over sodium sulfate. After evaporating the solvent under reduced pressure, the obtained residue was purified by silica gel chromatography (hexane:ethyl acetate=3:1) to obtain the desired product (9.45 g) as a colorless oil.
¹H NMR (CDCl₃, 400 MHz): δ 3.53 (3H, s), 3.96 (3H, s), 5.34 (2H, s), 7.28 (1H, d, J=8.6 Hz), 7.43-7.44 (2H, m), 9.88 (1H, s).

### <Example 245>

### Methyl 3-(3-Methoxy-4-methoxymethyloxyphenyl)-3-hydroxy-2,2-dimethyl propionate ester

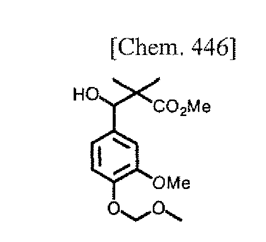

The compound of Example 244 (2.00 g) was dissolved in diethyl ether (100 mL) under an argon atmosphere, and dimethylketene methyltrimethylsilyl acetal (3.11 mL) and a boron trifluoride diethyl ether complex (1.94 mL) were added thereto, followed by stirring at room temperature for 1 hour. To the reaction liquid was added a saturated aqueous sodium hydrogen carbonate solution, followed by extraction three times with ethyl acetate, and the combined organic layer was washed with saturated brine and then dried over sodium sulfate. After evaporating the solvent under reduced pressure, the obtained residue was purified by silica gel chromatography (hexane:ethyl acetate=2:1) to obtain the desired product (3.00 g) as a colorless oil.
¹H NMR (CDCl₃, 400 MHz): δ 1.12 (3H, s), 1.16 (3H, s), 3.02 (1H, d, J=4.3 Hz), 3.52 (3H, s), 3.73 (3H, s), 3.88 (3H, s), 4.85 (1H, d, J=4.3 Hz), 5.22 (2H, s), 6.80 (1H, dd, J=8.6, 1.8 Hz), 6.90 (1H, d, J=1.8 Hz), 7.09 (1H, d, J=8.6 Hz).

### <Example 246>

### Methyl 3-(3-Methoxy-4-methoxymethyloxyphenyl)-2,2-dimethyl-3-oxopropionate ester

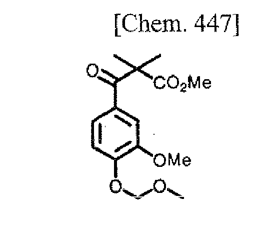

The compound of Example 245 (3.00 g) was dissolved in DMSO (70 mL) under an argon atmosphere, and triethylamine (14.1 mL) and a sulfur trioxide-pyridine complex (8.04 g) were added thereto, followed by stirring at room temperature for 1 hour. To the reaction liquid was added 1 mol/L hydrochloric acid, followed by extraction three times with hexaneethyl acetate (4-1), and the combined organic layer was washed with saturated brine and then dried over anhydrous sodium sulfate. After evaporating the solvent under reduced pressure, the obtained residue was purified by silica gel chromatography (hexane:ethyl acetate=3:1) to obtain the desired product (2.41 g) as a colorless oil.
¹H NMR (CDCl₃, 400 MHz): δ 1.54 (6H, s), 3.51 (3H, s), 3.65 (3H, s), 3.92 (3H, s), 5.29 (2H, s), 7.13 (1H, d, J=8.6 Hz), 7.37 (1H, dd, J=8.6, 2.4 Hz), 7.52 (1H, d, J=2.4 Hz).

### <Example 247>

### 5-(3-Methoxy-4-methoxymethyloxyphenyl)-4,4-dimethyl-2,4-dihydropyrazol-3-one

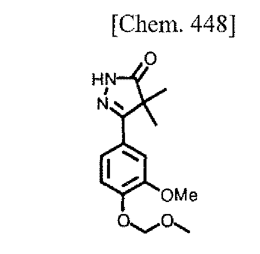

The compound of Example 246 (2.41 g) was dissolved in ethanol (50 mL), and hydrazine monohydrate (1.18 mL) was added thereto, followed by stirring for 10 hours to heating under reflux. To the reaction liquid was added water, followed by extraction three times with ethyl acetate, and the combined extracted layer was washed with saturated brine and then dried over anhydrous sodium sulfate. The solvent was evaporated under reduced pressure to obtain the desired product (2.17 g) as a colorless powder.
¹H NMR (CDCl₃, 400 MHz): δ 1.52 (6H, s), 3.53 (3H, s), 3.94 (3H, s), 5.28 (2H, s), 7.17 (1H, d, J=8.6 Hz), 7.23 (1H, dd, J=8.6, 1.8 Hz), 7.47 (1H, d, J=1.8 Hz), 8.62 (1H, s).

### <Example 248>

### 5-(3-Methoxy-4-hydroxyphenyl)-4,4-dimethyl-2,4-dihydropyrazol-3-one

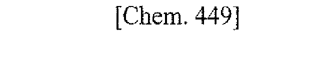

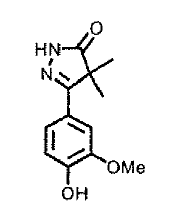

The compound of Example 247 (2.17 g) was dissolved in methanol (30 mL) and THF (30 mL), and 3 mol/L hydrochloric acid was added thereto, followed by stirring for 4 hours to heating under reflux. To the reaction liquid was added water, followed by extraction three times with ethyl acetate, and the combined extracted layer was washed with saturated brine and then dried over anhydrous sodium sulfate. After evaporating the solvent under reduced pressure, the obtained residue was washed with diisopropyl ether to obtain the desired product (1.60 g) as a colorless powder.
¹H NMR (DMSO-d₆, 400 MHz): δ 1.32 (6H, s), 3.78 (3H, s), 6.80 (1H, d, J=7.9 Hz), 7.19 (1H, dd, J=7.9, 1.8 Hz), 7.31 (1H, d, J=1.8 Hz), 9.50 (1H, s), 11.32 (1H, s).

### <Example 249>

### 2,3-Difluoro-4-methoxy-1 1-propionylbenzene

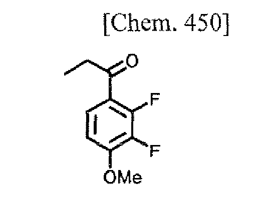

Aluminum chloride (9.25 g) was dissolved in nitromethane (100 mL) under an argon atmosphere, and propionic acid chloride (6.06 mL) and 2,3-difluoroanisole (4.0 g) that had been dissolved in nitromethane (30 mL) was added thereto, followed by stirring at room temperature for 18 hours. It was added with ice water, extracted three times with ethyl acetate, washed with saturated brine, dried over anhydrous sodium sulfate, and then passed through silica gel. The solvent was evaporated under reduced pressure to obtain the desired product (5.68 g) as a white powder.
¹H NMR (CDCl₃, 400 MHz): δ 1.20 (3H, t, J=7.6 Hz), 2.95-2.99 (2H, m), 3.96 (3H, s), 6.81 (1H, ddd, J=6.7, 6.7, 1.8 Hz), 7.69 (1H, ddd, J=7.9, 7.9, 2.4 Hz).

### <Example 250>

### 6-(2,3-Difluoro-4-methoxyphenyl)-5-methyl-4,5-dihydro-2H-pyridazin-3-one

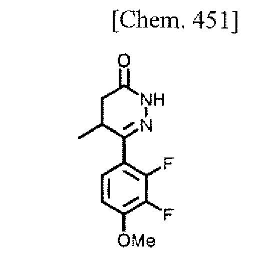

The compound of Example 249 (5.68 g) was dissolved in THF (200 mL) under an argon atmosphere, and a solution (1.0 mol/L, 31.2 mL) of lithium hexamethyl disilazane in THF was added thereto at 0°C, followed by stirring at 0°C for 30 minutes, and then t-butyl bromoacetate (5.41 mL) was added thereto, followed by stirring at room temperature for 1.5 hours. A saturated aqueous ammonium chloride solution was added thereto, followed by evaporation of tetrahydrofuran under reduced pressure. Then, the residue was extracted three times with ethyl acetate, washed with saturated brine, dried over anhydrous sodium sulfate, and then filtered. The solvent of the filtrate was evaporated under reduced pressure, and the obtained residue was dissolved in dichloromethane (50 mL), and trifluoroacetic acid (20 mL) was added thereto, followed by leaving to stand at room temperature for 16 hours. After evaporating the solvent under reduced pressure, the obtained residue was dissolved in ethanol (250 mL), and acetic acid (18.0 mL, 315 mmol) and hydrazine monohydrate (6.92 mL) were added thereto, followed by stirring for 7 hours under the condition of heating to reflux. After evaporating ethanol under reduced pressure, it was added with water to precipitate the crystal, collected by filtration, and then washed with diisopropyl ether to obtain the desired product (4.05 g) as a white powder.
¹H NMR (CDCl₃, 400 MHz): δ 1.22 (3H, d, J=7.3 Hz), 2.46 (1H, dd, J=17.1, 3.1 Hz), 2.75 (1H, dd, J=17.1, 6.7 Hz), 3.27-3.29 (1H, m), 3.95 (3H, s), 6.79 (1H, ddd, J=8.6, 7.3, 1.8 Hz), 7.34 (1H, ddd, J=8.6, 7.9, 2.4 Hz), 8.51 (1H, s).

### <Example 251>

### 6-(2,3-Difluoro-4-hydroxyphenyl)-5-methyl-4,5-dihydro-2H-pyridazin-3-one

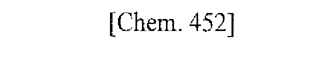

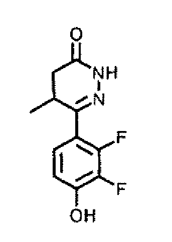

The compound of Example 250 (4.05 g) was dissolved in dichloromethane (170 mL) under an argon atmosphere, and aluminum chloride (45.9 g) was added thereto, followed by stirring at room temperature for 15 hours. Ice water was added thereto, followed by extraction three times with tetrahydrofuran, and the combined organic layer was washed with saturated brine and then dried over anhydrous sodium sulfate. After evaporating the solvent of the filtrate under reduced pressure, diisopropyl ether was added thereto, and the precipitated crystal was collected by filtration to obtain the desired product (3.42 g) as a yellow powder.
¹H NMR (CDCl₃, 400 MHz): δ 1.02 (3H, d, J=7.3 Hz), 2.20 (1H, dd, J=17.1, 3.1 Hz), 2.65 (1H, dd, J=17.1, 6.7 Hz), 3.10-3.12 (1H, m), 6.80-6.81 (1H, m), 7.23-7.24 (1H, m), 10.75 (1H, s), 10.95 (1H, s).

### <Example 252>

### t-Butyl 3-[2,3-difluoro-4-(2-iodoethoxy)-phenyl]-4-methyl-6-oxo-5,6-dihydro-4H-pyridazine-1-carboxylate ester

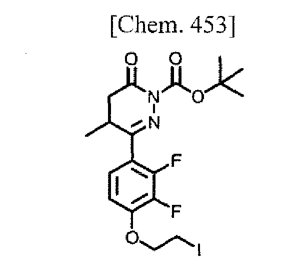

The reaction was carried out in the same manner as in Examples 240 to 243 using the compound of Example 251 to obtain the desired product as a colorless powder.
¹H NMR (CDCl₃, 400 MHz): δ 1.22 (3H, d, J=7.3 Hz), 2.54 (1H, dd, J=16.2, 3.4 Hz), 2.82 (1H, dd, J= 16.2, 6.1 Hz), 3.30-3.32 (1H, m), 3.45 (2H, t, J=7.0 Hz), 4.36 (2H, t, J=7.0 Hz), 6.76-6.80 (1H, m), 7.45-7.47 (1H, m).
ESIMS(+): 495 [M+H]⁺.

### <Examples 253 and 254>

### (+)-6-(4-Methoxyphenyl)-5-methyl-4,5-dihydro-2H-pyridazin-3-one

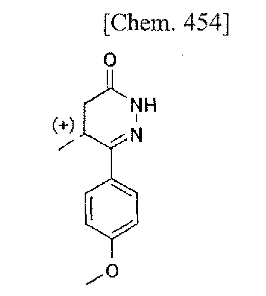

The compound of Example 166 was subjected to optical resolution by high performance liquid chromatography (Daicel CHIRALPAK AS-H column, Eluent: hexane/ethanol=40/60, Flow rate: 3.00 ml/min, Detection: 293 nm) to obtain a (+) form as a colorless powder from the previously eluted portion (Example 253).
Optical rotation: [α]_{D}²³ +449 (c0.53, DMSO).

### (-)-6-(4-Methoxyphenyl)-5-methyl-4,5-dihydro-2H-pyridazin-3-one

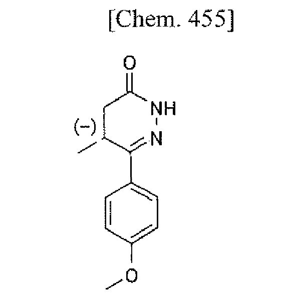

A (-) form was obtained as a colorless powder from the later eluted portion (Example 254).
Optical rotation: [α]_{D}²³ -467 (c 0.52, DMSO).

### <Example 255>

### (+)-6-(4-Hydroxyphenyl)-5-methyl-4,5-dihydro-2H-pyridazin-3-one

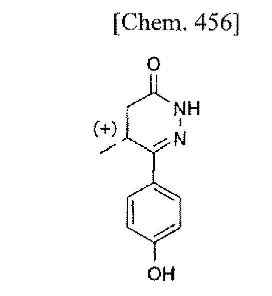

The compound of Example 253 (150 mg) was dissolved in dichloromethane (5.00 mL), and aluminum chloride (1.83 g) was added thereto under ice cooling, followed by stirring at room temperature for 22 hours. The reaction liquid was poured into ice water, followed by extraction with THF, and the extracted layer was dried over anhydrous magnesium sulfate. After evaporating the solvent under reduced pressure, the resulting powder was suspended in diisopropyl ether and collected by filtration to obtain the desired product (117 mg) as a white powder.
¹H NMR (DMSO-d₆, 400 MHz): δ 1.04 (3H, d, J=7.3 Hz), 2.18 (1H, d, J=15.9 Hz), 2.63 (1H, dd, J=1.8, 15.9 Hz), 3.28-3.33 (1H, m), 6.78-6.80 (2H, m), 7.59-7.63 (2H, m), 9.78 (1H, s), 10.8 (1H, s).
The optical purity was measured by means of HPLC.
Analysis condition: Column; Daicel CHIRALPAK (registered trademark) AS column (0.46cmϕ x 25cm), Developing solvent: hexane/ethanol=40/60, Flow rate: 0.5 ml/min., Detection: UV (293 nm).
Retention time: 12.1 minutes (98%ee).
Optical rotation: [α]_{D}²⁶ +378 (c 0.42, DMSO).

### <Example 256>

### (-)-6-(4-Hydroxyphenyl)-5-methyl-4,5-dihydro-2H-pyridazin-3-one

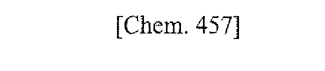

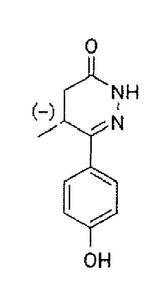

The reaction was carried out in the same manner as in Example 255 using the compound (150 mg) of Example 254 to obtain the desired product (112 mg) as a white powder.
¹H NMR (DMSO-d₆, 400 MHz): δ 1.04 (3H, d, J=7.3 Hz), 2.18 (H, d, J=15.9 Hz), 2.63 (1H, dd, J=1.8, 15.9 Hz), 3.28-3.33 (1H, m), 6.78-6.80 (2H, m), 7.59-7.63 (2H, m), 9.78 (1H, s), 10.8(1H,s).
The optical purity was measured by means of HPLC.
Analysis condition: column; Daicel CHIRALPAK (registered trademark) AS column (0.46cmϕ x 25cm), Developing solvent: hexane/ethanol=40/60, Flow rate: 0.5 ml/min., Detection: UV (293 nm).
Retention time: 21.5 minutes (>99%ee).
Optical rotation: [ϕ]_{D}²⁶ -395 (c 0.44, DMSO).

### <Example 257>

### 6-(4-Hydroxy-3-methoxyphenyl)-4,5-dihydro-2H-pyridazin-3-one

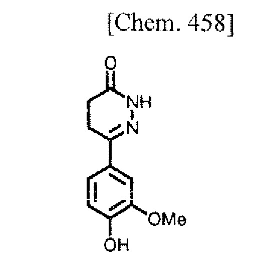

4-Acetyl-2-methoxy-1-methoxymethyloxybenzene (1.74 g) was dissolved in THF (40 mL) under an argon gas atmosphere, and lithium bistrimethylsilyl amide (1.00 mol/L THF solution, 9.93 mL) was added dropwise under ice cooling, followed by stirring at the same temperature for 30 minutes. Thereafter, tert-butyl bromoacetate (1.83 mL) was added thereto at the same temperature, followed by stirring at room temperature for 2 hours. To the reaction liquid was added a saturated aqueous ammonium chloride solution, followed by extraction with ethyl acetate. The extracted layer was washed with water and saturated brine in this order, and then dried over anhydrous sodium sulfate. The solvent was evaporated under reduced pressure, the obtained residue was dissolved in methylene chloride (10 mL), and trifluoroacetic acid (10.0 mL) was added thereto, followed by stirring at room temperature for 1 hour. The solvent was evaporated under reduced pressure, the obtained residue was dissolved in ethanol (60 mL), and hydrazine monohydrate (1.20 mL) was added thereto, followed by stirring for 4 hours to heating under reflux. After evaporating the solvent under reduced pressure, to the residue was added ice water, and the resulting solid was collected by filtration. The obtained solid was washed with water, cold ethanol, and diethyl ether in this order to obtain the desired product (470 mg) as a cream color powder. ¹H NMR (DMSO-d₆, 400 MHz): δ 2.36 (2H, dd, J=9.2, 7.9 Hz), 2.88 (2H, dd, J=9.2, 7.9 Hz), 3.78 (3H, s), 6.78 (1H, d, J=8.0 Hz), 7.14 (1H, dd, J=8.0, 1.8 Hz), 7.32 (1H, d, J=1.8 Hz), 9.37 (1H, s), 10.74 (1H, s).
EIMS (+): 220 [M]⁺.

### <Examples 258 and 259>

### Methyl (+)-4-(3-fluoro-4-methoxyphenyl)-3-methyl-4-oxobutanoate ester and

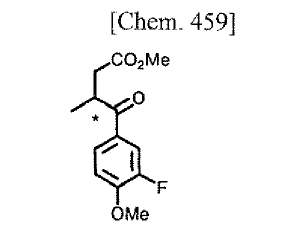

### (-)-4-(3-Fluoro-4-methoxyphenyl)-3-methyl-4-oxobutanoic acid

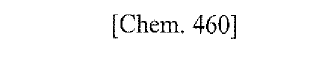

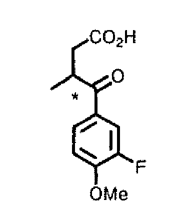

To a solution the compound of Example 192 (4.55 g) in acetone (45.0 mL) were added a phosphate buffer (pH 6.86, 135 mL) and lipase Amano PS(9.10 g), followed by stirring at room temperature for 62 hours. To the reaction liquid was added ethyl acetate (300 mL), followed by stirring, the insoluble materials were then removed by filtration through Celite, and the filtrate was extracted with ethyl acetate. The ethyl acetate layer was washed with a 3% aqueous sodium hydrogen carbonate solution (washing liquid A) and dried over anhydrous magnesium sulfate, and the solvent was evaporated under reduced pressure. The obtained residue was purified by silica gel column chromatography (hexane:ethyl acetate)=2:1) to obtain a pale yellow oily methyl 4-(3-fluoro-4-methoxyphenyl)-3-methyl-4-oxobutanoate ester (2.28 g) (Example 258).
Optical rotation: [α]_{D}²⁴ +16.0 (c 0.880, CHCl₃).
Further, the previous washing liquid A was adjusted to pH 2 with concentrated hydrochloric acid under ice cooling, followed by extraction with ethyl acetate, the extracted layer was dried over anhydrous magnesium sulfate, and the solvent was evaporated under reduced pressure to obtain a white solid 4-(3-fluoro-4-methoxyphenyl)-3-methyl-4-oxobutanic acid (1.67 g) (Example 259).
Optical rotation: [α]_{D}²⁴ -23.4 (c 0.530, CHCl₃).

### <Example 260>

### (+)-6-(3-Fluoro-4-methoxyphenyl)-5-methyl-4,5-dihydro-2H-pyridazin-3-one

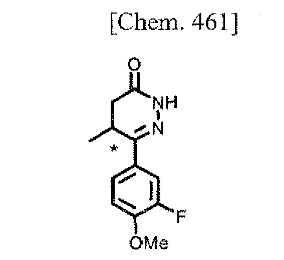

The reaction was carried out in the same manner as in Example 193 using the compound of Example 258 to obtain the desired product as a white solid. Optical rotation: [α]_{D}²⁴ +384.8 (c 0.510, CHCl₃).

### <Example 261>

### (-)-6-(3-Fluoro-4-methoxyphenyl)-5-methyl-4,5-dihydro-2H-pyridazin-3-one

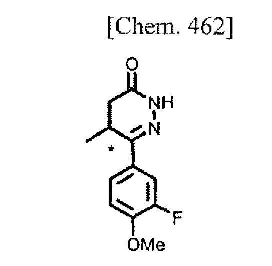

The reaction was carried out in the same manner as in Example 193 using the compound of Example 259 to obtain the desired product as a white solid. Optical rotation: [α]_{D}²⁴ -369.7 (c 0.690, CHCl₃).

### <Example 262>

### (+)-6-(3-Fluoro-4-hydroxyphenyl)-5-methyl-4,5-dihydro-2H-pyridazin-3-one

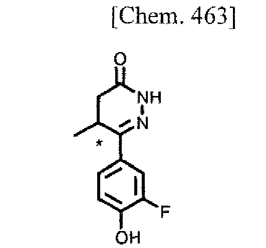

The reaction was carried out in the same manner as in Example 194 using the compound of Example 260 to obtain the desired product as a white solid. Optical rotation: [α]_{D}²⁴ +385.8 (c 0.0205, CHCl₃).

### <Example 263>

### (-)-6-(3-fluoro-4-hydroxyphenyl)-5-methyl-4,5-dihydro-2H-pyridazin-3-one

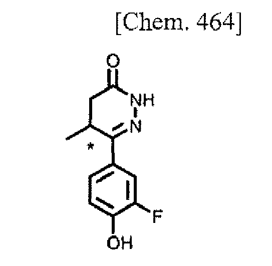

The reaction was carried out in the same manner as in Example 194 using the compound of Example 261 to obtain the desired product as a white solid.
Optical rotation: [α]_{D}²⁴ -385.1 (c 0.026, CHCl₃).

### <Example 264>

### 6-[4-(3-Bromopropoxy)-2,3-difluorophenyl]-5-methyl-4,5-dihydro-2H-pyridazin-3-one

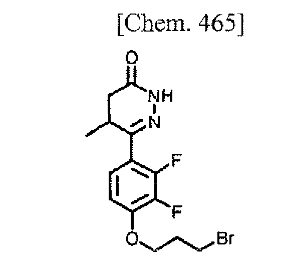

The compound of Example 251 (2.50 g) was dissolved in DMF (50 mL), and 1,3-dibromopropane (1.58 mL) and potassium carbonate (2.87 g) were added thereto, followed by stirring at 60°C for 50 minutes. To the reaction liquid was added water, followed by extraction three times with ethyl acetate. The obtained extracted layer was washed with saturated brine and dried over anhydrous sodium sulfate. The solvent was evaporated under reduced pressure, and the residue was then purified by silica gel column chromatography (hexane:ethyl acetate=1:3) to obtain the desired product (1.96 g) as a yellow powder.
¹H NMR (CDCl₃, 400 MHz): δ 1.22 (3H, d, J=7.3 Hz), 2.35-2.41 (2H, m), 2.45 (1H, dd, J=16.5, 3.1 Hz), 2.75 (1H, dd, J=16.5, 6.7 Hz), 3.23-3.31 (1H, m), 3.63 (2H, t, J=6.1 Hz), 4.24 (2H, t, J=5.8 Hz), 6.79-6.84 (1H, m), 7.31-7.33 (1H, m), 8.51 (1H, s).
ESIMS(+): 521 [M+H]⁺.

### <Example 265>

### 6-[4-(3-Bromopropoxy)-2,3-difluorophenyl]-2-t-butoxycarbonyl 5-methyl-4,5-dihydro-2H-pyridazin-3-one

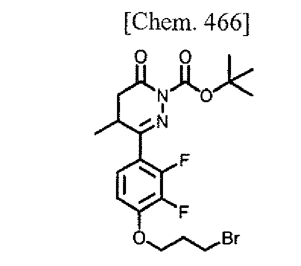

The reaction was carried out in the same manner as in Example 241 using the compound of Example 264 to obtain the desired product as a yellow powder.
¹H NMR (CDCl₃, 400 MHz): δ 1.22 (3H, d, J=7.3 Hz), 1.60 (9H, s), 2.34-2.40 (2H, m), 2.54 (1H, dd, J=16.2, 3.4 Hz), 2.82 (1H, dd, J=16.2, 6.4 Hz), 3.27-3.34 (1H, m), 3.63 (2H, t, J=6.4 Hz), 4.24 (2H, t, J=5.8 Hz), 6.81 (1H, m), 7.44-7.49 (1H, m).

### <Example 266>

### 6-[4-(3-Bromopropoxy)phenyl]-2-t-butoxycarbonyl 5-methyl-4,5-dihydro-2H-pyridazin-3-one

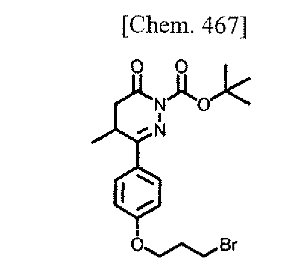

The reaction was carried out in the same manner as in Example 264 using the compound of Example 167 and subsequently, the reaction was carried out in the same manner as in Example 241 to obtain the desired product as a white powder.
¹H NMR (CDCl₃, 400 MHz): δ 1.25 (3H, d, J=7.3 Hz), 1.62 (9H, s), 2.31-2.37 (2H, m), 2.56 (1H, d, J=16.2 Hz), 2.78 (1H, dd, J=16.2, 6.4 Hz), 3.36-3.38 (1H, m), 3.62 (2H, t, J=6.4 Hz), 4.13 (2H, t, J=6.1 Hz), 6.95 (2H, d, J=8.6 Hz), 7.79 (2H, d, J=8.6 Hz).

### <Example 267>

### 6-(8-Methoxy-2-methylquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

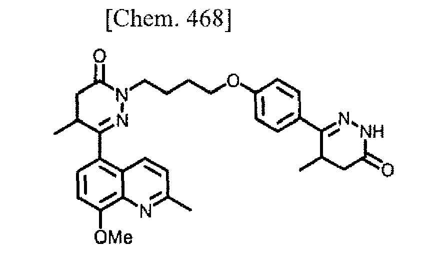

The compound of Example 148 (425 mg) was dissolved in DMF (6.00 mL) under an argon gas atmosphere, and the compound of Example 167 (208 mg) and potassium carbonate (281 mg) were added thereto, followed by stirring at 60°C for 5 hours. To the reaction liquid was added water, followed by extraction with ethyl acetate. The organic layer was washed with water and saturated brine in this order, and then dried over anhydrous sodium sulfate. After evaporating the solvent under reduced pressure, the residue was purified by silica gel column chromatography (ethyl acetate: methanol= 10:1) to obtain the desired product (474 mg) as a pale yellow amorphous.
Elemental analysis: Found value C 67.28%, H 6.34%, N 12.50%, Calculated value as C₃₁H₃₅N₅O₄ · 1/2H₂O C 67.62%, H 6.59%, N 12.72%.
FABMS (+): 542 [M]⁺.
¹H NMR (CDCl₃, 400 MHz): δ 1.17 (3H, t, J=7.3 Hz), 1.24 (3H, d, J=7.3 Hz), 1.88-1.95 (4H, m), 2.46 (1H, d, J=16.5 Hz), 2.55 (1H, dd, J=16.5, 3.6 Hz), 2.70 (1H, dd, 16.5, 7.3 Hz), 2.80 (3H, s), 2.86 (1H, dd, J=16.5, 7.3 Hz), 3.21-3.25 (1H, m), 3.29-3.34 (1H, m), 3.83-3.90 (1H, m), 4.05-4.09 (1H, m), 4.12 (3H, s), 6.89-6.92 (2H, m), 7.05 (1H, d, J=8.6 Hz), 7.35 (1H, d, J=8.6 Hz), 7.49 (1H, d, J=8.6 Hz), 7.66-7.69 (1H, m), 8.43 (1H, brs), 8.56 (1H, d, J=8.6 Hz).

### <Example 268>

### 6-(8-Methoxy-2-methylquuiolin-5-yl)-2-[4-[4-(4-methyl-6-oxo-l,4,5,6-tetrahydropyridazin-3-yl)phenoxy]butyl]-2H-pyridazin-3-one

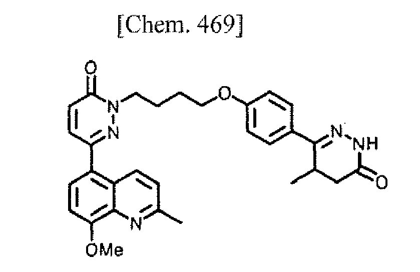

The reaction was carried out in the same manner as in Example 267 using the compound of Example 149 and the compound of Example 167 to obtain the desired product as a colorless amorphous.
Elemental analysis: Found value C 67.85%, H 6.02%, N 12.95%, Calculated value as C₃₀H₃₁N₅O₄ · 1/3H₂O C 67.78%, H 6.00%, N 13.17%.
ESIMS (+): 526 [M+H]⁺.
¹H NMR (CDCl₃, 400 MHz): δ 1.24 (3H, d, J=7.4 Hz), 1.90-1.97 (2H, m), 2.08-2.17 (2H, m), 2.46 (1H, d, J=16.8 Hz), 2.70 (1H, dd, J=16.8, 6.7 Hz), 2.82 (3H, s), 3.28-3.33 (1H, m), 4.07 (2H, t, J=6.1 Hz), 4.13 (3H, s), 4.36 (2H, J=7.3 Hz), 6.89-6.92 (2H, m), 7.06 (1H, d, J=9.8 Hz), 7.10 (1H, d, J=8.0 Hz), 7.36 (1H, d, J=8.6 Hz), 7.48 (1H, d, J=9.8 Hz), 7.51 (1H, d, J=8.0 Hz), 7.65-7.68 (2H, m), 8.35 (1H, d, J=8.6 Hz), 8.42 (1H, brs).

### <Example 269>

### 6-(8-Metuoxy-2-isopropylquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

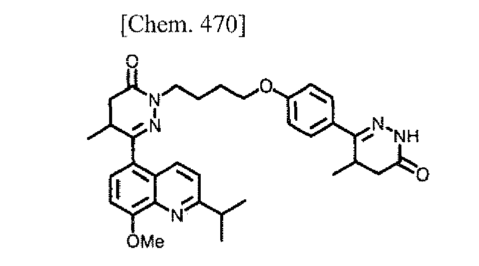

The reaction was carried out in the same manner as in Example 267 using the compound of Example 150 and the compound of Example 167 to obtain the desired product as a colorless amorphous.
Elemental analysis: Found value C 69.16%, H 7.02%, N 11.80%, Calculated value as C₃₃H₃₉N₅O₄ · 1/3H₂O C 69.03%, H 6.93%, N 12.20%.
ESIMS (+): 570 [M+H]⁺
¹H NMR (CDCl₃, 400 MHz): δ 1.16 (3H, d, J=7.4 Hz), 1.24 (3H, d, J=7.4 Hz), 1.38 (6H, dd, J=1.8, 7.1 Hz), 1.90-1.96 (4H, m), 2.45 (1H, d, J=16.8 Hz), 2.55 (1H, dd, J=3.0, 16.8 Hz), 2.70 (1H, dd, J=16.8, 6.7 Hz), 2.86 (1H, dd, J=16.8, 6.7 Hz), 3.21-3.25 (1H, m), 3.30-3.42 (2H, m), 3.83-3.89 (1H, m), 4.06-4.09 (3H, m), 4.12 (3H, s), 6.92 (2H, d, J=8.6 Hz), 7.05 (1H, d, J=8.6 Hz), 7.43 (1H, d, J=8.6 Hz), 7.49 (1H, d, J=8.6 Hz), 7.67 (2H, d, J=8.6 Hz), 8.39 (1H, brs), 8.62 (1H, d, J=8.6 Hz).

### <Example 270>

### 6-(8-Methoxy-2-isopropylquinolin-5-yl)-2-[4-[4-(4-methyl-6-oxo-l,4,5,6-tetrahydropyridazin-3-yl)phenoxy]butyl]-2H-pyridazin-3-one

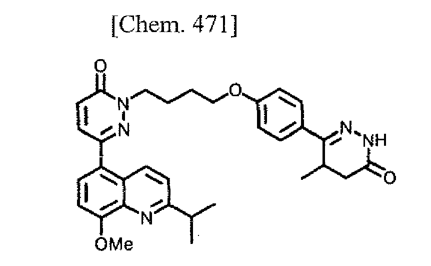

The reaction was carried out in the same manner as in Example 267 using the compound of Example 151 and the compound of Example 167 to obtain the desired product as a colorless amorphous.
Elemental analysis: Found value C 68.78%, H 6.40%, N 12.31%, Calculated value as C₃₂H₃₅N₅O₄ · 1/3H₂O C 68.68%, H 6.42%, N 12.39%.
ESIMS (+): 554 [M+H]⁺.
¹H NMR (CDCl₃, 400 MHz): δ 1.23 (3H, d, J=7.3 Hz), 1.39 (6H, d, J=7.3 Hz), 1.93 (2H, m), 2.10 (2H, m), 2.45 (1H, d, J=17.1 Hz), 2.70 (1H, dd, J=6.7, 17.1 Hz), 3.30-3.44 (2H, m), 4.07 (2H, t, J=6.1 Hz), 4.13 (3H, s), 4.36 (2H, J=7.3 Hz), 6.91 (2H, d, J=9.2 Hz), 7.06 (1H, d, J=9.8 Hz), 7.10 (1H, d, J=8.6 Hz), 7.44 (1H, d, J=9.2 Hz), 7.48 (1H, d, J=9.8 Hz), 7.50 (1H, d, J=8.6 Hz), 7.66 (2H, d, J=9.2 Hz), 8.40 (1H, d, J=9.2 Hz).

### <Example 271>

### 2-[4-[2,3-Difluoro-4-(6-oxo-1,4,5,6-tetrahydropyridazin-3-yl)phenoxy]butyl]-6-(8-methoxy-2-methylquinolin-5-yl)-2H-pyridazin-3-one

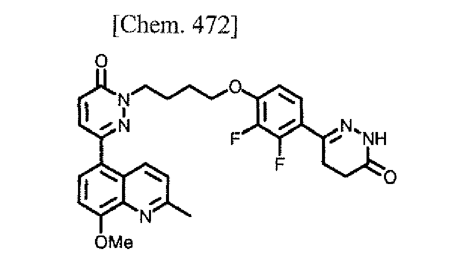

The reaction was carried out in the same manner as in Example 267 using the compound of Example 149 and the compound of Example 213 to obtain the desired product as a colorless amorphous.
Elemental analysis: Found value C 63.17%, H 4.98%, N 12.55%, Calculated value as C₂₉H₂₇F₂N₅O₄ · 1/5H₂O C 63.20%, H 5.01%, N 12.71%.
ESIMS (+): 548 [M+H]⁺.
¹H NMR (CDCl₃, 400 MHz): δ 1.93-2.00 (2H, m), 2.09-2.14 (2H, m), 2.59 (2H, dd, J=8.9, 6.7 Hz), 2.82 (3H, s), 2.98 (2H,dt, J=8.9,1.8 Hz), 4.13 (3H, s), 4.15 (2H, t, J=6.8 Hz), 4.36 (2H, t, J=7.3 Hz), 6.73-6.78 (1H, m), 7.06 (1H, d, J=9.8 Hz), 7.10 (1H, d, J=8.6 Hz), 7.27-7.32 (1H, m), 7.38 (1H, d, J=8.6 Hz), 7.49 (1H, d, J=9.8 Hz), 7.51 (1H, d, J=8.6 Hz), 8.3 (1H, d, J=8.6 Hz), 8.53 (1H, brs).

### <Example 272>

### 2-[4-[2-Fluoro-4-(6-oxo-l,4,5,6-tetrahydropyridazin-3-yl)phenoxy]butyl]-6-(8-methoxy-2-methylquinolin-5-yl)-2H-pyridazin-3-one

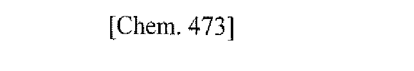

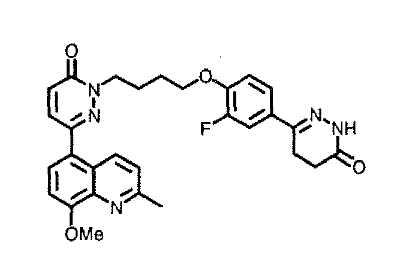

The reaction was carried out in the same manner as in Example 267 using the compound of Example 149 and the compound of Example 203 to obtain the desired product as a colorless amorphous.
Elemental analysis: Found value C 63.37%, H 5.21%, N 12.52%, Calculated value as C₂₉H₂₇F₂N₅O₄ · H₂O C 63.61 %, H 5.52%, N 12.79%.
ESIMS (+): 530 [M+H]⁺.
¹H NMR (CDCl₃, 400 MHz): δ 1.95-2.00 (2H, m), 2.09-2.15 (2H, m), 2.60 (2H, dd, J=7.3, 8.6 Hz), 2.82 (3H, s), 2.93 (2H, t, J=8.0 Hz), 4.13 (3H, s), 4.14 (2H, m), 4.36 (2H, t, J=7.3 Hz), 6.94 (1H, t, J=8.6 Hz), 7.06 (1H, d, J=9.8 Hz), 7.10 (1H, d, J=8.0 Hz), 7.33-7.36 (1H, m), 7.38 (1H, d, J=9.2 Hz), 7.47-7.52 (3H, m), 8.35 (1H, d, J=9.2 Hz), 8.49 (1H, brs).

### <Example 273>

### 2-[4-[2,3-Difluoro-4-(4-methyl-6-oxo-1,4,5,6-tetrahydropyridazin-3-yl)phenoxy]butyl]-6-(8-methoxy-2-methylquinolin-5-yl)-2H-pyridazin-3-one

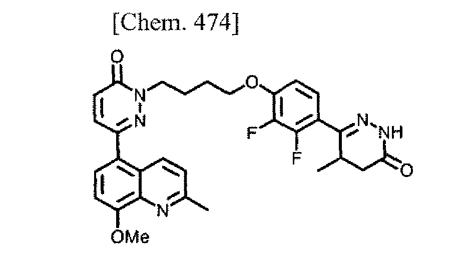

The reaction was carried out in the same manner as in Example 267 using the compound of Example 149 and the compound of Example 251 to obtain the desired product as a colorless amorphous.
Elemental analysis: Found value C 63.88%, H 5.27%, N 12.25%, Calculated value as C₃₀H₂₉F₂N₅O₄ C 64.16%, H 5.21%, N 12.47%.
ESIMS (+): 562 [M+H]⁺.
¹H NMR (CDCl₃, 400 MHz): δ 1.21 (3H, d, J=7.4 H), 1.93-2.00 (2H, m), 2.09-2.17 (2H, m), 2.44 (2H, dd, J=17.1, 3.1 Hz), 2.74 (1H, dd, J=17.1, 6.7 Hz), 2.82 (3H, s), 3.22-3.30 (1H, m), 4.10-4.17 (2H, m), 4.13 (3H, s), 4.36 (2H, t, J=7.3 Hz), 6.74-6.79 (1H, m), 7.06 (1H, d, J=8.6 Hz), 7.11 (1H, d, J=8.6 Hz), 7.25-7.29 (1H, m), 7.39 (1H, d, J=8.6 Hz), 7.49 (1H, d, J=8.6 Hz), 7.51 (1H, d, J=8.6 Hz), 8.35 (1H, d, J=8.6 Hz), 8.50 (1H, brs).

### <Example 274>

### 2-[4-[2,3-Difluoro-4-(6-oxo-1,4,5,6-tetrahydropyridazin-3-yl)phenoxy]butyl]-6-(7-methoxy-2-trifluoromethylbenzofuran-4-yl)-2H-pyridazin-3-one

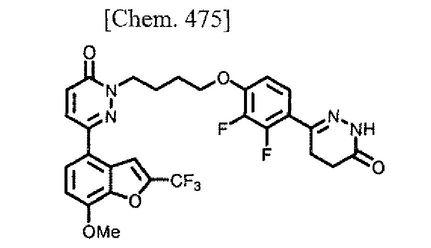

The reaction was carried out in the same manner as in Example 267 using the compound of Example 145 and the compound of Example 213 to obtain the desired product as a colorless powder.
Elemental analysis: Found value C 56.97%, H 3.96%, N 9.27%, Calculated value as C₂₈H₂₃F₅N₄O₅ C 56.95%, H 3.93%, N 9.49%.
ESIMS (+): 591 [M+H]⁺.
¹H NMR (CDCl₃, 400 MHz): δ 1.95-2.00 (2H, m), 2.11-2.17 (2H, m), 2.57-2.61 (2H, m), 2.96-3.00 (2H, m), 4.09 (3H, s), 4.15 (2H, t, J=6.1 Hz), 4.39 (2H, t, J=6.7 Hz), 6.73-6.77 (1H, m), 6.99 (1H, d, J=8.0 Hz), 7.06 (1H, d, J=9.8 Hz), 7.28-7.32 (1H, m), 7.50 (1H, d, J=8.0 Hz), 7.71 (1H, d, J=1.2 Hz), 7.73 (1H, d, J=9.8 Hz), 8.60 (1H, brs).

### <Example 275>

### 2-[4-[2,3-Difluoro-4-(4,4-dimethyl-5-oxo-4,5-dihydro-1H-pyrazol-3-yl)phenoxy]butyl]-6-(7-methoxy-2-trifluoromethylbenzofuran-4-yl)-2H-pyridazin-3-one

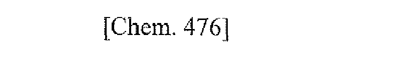

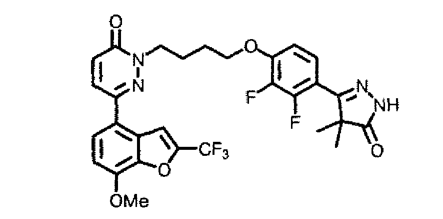

The reaction was carried out in the same manner as in Example 267 using the compound of Example 145 and the compound of Example 230 to obtain the desired product as a colorless powder.
Elemental analysis: Found value C 57.30%, H 4.01%, N 9.25%, Calculated value as C₂₉H₂₅F₅N₄O₅ C 57.62%, H 4.17%, N 9.27.
ESIMS (+): 604 [M+H]⁺.
¹H NMR (CDCl₃, 400 MHz): δ 1.40 (6H, s), 1.96-2.01 (2H, m), 2.12-2.17 (2H, m), 4.08 (3H, s), 4.17 (2H, t, J=6.1 Hz), 4.40 (2H, t, J=6.7 Hz), 6.76-6.80 (1H, m), 6.99 (1H, d, J=8.6 Hz), 7.06 (1H, d, J=9.8 Hz), 7.31-7.35 (1H, m), 7.50 (1H, d, J=8.6 Hz), 7.72 (1H, d, J=9.8 Hz), 7.72 (1H, s), 8.60 (1H, brs).

### <Example 276>

### 2-[4-[2,3-Difluoro-4-(4-methyl-6-oxo-l,4,5,6-tetrahydropyridazin-3-yl)phenoxy]butyl]-6-(7-methoxy-2-trifluoromethylbenzofuran-4-yl)-2H-pyridazin-3-one

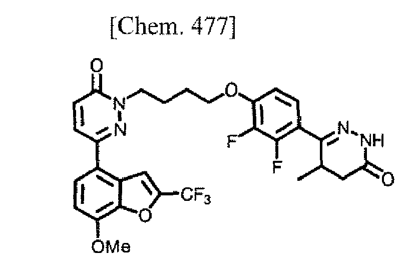

The reaction was carried out in the same manner as in Example 267 using the compound of Example 145 and the compound of Example 251 to obtain the desired product as a colorless powder.
Elemental analysis: Found value C 57.23%, H 4.06%, N 9.18 %, Calculated value as C₂₉H₂₅F₅N₄O₅ C 57,62%, H 4.17%, N 9.27%.
HRESIMS (+): 605.18351 (Calculated value as C₂₉H₂₆F₅N₄O₅ 605.18234).
¹H NMR (CDCl₃, 400 MHz): δ 1.21 (3H, d, J=7.3 Hz), 1.94-2.01 (2H, m), 2.11-2.17 (2H, m), 2.44 (1H, dd, J=17.1, 3.1 Hz), 2.74 (1H, dd, J=17.1, 6.7 Hz), 3.23-3.28 (1H, m), 4.08 (3H, s), 4.16 (2H, t, J=6.1 Hz), 4.39 (2H, t, J=6.7 Hz), 6.73-6.78 (1H, m), 6.99 (1H, d, J=8.0 Hz), 7.06 (1H, d, J=9.8 Hz), 7.25-7.29 (1H, m), 7.50 (1H, d, J=8.0 Hz), 7.71 (1H, d, J=1.2 Hz), 7.72 (1H, d, J=9.8 Hz), 8.49 (1H, brs).

### <Example 277>

### 2-[4-[2,3-Difluoro-4-(4,4-dimethyl-5-oxo-4,5-dihydro-1H-pyrazol-3-yl)phenoxy]butyl]-6-(4-methoxy-2-trifluoromethyl-1H-benzimidazol-7-yl)-2H-pyridazin-3-one

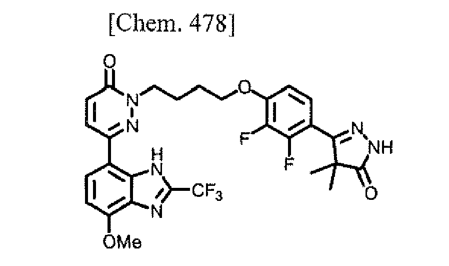

The reaction was carried out in the same manner as in Example 267 using the compound of Example 146 and the compound of Example 230 to obtain a condensed form. Then, the obtained condensed form (200 mg) was dissolved in THF (2.00 mL), and 3.00 mol/L hydrochloric acid (2.00 mL) was added thereto, followed by stirring at room temperature for 3 hours and at 50°C for 1 hour. To the reaction liquid was added water, and the resulting solid was then collected by filtration and washed with water. The obtained solid was dissolved in methanol-containing chloroform (chloroform:methanol=9:1) and then dried over anhydrous magnesium sulfate. After evaporating the solvent, the residue was purified by silica gel column chromatography (ethyl acetate:methanol=30:1) to obtain the desired product as a colorless powder.
Elemental analysis: Found value C 55.69%, H 4.15%, N 13.72%, Calculated value as C₂₈H₂₅F₅N₆O₄ C 55.63%, H 4.17%, N 13.90%.
¹H NMR (CDCl₃, 400 MHz): δ 1.40 (6H, s), 1.98-2.05 (2H, m), 2.16-2.23 (2H, m), 4.12 (3H, s), 4.18 (2H, t, J=6.1 Hz), 4.44 (2H, t, J=6.7 Hz), 6.76-6.81 (1H, m), 6.86 (1H, d, J=8.6 Hz), 7.12 (1H, d, J=9.8 Hz), 7.32-7.37 (1H, m), 7.67 (1H, d, J=8.6 Hz), 7.89 (1H, d, J=9.8 Hz), 8.52 (1H, brs), 11.19 (1H, brs).

### <Example 278>

### 2-[4-[2,3-Difluoro-4-(6-oxo-1,4,5,6-tetrahydropyridazin-3-yl)phenoxy]butyl]-6-(4-methoxy-2-trifluoromethyl-1H-benzimidazol-7-yl)-2H-pyridazin-3-one

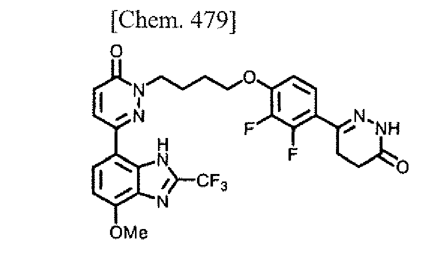

The reaction was carried out in the same manner as in Example 267 using the compound of Example 146 and the compound of Example 213, and the obtained compound was then allowed to react in the same manner as in Example 277 to obtain the desired product as a colorless powder.
HRESIMS (+): 591.17684 (Calculated value as C₂₇H₂₄F₅N₆O₄ 591.17792).
¹H NMR (CDCl₃, 400 MHz): δ 1.99-2.05 (2H, m), 2.15-2.21 (2H, m), 2.57-2.61 (2H, m), 2.95-3.00 (2H, m), 4.12 (3H, s), 4.17 (2H, t, J=6.1 Hz), 4.43 (2H, t, J=6.7 Hz), 6.73-6.77 (1H, m), 6.86 (1H, d, J=8.6 Hz), 7.11 (1H, d, J=9.8 Hz), 7.26-7.32 (1H, m), 7.67 (1H, d, J=8.6 Hz), 7.88 (1H, d, J=9.8 Hz), 8.50 (1H, brs), 11.18 (1H, brs).

### <Example 279>

### 2-[4-[2,3-Difluoro-4-(4-methyl-6-oxo-1,4,5,6-tetrahydropyridazin-3-yl)phenoxy]butyl]-6-(4-methoxy-2-trifluoromethyl-1H-benzimidazol-7-yl)-2H-pyridazin-3-one

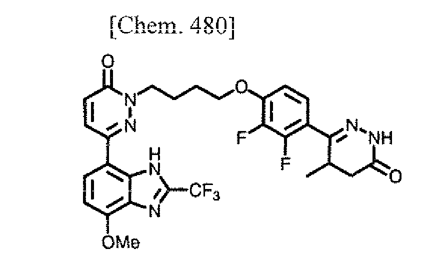

The reaction was carried out in the same manner as in Example 267 using the compound of Example 146 and the compound of Example 251, and the obtained compound was then allowed to react in the same manner as in Example 277 to obtain the desired product as a colorless powder.
Elemental analysis: Calculated value C 55.63%, H 4.17%, N 13.90%, Found value as C₂₈H₂₅F₅N₆O₄ C 55.56%, H 4.12%, N 13.68%.
¹H NMR (CDCl₃, 400 MHz): δ 1.20 (3H, d, J=7.3 Hz), 1.99-2.05 (2H, m), 2.15-2.21 (2H, m), 2.44 (1H, dd, J=3.1, 16.5 Hz), 2.74 (1H, dd, J=6.7, 16.5 Hz), 3.23-3.27 (1H, m), 4.12 (3H, s), 4.17 (2H, t, J=6.1 Hz), 4.43 (2H, t, J=6.7 Hz), 6.74-6.78 (1H, m), 6.86 (1H, d, J=8.6 Hz), 7.1 (1H, d, J=9.8 Hz), 7.26-7.31 (1H, m), 7.67 (1H, d, J=8.6 Hz), 7.88 (1H, d, J=9.8 Hz), 8.50 (1H, brs), 11.19 (1H, brs).

### <Example 280>

### 6-(2-ethyl-8-Methoxyquinolin-5-yl)-2-[4-[4-(4-methyl-6-oxo-1,4,5,6-tetrahydroppyridazin-3-yl)phenoxy]butyl]-2H-pyridazin-3-one

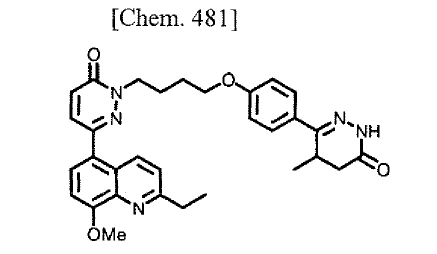

The reaction was carried out in the same manner as in Example 267 using the compound of Example 147 and the compound of Example 167 to obtain the desired product as a colorless amorphous.
¹H NMR (CDCl₃, 400 MHz): δ 1.24 (3H, d, J=7.3 Hz), 1.41 (3H, t, J=7.3 Hz), 1.87-1.99 (2H, m), 2.06-2.17 (2H, m), 2.45 (1H, dd, J=17.1, 1.2 Hz), 2.70 (1H, dd, J=17.1, 6.7 Hz), 3.10 (2H, q, J=7.3 Hz), 3.26-3.37 (1H, m), 4.07 (2H, t, J=6.7 Hz), 4.13 (3H, s), 4.36 (2H, t, J=7.3 Hz), 6.90 (2H, d, J=9.2 Hz), 7.06 (1H, d, J=9.2 Hz), 7.10 (1H, d, J=8.6 Hz), 7.40 (1H, d, J=9.2 Hz), 7.48 (1H, d, J=8.6 Hz), 7.51 (1H, d, J=8.6 Hz), 7.66 (2H, d, J=9.2 Hz), 8.3 8 (1H, d, J=8.6 Hz), 8.41 (1H, brs).
ESIMS (+): 540 [M+H]⁺.
Elemental analysis: Found value C 68.36%, H 6.10%, N 12.72%, Calculated value as C₃₁H₃₃N₅O₄ · 1/5H₂O C 68.54%, H 6.20%, N 12.89%.

### <Example 281>

### 6-(2-Ethyl-8-methoxyquinolin-5-yl)-2-[4-[4-(6-oxo-1,4,5,6-tetrahydropyridazin-3-yl)phenoxy]butyl]-2H-pyridazrin-3-one

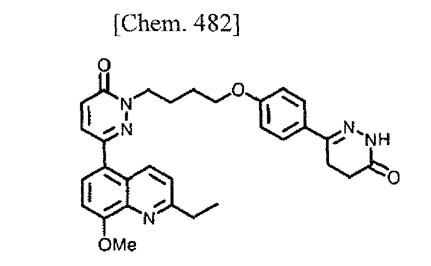

The reaction was carried out in the same manner as in Example 267 using the compound of Example 147 and the compound of Example 175 to obtain the desired product as a colorless amorphous.
¹H NMR (CDCl₃, 400 MHz): δ 1.41 (3H, t, J=7.9 Hz), 1.87-1.99 (2H, m), 2.06-2.18 (2H, m), 2.59 (2H, t, J=8.6 Hz), 2.95 (2H, t, J=8.6 Hz), 3.10 (2H, q, J=7.9 Hz), 4.07 (2H, t, J=6.1 Hz), 4.13 (3H, s), 4.36 (2H, t, J=7.3 Hz), 6.90 (2H, d, J=9.2 Hz), 7.06 (1H, d, J=9.8 Hz), 7.10 (1H, d, J=8.6 Hz), 7.40 (1H, d, J=8.6 Hz), 7.48 (1H, d, J=9.8 Hz), 7.50 (1H, d, J=8.6 Hz), 7.63 (2H, d, J=9.2 Hz), 8.38 (1H, d, J=8.6 Hz), 8.41 (1H, brs).
ESIMS (+): 526 [M+H]⁺.
Elemental analysis: Found value C 68.07%, H 5.89%, N 13.05%, Calculated value as C₃₀H₃₁N₅O₄ · 1/5H₂O C 68.09%, H 5.98%, N 13.23%.

### <Example 282>

### 6-(2-Ethyl-8-methoxyquinolin-5-yl)-2-[4-[4-(4,4-dimethyl-5-oxo-4,5-dihydro-1H-pyrazol-3-yl)phenoxy]butyl]-2H-pyridazin-3-one

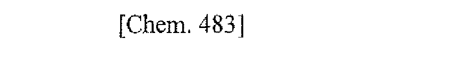

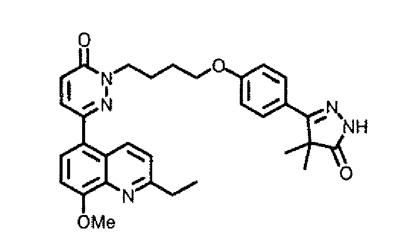

The reaction was carried out in the same manner as in Example 267 using the compound of Example 147 and the compound of Example 182 to obtain the desired product as a white crystal.
¹H NMR (CDCl₃, 400 MHz): δ 1.41 (3H, t, J=7.9 Hz), 1.49 (6H, s), 1.87-1.99 (2H, m), 2.06-2.19 (2H, m), 3.10 (2H, q, J=7.9 Hz), 4.07 (2H, t, J=6.7 Hz), 4.13 (3H, s), 4.36 (2H, t, J=7.3 Hz), 6.92 (2H, d, J=9.2 Hz), 7.06 (1H, d, J=9.8 Hz), 7.10 (1H, d, J=7.9 Hz), 7.41 (1H, d, J=9.8 Hz), 7.48 (1H, d, J=9.8 Hz), 7.51 (1H, d, J=7.9 Hz), 7.71 (2H, d, J=9.2 Hz), 8.38 (1H, d, J=9.2 Hz), 8.38 (1H, brs).
HRESIMS (+): 540.25972 (Calculated value as C₃₁H₃₄N₅O₄ 540.26108).

### <Example 283>

### 6-(2-Ethyl-8-methoxyquinolin-5-yl)-2-[4-[2,3-difluoro-4-(6-oxo-1,4,5,6-tetrahydropyridazin-3-yl)phenoxy]butyl]-2H-pyridazin-3-one

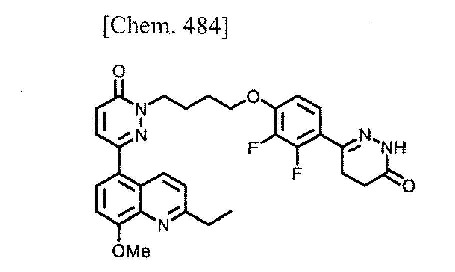

The reaction was carried out in the same manner as in Example 267 using the compound of Example 147 and the compound of Example 213 to obtain the desired product as a colorless amorphous.
¹H NMR (CDCl₃, 400 MHz): δ 1.41 (3H, t, J=7.9 Hz), 1.91-2.02 (2H, m), 2.07-2.18 (2H, m), 2.55-2.62 (2H, m), 2.94-3.02 (2H, m), 3.10 (2H, q, J=7.9 Hz), 4.13 (3H, s), 4.15 (2H, t, J=6.7 Hz), 4.36 (2H, t, J=6.7 Hz), 6.72-6.79 (1H, m), 7.06 (1H, d, J=9.8 Hz), 7.10 (1H, d, J=8.6 Hz), 7.27-7.33 (1H, m), 7.43 (1H, d, J=9.2 Hz), 7.49 (1H, d, J=9.8 Hz), 7.51 (1H, d, J=8.6 Hz), 8.38 (1H, d, J=8.6 Hz), 8.57 (1H, brs).
ESIMS (+): 562 [M+H]⁺.
Elemental analysis: Found value C 63.98%, H 5.26%, N 12.12%, Calculated value as C₃₀H₂₉F₂N₅O₄ · 1/5H₂O C 63.75%, H 5.24%, N 12.39%

### <Example 284>

### 6-(2-Ethyl-8-methoxyquinolin-5-yl)-2-[4-[2-fluoro-4-(6-oxo-1,4,5,6-tetrahydropyridazin-3-yl)phenoxy]buty]-2H-pyridazin-3-one

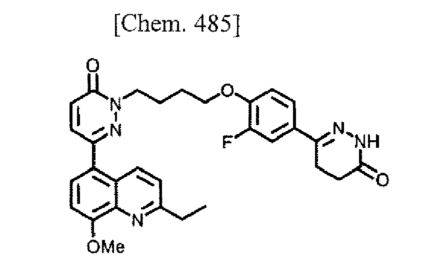

The reaction was carried out in the same manner as in Example 267 using the compound of Example 147 and the compound of Example 203 to obtain the desired product as a colorless amorphous.
¹H NMR (CDCl₃, 400 MHz): δ 1.41 (3H, t, J=7.3 Hz), 1.92-2.02 (2H, m), 2.08-2.18 (2H, m), 2.60 (2H, t, J=7.9 Hz), 2.92 (2H, t, J=7.9 Hz), 3.10 (2H, q, J=7.3 Hz), 4.13 (3H, s), 4.14 (2H, t, J=6.1 Hz), 4.37 (2H, t, J=6.7 Hz), 6.95 (1H, t, J=8.6 Hz), 7.06 (1H, d, J=9.2 Hz), 7.10 (1H, d, J=8.6 Hz), 7.32-7.37 (1H, m), 7.42 (1H, d, J=8.6 Hz), 7.47-7.53 (1H, m), 7.49 (1H, d, J=9.2 Hz), 7.51 (1H, d, J=8.6 Hz), 8.39 (1H, d, J=8.6 Hz), 8.49 (1H, brs).
HRESIMS (+): 544.23281 (Calculated value as C₃₀H₃₀FN₅O₄ 544.23601).

### <Example 285>

### 6-(2-Ethyl-8-methoxyquinolin-5-yl)-2-[4-[2-fluoro-4-(4,4-dimethyl-5-oxo-4,5-dihydro-1H-pyrazol-3-yl)phenoxy]butyl]-2H-pyridazin-3-one

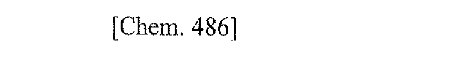

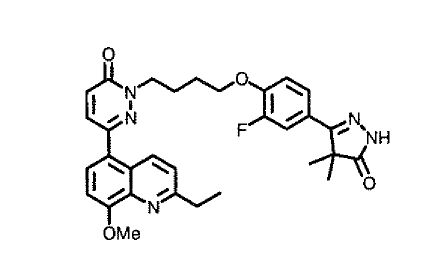

The reaction was carried out in the same manner as in Example 267 using the compound of Example 147 and the compound of Example 217 to obtain the desired product as a white crystal.
¹HNMR (CDCl₃, 400 MHz): δ 1.41 (3H, t, J=7.9 Hz), 1.48 (6H, s), 1.92-2.03 (2H, m), 2.08-2.19 (2H, m), 3.10 (2H, q, J=7.9 Hz), 4.15 (3H, s), 4.15 (2H, t, J=6.1 Hz), 4.37 (2H, t, J=7.3 Hz), 6.97 (1H, t, J=8.6 Hz), 7.06 (1H, d, J=9.2 Hz), 7.10 (1H, d, J=7.9 Hz), 7.41-7.46 (2H, m), 7.43 (2H, d, J=8.6 Hz), 7.49 (1H, d, J=9.2 Hz), 7.51 (1H, d, J=7.9 Hz), 7.55 (1H, dd, J=12.2, 1.8 Hz), 8.39 (1H, d, J=8.6 Hz), 8.47 (1H, brs).
ESIMS (+): 558 [M+H]⁺.
Elemental analysis: Found value C 66.61%, H 5.72%, N 12.36%, Calculated value as C₃₁H₃₂FN₅O₄ C 66.77%, H 5.78%, N 12,56%.

### <Example 286>

### 6-(2-Ethyl-8-methoxyquinolin-5-yl)-2-[4-[2-methoxy-4-(4,4-dimethyl-5-oxo-4,5-dihydro-11]-pyrazol-3-yl)phenoxy]butyl]-2H-pyridazin-3-one

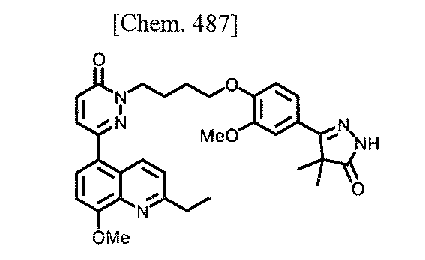

The reaction was carried out in the same manner as in Example 267 using the compound of Example 147 and the compound of Example 248 to obtain the desired product as a white crystal.
¹H NMR (CDCl₃, 400 MHz): δ 1.40 (3H, t, J=7.9 Hz), 1.51 (6H, s), 1.94-2.06 (2H, m), 2.06-2.18 (2H, m), 3.09 (2H, q, J=7.9 Hz), 3.87 (3H, s), 4.13 (3H, s), 4.13 (2H, t, J=6.1 Hz), 4.36 (2H, t, J=7.3 Hz), 6.86 (1H, d, J=8.6 Hz), 7.06 (1H, d, J=9.2 Hz), 7.10 (1H, d, J=8.6 Hz), 7.22 (1H, dd, J=8.6, 1.8 Hz), 7.41 (1H, d, J=9.2 Hz), 7.42 (1H, d, J=1.8 Hz), 7.48 (1H, d, J=9.2 Hz), 7.50 (1H, d, J=8.6 Hz), 8.38 (1H, d, J=9.2 Hz), 8.39 (1H, brs).
HRESIMS (+): 570.27348 (Calculated value as C₃₂H₃₅N₅O₅ 570.27164).

### <Example 287>

### 6-(2-Ethyl-8-methoxyquinolin-5-yl)-2-[4-[2-fluoro-4-(4-methyl-6-oxo-1,4,5,6-tetrahydropyridazin-3-yl)phenoxy]butyl]-2H-pyridazin-3-one

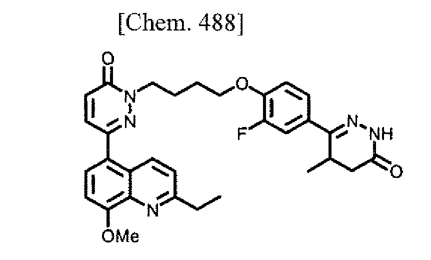

The reaction was carried out in the same manner as in Example 267 using the compound of Example 147 and the compound of Example 194 to obtain the desired product as a colorless amorphous.
¹H NMR (CDCl₃, 400 MHz): δ 1.23 (3H, d, J=7.3 Hz), 1.41 (3H, t, J=7.9 Hz), 1.92-2.02 (2H, m), 2.09-2.18 (2H, m), 2.46 (1H, dd, J=17.1, 1.2 Hz), 2.70 (1H, dd, J=17.1, 7.3 Hz), 3.10 (2H, q, J=7.9 Hz), 3.21-3.32 (1H, m), 4.13 (3H, s), 4.15 (2H, t, J=6.1 Hz), 4.37 (2H, t, J=7.3 Hz), 6.95 (1H, t, J=8.6 Hz), 7.06 (1H, d, J=9.2 Hz), 7.10 (1H, d, J=8.6 Hz), 7.35-7.41 (1H, m), 7.42 (1H, d, J=9.2 Hz), 7.49 (1H, d, J=8.6 Hz), 7.51 (1H, d, J=8.6 Hz), 7.53 (1H, dd, J=12.8, 2.4 Hz), 8.39 (1H, d, J=8.6 Hz), 8.49 (1H, brs).
HRESIMS (+): 558.25340 (Calculated value as C₃₁H₃₃FN₅O₄ 558.25166).

### <Example 288>

### 6-(2-Ethyl-8-methoxyquinolin-5-yl)-2-[4-[2-methoxy-4-(4-methyl-6-oxo-1,4,5,6-tetrahydropyridazin-3-yl)phenoxy]butyl]-2H-pyridazin-3-one

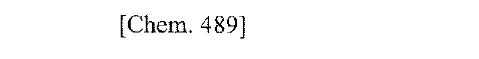

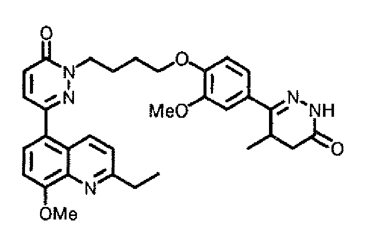

The reaction was carried out in the same manner as in Example 267 using the compound of Example 147 and the compound of Example 201 to obtain the desired product as a colorless amorphous.
¹H NMR (CDCl₃, 400 MHz): δ 1.24 (3H, d, J=7.3 Hz), 1.40 (3H, t, J=7.3 Hz), 1.94-2.04 (2H, m), 2.06-2.18 (2H, m), 2.46 (1H, dd, J=17.1, 1.2 Hz), 2.70 (1H, dd, J=17.1, 6.7 Hz), 3.09 (2H, q, J=7.3 Hz), 3.27-3.38 (1H, m), 3.87 (3H, s), 4.12-4.16 (2H, m), 4.13 (3H, s), 4.36 (2H, t, J=7.3 Hz), 6.86 (1H, d, J=8.6 Hz), 7.05 (1H, d, J=9.2 Hz), 7.09 (1H, d, J=8.6 Hz), 7.14 (1H, dd, J=8.6, 2.4 Hz), 7.40 (1H, d, J=9.2 Hz), 7.42 (1H, d, J=2.4 Hz), 7.48 (1H, d, J=9.2 Hz), 7.50 (1H, d, J=9.2 Hz), 8.38 (1H, d, J=9.2 Hz), 8.44 (1H, brs).
HRESIMS (+): 570.27136 (Calculated value as C₃₂H₃₆N₅O₄ 570.27164).

### <Example 289>

### 6-(2-Ethyl-8-methoxyquinolin-5-yl)-2-[4-[2,3-difluoro-4-(4-methyl-6-oxo-1,4,5,6-tetrahydropyridazin-3-yl)phenoxy]butyl]-2H-pyridazin-3-one

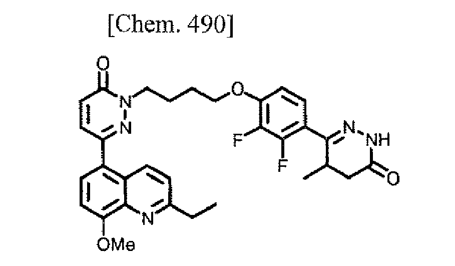

The reaction was carried out in the same manner as in Example 267 using the compound of Example 147 and the compound of Example 251 to obtain the desired product as a colorless amorphous.
¹H NMR (CDCl₃, 400 MHz): δ 1.20 (3H, d, J=7.3 Hz), 1.41 (3H, t, J=7.9 Hz), 1.92-2.02 (2H, m), 2.07-2.18 (2H, m), 2.44 (1H, dd, J=17.1, 3.1 Hz), 2.74 (1H, dd, J=17.1, 6.7 Hz), 3.10 (2H, q, J=7.9 Hz), 3.20-3.31 (1H, m), 4.13 (3H, s), 4.16 (2H, t, J=6.1 Hz), 4.37 (2H, t, J=7.3 Hz), 6.73-6.80 (1H, m), 7.06 (1H, d, J=9.2 Hz), 7.10 (1H, d, J=7.9 Hz), 7.23-7.30 (1H, m), 7.43 (1H, d, J=9.2 Hz), 7.49 (1H, d, J=9.2 Hz), 7.51 (1H, d, J=7.9 Hz), 8.38 (1H, d, J=9.2 Hz), 8.53 (1H, brs).
ESIMS (+): 576 [M+H]⁺,
HRESIMS (+): 576.24262 (Calculated value as C₃₁H₃₂F₂N₅O₄ 576.24223).

### <Example 290>

### 6-(2-Ethyl-8-methoxyquinolin-5-yl)-2-[4-[2,3-difluoro-4-(4,4-dimethyl-5-oxo-4,5-dihydro-1H-pyrazol-3-yl)phenoxy]butyl]-2H-pyridazin-3-one

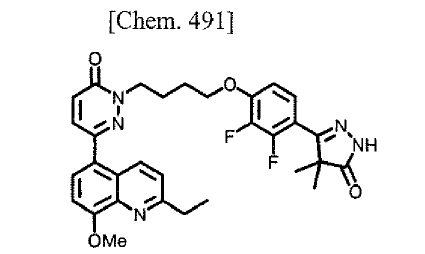

The reaction was carried out in the same manner as in Example 267 using the compound of Example 147 and the compound of Example 230 to obtain the desired product as a white powder.
¹H NMR (CDCl₃, 400 MHz): δ 1.40 (3H, s), 1.40 (3H, s), 1.41 (3H, t, J=7.3 Hz), 1.93-2.03 (2H, m), 2.08-2.19 (2H, m), 3.10 (2H, q, J=7.3 Hz), 4.13 (3H, s), 4.17 (2H, t, J=6.1 Hz), 4.37 (2H, t, J=7.3 Hz), 6.75-6.82 (1H, m), 7.07 (1H, d, J=9.8 Hz), 7.10 (1H, d, J=8.6 Hz), 7.30-7.37 (1H, m), 7.43 (1H, d, J=8.6 Hz), 7.49 (1H, d, J=9.8 Hz), 7.51 (1H, d, J=8.6 Hz), 8.38 (1H, d, J=8.6 Hz), 8.60 (1H, brs).
HRESIMS (+): 576.24078 (Calculated value as C₃₁H₃₂E₂N₅O₄ 576.24223).

### <Example 291>

### 6-(2-Ethyl-8-methoxyquinolin-5-yl)-2-[4-[2-methoxy-4-(6-oxo-1,4,5,6-tetrahydropyridazin-3-yl)phenoxy]butyl]-2H-pyridazin-3-one

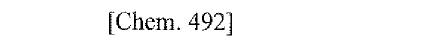

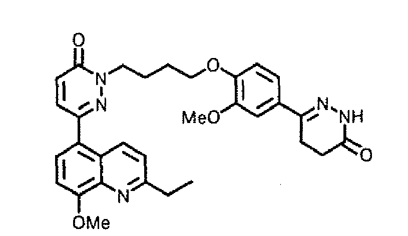

The reaction was carried out in the same manner as in Example 267 using the compound of Example 147 and the compound of Example 257 to obtain the desired product as a colorless amorphous.
¹H NMR (CDCl₃, 400 MHz): δ 1.40 (3H, t, J=7.9 Hz), 1.93-2.04 (2H, m), 2.06-2.18 (2H, m), 2.59 (2H, t, J=8.6 Hz), 2.95 (2H, t, J=8.6 Hz), 3.09 (2H, q, J=7.9 Hz), 3.86 (3H, s), 4.12 (2H, t, J=6.7 Hz), 4.13 (3 H, s), 4.36 (2H, t, J=7.3 Hz), 6.85 (1H, d, J=8.6 Hz), 7.05 (1H, d, J=9.2 Hz), 7.09 (1H, d, J=7.9 Hz), 7.11 (1H, dd, J=8.6, 2.4 Hz), 7.38 (1H, s), 7.40 (1H, d, J=8.6 Hz), 7.48 (1H, d, J=9.2 Hz), 7.50 (1H, d, J=7.9 Hz), 8.38 (1H, d, J=8.6 Hz), 8.44 (1H, brs). HRESIMS (+): 556.25454 (Calculated value as C₃₁H₃₄N₅O₅ 556.25599).

### <Example 292>

### 6-(2-Ethyl-8-methoxyquinolin-5-yl)-5-methyl-2-[3-[4-(4-methyl-6-oxo-1,4,5,6-tetrahydropyridazin-3-yl)phenoxy]propyl]-4,5-dihydro-2H-pyridazin-3-one

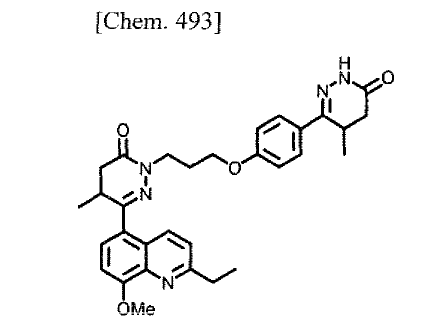

The reaction was carried out in the same manner as in Example 267 using the compound of Example 266 and the compound of Example 72, the obtained compound (196 mg) was then dissolved in dichloromethane (3.0 mL), and trifluoroacetic acid (1.0 mL) was added thereto, followed by stirring at room temperature for 1 hour. A saturated aqueous sodium hydrogen carbonate solution was added thereto, followed by extraction three times with ethyl acetate, and the combined organic solvent was washed with saturated brine and then dried over anhydrous sodium sulfate. The solvent was evaporated under reduced pressure, and the residue was then purified by silica gel chromatography (ethyl acetate→ethyl acetate:methanol= 20:1) to obtain the desired product (113 mg) as a yellow amorphous. HRESIMS (+): 542.27909 (Calculated value as C₃₁H₃₆N₅O₄ 542.27673).
¹H NMR (CDCl₃, 400 MHz): δ 1.17 (3H, d, J=7.3 Hz), 1.24 (3H, d, J=7.3 Hz), 1.38 (3H, t, J=7.6 Hz), 2.27-2.28 (2H, m), 2.46 (1H, d, J=16.5 Hz), 2.55 (1H, dd, J=16.5, 3.7 Hz), 2.71 (1H, dd, J=16.5, 6.1 Hz), 2.85 (1H, dd, J=16.5, 6.7 Hz), 3.06 (2H, q, J=7.6 Hz), 3.23-3.25 (1H, m), 3.31-3.32 (1H, m), 4.04-4.05 (1H, m), 4.11 (3H, s), 4.12-4.13 (2H, m), 4.19-4.20 (1H, m), 6.92 (2H, d, J=9.2 Hz), 7.04 (1H, d, J=8.6 Hz), 7.29 (1H, d, J=8.6 Hz), 7.29 (1H, d, J=8.6 Hz), 7.47 (1H, d, J=8.6 Hz), 7.67 (2H, d, J=9.2 Hz), 8.41 (1H, s), 8.58 (1H, d, J=8.6 Hz).

### <Example 293>

### 6-(2-Ethyl-8-methoxyquinolin-5-yl)-5-methyl-2-[4-[2-methoxy-4-(4-methyl-6-oxo-1,4,5,6-tetrahydropyridazin-3-yl)phenoxy]butyl]-4,5-dihydro-2H-pyridazin-3-one

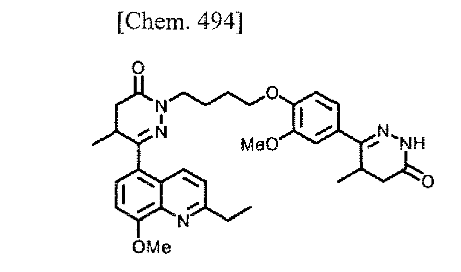

The reaction was carried out in the same manner as in Example 267 using the compound of Example 152 and the compound of Example 201 to obtain the desired product as a yellow amorphous.
HRESIMS (+): 586.30063 (Calculated value as C₃₃H₄₀N₅O₅ 586.30294).
¹H NMR (CDCl₃,400 MHz): δ 1.15 (3H, d, J=7.3 Hz), 1.24 (3H, d, J=7.3 Hz), 1.39 (3H, t, J=7.6 Hz), 1.94-1.97 (4H, m), 2.46 (1H, d, J=16.8 Hz), 2.54 (1H, dd, J=3.4, 16.8 Hz), 2.70 (1H, dd, J=16.8, 6.7 Hz), 2.85 (1H, dd, J=6.7, 16.8 Hz), 3.07 (2H, q, J=7.6 Hz), 3.22-3.23 (1H, m), 3.32-3.33 (1H, m), 3.86 (3H, s), 3.86-3.90 (1H, m), 4.06-4.14 (3H, m), 4.11 (3H, s), 6.86 (1H, d, J=8.6 Hz), 7.04 (1H, d, J=8.6 Hz), 7.15 (1H, dd, J=8.6, 2.1 Hz), 7.40-7.41 (2H, m), 7.48 (1H, d, J=8.6 Hz), 8.45 (1H, s), 8.59 (1H, d, J=8.6 Hz).

### <Example 294>

### 6-(2-Ethyl-8-methoxyquinolin-5-yl)-5-methyl-2-[4-[2-fluoro-4-(4-methyl-6-oxo-1,4,5,6-tetrahydropyridazin-3-yl)phenoxy]butyl]-4,5-dihydro-2H-pyridazin-3-one

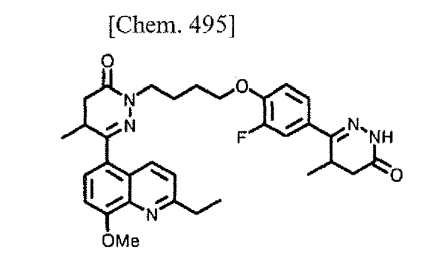

The reaction was carried out in the same manner as in Example 267 using the compound of Example 152 and the compound of Example 194 to obtain the desired product as a yellow amorphous.
HRESIMS (+): 574.28391 (Calculated value as C₃₂H₃₇FN₅O₄ 574.28296).
¹H NMR (CDCl₃, 400 MHz): δ 1.16 (3H, d, J=7.3 Hz), 1.23 (3H, d, J=7.3 Hz), 1.40 (3H, t, J=7.6 Hz), 1.90-2.00 (4H, m), 2.46 (1H, d, J=16.5 Hz), 2.55 (1H, dd, J=16.5, 3.7 Hz), 2.70 (1H, dd, J=16.5, 6.7 Hz), 2.86 (1H, dd, J=16.5, 6.7 Hz), 3.08 (2H, q, J=7.6 Hz), 3.21-3.29 (2H, m), 3.85-3.87 (1H, m), 4.09-4.14 (3H, m), 4.11 (3H, s), 6.96 (1H, t, J=8.6 Hz), 7.05 (1H, d, J=7.9 Hz), 7.38-7.39 (1H m), 7.42 (1H, d, J=8.6 Hz), 7.49 (1H, d, J=7.9 Hz), 7.53 (1H, dd, J=12.8, 1.8 Hz), 8.47 (1H, s), 8.59 (1H, d, J=8.6 Hz).

### <Example 295>

### 6-(2-Ethyl-8-methoxyquinolin-5-yl)-5-methyl-2-[4-[2,3-difluoro-4-(4-methyl-6-oxo-1,4,5,6-tetrahydropyridazin-3-yl)phenoxy]butyl]-4,5-dihydro-2H-pyridazin-3-one

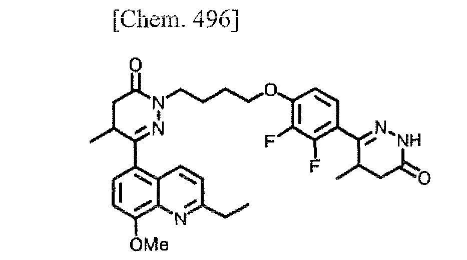

The reaction was carried out in the same manner as in Example 267 using the compound of Example 152 and the compound of Example 251 to obtain the desired product as a yellow amorphous.
HRESIMS (+): 592.27502 (Calculated value as C₃₂H₃₆F₂N₅O₄ 592.27353).
¹H NMR (CDCl₃, 400 MHz): δ 1.17 (3H, d, J=7.3 Hz), 1.20 (3H, d, J=7.3 Hz), 1.41 (3H, t, J=7.6 Hz), 1.95 (4H, m), 2.44 (1H, dd, J=16.5, 3.1 Hz), 2.55 (1H, dd, J=16.5, 3.7 Hz), 2.74 (1H, dd, J=16.5, 6.7 Hz), 2.86 (1H, dd, J=16.5, 6.7 Hz), 3.09 (2H, q, J=7.6 Hz), 3.21-3.27 (2H, m), 3.86 (1H, t, J=6.7 Hz), 4.09-4.17 (3H, m), 4.12 (3H, s), 6.77 (1H, t, J=8.3 Hz), 7.05 (1H, d, J=8.6 Hz), 7.25-7.30 (1H, m), 7.43 (1H, d, J=8.6 Hz), 7.49 (1H, d, J=8.6 Hz), 8.51 (1H, s), 8.58 (1H, d, J=8.6 Hz).

### <Example 296>

### 6-(2-Ethyl-8-methoxyquinolin-5-yl)-5-methyl-2-[4-[4-(6-oxo-1,4,5,6-tetrahydropyridazin-3-yl)phenoxy]butyl]-4,5-dihydro-2H-pyridazin-3-one

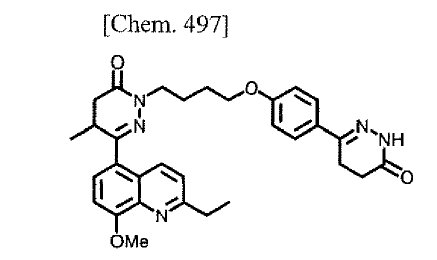

The reaction was carried out in the same manner as in Example 267 using the compound of Example 152 and the compound of Example 175 to obtain the desired product as a yellow solid.
HREIMS (+): 542.27434 (Calculated value as C₃₁H₃₆N₅O₄ 542.27673).
¹H NMR (CDCl₃, 400 MHz): δ 1.17 (3H, d, J=7.3 Hz), 1.40 (3H, t, J=7.6 Hz), 1.90-1.96 (4H, m), 2.55-2.59 (3H, m), 2.86 (1H dd, J=16.5, 6.7 Hz), 2.96 (2H, t, J=8.3 Hz), 3.09 (2H, q, J=7.6 Hz), 3.20-3.28 (1H, m), 3.86-3.88 (1H, m), 4.07-4.08 (3H, m), 4.12 (3H, s), 6.91 (2H, d, J=8.6 Hz), 7.06 (1H, d, J=8.6 Hz), 7.40 (1H, d, J=8.6 Hz), 7.50 (1H, d, J=8.6 Hz), 7.64 (2H, d, J=8.6 Hz), 8.41 (1H, s), 8.60 (1H, d, J=8.6 Hz).

### <Example 297>

### 6-(2-Ethyl-8-methoxyquinolin-5-yl)-5-methyl-2-[4-[2-methoxy-4-(6-oxo-1,4,5,6-tetrahydropyridazin-3-yl)phenoxy]butyl]-4,5-dihydro-2H-pyridazin-3-one

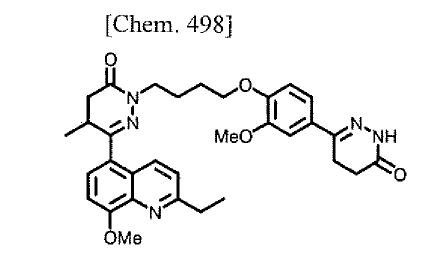

The reaction was carried out in the same manner as in Example 267 using the compound of Example 152 and the compound of Example 257 to obtain the desired product as a yellow amorphous.
HRESIMS (+): 572.28450 (Calculated value as C₃₂H₃₈N₅O₅ 572.28729).
¹H NMR (CDCl₃, 400 MHz): δ 1.16 (3H, d, J=7.3 Hz), 1.40 (3H, t, J=7.6 Hz), 1.94-1.97 (4H, m), 2.52-2.62 (3H, m), 2.85 (1H, dd, J=16.5, 6.7 Hz), 2.96 (2H, t, J=7.9 Hz), 3.08 (2H, q, J=7.7 Hz), 3.21-3.24 (1H, m), 3.86 (3H, s), 3.86-3.90 (1H, m), 4.07-4.15 (3H, m), 4.12 (3H, s), 6.86 (1H, d, J=8.6 Hz), 7.05 (1H, d, J=8.6 Hz), 7.12 (1H, dd, J=8.6, 1.8 Hz), 7.39-7.40 (2H, m), 7.49 (1H, d, J=8.6 Hz), 8.46 (1H, s), 8.59 (1H, d, J=8.6 Hz).

### <Example 298>

### 6-(2-Ethyl-8-methoxyquinolin-5-yl)-5-methyl-2-[4-[2-fluoro-4-(6-oxo-1,4,5,6-tetrahydropyridazin-3-yl)phenoxy]butyl]-4,5-dihydro-2H-pyridazin-3-one

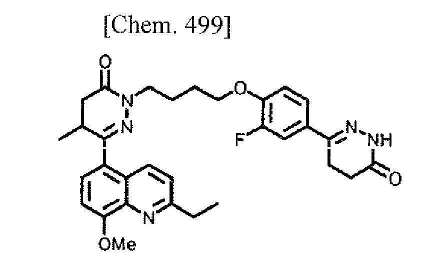

The reaction was carried out in the same manner as in Example 267 using the compound of Example 152 and the compound of Example 203 to obtain the desired product as a yellow amorphous.
HRESIMS (+): 560.26489 (Calculated value as C₃₁H₃₅FN₅O₄ 560.26731).
¹H NMR (CDCl₃, 400 MHz): δ 1.16 (3H, d, J=7.3 Hz), 1.40 (3H, t, J=7.6 Hz), 1.92-1.97 (4H, m), 2.55 (1H, dd, J=16.5, 3.1 Hz), 2.60 (2H, t, J=8.3 Hz), 2.86 (1H, dd, J=16.5, 6.7 Hz), 2.93 (2H, t, J=8.3 Hz), 3.08 (2H, q, J=7.6 Hz), 3.22-3.24 (1H, m), 3.85-3.87 (1H, m), 4.10-4.13 (3H, m), 4.11 (3H, s), 6.95 (1H, t, J=8.6 Hz), 7.05 (1H, d, J=7.9 Hz), 7.39-7.36 (1H, m), 7.42 (1H, d, J=9.2 Hz), 7.48 (1H, d, J=7.9 Hz), 7.50 (1H, dd, J=12.2, 2.4 Hz), 8.45 (1H, s), 8.59 (1H, d, J=9.2 Hz).

### <Example 299>

### 6-(2-Ethyl-8-methoxyquinolin-5-yl)-5-methyl-2-[4-[2,3-difluoro-4-(6-oxo-1,4,5,6-tetrahydropyridazin-3-yl)phenoxy]butyl]-4,5-dihydro-2H-pyridazin-3-one

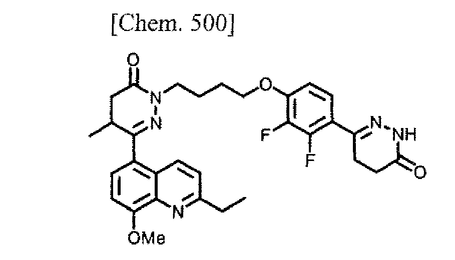

The reaction was carried out in the same manner as in Example 267 using the compound of Example 152 and the compound of Example 213 to obtain the desired product as a yellow amorphous.
HRESIMS (+): 578.25636 (Calculated value as C₃₁H₃₄F₂N₅O₄ 578.25788).
¹H NMR (CDCl₃, 400 MHz): δ 1.17 (3H, d, J=7.3 Hz), 1.40 (3H, t, J=7.6 Hz), 1.94 (4H, m), 2.52-2.61 (3H, m), 2.86 (1H, dd, J=16.5, 6.7 Hz), 2.97-2.99 (2H, m), 3.09 (2H, q, J=7.6 Hz), 3.21-3.25 (1H, m), 3.85-3.87 (1H, m), 4.08-4.16 (3H, m), 4.11 (3H, s), 6.75-6.78 (1H, m), 7.05 (1H, d, J=8.6 Hz), 7.27-7.32 (1H, m), 7.43 (1H, d, J=8.6 Hz), 7.49 (1H, d, J=8.6 Hz), 8.49 (1H, s), 8.59 (1H, d, J=8.6 Hz).

### <Example 300>

### 6-(2-Ethyl-8-methoxyquinolin-5-yl)-5-methyl-2-[4-[4-(4,4-dimethyl-5-oxo-4,5-dihydro-1H-pyrazol-3-yl)phenoxy]butyl]-4,5-dihydro-2H-pyridazin-3-one

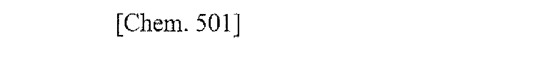

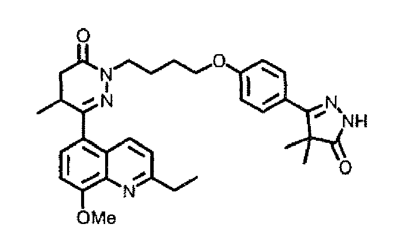

The reaction was carried out in the same manner as in Example 267 using the compound of Example 152 and the compound of Example 182 to obtain the desired product as a yellow amorphous.
HRESIMS (+): 556.29311 (Calculated value as C₃₂H₃₈N₅O₄ 556.29311).
¹H NMR (CDCl₃, 400 MHz): δ 1.17 (3H, d, J=7.3 Hz), 1.40 (3H, t, J=7.6 Hz), 1.50 (6H, s), 1.90-1.96 (4H, m), 2.56 (1H, dd, J=16.5, 3.7 Hz), 2.86 (1H, dd, J=16.5, 6.7 Hz), 3.09 (2H, q, J=7.6 Hz), 3.23-3.24 (1H, m), 3.83-3.90 (1H, m), 4.08-4.09 (3H, m), 4.12 (3H, s), 6.93 (2H, d, J=9.2 Hz), 7.06 (1H, d, J=8.6 Hz), 7.41 (1H, d, J=8.6 Hz), 7.50 (1H, d, J=8.6 Hz), 7.72 (2H, d, J=9.2 Hz), 8.39 (1H, s), 8.60 (1H, d, J=8.6 Hz).

### <Example 301>

### 6-(2-Ethyl-8-methoxyquinolin-5-yl)-5-methyl-2-[4-[2-methoxy-4-(4,4-dimethyl-5-oxo-4,5-dihydro-1H-pyrazol-3-yl)phenoxy]butyl]-4,5-dihydro-2H-pyridazin-3-one

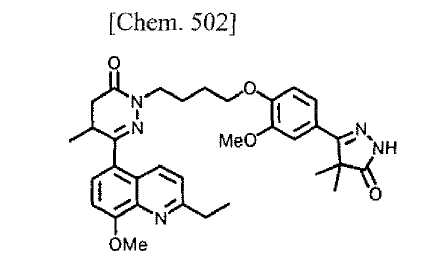

The reaction was carried out in the same manner as in Example 267 using the compound of Example 152 and the compound of Example 248 to obtain the desired product as a yellow amorphous.
HRESIMS (+): 586.29906 (Calculated value as C₃₃H₄₀N₅O₅ 586.30294).
¹H NMR (CDCl₃, 400 MHz): δ 1.16 (3H, d, J=7.3 Hz), 1.40 (3H, t, J=7.6 Hz), 1.51 (6H, s), 1.94-1.97 (4H, m), 2.55 (1H, dd, J=16.5, 3.7 Hz), 2.85 (1H, dd, J=16.5, 6.7 Hz), 3.08 (2H, q, J=7.6 Hz), 3.22-3.24 (1H, m), 3.87-3.89 (1H, m), 3.87 (3H, s), 4.09-4.12 (3H, m), 4.12 (3H, s), 6.87 (1H, d, J=8.6 Hz), 7.05 (1H, d, J=8.6 Hz), 7.22 (1H, dd, J=8.6, 1.8 Hz), 7.40-7.41 (2H, m), 7.49 (1H, d, J=8.6 Hz), 8.38 (1H, s), 8.59 (1H, d, J=8.6 Hz).

### <Example 302>

### 6-(2-Ethyl-8-methoxyquinolin-5-yl)-5-methyl-2-[4-[2-fluoro-4-(4,4-dimethyl-5-oxo-4,5-dihydro-1H-pyrazol-3-yl)phenoxy]butyl]-4,5-dihydro-2H-pyridazin-3-one

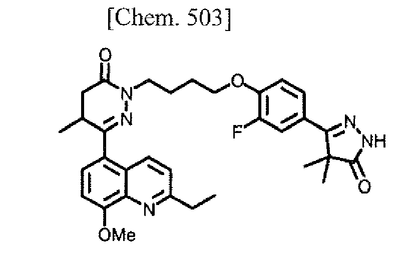

The reaction was carried out in the same manner as in Example 267 using the compound of Example 152 and the compound of Example 217 to obtain the desired product as a yellow amorphous.
HRESIMS (+): 574.28203 (Calculated value as C₃₂H₃₇FN₅O₄ 574.28296).
¹H NMR (CDCl₃, 400 MHz): δ 1.16 (3H, d, J=7.3 Hz), 1.40 (3H, t, J=7.65 Hz), 1.48 (6H, s), 1.91-1.95 (4H, m), 2.55 (H, dd, J=16.5, 3.1 Hz), 2.86 (1H, dd, J=16.5, 6.7 Hz), 3.08 (2H, q, J=7.6 Hz), 3.22-3.24 (1H, m), 3.84-3.86 (1H, m), 4.09-4.14 (3H, m), 4.11 (3H, s), 6.97 (1H, t, J=8.6 Hz), 7.05 (1H, d, J=8.6 Hz), 7.42-7.44 (2H, m), 7.49 (1H, d, J=8.6 Hz), 7.55 (1H, dd, J=12.5, 2.1 Hz), 8.45 (1H, s), 8.59 (1H, d, J=8.6 Hz).

### <Example 303>

### 6-(2-Ethyl-8-methoxyquinolin-5-yl)-5-methyl-2-[4-[2,3-difluoro-4-(4,4-dimethyl-5-oxo-4,5-dihydro-1H-pyrazol-3-yl)phenoxy]butyl]-4,5-dihydro-2H-pyridazin-3-one

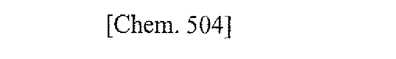

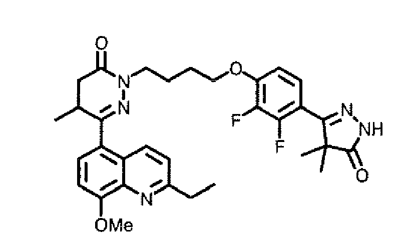

The reaction was carried out in the same manner as in Example 267 using the compound of Example 152 and the compound of Example 230 to obtain the desired product as a white solid.
HREIMS (+): 592.27256 (Calculated value as C₃₂H₃₆F₂N₅O₄ 592.27353).
¹H NMR (CDCl₃, 400 MHz): δ 1.17 (3H, d, J=7.3 Hz), 1.39-1.42 (9H, m), 1.96 (4H, s), 2.55 (1H, dd, J=16.5, 3.7 Hz), 2.86 (1H, dd, J=16.5, 6.7 Hz), 3.09 (2H, q, J=7.6 Hz), 3.23-3.25 (1H, m), 3.86-3.87 (1H,m), 4.07-4.18 (3H, m), 4.12 (3H, s), 6.78-6.80 (1H, m), 7.05 (1H, d, J=8.6 Hz), 7.31-7.36 (1H, m), 7.43 (1H, d, J=8.6 Hz), 7.49 (1H, d, J=8.6 Hz), 8.51 (1H, s), 8.58 (1H, d, J=8.6 Hz).

### <Example 304>

### 2-[4-[2,3-Difluoro-4-(4-methyl-6-oxo-1,4,5,6-tetrahydropyridazin-3-yl)phenoxy]butyl]-6-(5-methoxy-2-trifluoromethyl-[1,2,4]triazolo[1,5-a]pyridin-8-yl)-2H-pyridazin-3-one

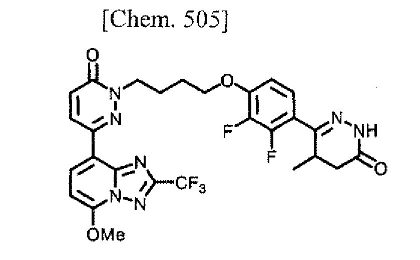

The reaction was carried out in the same manner as in Example 267 using the compound of Example 153 and the compound of Example 251 to obtain the desired product as a pale yellow solid.
HRESIMS (+): 606.18762 (Calculated value as C₂₇H₂₅F₆N₇O₄ 606.18882).
¹H NMR (CDCl₃, 400 MHz): δ 1.20 (3H, d, J=7.3 Hz), 1.95-1.97 (2H, m), 2.11-2.13 (2H, m), 2.44 (1H, dd, J=16.5, 3.1 Hz), 2.74 (1H, dd, J=16.5, 6.7 Hz), 3.24-3.26 (1H, m), 4.15 (2H, t, J=6.1 Hz), 4.29 (3H, s), 4.37 (2H, t, J=7.0 Hz), 6.63 (1H, d, J=8.6 Hz), 6.75-6.79 (1H, m), 7.09 (1H, d, J=9.8 Hz), 7.27-7.29 (1H, m), 8.37 (1H, d, J=8.6 Hz), 8.48 (1H, s), 8.73 (1H, d, J=9.8 Hz).

### <Example 305>

### 2-[4-[2,3-Difluoro-4-(6-oxo-1,4,5,6-tetrahydropyridazin-3-yl)phenoxy]butyl]-6-(5-methoxy-2-trifluoromethyl-[1,2,4]triazolo[1,5-a]pyridin-8-yl)-2H-pyridazin-3-one

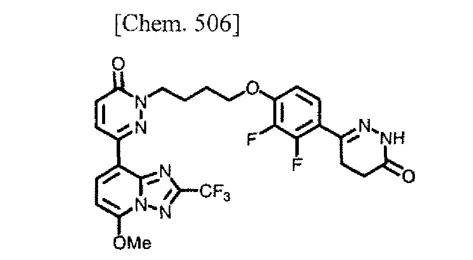

The reaction was carried out in the same manner as in Example 267 using the compound of Example 153 and the compound of Example 213 to obtain the desired product as a white solid.
HRESIMS (+): 592.17271 (Calculated value as C₂₆H₂₃F₅N₇O₄ 592.17317).
¹H NMR (CDCl₃, 400 MHz): δ 1.92-1.99 (2H, m), 2.09-2.17 (2H, m), 2.59 (2H, t, J=7.9 Hz), 2.95-3.00 (2H, m), 4.15 (2H, t, J=6.1 Hz), 4.29 (3H, s), 4.37 (2H, t, J=7.0 Hz), 6.63 (1H, d, J=8.6 Hz), 6.74-6.78 (1H, m), 7.09 (1H, d, J=9.8 Hz), 7.27-7.32 (1H, m), 8.36 (1H, d, J=8.6 Hz), 8.48 (1H, s), 8.73 (1H, d, J=9.8 Hz).

### <Example 3 06>

### 2-[4-[2,3-Difluoro-4-(4,4-dimethyl-5-oxo-4,5-dihydro-1H-pyrazol-3-yl)phenoxy]butyl]-6-(5-methoxy-2-trifluoromethyl-[1,2,4]triazolo[1,5-a]pyridin-8-yl)-2H-pyridazin-3-one

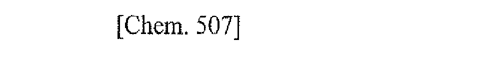

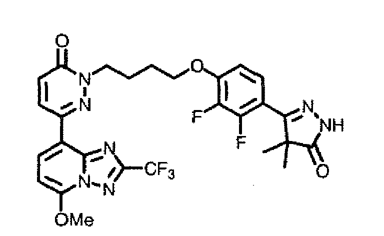

The reaction was carried out in the same manner as in Example 267 using the compound of Example 153 and the compound of Example 230 to obtain the desired product as a white solid.
Elemental analysis: Found value C 53.49%, H 3.99%, N 15.83%, Calculated value as C₁₆H₁₄F₃N₃O₂ C 53.56%, H 4.00%, N 16.20%.
HRESIMS (+): 606.18769 (Calculated value as C₂₇H₂₅F₅N₇O₄ 606.18882).
¹H NMR (CDCl₃, 400 MHz): δ 1.40 (3H, s), 1.40 (3H, s), 1.96-1.98 (2H, m), 2.12-2.14 (2H, m), 4.17 (2H, t, J=6.1 Hz), 4.29 (3H, s), 4.38 (2H, t, J=7.3 Hz), 6.63 (1H, d, J=8.6 Hz), 6.78-6.81 (1H, m), 7.09 (1H, d, J=9.8 Hz), 7.32-7.34 (1H, m), 8.37 (1H, d, J=8.6 Hz), 8.48 (1H, s), 8.73 (1H, d, J=9.8 Hz).

### <Example 307>

### 2-[4-[2,3-Difluoro-4-(4-methyl-6-oxo-1,4,5,6-tetrahydropyridazin-3-yl)phenoxy]butyl]-6-(4-methoxy-2-trifluoromethylbenzothiazol-7-yl)-2H-pyridazin-3-one

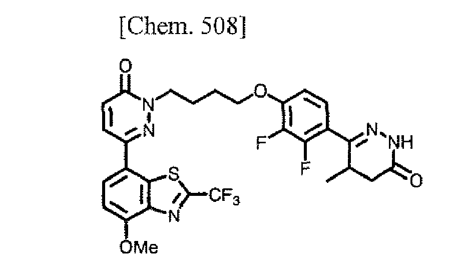

The reaction was carried out in the same manner as in Example 267 using the compound of Example 154 and the compound of Example 251 to obtain the desired product as a white solid.
Elemental analysis: Found value C 54.24%, H 3.90%, N 10.93%, Calculated value as C₂₈H₂₄F₅N₃O₄S C 54.10%, H 3.89%, N 11.27%.
¹H NMR (CDCl₃, 400 MHz): δ 1.20 (3H, d, J=6.7 Hz), 1.96-2.03 (2H, m), 2.18-2.25 (2H, m), 2.44 (1H, dd, J=17.1, 3.1 Hz), 2.74 (1H, dd, J=17.1, 6.7 Hz), 3.25-3.26 (1H, m), 4.16 (3H, s), 4.17 (2H, t, J=6.7 Hz), 4.47 (2H, t, J=7.0 Hz), 6.74-6.78 (1H, m), 7.11 (1H, d, J=9.8 Hz), 7.13 (1H, d, J=8.6 Hz), 7.26-7.30 (1H, m), 7.88 (1H, d, J=8.6 Hz), 7.90 (1H, d, J=9.8 Hz), 8.47 (1H, s).

### <Example 308>

### 2-[4-[2,3-Difluoro-4-(6-oxo-1,4,5,6-tetrahydropyridazin-3-yl)phenoxy]butyl]-6-(4-methoxy-2-trifluoromethylbenzothiazol-7-yl)-2H-pyridazin-3-one

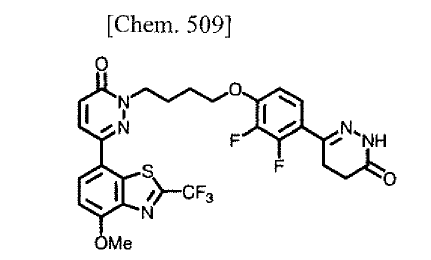

The reaction was carried out in the same manner as in Example 267 using the compound of Example 154 and the compound of Example 213 to obtain the desired product as a white solid.
Elemental analysis: Found value C 53.56%, H 3.61%, N 11.33%, Calculated value as C₂₇H₂₂F₅N₃O₄S C 53.38%, H 3.65%, N 11.53%.
¹H NMR (CDCl₃, 400 MHz): δ 1.98-2.00 (2H, m), 2.21-2.22 (2H, m), 2.58-2.60 (2H, m), 2.97-2.99 (2H, m), 4.16 (3H, s), 4.17 (2H, t, J=6.7 Hz), 4.46 (2H, t, J=7.0 Hz), 6.74-6.76 (1H, m), 7.11 (1H, d, J=9.8 Hz), 7.12 (1H, d, J=8.6 Hz), 7.27-7.32 (1H, m), 7.88 (1H, d, J=8.6 Hz), 7.90 (1H, d, J=9.8 Hz), 8.47 (1H, s).

### <Example 309>

### 2-[4-[2,3-Difluoro-4-(4,4-dimethyl-5-oxo-4,5-dihydro-1H-pyrazol-3-yl)phenoxy]butyl]-6-(4-methoxy-2-trifluoromethylbenzothiazol-7-yl)-2H-pyridazin-3-one

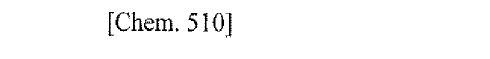

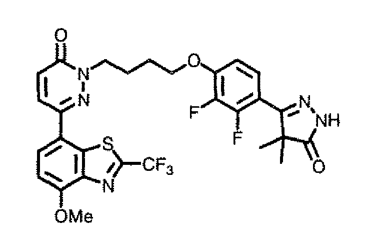

The reaction was carried out in the same manner as in Example 267 using the compound of Example 154 and the compound of Example 230 to obtain the desired product as a white solid.
HRESIMS (+): 622.15899 (Calculated value as C₂₈H₂₅F₅N₅O₄S 622.15474).
¹H NMR (CDCl₃, 400 MHz): δ 1.40 (3H, s), 1.40 (3H, s), 1.99-2.01 (2H, m), 2.22-2.24 (2H, m), 4.16 (3H, s), 4.19 (2H, t, J=6.4 Hz), 4.47 (2H, t, J=7.0 Hz), 6.79-6.81 (1H, m), 7.11 (1H, d, J=9.8 Hz), 7.13 (1H, d, J=8.6 Hz), 7.32-7.34 (1H, m), 7.88 (1H, d, J=8.6 Hz), 7.90 (1H, d, J=9.8 Hz), 8.43 (1H, s).

### <Example 310>

### 6-(7-Methoxy-2-trifluoromethylbenzofuran-4-yl)-5-methyl-2-[4-[4-(4-methyl-6-oxo-1,4,5,6-tetrahydropyridazin-3-yl)phenoxy]butyl]-2H-pyridazin-3-one

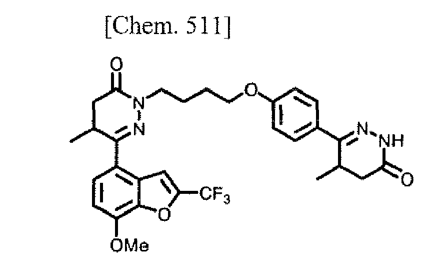

The reaction was carried out in the same manner as in Example 267 using the compound of Example 155 and the compound of Example 167 to obtain the desired product as a colorless amorphous.
HRFABMS (+): 585.2312 (Calculated value as C₃₀H₃₂F₃N₄O₅ 585.2325).
(as a 1:1 diastereomer mixture)
¹H NMR (CDCl₃, 400 MHz): δ 1.23 (3H, d, J=7.3 Hz), 1.25 (3H, d, J=7.3 Hz), 1.87-1.99 (4H, m), 2.45 (1H, d, J=17.1 Hz), 2.53 (1H, dd, J=17.1, 1.8 Hz), 2.67-2.77 (2H, m), 3.28-3.42 (2H, m), 3.94-3.96 (1H, m), 4.08-4.13 (3H, m), 4.08 (3H, s), 6.89 (2H, d, J=8.0 Hz), 6.94 (1H, d, J=8.0 Hz), 7.46 (1H, d, J=8.0 Hz), 7.65 (2H, d, J=8.0 Hz), 7.90 (1H, s), 8.43 (1H, brs).

### <Example 311>

### 6-(7-Methoxy-2-trifluoromethylbenzo[b]thiophen-4-yl)-5-methyl-2-[4-[4-(4-methyl-6-oxo-1,4,5,6-tetrahydropyridazin-3-yl)pbenoxy]butyl]-2H-pyridazin-3-one

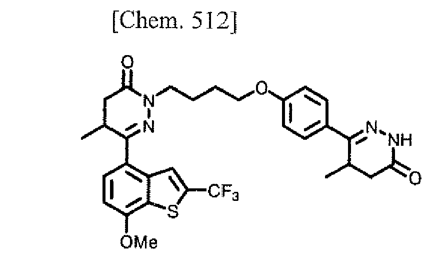

The reaction was carried out in the same manner as in Example 267 using the compound of Example 156 and the compound of Example 167 to obtain the desired product as a colorless powder.
Elemental analysis: Found value C 59.46%, H 5.28%, N 9.42%, Calculated value as C₃₀H₃₁F₃N₄O₄S · 1/5H₂O C 59.63%, H 5.24%, N 9.27%.
FARMS (+): 601 [M+H]⁺.
¹H NMR (CDCl₃, 400 MHz): δ 1.24 (6H, d, J=8.0 Hz), 1.25 (6H, d, J=7.3 Hz), 1.88-2.00 (8H, m), 2.43-2.56 (4H, m), 2.67-2.80 (4H, m), 3.30-3.41 (4H, m), 3.92-4.10 (4H, m), 4.07 (6H, s), 6.90 (4H, d, J=8.6 Hz), 6.90 (2H, d, J=8.6 Hz), 7.59 (2H, d, J=8.0 Hz), 7.65 (4H, d, J=8.6 Hz), 8.43 (2H, brs), 8.63 (1H, s, diastereomer), 8.64 (1H, s, diastereomer).

### <Example 312>

### 6-(2-Ethyl-8-melhoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

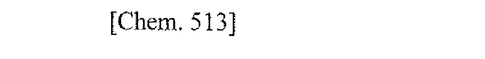

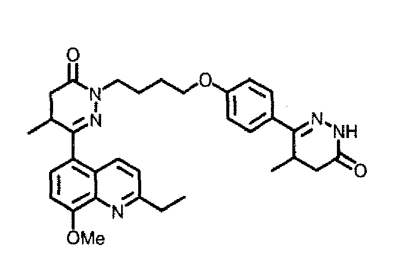

The reaction was carried out in the same manner as in Example 267 using the compound of Example 152 and the compound of Example 167 to obtain the desired product as a colorless amorphous.
HRFABMS (+): 556.2924 (Calculated value as C₃₂H₃₈N₅O₄ 556.2924).
(as a 1:1 diastereomer mixture)
¹H NMR (CDCl₃, 400 MHz): δ 1.17 (3H, d, J=6.7 Hz), 1.24 (3H, d, J=7.3 Hz), 1.40 (3H, t, J=7.3 Hz), 1.90-1.96 (4H, m), 2.46 (1H, d, J=17.1 Hz), 2.56 (1H, dd, J=17.1, 3.8 Hz), 2.70 (1H, dd, J=17.1, 6.7 Hz), 2.86 (1H, dd, J=16.5, 6.7 Hz), 3.08 (2H, q, J=7.3 Hz), 3.21-3.34 (2H, m), 3.83-3.90 (1H, m), 4.05-4.12 (1H, m), 4.12 (3H, s), 6.92 (2H, d, J=9.2 Hz), 7.06 (1H, d, J=8.0 Hz), 7.40 (1H, d, J=8.6 Hz), 7.49 (1H, d, J=8.0 Hz), 7.67 (2H, d, J=9.2 Hz), 8.49 (1H, brs), 8.59 (1H, d, J=8.6 Hz).

### <Example 313>

### 6-(4-Methoxy-2-trifluoromethylbenzothiazol-7-yl)-5-methyl-2-[4-[4-(4-methyl-6-oxo-1,4,5,6-tetrahydropyridazin-3-yl)phenoxy]butyl]-4,5-dihydro-2H-pyridazin-3-one

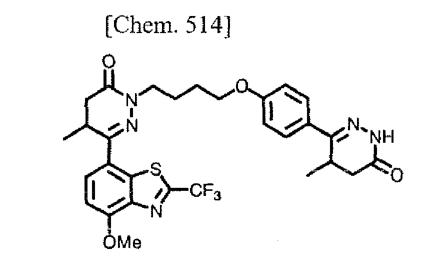

The reaction was carried out in the same manner as in Example 267 using the compound of Example 157 and the compound of Example 167 to obtain the desired product as a colorless powder.
HRFABMS (+): 602.2085 (Calculated value as C₂₉H₃₁F₃N₅O₄S 602.2049).
(as a 1:1 diastereomer mixture)
¹H NMR (CDCl₃, 400 MHz): δ 1.23 (3H, d, J=7.3 Hz), 1.28 (3H, d, J=7.3 Hz), 1.87-1.94 (4H, m), 2.45 (1H, d, J=17.1 Hz), 2.59 (1H, dd, J=17.1, 1.3 Hz), 2.68-2.78 (2H, m), 3.28-3.34 (1H, m), 3.48-3.54 (1H, m), 4.03-4.15 (4H, m), 4.1 (3H, s), 6.88-6.90 (2H, m), 7.08 (1H, d, J=8.6 Hz), 7.64-7.66 (2H, m), 7.75 (1H, d, J=8.6 Hz), 8.41 (1H, brs).

### <Example 314>

### 6-(4-Methoxy-2-trifluoromethyl-1H-benzimidazol-7-yl)-5-methyl-2-[4-[4-(4-methyl-6-oxo-1,4,5,6-tetrahydropyridazin-3-yl)phenoxy]butyl]-4,5-dihydro-2H-pyridazin-3-one

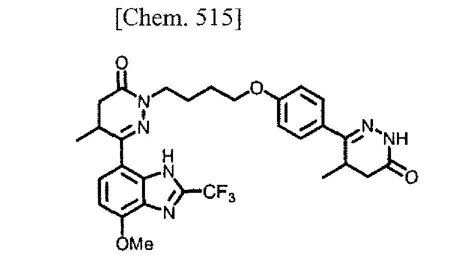

The reaction was carried out in the same manner as in Example 277 using the compound of Example 159 and the compound of Example 167 to obtain the desired product as a colorless powder.
HRFABMS (+): 585.2428 (Calculated value as C₂₉H₃₂F₃N₆O₄ 585.2437).
(as a 1:1 diastereomer mixture)
¹H NMR (CDCl₃, 400 MHz): δ 1.21-1.29 (6H, m), 1.89-2.02 (4H, m), 2.43-2.50 (1H, m), 2.55-2.59 (1H, m), 2.67-2.78 (2H, m), 3.28-3.35 (1H, m), 3.47-3.51 and 5.22-5. 37 (1H, m), 3.99-4.14 (4H, m), 4.11(3H, s), 6.81 (1H, d, J=8.6 Hz), 6.86-6.89 (2H, m), 7.55 (1H, d, J=8.6 Hz), 7.63-7.70 (2H, m), 8.41 (1H, brs), 11.4 (1H, brs).

### <Example 315>

### 6-(2-Ethyl-8-methoxyquinolin-5-yl)-5-methyl-2[2-[4-(4-methyl-6-oxo-1,4,5,6-tetrahydropyridazin-3-yl)phenoxy]ethyl]-4,5-dihydro-2H-pyridazin-3-one

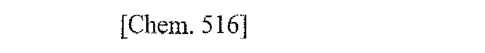

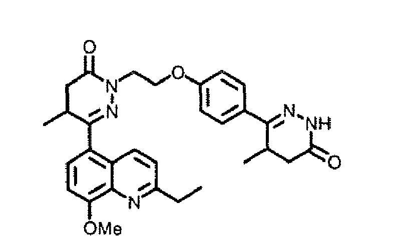

The obtained compound was allowed to react in the same manner as in Example 292 using the compound of Example 72 and the compound of Example 243 to obtain the desired product as a colorless powder.
HRESIMS (+): 528.25850 (Calculated value as C₃₀H₃₄N₅O₄ 528.26108).
(as a 1:1 diastereomer mixture)
¹H NMR (CDCl₃, 400 MHz): δ 1.15 (3H, d, J=7.3 Hz), 1.25 (3H, d, J=7.3 Hz), 1.38 (3H, t, J=7.3 Hz), 2.46 (1H, d, J=16.5 Hz), 2.59 (1H, dd, J=116.5, 3.1 Hz), 2.70 (1H, dd, J=16.5, 6.7 Hz), 2.89 (1H, dd, J=16.5, 6.7 Hz), 3.06 (2H, q, J=7.3 Hz), 3.22-3.26 (1H, m), 3.30-3.35 (1H, m), 4.11 (3H, s), 4.11-4.16 (1H, m), 4.33-4.40 (2H, m), 4.52-4.57 (1H, m), 6.98 (2H, d, J=9.2 Hz), 7.05 (1H, d, J=8.6 Hz), 7.26 (1H, d, J=8.6 Hz), 7.49 (1H, d, J=8.6 Hz). 7.71 (2H, d, J=9.2 Hz), 8.46 (1H, brs), 8.63 (1H, d, J=8.6 Hz).

### <Example 316>

### 6-(8-Methoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

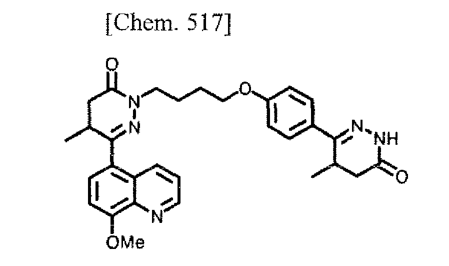

The reaction was carried out in the same manner as in Example 267 using the compound of Example 160 and the compound of Example 167 to obtain the desired product as a colorless amorphous.
HRESIMS (+): 528.25930 (Calculated value as C₃₀H₃₃N₅O₄ 528.26108).
¹H NMR (CDCl₃, 400 MHz): δ 1.17 (3H, d, J=7.4 Hz), 1.24 (3H, d, J=7.4 Hz), 1.86-1.95 (3H, d, J=17.1 Hz), 2.56 (1H, dd. J=16.5, 3.6 Hz), 2.70 (1H, dd, J=17.1, 6.7 Hz), 2.87 (1H, dd, J=16.5, 6.7 Hz), 3.20-3.36 (2H, m), 3.85-3.91 (1H, m), 4.06-4.11 (3H, m), 4.14 (3H, s), 6.91 (2H, d, J=9.2 Hz), 7.08 (1H, d, J=8.0 Hz), 7.48 (1H, dd, J=8.6, 5.6 Hz), 7.57 (1H, d, J=8.0 Hz), 7.68 (2H, d, J=9.2 Hz), 8.46 (1H, s), 8.69 (1H, dd, J=8.6, 1.8 Hz), 8.97 (1H, dd, J=5.6, 1.8 Hz).

### <Example 317>

### 2-(4-Bromobutyl)-6-(8-methoxyquinolin-5-yl)-3-(2H)-pyridazinone

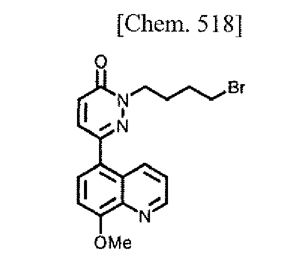

The reaction was carried out in the same manner as in Example 145 using the compound of Example 88 to obtain the desired product as a pale yellow oil.
EIMS (+): 387 [M+].

### <Example 318>

### 6-(8-Methoxyquinolin-5-yl)-2-[4-[4-(4-methyl-6-oxo-1,4,5,6-tetrahydropyridazin-3-yl)phenoxylbutyl]-2H-pyridazin-3-one

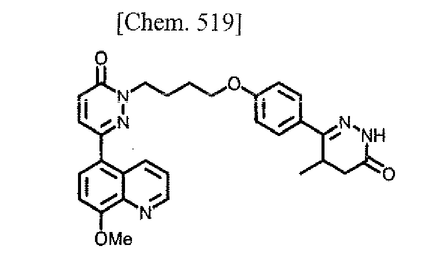

The reaction was carried out in the same manner as in Example 267 using the compound of Example 317 and the compound of Example 167 to obtain the desired product as a pale yellow solid.
HRESIMS (+): 512.22774 (Calculated value as C₂₉H₃₀N₅O₄ 512.22978).
¹H NMR (CDCl₃, 400 MHz): δ 1.23 (3H, d, J=7.4 Hz), 1.90-1.97 (2H, m), 2.08-2.16 (2H, m), 2.45 (1H, d, 1=16.5 Hz), 2.70 (1H, dd. J=16.5, 6.7 Hz), 3.28-3.35 (1H, m), 4.07 (2H, t, J=6.1 Hz), 4.15 (3H, s), 4.37 (2H, t, J=7.4 Hz), 6.91 (2H, d, J=9.2 Hz), 7.07 (1H, d, J=9.8 Hz), 7.13 (1H, d, J=8.0 Hz), 7.47-7.50 (2H, m), 7.58 (2H, d, J=8.0 Hz), 7.66 (1H, d, J=6.7 Hz), 8.42 (1H, s), 8.47 (1H, dd, J=9.2, 1.8 Hz), 8.99 (1H, dd, J=4.3, 1.8 Hz).

### <Example 319>

### 2-[4-[2,3-Difluoro-4-(4-methyl-6-oxo-1,4,5,6-tetrahydropyridazin-3-yl)phenoxy]butyl]-6-(8-methoxyquinolin-5-yl)-2H-pyridazin-3-one

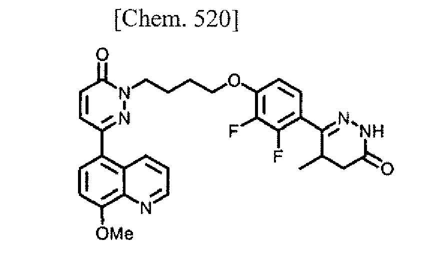

The reaction was carried out in the same manner as in Example 267 using the compound of Example 317 and the compound of Example 251 to obtain the desired product as a colorless amorphous.
HRESIMS (+): 548.20769 (Calculated value as C₂₉H₂₈F₂N₅O₄ 548.21093).
¹H NMR (CDCl₃, 400 MHz): δ 1.20 (3H, d, J=7.4 Hz), 1.93-2.00 (2H, m), 2.09-2.17 (2H, m), 2.44 (1H, dd, J=17.2, 3.1 Hz), 2.74 (1H, dd. J=17.2, 6.7 Hz), 3.22-3.28 (1H, m), 4.13-4.17 (2H, 4.16 (3H, s), 4.37 (2H, t, J=7.4 Hz), 6.76(1H, ddd, J=9.2, 7.3, 1.8 Hz), 7.07 (1H, d, J=8.6 Hz), 7.14 (1H, d, J=8.6 Hz), 7.25-7.28 (1H, m), 7.49 (1H, d, J=8.6 Hz), 7.50 (1H, dd, J=8.6, 4.3 Hz), 7.59 (1H, d, J=8.6 Hz), 8.48 (1H, dd, J=8.6, 1.8 Hz), 8.55 (1H, brs), 9.00 (1H, dd, J=4.3, 1.8 Hz).

### <Example 320>

### 2-[4-[2,3-Difluoro-4-(6-oxo-1,4,5,6-tetrahydropyridazin-3-yl)phenoxy]butyl]-6-(8-methoxyquinolin-5-yl)-2H-pyridazin-3-one

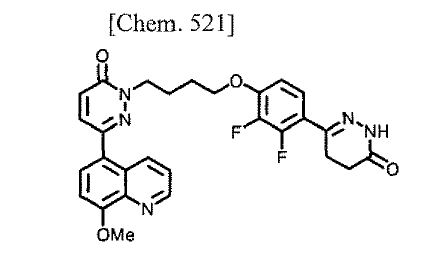

The reaction was carried out in the same manner as in Example 267 using the compound of Example 317 and the compound of Example 213 to obtain the desired product as a white amorphous.
HREIMS (+): 534.19441 (Calculated value as C₂₈H₂₆F₂N₅O₄ 534.19528).
¹H NMR (CDCl₃, 400 MHz): δ 1.93-1.99 (2H, m), 2.09-2.16 (2H, m), 2.59 (1H, dd, J=8.6, 7.9 Hz), 2.98 (2H, ddd, J=7.3, 6.1, 1.8 Hz), 4.13-4.17 (2H, m), 4.15 (3H, s), 4.37 (2H, t, J=7.4 Hz), 6.76 (1H, ddd, J=9.2, 7.3, 1.8 Hz), 7.07 (1H, d, J=8.6 Hz), 7.13 (1H, d, J=8.6 Hz), 7.32 (1H, ddd, J=7.3, 6.8, 1.8 Hz), 7.49 (1H, d, J=8.6 Hz), 7.50 (1H, dd, J=8.6, 4.3 Hz), 7.59 (1H, d, J=8.6 Hz), 8.48 (1H, dd, J=8.6, 1.8 Hz), 8.50 (1H, brs), 9.00 (1H, dd, J=4.3, 1.8 Hz).

### <Example 321>

### 6-(8-Methoxy-2-trifluoromethylquinotin-5-yl)-2-[4-[4-(4-methyl-6-oxo-1,4,5,6-tetrahydropyridazin-3-yl)phenoxy]butyl]-2H-pyridazin-3-one

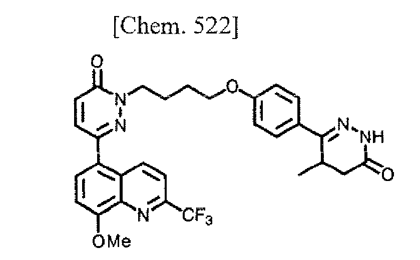

The reaction was carried out in the same manner as in Example 267 using the compound of Example 161 and the compound of Example 167 to obtain the desired product as a white solid.
HRFABMS (+): 580.2204 (Calculated value as C₃₀H₂₈F₃N₅O₄ 580.2172).
¹H NMR (CDCl₃, 400 MHz): δ 1.24 (3H, d, J=7.3 Hz), 1.90-1.97 (2H, m), 2.08-2.18 (2H, m), 2.46 (1H, d, J=16.5 Hz), 2.71 (1H, dd, J=16.5, 6.7 Hz), 3.30-3.33 (1H, m), 4.07 (2H, t, J=6.1 Hz), 4.16 (3H, s), 4.37 (2H, t, J=7.3 Hz), 6.90 (2H, d, J=9.2 Hz), 7.10 (1H, d, J=9.8 Hz), 7.21 (1H, d, J=8.6 Hz), 7.50 (1H, d, J=9.8 Hz), 7.66 (2H, d, J=9.2 Hz), 7.70 (1H, d, J=8.6 Hz), 7.80 (1H, J=8.6 Hz), 8.43 (1H, brs), 8.74 (1H, d, J=8.6 Hz).

### <Examples 322 and 323>

### (+)-6-(2-Ethyl-8-methoxyquinolin-5-yl)-2-[4-[2,3-difluoro-4-(4-methyl-6-oxo-1,4,5,6-tetrahydropyridazin-3-yl)phenoxy]butyl]-2H-pyridazin-3-one and (-)-6-(2-ethyl-8-methoxyquinolin-5-yl)-2-[4-[2,3-difluoro-4-(4-methyl-6-oxo-1,4,5,6-tetrahydropyridazin-3-yl)phenoxy]butyl]-2H-pyridazin-3-one

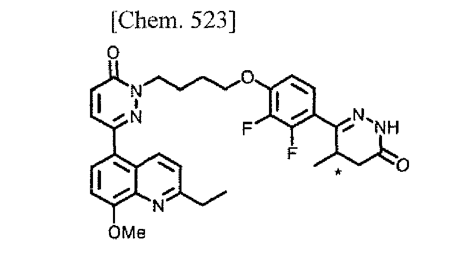

The compound of Example 289 was subjected to optical resolution by high performance liquid chromatography.
Resolution condition: Column: DAICEL CHIRALCEL (registered trademark) OJ
Developing solvent: ethanol
(+)-6-(2-ethyl-8-methoxyquinolin-5-yl)-2-[4-[2,3-difluoro-4-(4-methyl-6-oxo-1,4,5,6-tetrahydropyridazin-3-yl)phenoxylbutyl]-2H-pyridazin-3-one (Example 322)
White powder,
Optical rotation: [α]_{D}²² +89.3 (c 0.42, CHCl₃).
Elemental analysis: Found value C 64.35%, H 5.42%, N 12.09%, Calculated value as C₃₁H₃₁F₂N₅O₄ C 64.69%, H 5.43%, N 12.17%.
(-)-6-(2-Ethyl-8-methoxyquinolin-5-yl)-2-[4-[2,3-difluoro-4-(4-methyl-6-oxo-1,4,5,6-tetrahydropyridazin-3-yl)phenoxy]butyl]-2H-pyridazin-3-one (Example 323)
White powder,
Optical rotation: [a]_{D}²² -87.1 (c 0.42, CHCl₃).
Elemental analysis: Found value C 64.45%, H 5.41%, N 12.06%, Calculated value as C₃₁H₃₁F₂N₅O₄ C 64.69%, H 5.43%, N 12.17%.

### <Example 324>

### 6-(2-Ethyl-8-methoxyquinolin-5-yl)-2-[2-[2,3-difluoro-4-(4-methyl-6-oxo-1,4,5,6-tetrahydropyridazin-3-yl)phenoxy]ethyl]-2H-pyridazin-3-one

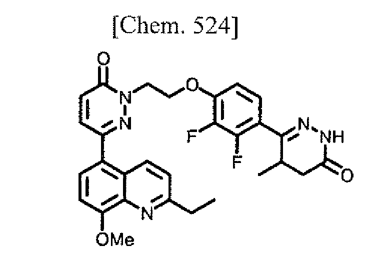

The compound of Example 95 (100 mg) was dissolved in DMF (3.0 mL) under an argon atmosphere, and 60% sodium hydride (16.3 mg) was added thereto at 0°C, followed by stirring at room temperature for 30 minutes. A solution of the compound of Example 252 (211 ing) in DMF (0.5 mL) was added thereto at 0°C, followed by stirring at room temperature for 2 hours. To the reaction liquid was added a saturated aqueous ammonium chloride solution, followed by extraction three times with ethyl acetate. The obtained extracted layer was washed with saturated brine chloride, dried over anhydrous sodium sulfate, and filtered. After evaporating the solvent of the obtained filtrate under reduced pressure, the residue was dissolved in dichloromethane (3.0 mL), and trifluoroacetic acid (1.0 mL) was added thereto, followed by stirring at room temperature for 1 hour. To the reaction liquid was added a saturated aqueous sodium hydrogen carbonate solution, followed by extraction three times with ethyl acetate. The obtained extracted layer was washed with saturated brine, dried over anhydrous sodium sulfate, and filtered. After evaporating the solvent of the obtained filtrate under reduced pressure, the residue was purified by silica gel column chromatography (ethyl acetate:methanol=20:1) to obtain the desired product (135 mg) as a yellow amorphous.
¹H NMR (CDCl₃, 400 MHz): δ 1.20 (3H, d, J=7.3 Hz), 1.41 (3H, t, J=7.6 Hz), 2.44 (1H, dd, J=17.1, 3.1 Hz), 2.74 (1H, dd, J=17.1, 6.7 Hz), 3.10 (2H, q, J=7.6 Hz), 3.24-3.26 (1H, m), 4.13 (3H, s), 4.61 (2H, t, J=5.5 Hz), 4.72 (2H, t, J=5.5 Hz), 6.82-6.87 (1H, m), 7.08 (1H, d, J=9.2 Hz), 7.11 (1H, d, J=7.9 Hz), 7.30-7.32 (1H, m), 7.40 (1H, d, J=7.9 Hz), 7.50 (1H, d, J=7.9 Hz), 7.54 (1H, d, J=7.9 Hz), 8.49 (1H, d, J=9.2 Hz), 8.50 (1H, brs).
HREIMS (+): 548.20859 (Calculated value as C₂₉H₂₈F₂N₅O₄ 548.21093).

### <Example 325>

### 6-(2-Ethyl-8-methoxyquinolin-5-yl)-2-[3-[2,3-difluoro-4-(4-methyl-6-oxo-1,4,5,6-tetrahydropyridazin-3-yl)phenoxy]propyl]-2H-pyridazin-3-one

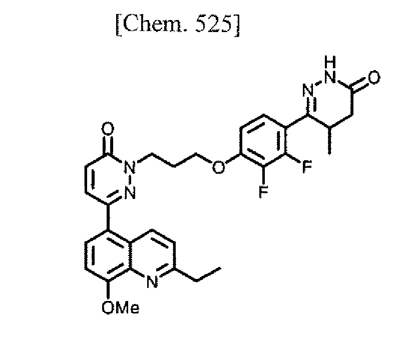

¹H NMR (CDCl₃, 400 MHz): δ 1.19 (3H, d, J=7.3 Hz), 1.40 (3H, t, J=7.6 Hz), 2.43-2.48 (3H, m), 2.76 (1H, dd, J=17.1, 6.7 Hz), 3.09 (2H, q, J=7.5 Hz), 3.22-3.24 (1H, m), 4.12 (3H, s), 4.24 (2H, t, J=6.1 Hz), 4.52 (2H, t, J=6.7 Hz), 6.71-6.76 (1H, m), 7.05 (1H, d, J=9.8 Hz), 7.08 (1H, d, J=7.9 Hz), 7.22-7.24 (1H, m), 7.37 (1H,d. J=8.6 Hz), 7.46 (1H, d, J=7.9 Hz), 7.47 (1H, d, J=7.9 Hz), 7.49 (1H, d, J=9.8 Hz), 8.37 (1H, d, J=8.6 Hz), 8.47 (1H, s).
HRESIMS (+): 562.22710 (Calculated value as C₃₀H₃₀F₂N₅O₄ 562.22658).

### <Example 326>

### 6-(2-Ethyl-8-methoxyquinolin-5-yl)-2-[3-[2,3-difluoro-4-(6-oxo-1,4,5,6-tetrahydropyridazin-3-yl)phenoxy]propyl]-2H-pyridazin-3-one

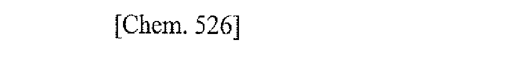

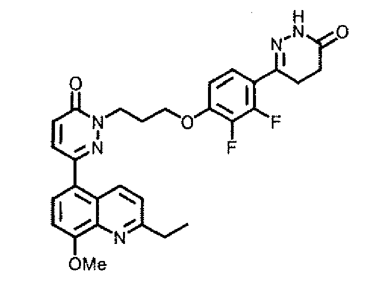

The reaction was carried out in the same manner as in Example 267 using the compound of Example 162 and the compound of Example 213 to obtain the desired product as a colorless powder.
¹H NMR (CDCl₃, 400 MHz): δ 1.40 (3H, t, J=7.6 Hz), 2.45-2.46 (2H, m), 2.60 (2H, t, J=8.3 Hz), 2.95-2.96 (2H, m), 3.09 (2H, q, J=7.7 Hz), 4.12 (3H, s), 4.23 (2H, t, J=5.8 Hz), 4.52 (2H, t, J=6.7 Hz), 6.71-6.73 (1H, m), 7.05 (1H, d, J=9.8 Hz), 7.07 (1H, d, J=9.2 Hz), 7.23-7.25 (1H, m), 7.36 (1H, d, J=8.6 Hz), 7.46 (1H, d, J=8.6 Hz), 7.49 (1H, d, J=9.8 Hz), 8.37 (1H, d, J=9.2 Hz), 8.49 (1H, s).
HRESIMS (+): 548.20862 (Calculated value as C₂₉H₂₈F₂N₅O₄ 548.21093).

### <Example 327>

### 6-(2-Ethyl-8-methoxyquinolin-5-yl)-2-[3-[2,3-difluoro-4-(4,4-dimethyl-5-oxo-4,5-dihydro-1H-pyrazol-3-yl)phenoxy]propyl]-2H-pyridazin-3-one

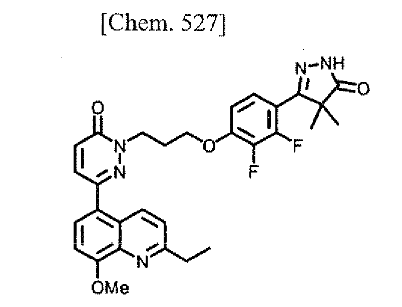

The reaction was carried out in the same manner as in Example 267 using the compound of Example 162 and the compound of Example 230 to obtain the desired product as a colorless powder.
¹H NMR (CDCl₃, 400 MHz): δ 1.39 (6H, s), 1.40 (3H, t, J=7.6 Hz), 1.40 (3H, t, J=7.6 Hz), 2.47-2.48 (2H, m), 3.09 (2H, q, J=7.6 Hz), 4.12 (3H, s), 4.26 (2H, t, J=5.8 Hz), 4.52 (2H, t, J=6.7 Hz), 6.76-6.78 (1H, m), 7.06 (1H, d, J=9.8 Hz), 7.08 (1H, d, J=8.6 Hz), 7.28-7.33 (1H, m), 7.38 (1H, d, J=9.2 Hz), 7.48 (1H, d, J=9.2 Hz), 7.50 (1H, d, J=9.8 Hz), 8.38 (1H, d, J=8.6 Hz), 8.57 (1H, s).
HRESIMS (+): 562.22323 (Calculated value as C₃₀H₃₀F₂N₅O₄ 562.22658).

### <Example 328>

### (+)-6-(2-Ethyl-8-methoxyquinolin-5-yl)-2-[4-[2-fluoro-4-(4-methyl-6-oxo-1,4,5,6-tetrahydropyridazin-3-yl)phenoxy]butyl]-2H-pyridazin-3-one

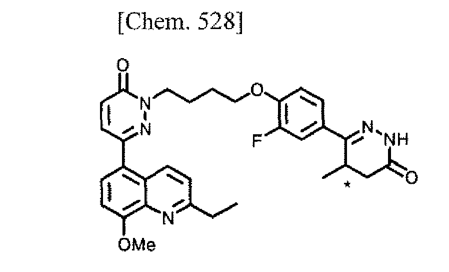

The reaction was carried out in the same manner as in Example 267 using the compound of Example 147 and the compound of Example 262 to obtain the desired product as a colorless amorphous.

Further, the optical purity was determined by means of HPLC using a chiral column. The separation condition was as follows.
Column: Daicel CHIRALCEL (registered trademark) OJ column
Developing solvent: ethanol/hexane=90/10
Flow rate: 0.5 mL/min
Detection: UV (293 nm)
Retention time: t=50.4 minutes (93%ee)
Optical rotation: [α]_{D}²⁵ +170 (c 0.75, CHCl₃).
¹H NMR (CDCl₃, 400 MHz): δ 1.23 (3H, d, J=7.3 Hz), 1.41 (3H, t, J=7.3 Hz), 1.91-2.03 (2H, m), 2.08-2.19 (2H, m), 2.46 (1H, d, J=17.1 Hz), 2.70 (1H, dd, J=17.1, 7.3 Hz), 3.10 (2H, q, J=7.3 Hz), 3.21-3.33 (1H, m), 4.13 (3H, s), 4.15 (2H, t, J=6.7 Hz), 4.37 (2H, t, J=7.3 Hz), 6.95 (1H, t, J=8.6 Hz), 7.06 (1H, d, J=9.2 Hz), 7.10 (1H, d, J=8.6 Hz), 7.38 (1H, d, J=8.6 Hz), 7.42 (1H, d, J=9.2 Hz), 7.49 (1H, d, J=9.2 Hz), 7.50-7.56 (1H, m), 7.51 (1H, d, J=8.6 Hz), 8.39 (1H, d, J=8.6 Hz), 8.45 (1H, brs).
HRESIMS (+): 558.25171 (Calculated value as C₃₁H₃₂FN₅O₄ 558.25166).

### <Example 329>

### (-)-6-(2-Ethyl-8-methoxyquinolin-5-yl)-2-[4-[2-fluoro-4-(4-methyl-6-oxo-1,4,5,6-tetrahydropyridazin-3-yl)phenoxy]butyl]-2H-pyridazin-3-one

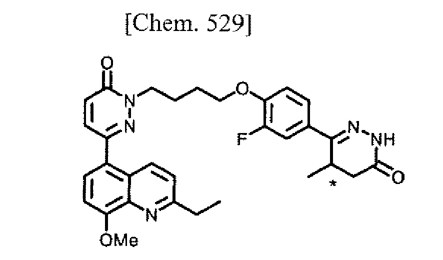

The reaction was carried out in the same manner as in Example 267 using the compound of Example 147 and the compound of Example 263 to obtain the desired product as a colorless amorphous.

Further, the optical purity was determined by means of HPLC using a chiral column. The separation condition was as follows.
Column: Daicel CHIRALCEL (registered trademark) OJ column
Developing solvent: ethanol/hexane=90/10
Flow rate: 0.5 mL/min
Detection: UV (293 nm)
Retention time: t=32.9 minutes (93%ee)
Optical rotation: [α]_{D}²⁵ -164 (c 0.71, CHCl₃).
¹H NMR (CDCl₃, 400 MHz): δ 1.23 (3H, d, J=7.3 Hz), 1.41 (3H, t, J=7.3 Hz), 1.92-2.03 (2H, m), 2.08-2.19 (2H, m), 2.46 (1H, dd, J=17.1, 1.2 Hz), 2.70 (1H, dd, J=17.1, 6.7 Hz), 3.10 (2H, q, J=7.3 Hz), 3.22-3.32 (1H, m), 4.13 (3H, s), 4.15 (2H, t, J=6.1 Hz), 4.37 (2H, t, J=7.3 Hz), 6.96 (1H, t, J=8.6 Hz), 7.06 (1H, d, J=9.2 Hz), 7.10 (1H, d, J=7.9 Hz), 7.35-7.41 (1H, m), 7.43 (1H, d, J=8.6 Hz), 7.49 (1H, d, J=9.2 Hz), 7.50-7.56 (1H, m), 7.51 (1H, d, J=7.9 Hz), 8.39 (1H, d, J=8.6 Hz), 8.47 (1H, brs).
HRESIMS (+): 558.25110 (Calculated value as C₃₁H₃₂FN₅O₄ 558.25166).

### <Example 330>

### 2-[4-[2,3-Difluoro-4-(4-methyl-6-oxo-1,4,5,6-tetrahydropyridazin-3-yl)phenoxy]butyl]-6-(7-methoxy-2-trifluoromethylbenzo[b]thiophen-4-yl)-2H-pyridazin-3-one

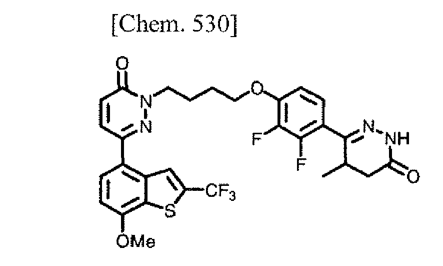

The reaction was carried out in the same manner as in Example 267 using the compound of Example 163 and the compound of Example 251 to obtain the desired product as a white solid.
¹H NMR (CDCl₃, 400 MHz): δ 1.20 (3H, d, J=7.3 Hz), 1.93-2.02 (2H, m), 2.18-2.20 (2H, m), 2.44 (1H, dd, J=17.1, 3.1 Hz), 2.74 (1H, dd, J=17.1, 6.7 Hz), 3.21-3.30 (1H, m), 4.07 (3H, s), 4.15 (2H, t, J=6.4 Hz), 4.38 (2H, t, J=7.3 Hz), 6.71-6.79 (1H, m), 6.96 (1H, d, J=7.9 Hz), 7.07 (1H, d, J=9.8 Hz), 7.24-7.29 (1H, m), 7.56 (1H, d, J=7.9 Hz), 7.62 (1H, d, J=9.8 Hz), 8.20-8.25 (1H, m), 8.50-8.55 (1H, m).
ESIMS (+): 621 [M+H]⁺
Elemental analysis: Found value C 56.00%, H 4.01%, N 8.89%, Calculated value as C₂₉H₂₅F₅N₄O₄S C 56.13%, H 4.06%, N 9.03%.

### <Example 331>

### 2-[4-[2,3-Difluoro-4-(6-oxo-1,4,5,6-tetrahydropyridazin-3-yl)phenoxy]butyl]-6-(7-methoxy-2-trifluoromethylbenzo[b]thiophen-4-yl)-2H-pyridazin-3-one

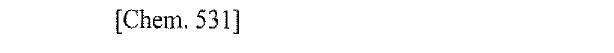

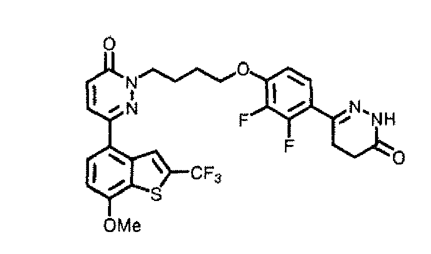

The reaction was carried out in the same manner as in Example 267 using the compound of Example 163 and the compound of Example 213 to obtain the desired product as a white solid.
¹H NMR (CDCl₃, 400 MHz): δ 1.92-2.02 (2H, m), 2.08-2.18 (2H, m), 2.54-2.62 (2H, m), 2.94-3.01 (2H, m), 4.07 (3H, s), 4.15 (2H, t, J=6.1 Hz), 4.38 (2H, t, J=7.3 Hz), 6.75 (1H, ddd, J=16.5, 7.4, 1.8 Hz), 6.96 (1H, d, J=8.6 Hz), 7.07 (1H, d, J=9.2 Hz), 7.25-7.35 (1H, m), 7.56 (1H, d, J=8.6 Hz), 7.63 (1H, d, J=9.2 Hz), 8.23 (1H, s), 8.53 (1H, s).
ESIMS (+): 607 [M+H]⁺.
Elemental analysis: Found value C 55.21%, H 3.67%, N 9.23%, Calculated value as C₂₈H₂₃F₅N₄O₄S C 55.44%, H 3.82%, N 9.24%.

### <Example 332>

### 2-[4-[2,3-Difluoro-4-(4,4-dimethyl-5-oxo-4,5-dihydro-1H-pyrazol-3-yl)phenoxy]butyl]-6-(7-methoxy-2-trifluoromethylbenzo[b]thionophen-4-yl)-2H-pyridazin-3-one

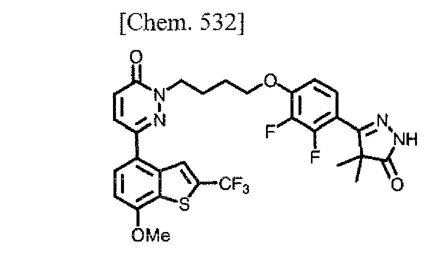

The reaction was carried out in the same manner as in Example 267 using the compound of Example 163 and the compound of Example 230 to obtain the desired product as a white solid.
¹H NMR (CDCl₃, 400 MHz): δ 1.398 (1H, s), 1.401 (1H, s), 1.93-2.03 (2H, m), 2.10-2.19 (2H, m), 4.07 (3H, s), 4.17 (2H, t, J=6.1 Hz), 4.39 (2H, t, J=7.0 Hz), 6.74-6.81 (1H, m), 6.95 (1H, d, J=8.6 Hz), 7.08 (1H, d, J=9.8 Hz), 7.29-7.36 (1H, m), 7.57 (1H, d, J=8.6 Hz), 7.63 (1H, d, J=9.8 Hz), 8.24 (1H, s), 8.66 (1H, s).
ESIMS (+): 621 [M+H]⁺.
Elemental analysis: Found value C 55.71%, H 3.99%, N 8.95%, Calculated value as C₂₉H₂₅F₅N₄O₄S C 56.13%, H 4.06%, N 9.03%.

### <Example 333>

### 6-(2-Ethyl-4-methoxybenzooxazol-7-yl)-2-[4-[2,3-difluoro-4-(6-oxo-1,4,5,6-tetrahydropyridazin-3-yl)phenoxy]butyl]-2H-pyridazin-3-one

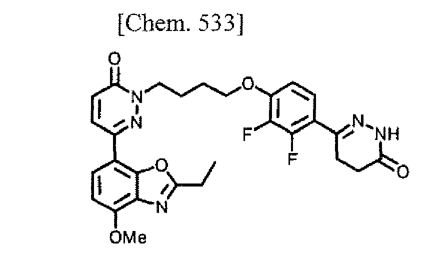

The reaction was carried out in the same manner as in Example 267 using the compound of Example 165 and the compound of Example 213 to obtain the desired product as a white solid.
¹H NMR (CDCl₃, 400 MHz): δ 1.49 (3H, t, J=7.6 Hz), 1.93-2.00 (2H, m), 2.10-2.17 (2H, m), 2.59 (2H, t, J=8.3 Hz), 2.96-3.04 (4H, m), 4.08 (3H, s), 4.15 (2H, t, J=6.1 Hz), 4.36 (2H, t, J=7.0 Hz), 6.73-6.79 (1H, m), 6.88 (1H, d, J=8.6 Hz), 7.05 (1H, d, J=9.2 Hz), 7.25-7.32 (1H, m), 7.78 (1H, d, J=8.6 Hz), 8.00 (1H, d, J=9.2 Hz), 8.54 (1H, s).
ESIMS (+): 552 [M+H]⁺.
Elemental analysis: Found value C 59.26%, H 4.95%, N 12.45%, Calculated value as C₂₈H₂₇F₂N₅O₅·H₂O C 59.05%, H 5.13%, N 12.30%.

### <Example 334>

### 6-(2-Ethyl-4-methoxybenzooxazol-7-yl)-2-[4-[2,3-difluoro-4-(4-methyl-6-oxo-1,4,5,6-tetrahydropyridazin-3-yl)phenoxy]butyl]-2H-pyridazin-3-one

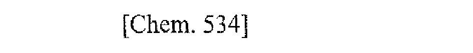

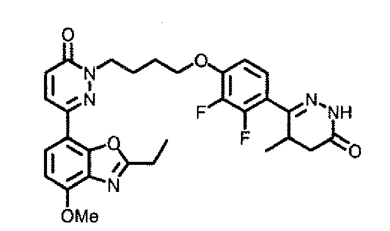

The reaction was carried out in the same manner as in Example 267 using the compound of Example 165 and the compound of Example 251 to obtain the desired product as a white solid.
¹H NMR (CDCl₃, 400 MHz): δ 1.20 (3H, d, J=7.3 Hz), 1.49 (3H, t, J=7.6 Hz), 1.95-1.99 (2H, m), 2.10-2.16 (2H, m), 2.44 (1H, dd, J=17.1, 3.1 Hz), 2.74 (1H, dd, J=17.1, 6.7 Hz), 3.01 (2H, q, J=7.6 Hz), 3.21-3.29 (1H, m), 4.08 (3H, s), 4.16 (2H, t, J=6.1 Hz), 4.37 (2H, t, J=7.0 Hz), 6.73-6.79 (1H, m), 6.88 (1H, d, J=8.6 Hz), 7.06 (1H, d, J=9.8 Hz), 7.23-7.29 (1H, m), 7.79 (1H, d, J=8.6 Hz), 8.00 (1H, d, J=9.8 Hz), 8.50-8.56 (1H, m).
ESIMS (+): 566 [M+H]⁺.
Elemental analysis: Found value C 61.38%, H 5.19%, N 12.10%, Calculated value as C₂₉H₂₉F₂N₅O₅ C 61.59%, H 5.17%, N 12.38%.

### <Example 335>

### 6-(2-Ethyl-4-methoxybenzooxazol-7-yl)-2-[4-[2,3-difluoro-4-(4,4-dimethyl-5-oxo-4,5-dihydro-1H-pyrazol-3-yl)phenoxy]butyl]-2H-pyridazin-3-one

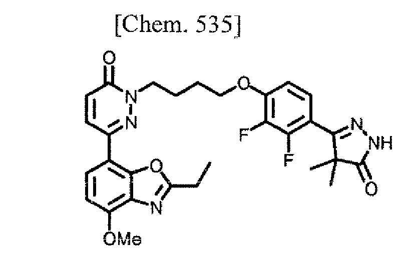

The reaction was carried out in the same manner as in Example 267 using the compound of Example 165 and the compound of Example 230 to obtain the desired product as a white solid.
¹H NMR (CDCl₃, 400 MHz): δ 1.399 (3H, s), 1.402 (3H, s), 1.49 (3H, t, J=7.6 Hz), 1.94-2.01 (2H, m), 2.11-2.18 (2H, m), 3.01 (2H, q, J=7.6 Hz), 4.08 (3H, s), 4.17 (2H, t, J=6.4 Hz), 4.37 (2H, t, J=7.0 Hz), 6.76-6.82 (1H, m), 6.88 (1H, d, J=8.6 Hz), 7.06 (1H, d, J=9.8 Hz), 7.30-7.36 (1H, m), 7.79 (1H, d, J=8.6 Hz), 8.01 (1H, d, J=9.8 Hz), 8.60 (1H, s).
ESIMS (+): 566 [M+H]⁺.
Elemental analysis: Found value C 61.33%, H 5.07%, N 12.32%, Calculated value as C₂₉H₂₉F₂N₅O₅ C 61.59%, H 5.17%, N 12.38%.

### <Example 336>

### 2-[4-[2,3-Difluoro-4-(4-methyl-6-oxo-1,4,5,6-tetrahydropyridazin-3-yl)phenoxyl]butyl]-6-(8-methoxy-2-trifluoromethylimidazo[1,2-a]pyridin-5-yl)-2H-pyridazirdazin-3-one

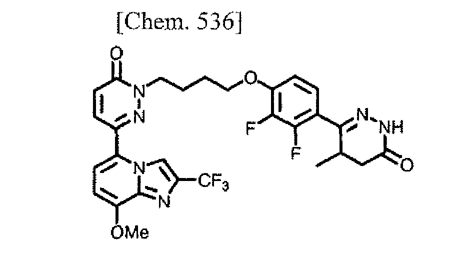

The reaction was carried out in the same manner as in Example 267 using the compound of Example 164 and the compound of Example 251 to obtain the desired product as a white solid.
¹H NMR (CDCl₃, 400 MHz): δ 1.20 (3H, d, J=7.3 Hz), 1.94-2.02 (2H, m), 2.10-2.19 (2H, m), 2.44 (1H, dd, J=17.1, 3.1 Hz), 2.74 (1H, dd, J=17.1, 6.7 Hz), 3.22-3.29 (1H, m), 4.11 (3H, s), 4.16 (2H, t, J=6.1 Hz), 4.42 (2H, t, J=7.0 Hz), 6.70 (1H, d, J=7.9 Hz), 6.72-6.79 (1H, m), 7.11 I (1H, d, J=9.8 Hz), 7.18 (1H, d, J=7.9 Hz), 7.26-7.31 (1H, m), 7.67 (1H, d, J=9.8 Hz), 8.51 (1H, s), 8.93 (1H, d, J=1.2 Hz).
HREIMS (+): 605.1925 (Calculated value as C₂₈H₂₆F₅N₆O₄ 605.1936).

### <Example 337>

### 2-[4-[2,3-Difluoro-4-(6-oxo-1,4,5,6-tetrallydropyridazin-3-yl)phenoxy]butyl]-6-(8-methoxy-2-trifluoromethylimidazo[1,2-a]pyridin-5-yl)-2H-pyridazin-3-one

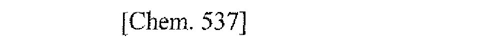

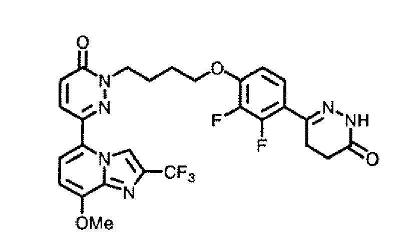

The reaction was carried out in the same manner as in Example 267 using the compound of Example 164 and the compound of Example 213 to obtain the desired product as a white solid.
¹H NMR (CDCl₃, 400 MHz): δ 1.94-2.02 (2H, m), 2.10-2.19 (2H, m), 2.56-2.62 (2H, m), 2.95-3.01 (2H, m), 4.11 (3H, s), 4.15 (2H, t, J=6.1 Hz), 4.41 (2H, t, J=7.0 Hz), 6.70 (1H, d, J=7.9 Hz), 6.73-6.78 (1H, m), 7.11 (1H, d, J=9.8 Hz), 7.18 (1H, d, J=9.8 Hz), 7.27-7.34 (1H, m), 7.67 (1H, d, J=9.8 Hz), 8.52 (1H, s), 8.92 (1H, s).
HRESIMS (+): 591.1748 (Calculated value as C₂₇H₂₄F₅N₆O₄ 591.1779).

### <Example 338>

### 2-[4-[2,3-Difluoro-4-(4,4-dimethyl-5-oxo-4,5-dihydro-1H-pyrazol-3-yl)phenoxy]butyl]-6-(8-methoxy-2-trifluoromethylimidazo[1,2-a]pyridin-5-yl)-2H-pyridazin-3-one

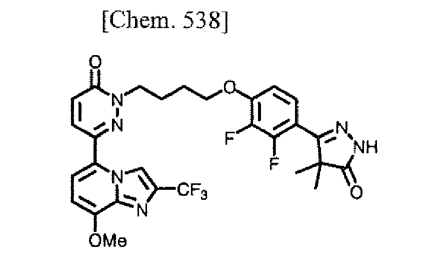

The reaction was carried out in the same manner as in Example 267 using the compound of Example 164 and the compound of Example 230 to obtain the desired product as a white solid.
HRESIMS (+): 605.1961 (Calculated value as C₂₈H₂₆F₅N₆O₄ 605.1936).
¹H NMR (CDCl₃, 400 MHz): δ 1.399 (3H, s), 1.402 (3H, s), 1.96-2.02 (2H, m), 2.13-2.18 (2H, m), 4.11 (3H, s), 4.17 (2H, t, J=6.1 Hz), 4.42 (2H, t, J=7.0 Hz), 6.70 (1H, d, J=8.6 Hz), 6.76-6.81 (1H, m), 7.11 (1H, d, J=9.8 Hz), 7.18 (1H, d, J=8.6 Hz), 7.28-7.38 (1H, m), 7.67 (1H, d, J=9.8 Hz), 8.60 (1H, s), 8.93 (1H, s).

### <Examples 339>

### 6-(2-Ethyl-8-methoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

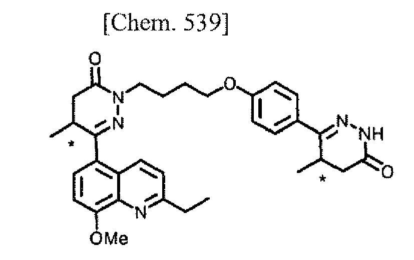

The compound of Example 96 was allowed to react in the same manner in Example 145, and the obtained compound and the compound of Example 255 were then reacted in the same manner as in Example 267 to obtain the desired product as a colorless amorphous. HRESIMS (+); 556.29193 (Calculated value as C₃₂H₃₈N₅O₄ 556.29238).
¹H NMR (CDCl₃, 400 MHz): δ 1.17 (3H, d, J=7.3 Hz), 1.24 (3H, d, J=7.3 Hz), 1.40 (3H, t, J=7.3 Hz), 1.90-1.96 (4H, m), 2.46 (1H, d, J=17.1 Hz), 2.55 (1H, dd, J=17.1, 3.7 Hz), 2.70 (1H, dd, J=17.1, 6.7 Hz), 2.86 (1H, dd, J=17.1, 6.7 Hz), 3.08 (2H, q, J=7.3 Hz), 3.21-3.25 (1H, m), 3.30-3.34 (1H, m), 3.83-3.90 (1H, m), 4.06-4.15 (3H, m), 4.12 (3H, s), 6.90-6.93 (2H, m), 7.05 (1H, d, J=8.6 Hz), 7.40 (1H, d, J=9.2 Hz), 7.49 (1H, d, J=8.6 Hz), 7.66-7.68 (2H, m), 8.43 (1H, brs), 8.59 (1H, d, J=9.2 Hz).
Further, the optical purity was determined by means of HPLC using a chiral column. The separation condition was as follows.
Column: Daicel CHIRALPAK (registered trademark) IA column
Developing solvent: hexane/THF=55/45
Flow rate: 0.5 mL/min
Detection: UV (293 nm)
Retention time: t=24.7 minutes (96%ee)

### <Example 340>

### 6-(2-Ethyl-8-methoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

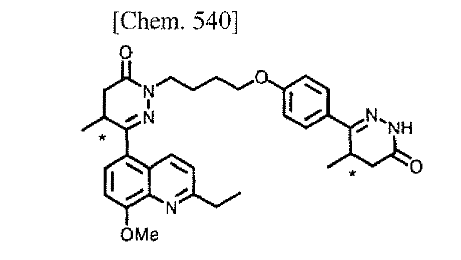

The compound of Example 96 was allowed to react in the same manner in Example 145, and the obtained compound and the compound of Example 256 were then reacted in the same manner as in Example 267 to obtain the desired product as a colorless amorphous substance.
HRESIMS (+): 556.29094 (Calculated value as C₃₂H₃₈N₅O₄ 556.29238).
¹H NMR (CDCl₃, 400 MHz): δ 1.17 (3H, d, J=7.3 Hz), 1.24 (3H, d, J=7.3 Hz), 1.40 (3H, t, J=7.3 Hz), 1.90-1.96 (4H, m), 2.46 (1H, d, J=17.1 Hz), 2.55 (1H, dd, J=17.1, 3.7 Hz), 2.70 (1H, dd, J=17.1, 6.7 Hz), 2.86 (1H, dd, J=17.1, 6.7 Hz), 3.08 (2H, q, J=7.3 Hz), 3.21-3.25 (1H, m), 3.30-3.34 (1H, m), 3.83-3.90 (1H, m), 4.06-4.15 (3H, m), 4.12 (3H, s), 6.90-6.93 (2H, m), 7.05 (1H, d, J=8.6 Hz), 7.40 (1H, d, J=9.2 Hz), 7.49 (1H, d, J=8.6 Hz), 7.66-7.68 (2H, m), 8.43 (1H, brs), 8.59 (1H, d, J=9.2 Hz).
Further, the optical purity was determined by means of HPLC using a chiral column. The separation condition was as follows.
Column: Daicel CHIRALPAK (registered trademark) IA column
Developing solvent: hexane/THF=55/45
Flow rate: 0.5 mL/min
Detection: UV (293 nm)
Retention time: t=52.9 minutes (96%ee)

### <Example 341>

### 6-(2-Ethyl-8-methoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

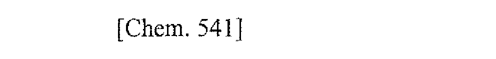

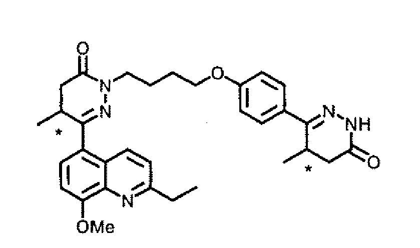

The compound of Example 97 was allowed to react in the same manner in Example 145, and the obtained compound and the compound of Example 255 were then reacted in the same manner as in Example 267 to obtain the desired product as a colorless amorphous. HRESIMS (+): 556.29100 (Calculated value as C₃₂H₃₈N₅O₄ 556.29238).
¹H NMR (CDCl₃, 400 MHz): δ 1.17 (3H, d, J=7.3 Hz), 1.24 (3H, d, J=7.3 Hz), 1.40 (3H, t, J=7.3 Hz), 1.90-1.96 (4H, m), 2.46 (1H, d, J=17.1 Hz), 2.55 (1H, dd, J=17.1, 3.7 Hz), 2.70 (1H, dd, J=17.1, 6.7 Hz), 2.86 (1H, dd, J=17.1, 6.7 Hz), 3.08 (2H, q, J=7.3 Hz), 3.21-3.25 (1H, m), 3.30-3.34 (1H, m), 3.85-3.88 (1H, m), 4.06-4.12 (3H, m), 4.12 (3H, s), 6.90-6.93 (2H, m), 7.05 (1H, d, J=8.0 Hz), 7.40 (1H, d, J=8.6 Hz), 7.49 (1H, d, J=8.0 Hz), 7.66-7.68 (2H, m), 8.41 (1H, brs), 8.59 (1H, d, J=8.6 Hz).
Further, the optical purity was determined by means of HPLC using a chiral column. The separation conditions were as follows.
Column: Daicel CHIRALPAK (registered trademark) IA column
Developing solvent: hexane/THF=55/45
Flow rate: 0.5 mL/min
Detection: UV (293 nm)
Retention time: t=22.2 minutes (98%ee)

### <Example 342>

### 6-(2-Ethyl-8-methoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one

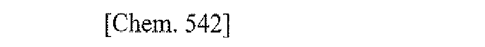

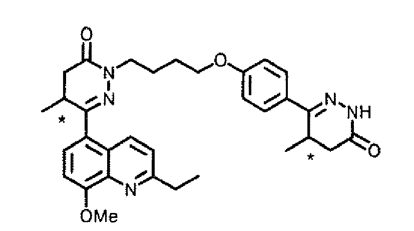

The compound of Example 97 was allowed to react in the same manner in Example 145, and the obtained compound and the compound of Example 256 were then reacted in the same manner as in Example 267 to obtain the desired product as a colorless amorphous. HRESIMS (+): 556.29155 (Calculated value as C₃₂H₃₈N₅O₄ 556.29238).
¹H NMR (CDCl₃, 400 MHz): δ 1.17 (3H, d, J=7.3 Hz), 1.24 (3H, d, J=7.3 Hz), 1.40 (3H, t, J=7.3 Hz), 1.90-1.96 (4H, m), 2.46 (1H, d, J=17.1 Hz), 2.55 (1H, dd, J=17.1, 3.7 Hz), 2.70 (1H, dd, J=17.1, 6.7 Hz), 2.86 (1H, dd, J=17.1, 6.7 Hz), 3.08 (2H, q, J=7.3 Hz), 3.21-3.25 (1H, m), 3.30-3.34 (1H, m), 3.85-3.88 (1H, m), 4.06-4.12 (3H, m), 4.12 (3H, s), 6.90-6.93 (2H, m), 7.05 (1H, d, J=8.0 Hz), 7.40 (1H, d, J=8.6 Hz), 7.49 (1H, d, J=8.0 Hz), 7.66-7.68 (2H, m), 8.41 (1H, brs), 8.59 (1H, d, J=8.6 Hz).
Further, the optical purity was determined by means of HPLC using a chiral column. The separation conditions were as follows.
Column: Daicel CHIRALPAK (registered trademark) IA column
Developing solvent: hexane/THF=55/45
Flow rate: 0.5 mL/min
Detection: UV (293 nm)
Retention time: t=56.2 minutes (98%ee)

### <Test Example 1> Phosphodiesterase inhibitory activity

RT-PCR was each performed to isolate the cDNA of a PDE3A catalytic domain (hereinafter simply referred to as "cat") and PDE4Bcat from human-derived RNA. The isolated cDNA fragments were inserted into Sf9 insect cells using Gateway system (manufactured by Invitrogen Corporation) and Bac-to-Bac (registered trademark) Baculovirus Expression system (manufactured by Invitrogen Corporation) to express these PDE protein. These recombinant PDE3Acat, PDE4Bcat, PDE5Acat, and PDE10A1 were purified from the culture supernatants or cell extracts of Sf9 cells expressing high levels of the PDE protein by ion exchange chromatography and used for the experiments as shown below.

A 4 mmol/L solution of each test compound was stepwisely diluted four-fold with a 15% DMSO solution to prepare solutions at concentrations of 15 nmol/L to 4 mmol/L (the final concentrations used in the experiments were 1.5 nmol/L to 400 µmol/L). 10 µL of the prepared test compound solutions, [³H] cAMP diluted with a buffer solution [40 mmol/L Tris-HCl (pH 7.4), 10 mmol/L MgCl₂], and 40 µL of the recombinant human-derived PDE protein at 2 × 10⁻⁶ units (wherein 1 unit is defined as an amount of PDE that degrades 1 µmol/L of cAMP in one minute under the conditions of a pH of 7.5 and 30°C) were added to a 96-well plate, and the mixtures were reacted at 30°C for 20 minutes. Then, the mixtures were reacted at 65°C for 2 minutes, 25 µL of a 1 mg/mL 5'-nucleotidase (Crotalus atrox venom, manufactured by Sigma) was then added thereto, and the mixtures were then reacted at 30°C for 10 minutes. After completion of the reaction, 200 µL of a solution of Dowex [300 mg/mL Dowex 1x8-400 (manufactured by Sigma Aldrich), 33% ethanol] was added thereto, and the mixtures were mixed and shaken at 4°C for 20 minutes. Subsequently, 200 µL of MicroScint 20 (manufactured by Packard) was added thereto, and measurement was performed using a scintillation counter (Topcount, manufactured by Packard). IC₅₀ values were calculated using Graph Pad Prism v3.03 (manufactured by GraphPad Software).

Further, the indications were as follows: IC₅₀ value≥10 µmol/L(-), 10 µmol/L>IC₅₀ value≥1 µmol/L (+), 1 µmol/L>IC₅₀ value≥0.1 µmol/L(++), and 0.1 µmol/L>IC₅₀ value (+++).

The results are shown in Table 1.

[Table 1]

**Table 1**

| Example No. | IC₅₀(µmol/L) PDE3 | IC₅₀(µmol/L) PDE4 | Example No. | IC₅₀(µmol/L) PDE3 | IC₅₀(µmol/L) PDE4 | Example No. | IC₅₀(µmol/L) PDE3 | IC₅₀(µmol/L) PDE4 |
|---|---|---|---|---|---|---|---|---|
| 267 | ++ | +++ | 292 | + | ++ | 318 | +++ | ++ |
| 268 | ++ | ++ | 293 | ++ | ++ | 319 | +++ | ++ |
| 269 | +++ | ++ | 294 | +++ | +++ | 320 | +++ | +++ |
| 270 | +++ | ++ | 295 | +++ | +++ | 321 | ++ | ++ |
| 271 | +++ | +++ | 296 | + | +++ | 322 | +++ | +++ |
| 272 | ++ | +++ | 297 | + | ++ | 323 | +++ | +++ |
| 273 | +++ | +++ | 298 | +++ | +++ | 324 | +++ | ++ |
| 274 | +++ | +++ | 299 | +++ | +++ | 325 | ++ | ++ |
| 275 | +++ | ++ | 300 | + | +++ | 326 | + | + |
| 276 | +++ | ++ | 301 | - | ++ | 327 | + | ++ |
| 277 | +++ | + | 302 | + | +++ | 328 | +++ | ++ |
| 278 | +++ | + | 303 | ++ | +++ | 329 | +++ | +++ |
| 279 | +++ | ++ | 304 | +++ | + | 330 | +++ | + |
| 280 | +++ | ++ | 305 | ++ | + | 331 | +++ | + |
| 281 | ++ | ++ | 306 | + | + | 332 | ++ | + |
| 282 | - | ++ | 307 | +++ | +++ | 333 | +++ | ++ |
| 283 | +++ | ++ | 308 | +++ | +++ | 334 | +++ | ++ |
| 284 | +++ | ++ | 309 | +++ | +++ | 335 | ++ | ++ |
| 285 | + | + | 310 | ++ | +++ | 336 | +++ | ++ |
| 286 | - | + | 311 | +++ | + | 337 | ++ | ++ |
| 287 | +++ | +++ | 312 | ++ | +++ | 338 | ++ | ++ |
| 288 | ++ | ++ | 313 | ++ | +++ | 339 | + | +++ |
| 289 | +++ | +++ | 314 | +++ | +++ | 340 | +++ | +++ |
| 290 | +++ | +++ | 315 | + | +++ | 341 | + | +++ |
| 291 | + | ++ | 316 | +++ | ++ | 342 | +++ | ++ |

### <Test Example 2> Histamine-induced bronchoconstriction reaction in guinea pigs

Guinea pigs were anesthetized with pentobarbital (30 mg/kg, i.p.). A cannula for intravenous administration was inserted into the left external jugular vein, a cannula for collecting blood and measuring blood pressure was inserted into the right internal carotid artery, and a tracheal cannula was inserted into the trachea. The guinea pigs were maintained on artificial respiration under the conditions of 60 times/min and 10 mL/kg/stroke. The airflow from the side branch of the tracheal cannula was measured by a bronchospasm transducer (Ugo-Basile) and recorded on a computer via Power Lab (ADInstruments Japan). The guinea pigs were immobilized with gallamine (10 mg/kg, i.v.), and histamine (12.5 µg/kg, i.v.) was administered at 10-minute intervals.
After the histamine-induced bronchoconstriction became stable, the compound (0.3 mg/kg, i.v.) was administered. The histamine-induced bronchoconstriction reaction was measured 30 seconds after the compound administration to examine the bronchoconstriction inhibitory activity of the compound. The bronchoconstriction was recorded as the airflow value, and the results were represented by the ratio of the maximum value of the histamine-induced airflow 30 seconds after the administration to the maximum value of the airflow before the administration. Also, the test compounds dissolved in DMSO were used. Further, the indications were as follows: inhibition rate≥90% (+++), 90%>inhibition rate≥80% (++), and 80%>inhibition rate≥70% (+). [ ] means the results in the case of 0.1 mg/kg, i.v.

The results are shown in Table 2.

[Table 2]

**Table 2**

| Example No. | Inhibition rate | Example No. | Inhibition rate | Example No. | Inhibition rate |
|---|---|---|---|---|---|
| 267 | +++ | 291 | + | 304 | +++ |
| 269 | [++] | 292 | ++ | 305 | +++ |
| 271 | +++ | 293 | [+] | 312 | [+] |
| 272 | + | 294 | [+++] | 314 | ++ |
| 273 | +++ | 295 | [++] | 315 | + |
| 277 | ++ | 296 | [++] | 316 | [+++] |
| 278 | +++ | 297 | +++ | 318 | +++ |
| 279 | +++ | 298 | [+++] | 319 | [+++] |
| 287 | +++ | 299 | [+++] | 320 | +++ |
| 288 | ++ | 300 | + | 323 | + |
| 289 | +++ | 301 | ++ | 340 | +++ |
| 290 | +++ | 303 | ++ | 342 | +++ |

| | | | | | |
|---|---|---|---|---|---|
| [0.1 mg/kg] | | | | | |

### <Test Example 3> LPS acute inflammation model in rats

1 mg/kg of the compound was orally administered to rat one hour before inhalation of a lipopolysaccharide from E. coli serotype 055:B5 (LPS), and the rat was made to inhale 50 mL of the LPS solution nebulized using a nebulizer for 30 minutes. Then, 3 hours after the LPS inhalation, the rat was euthanized with 20% urethane (5 ml/rat, i.p.). 5 ml of physiological saline for bronchoalveolar lavage was injected into the bronchial tubes and alveoli through the airway, and the bronchial tubes and alveoli were washed three times using a 5 mL syringe. This operation was repeated twice, and the solution was collected as bronchoalveolar lavage fluid (BALF). The collected BALF was centrifuged at 1200 rpm and 4°C for 10 minutes (Hirtachi; himac CR 5 DL). The pellet was re-suspended in 10 mL of a 0.1% bovine serum albumin-physiological saline, and an equivalent amount of Turk's solution was added thereto to stain leukocytes. The total number of leukocytes was counted under a microscope to calculate the inhibition rate. Further, the indications were as follows: inhibition rate≥90% (++) and 90%>inhibition rate≥50% (+). [ ] means the results of the drug administration after fasting overnight.

The results are shown in Table 3.

[Table 3]

**Table 3**

| Example No. | Inhibition rate | Example No. | Inhibition rate |
|---|---|---|---|
| 271 | [+] | 319 | [+] |
| 283 | [++] | 320 | [+] |
| 289 | + | 322 | [+] |
| 299 | [+] | 323 | [++] |
| 312 | + | 339 | + |
| 314 | + | 340 | + |

As described above, the compounds represented by the general formula (1) of the present invention have a PDE inhibitory activity, and the effectiveness of the compounds have been confirmed in the experimental models of various animals.

### Industrial Availability

As described above, according to the present invention, it has been found that a novel pyridazinone derivative and an addition salt thereof have an excellent PDE inhibitory action. Such a compound having a PDE inhibitory action is useful as an agent for treating angina pectoris, cardiac failure, hypertension, or the like, as a platelet aggregation inhibitor, as an agent for preventing or treating bronchial asthma, chronic obstructive pulmonary disease (COPD), interstitial pneumonitis, allergic rhinitis, atopic dermatitis, rheumatoid arthritis, multiple sclerosis, Crohn's disease, or inflammatory bowel disease, as an agent for preventing or treating various psychiatric disorders such as Huntington's disease, Alzheimer's disease, dementia, Parkinson's disease, depression, schizophrenia, and the like, as an agent for preventing or treating obesity, metabolic syndrome, and the like, and as an agent for treating male erectile dysfunction.

## Claims

1. A pyridazinone derivative, optically active compound thereof, pharmaceutically acceptable salt thereof, or hydrate thereon, wherein the pyridazinone derivative is represented by the following general formula (1): 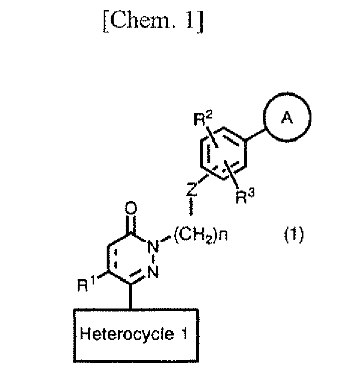 [wherein R¹ represents a hydrogen atom or an alkyl group having 1 to 6 carbon atoms,
R² and R³ are the same as or different from each other and represent a hydrogen atom, a halogen atom, or an alkoxy group having 1 to 6 carbon atoms,
Z represents an oxygen atom or a sulfur atom,
A represents a substituent represented by the general formula: 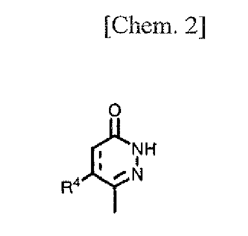 (wherein R⁴ represents a hydrogen atom or an alkyl group having 1 to 6 carbon atoms, and
[Chem. 3] - - -
represents a single bond or a double bond) or a substituent represented by the general formula: 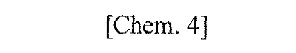 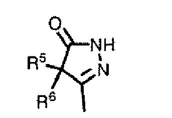 (wherein R⁵ and R⁶ are the same as or different from each other and represent an alkyl group having 1 to 6 carbon atoms),
Heterocycle 1 represents a heterocyclic compound represented by the following general formula (2): 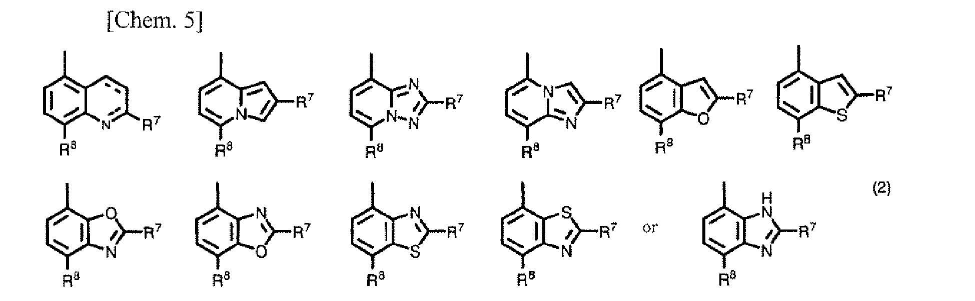 (wherein R⁷ represents a hydrogen atom or an alkyl group having 1 to 6 carbon atoms which may be substituted with halogen atom(s),
R⁸ represents a hydrogen atom or an alkoxy group having 1 to 6 carbon atoms,
[Chem. 6] - - -
represents a single bond or a double bond),
n represents an integer of 1 to 5, and
[Chem. 7] - - -
represents a single bond or a double bond].

2. The pyridazinone derivative, optically active compound thereof, pharmaceutically acceptable salt thereof, or hydrate thereof according to claim 1, wherein the compound represented by the general formula (1) is represented by the general formula (1a): 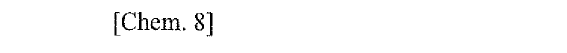 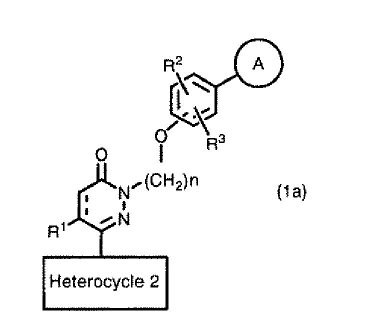 [wherein Heterocycle 2 represents the following general formula (2a): 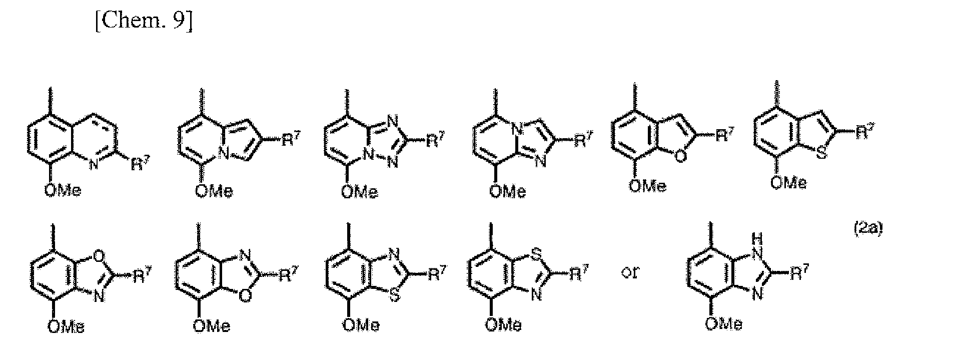 (wherein R⁷ and
[Chem. 10] - - -
are as defined above), and R¹, R², R³, A, n and
[Chem. 11] - - -
are as defined above].

3. The pyridazinone derivative, optically active compound thereof, pharmaceutically acceptable salt thereof, or hydrate thereof according to claim 1, wherein the compound represented by the general formula (1) is
6-(2-ethyl-8-methoxyquinolin-5-yl)-5-methyl-2-[4-[4-(4-methyl-6-oxo-1,4,5,6-tetrahydropyridazin-3-yl)phenoxy]butyl]-4,5-dihydro-2H-pyridazin-3-one,
6-(2-ethyl-8-methoxyquinolin-5-yl)-2-[4-[2,3-difluoro-4-(6-oxo-1,4,5,6-tetrahydropyridazin-3-yl)phenoxy]butyl]-2H-pyridazin-3-one,
6-(2-ethyl-8-methoxyquinolin-5-yl)-2-[4-[4-(6-oxo-1,4,5,6-tetrahydropyridazin-3-yl)phenoxy]butyl]-2H-pyridazin-3-one,
2-[4-[2-fluoro-4-(6-oxo-1,4,5,6-tetrahydropyridazin-3-yl)phenoxylbutyl]-6-(8-methoxy-2-methylquinolin-5-yl)-2H-pyridazin-3-one,
6-(2-ethyl-8-methoxyquinolin-5-yl)-5-methyl-2-[4-[2,3-difluoro-4-(6-oxo-1,4,5,6-tetrahydropyridazin-3-yl)phenoxy]butyl]-4,5-dihydro-2H-pyridazin-3-one,
6-(2-ethyl-8-methoxyquinolin-5-yl)-2-[4-[2,3-difluoro-4-(4-methyl-6-oxo-1,4,5,6-tetrahydropyridazin-3-yl)phenoxy]butyl]-2H-pyridazin-3-one,
6-(4-methoxy-2-trifluoromethyl-1H-benzimidazol-7-yl)-5-methyl-2-[4-[4-(4-methyl-6-oxo-1,4,5,6-tetrahydropyridazin-3-yl)phenoxy]butyl]-4,5-dihydro-2H-pyridazin-3-one,
2-[4-[2,3-difluoro-4-(6-oxo-1,4,5,6-tetrahydropyridazin-3-yl)phenoxy]butyl]-6-(8-methoxy-2-methylquinolin-5-yl)-2H-pyridazin-3-one,
2-[4-[2,3-difluoro-4-(4-methyl-6-oxo-1,4,5,6-tetrahydropyridazin-3-yl)phenoxy]butyl]-6-(8-methoxyquinolin-5-yl)-2H-pyridazin-3-one, or
2-[4-[2,3-difluoro-4-(6-oxo-1,4,5,6-tetrahydropyridazin-3-yl)phenoxy]butyl]-6-(8-methoxyquinolin-5-yl)-2H-pyridazin-3-one.

4. A phosphodiesterase (PDE) inhibitor comprising, as an active ingredient, the pyridazinone derivative, optically active compound thereof, pharmaceutically acceptable salt thereof, or hydrate thereof according to any one of claims 1 to 3.

5. A pharmaceutical agent comprising, as an active ingredient, the pyridazinone derivative, optically active compound thereof, pharmaceutically acceptable salt thereof, or hydrate thereof according to any one of claims 1 to 3.

6. The pharmaceutical agent according to claim 5, which is an agent for preventing or treating angina pectoris, cardiac failure, hypertension, bronchial asthma, chronic obstructive pulmonary disease (COPD), interstitial pneumonitis, allergic rhinitis, atopic dermatitis, rheumatoid arthritis, multiple sclerosis, Crohn's disease, inflammatory bowel disease, Alzheimer, dementia, Parkinson's disease, or depression.
